# EUROPEAN PATENT APPLICATION

(11) **EP 4 556 476 A1**
(43) Date of publication of application: **21.05.2025**
(21) Application number: 23839976.0
(22) Date of filing: 12.07.2023
(51) Int. Cl.: C07D 487/04, A61K 31/5377, A61P 35/00, A61P 29/00, A61P 25/00, C07D 473/00, C07D 473/34, C07D 471/04, C07D 401/04, A61K 31/52

(54) **NOVEL BICYCLIC HETEROARYL COMPOUND AND USE THEREOF**

(30) Priority: 13.07.2022 KR 20220086439; 28.07.2022 KR 20220093941; 02.12.2022 KR 20220167002
(71) Applicant: Ildong Pharmaceutical Co., Ltd., Seoul 06752 (KR); Arbormed Co., Ltd., Seoul 06237 (KR)
(72) Inventor: PARK, Sunyoung, Hwaseong-si, Gyeonggi-do 18449 (KR); SONG, Hyojung, Hwaseong-si, Gyeonggi-do 18449 (KR); JANG, Kyusic, Hwaseong-si, Gyeonggi-do 18449 (KR); CHANG, Min Whan, Seongnam-si, Gyeonggi-do 13519 (KR); KIM, Minkyung, Hwaseong-si, Gyeonggi-do 18449 (KR); JEONG, Il Ji, Hwaseong-si, Gyeonggi-do 18449 (KR); LIM, Jongha, Hwaseong-si, Gyeonggi-do 18449 (KR); YOO, Jisu, Seoul 04974 (KR); JEON, Woojin, Hwaseong-si, Gyeonggi-do 18449 (KR); SONG, Yoonsung, Hwaseong-si, Gyeonggi-do 18449 (KR); YOON, Hong Chul, Hwaseong-si, Gyeonggi-do 18449 (KR); PARK, Joon Tae, Hwaseong-si, Gyeonggi-do 18449 (KR); LEE, Jung Woo, Hwaseong-si, Gyeonggi-do 18449 (KR); AN, Kyung Mi, Hwaseong-si, Gyeonggi-do 18449 (KR); PARK, Dongsik, Suwon-si, Gyeonggi-do 16487 (KR); KIM, Dasol, Yongin-si, Gyeonggi-do 16944 (KR); IM, Weonbin, Yongin-si, Gyeonggi-do 16899 (KR); EUN, So-Young, Yongin-si, Gyeonggi-do 16911 (KR); MIN, Byong-Keol, Yongin-si, Gyeonggi-do 16838 (KR); LEE, Hong Jae, Suwon-si, Gyeonggi-do 16509 (KR); KIM, Eunjung, Seoul 06760 (KR); PARK, Eok, Seoul 07630 (KR)
(74) Representative: Cabinet Becker et Associés
(86) International application number: PCT/KR2023/009985
(87) International publication number: WO 2024/014885

(57) **Abstract**

The present invention provides a novel compound represented by Chemical Formula 1, an optical isomer thereof, or a pharmaceutically acceptable salt thereof. With an excellent inhibitory activity against PIKfyve, the novel compound of the present invention is useful as a therapeutic agent for PIKfyve activity-related cancer disease, inflammatory disease, or lysosomal storage disease.

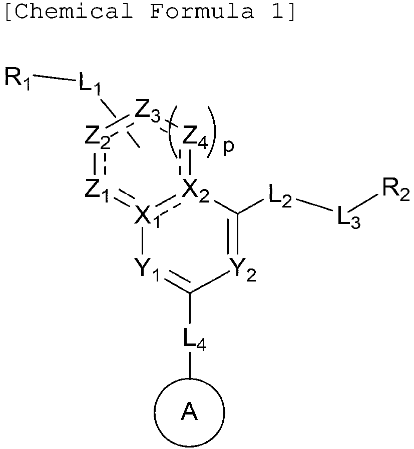

## Description

### [Technical Field]

The present disclosure relates to a novel bicyclic heteroaryl compound, a pharmaceutical composition comprising the compound, and uses of the compound.

### [Background Art]

Phosphorylated derivatives of phosphatidylinositol (PI) regulate the cytoskeleton, membrane trafficking, and secondary signaling of cells and organelles by recruiting protein complexes to lipid membranes of cells. The degree and location of phosphorylation of the inositol ring is regulated by various phosphorylation and dephosphorylation enzymes. PIKfyve protein is a kinase that phosphorylates phosphatidylinositol 3-phosphate (PI3P) and phosphatidylinositol (PI), generating phosphatidylinositol 3,5-bisphosphate (PI(3,5)P2) and phosphatidylinositol 5-phosphate (PI5P), respectively.

Phosphatidylinositol 3,5-bisphosphate (PI(3,5)P2) produced by PIKfyve regulates the division, fusion, and maturation of endosomes to maintain endomembrane homeostasis. Therefore, when the function of PIKfyve is inhibited, endosomal expansion and cytoplasmic vacuolization occur due to abnormal endosomal action in the cell. Endosomes mature into endolysosomes/lysosomes, fuse with autophagosomes, and promote autophagy degradation. However, inhibition of PIKfyve function also impairs autophagy degradation. PI(3,5)P2 also directly binds to and activates the function of ion channel proteins located in endosomes and lysosomes, and PIKfyve inhibitor-induced endosome expansion and cytoplasmic vacuolization are reversibly alleviated by supplementation with PI(3,5)P2 or activation of ion channel proteins.

Phosphatidylinositol 5-phosphate (PI5P) is generated by direct phosphorylation of PI by PIKfyve, or by dephosphorylation of PI(3,5)P2 or phosphatidylinositol 4,5-bisphosphate (PI(4,5)P2). PI5P acts as a second signaling molecule in endosomes and nuclear membranes, or interacts with external virulence factors at the cell membrane to regulate endosome entry. In addition, PI5P produced by PIKfyve was recently reported to be required for autophagosome generation by AMP-activated kinase (AMPK) activation, suggesting another link to autophagy.

Small molecule compounds such as Apilimod, APY0201, and YM201636 have been reported as inhibitors of PIKfyve. Apilimod, in particular, was first identified as an inhibitor of the inflammatory cytokines interleukin-12 and -23 (IL-12, IL-23) and has been evaluated for inflammatory and autoimmune diseases such as Crohn's disease, rheumatoid arthritis, and psoriasis. Since PIKfyve regulates the expression of IL12/23p40, a common subunit of IL-12 and IL-23, it has been suggested that inhibition of IL-12 and IL-23 production through PIKfyve inhibitors could prevent and treat inflammatory and autoimmune diseases.

PIKfyve has also been reported as a pharmacologic target in various cancers. PIKfyve inhibition can induce disruption of autophagy/lysosome-mediated nutrient regeneration and endosome/lysosome-dependent nutrient uptake pathways in some carcinomas, and also induce Rac1 inactivation, a GTPase, which may reduce the invasiveness of cancer cells. PIKfyve inhibitors have been reported to induce apoptosis or methuosis cell death in some cancer types and to exhibit anticancer activity in cancers such as non-Hodgkin's lymphoma, multiple myeloma, melanoma, liver cancer, glioblastoma, and colorectal adenocarcinoma.

In addition, reports have suggested that modulation of the activity of PIKfyve increases the concentration of PI3P, a substrate of PIKfyve, and normalizes lysosomes in motor neurons, suggesting the possibility of treating rare genetic diseases caused by reduced lysosomal activity. In Amyotrophic Lateral Sclerosis (ALS), a disease characterized by severe paralysis of the motor nerves, and Frontotemporal dementia (FTD), a neurodegenerative dementia, about 10% of cases are caused by mutations in genes involved in the lysosomal transport system (e.g., intronic repeats in the C9ORF72 gene). When Apilimod, a PIKfyve inhibitor, was treated to motor neurons derived from ALS/FTD patients with such mutations, the number of lysosomes increased and cell death was reduced. This was also confirmed in in vivo experiments using a c9orf72-deficient ALS animal model. Thus, inhibition of PIKfyve activity can be seen as a possibility for ALS treatment that increases PI3P (a substrate of PIKfyve), which is involved in endosome maturation and lysosomal biogenesis, thereby compensating for the reduced lysosomal activity caused by the mutation of C90RF72 and reducing cytotoxicity by effectively removing toxic dipeptide repeat-proteins made of C9ORF72-intronic repeats through lysosomal activity.

Against this background, the present disclosure synthesized novel PIKfyve inhibitor compounds and confirmed that these compounds exhibit excellent inhibitory activity as PIKfyve inhibitors.

### [Disclosure]

### [Technical Problem]

An object of the present disclosure is to provide a compound represented by the following Chemical Formula 1, an optical isomer thereof, or a pharmaceutically acceptable salt thereof:

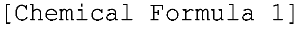

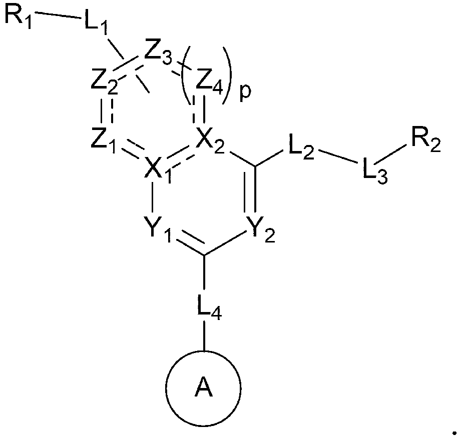

Another object of the present disclosure is to provide a pharmaceutical composition comprising: the compound represented by Chemical Formula 1 above, an optical isomer thereof, or a pharmaceutically acceptable salt thereof.

Still another object of the present disclosure is to provide a pharmaceutical composition comprising: the compound represented by Chemical Formula 1 above, an optical isomer thereof, or a pharmaceutically acceptable salt thereof; and a pharmaceutically acceptable carrier.

Still another object of the present disclosure is to provide a pharmaceutical composition for preventing or treating cancer comprising: the compound represented by Chemical Formula 1 above, an optical isomer thereof, or a pharmaceutically acceptable salt thereof.

Still another object of the present disclosure is to provide a pharmaceutical composition for preventing or treating inflammatory diseases comprising: the compound represented by Chemical Formula 1 above, an optical isomer thereof, or a pharmaceutically acceptable salt thereof.

Still another object of the present disclosure is to provide a pharmaceutical composition for preventing or treating lysosomal storage diseases (LSDs) comprising: the compound represented by Chemical Formula 1 above, an optical isomer thereof, or a pharmaceutically acceptable salt thereof.

Still another object of the present disclosure is to provide a pharmaceutical composition for preventing or treating neurological diseases comprising: the compound represented by Chemical Formula 1 above, an optical isomer thereof, or a pharmaceutically acceptable salt thereof.

Still another object of the present disclosure is to provide a PIKfyve inhibitor comprising: the compound represented by Chemical Formula 1 above, an optical isomer thereof, or a pharmaceutically acceptable salt thereof.

Still another object of the present disclosure is to provide a method for treating cancer, comprising: administering, to a subject in need thereof, a therapeutically effective amount of the compound represented by Chemical Formula 1 above, an optical isomer thereof, or a pharmaceutically acceptable salt thereof.

Still another object of the present disclosure is to provide a method for treating inflammatory diseases, comprising: administering, to a subject in need thereof, a therapeutically effective amount of the compound represented by Chemical Formula 1 above, an optical isomer thereof, or a pharmaceutically acceptable salt thereof.

Still another object of the present disclosure is to provide a method for treating lysosomal storage diseases (LSDs), comprising: administering, to a subject in need thereof, a therapeutically effective amount of the compound represented by Chemical Formula 1 above, an optical isomer thereof, or a pharmaceutically acceptable salt thereof.

Still another object of the present disclosure is to provide a method for treating neurological diseases, comprising: administering, to a subject in need thereof, a therapeutically effective amount of the compound represented by Chemical Formula 1 above, an optical isomer thereof, or a pharmaceutically acceptable salt thereof.

Still another object of the present disclosure is to provide use of the compound represented by Chemical Formula 1, an optical isomer thereof, or a pharmaceutically acceptable salt thereof for the manufacture of medicaments for the treatment of cancer.

Still another object of the present disclosure is to provide use of the compound represented by Chemical Formula 1, an optical isomer thereof, or a pharmaceutically acceptable salt thereof for the manufacture of medicaments for the treatment of inflammatory diseases.

Still another object of the present disclosure is to provide use of the compound represented by Chemical Formula 1, an optical isomer thereof, or a pharmaceutically acceptable salt thereof for the manufacture of medicaments for the treatment of lysosomal storage diseases (LSDs).

Still another object of the present disclosure is to provide use of the compound represented by Chemical Formula 1, an optical isomer thereof, or a pharmaceutically acceptable salt thereof for the manufacture of medicaments for the treatment of neurological diseases.

### [Technical Solution]

Terms used in the present application are only used to describe specific embodiments and are not intended to limit the present disclosure. Singular expressions include plural expressions unless the context clearly indicates otherwise. In the present specification, terms such as "comprise", "have", and the like are intended to designate the presence of features, steps, structures, or combinations thereof described in the specification and it should not be understood as precluding the possibility of the presence or addition of one or more other features, steps, structures, or combinations thereof.

As used herein, the term "halogen" means a substituent selected from fluorine (F), chloro (Cl), bromo (Br) and iodo (I).

In the present disclosure, "Cₓ-C_{y}" means having a carbon number of x or more and y or less.

As used herein, the term "substituted" refers to a moiety having a substituent that replaces hydrogen on one or more carbons of the main chain. "Substitution" or "substituted with" depends on whether such substitution is permissible for the substituted atom and substituent, and it is defined to include the implicit conditions that leads to stable compounds by substitution, for example, compounds that are not naturally modified by rearrangement, cyclization, elimination, and the like.

As used herein, the term "single bond" refers to the direct connection between two connected radicals. For example, if L represents a single bond in A-L-Z, this structure is essentially A-Z.

As used herein, the term "C₁-C₆ alkyl" means C₁-C₆ straight or branched chain saturated hydrocarbon such as methyl, ethyl, propyl, isopropyl, butyl, pentyl, or hexyl. Preferred alkyl groups include about 1, 2, 3, 4, 5 or 6 carbon atoms in the chain. Side chain means that at least one lower alkyl group, such as methyl, ethyl or propyl, is attached to the linear alkyl chain. "Lower alkyl" means a group having from about 1 to about 6 carbon atoms in the chain, which may be straight or branched. "Alkyl" may be unsubstituted or may be optionally substituted by one or more substituents that may be the same or different. Each substituent may be halogen, alkyl, aryl, heteroaryl, cycloalkyl, heterocycloalkyl, cyano, hydroxy, alkoxy, alkylthio, amino, carboxy, or the like. Each of these substituents may follow any of the definitions for each substituent referenced herein.

The term "C₁-C₆ haloalkyl" as used herein means C₁-C₆ straight or branched chain saturated hydrocarbon in which at least one hydrogen atom is substituted with a halogen atom (i.e., F, Cl, Br, or I). Examples thereof may include, but are not limited to, CH₂F, CHF₂, CF₃, etc.

The term "C₆-C₁₂ aryl" as used herein refers to an aromatic hydrocarbon containing 6 to 12 carbon atoms. For example, the aryl may refer to monocyclic (for example, phenyl) or bicyclic (for example, indenyl, naphthalenyl, tetrahydronaphthyl, tetrahydroindenyl) ring system. Preferably, aryl may be a phenyl group with the Chemical Formula C₆H₅, in which six carbon atoms are arranged in a cyclic ring structure. The phenyl group is a very stable and is found in many organic compounds as a type of aromatic hydrocarbon. In addition, the aryl may be substituted or unsubstituted, and when substituted, the hydrogen at the ortho, meta, or para position of the phenyl ring may be substituted with a halogen atom, C₁-C₆ alkyl, C₁-C₆ haloalkyl, -O-C₁-C₆ alkyl, -O-C₁-C₆ haloalkyl, oxo(=O), -OH, -CN, -N-(C₁-C₆ alkyl)₂ or -NH₂, but is not limited thereto.

The term "C₃-C₁₂ heteroaryl" as used herein refers to an optionally substituted aromatic ring containing 3 to 12 carbon atoms, in which at least one of the ring carbon atoms is substituted with a heteroatom selected from oxygen (O), nitrogen (N) and sulfur (S), or an aromatic ring (e.g., a bicyclic or tricyclic ring system) fused to one or more rings such as a heteroaryl ring, an aryl ring, a heterocyclic ring, or a carbocyclic ring, each of which may have an optional substituent.

The term "C₃-C₈ cycloalkyl" as used herein refers to a saturated hydrocarbon ring containing 3 to 8 carbon atoms, and the saturated hydrocarbon ring includes both monocyclic and polycyclic rings, and ring structures in which two or more rings share one or more pairs of carbon atoms (e.g., a fused ring, a spiro ring, a bridged ring, etc.). Cycloalkyl includes, but is not limited to, cyclopropyl, cyclobutyl, cyclopentyl, cyclohexyl, cycloheptanyl, cyclooctanyl, etc. In addition, the cycloalkyl may be substituted or unsubstituted, and when substituted, may be substituted with a halogen atom, C₁-C₆ alkyl, C₁-C₆ haloalkyl, -O-C₁-C₆ alkyl, -O-C₁-C₆ haloalkyl, oxo(=O), -OH, -CN, -N-(C₁-C₆ alkyl)₂ or - NH₂, but is not limited thereto. A specific example may include cyclobutanone. Further, one ring of a polycyclic cycloalkyl group may be aromatic when the polycyclic cycloalkyl group is attached to the parent structure through a non-aromatic carbon.

As used herein, "C₂-C₈ heterocycloalkyl" includes a saturated monocyclic or polycyclic heterocyclic ring containing 1 to 4 heteroatoms independently selected from nitrogen (N), oxygen (O), and sulfur (S), or a ring structure in which two or more rings share one or more pairs of carbon atoms (e.g., a fused ring, a spiro ring, a bridged ring, etc.). In addition, the heterocycloalkyl may be substituted or unsubstituted, and when substituted, may be substituted with a halogen atom, C₁-C₆ alkyl, C₁-C₆ haloalkyl, -O-C₁-C₆ alkyl, -O-C₁-C₆ haloalkyl, oxo(=O), -OH, -CN, -N-(C₁-C₆ alkyl)₂ or -NH₂, but is not limited thereto. When nitrogen is present in a heterocycloalkyl ring, it may exist in an oxidized state (i.e., N⁺-O⁻) as long as the properties of adjacent atoms and groups allow. Examples thereof may include piperidinyl N-oxide and morpholinyl-N-oxide. Further, when sulfur is present in a heterocycloalkyl ring, it may exist in an oxidized state (i.e., S⁺-O⁻ or -SO₂⁻) as long as the properties of adjacent atoms and groups allow. Examples thereof may include thiomorpholine S-oxide and thiomorpholine S,S-dioxide. Further, one ring of the polycyclic heterocycloalkyl group may be aromatic (e.g., aryl or heteroaryl) when the polycyclic heterocycloalkyl group is attached to the parent structure through a non-aromatic carbon or nitrogen atom.

As used herein, "C₂-C₈ heterocycloalkene" refers to a non-aromatic ring containing 1 to 4 heteroatoms independently selected from nitrogen (N), oxygen (O), and sulfur (S), and having at least one double bond derived by the removal of one molecule of hydrogen from an adjacent carbon atom, an adjacent nitrogen atom, or adjacent carbon and nitrogen atoms of the corresponding heterocycloalkyl. Heterocycloalkenyl groups include cyclic or polycyclic (e.g., bicyclic, tricyclic) or ring structures in which two or more rings share one or more pairs of carbon atoms (e.g., fused rings, spiro rings, bridged rings, etc.). When nitrogen is present in a heterocycloalkenyl ring, it may exist in an oxidized state (i.e., N⁺-O⁻) as long as the properties of adjacent atoms and groups allow. Further, when sulfur is present in a heterocycloalkenyl ring, it may exist in an oxidized state (i.e., S⁺-O⁻ or -SO₂⁻) as long as the properties of adjacent atoms and groups allow. Further, one ring of the polycyclic heterocycloalkenyl group may be aromatic (e.g., aryl or heteroaryl) when the polycyclic heterocycloalkenyl group is attached to the parent structure through a non-aromatic carbon or nitrogen atom. As discussed herein, whether each ring is considered to be an aryl, heteroaryl, cycloalkyl, cycloalkenyl, heterocycloalkyl, or heterocycloalkenyl group is determined by the atoms to which that moiety is bonded to the parent structure.

As used herein, the term "alkoxy" is an alkyl (carbon and hydrogen chain) group bonded to oxygen, and means C₁-C₆ straight or branched chain alkoxy such as methoxy, ethoxy, etc.

As used herein, the term "alkylthio" means an alkyl (carbon and hydrogen chain) group bonded to sulfur, and may preferably be C₁-C₆ straight or branched chain alkylthio.

As used herein, the term "amino" means a form in which hydrogen is bonded to a nitrogen atom, and this hydrogen atom may be substituted with C₁-C₆ straight or branched-chain alkyl, aryl, etc.

As used herein, "carboxy" means a substituent represented by -COOH or -CO₂H.

As used herein, "optical isomer (stereoisomer)" refers to two molecules having optical activity in a mirror-symmetric relationship. It is commonly used synonymously with enantiomer, which includes the R-form, S-form, or racemic compounds, respectively.

Further, unless defined otherwise, all terms used herein, including technical or scientific terms, have the same meaning as commonly understood by one of ordinary skill in the art to which the present disclosure belongs. Terms such as those defined in commonly used dictionaries should be interpreted as having a meaning consistent with the meaning in the context of the related art, and unless explicitly defined in the present application, it is not to be construed in an idealized or overly formal sense.

### Novel bicyclic heteroaryl compound, optical isomer thereof or pharmaceutically acceptable salt thereof

In one general aspect, there are provided a compound represented by the following Chemical Formula 1, an optical isomer thereof or a pharmaceutically acceptable salt thereof: 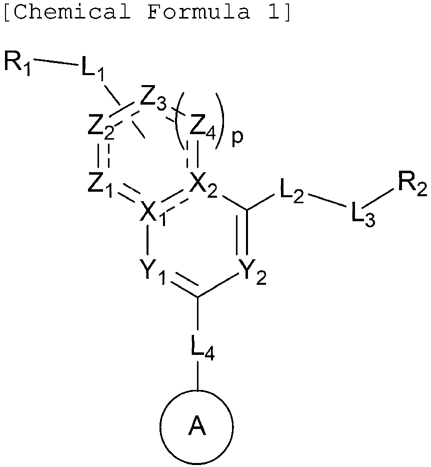 in Chemical Formula 1 above,
each of X₁ and X₂ is independently C or N;
each of Y₁ and Y₂ is independently C or N;
each of Z₁, Z₂, Z₃ and Z₄ is independently C, C-R', CH, CH₂, N, N-R' or S;
p is 0 or 1;
R' is a hydrogen atom or C₁-C₆ alkyl;
each of -̅ -̅ -̅ is optionally a single bond or a double bond, provided that two double bonds are not adjacent to each other;
L₁, L₂ and L₃ are each independently a single bond, C₁-C₆ alkyl, C₁-C₆ haloalkyl, C₁-C₆ alkyl-C=O, -C=O-, -C=O-C₁-C₆ alkyl, -C=O-NH, -SO₂, C₁-C₆ alkyl-Rₐ, C₁-C₆ alkyl-SO₂, -NH, - NH-C₁-C₆ alkyl, C₁-C₆ alkyl-NH-, C₁-C₆ alkyl-O-, -NH-N=CH-, - N=CH-, -NH-NH-C=O-, -C=O-NH-N=CH-, -O-, -O-C₁-C₆ alkyl, substituted or unsubstituted C₂-C₈ heterocycloalkyl containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted heteroaryl is substituted with at least one halogen atom, C₁-C₆ alkyl, C₁-C₆ haloalkyl, -O-C₁-C₆ alkyl, CN or NH₂;
Rₐ is substituted or unsubstituted C₃-C₈ cycloalkyl or substituted or unsubstituted C₂-C₈ heterocycloalkyl containing a heteroatom selected from oxygen, nitrogen and sulfur;
R₁ is a hydrogen atom, substituted or unsubstituted C₁-C₆ alkyl, C₁-C₆ haloalkyl, C₁-C₆ alkyl-OH, -O-C₁-C₆ alkyl, -O-C₁-C₆ haloalkyl, -O-C₃-C₈ cycloalkyl, -SO₂-C₁-C₆ alkyl, -N-(C₁-C₆ alkyl)₂, -C=O-R_{b}, substituted or unsubstituted C₃-C₈ cycloalkyl, substituted or unsubstituted C₂-C₈ heterocycloalkyl containing a heteroatom selected from oxygen, nitrogen and sulfur, substituted or unsubstituted C₂-C₈ heterocycloalkene containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted alkyl, cycloalkyl, heterocycloalkyl, heterocycloalkene, aryl, or heteroaryl is substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
R_{b} is C₁-C₆ alkyl, -N-(C₁-C₆ alkyl)₂, -O-C₁-C₆ alkyl, substituted or unsubstituted C₂-C₈ heterocycloalkyl containing a heteroatom selected from oxygen, nitrogen and sulfur, substituted or unsubstituted C₆-C₁₂ aryl, substituted or unsubstituted C₃-C₁₂ heteroaryl containing a heteroatom selected from oxygen, nitrogen and sulfur, wherein the substituted heterocycloalkyl, aryl and heteroaryl are substituted with at least one halogen atom, C₁-C₆ alkyl, C₁-C₆ haloalkyl, -O-C₁-C₆ alkyl, oxo(=O), -OH, -CN or -NH₂;
R₂ is a hydrogen atom, C₁-C₆ haloalkyl, substituted or unsubstituted C₂-C₈ heterocycloalkyl containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted heterocycloalkyl, aryl and heteroaryl are substituted with at least one halogen atom, C₁-C₆ alkyl, C₁-C₆ haloalkyl, -O-C₁-C₆ alkyl, CN or NH₂;
L₄ is a single bond, C₁-C₆ alkyl, -NH, -NH-C₁-C₆ alkyl, C₁-C₆ haloalkyl, or -O-C₁-C₆ alkyl; and 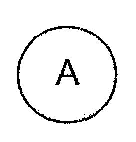
is substituted or unsubstituted C₂-C₈ heterocycloalkyl containing a heteroatom selected from oxygen, nitrogen and sulfur or substituted or unsubstituted C₂-C₈ heterocycloalkene containing a heteroatom selected from oxygen, nitrogen and sulfur, wherein the substituted heterocycloalkyl and heterocycloalkene are substituted with at least one halogen, C₁-C₆ alkyl, C₁-C₆ haloalkyl, C₁-C₆ alkyl-OH, -O-C₁-C₆ alkyl, oxo(=O), CN or NH₂.

In Chemical Formula 1 above, each X₁ and X₂ may independently be C or N, wherein at least one of X₁ and X₂ may be C; and each Y₁ and Y₂ may independently be C or N, wherein at least one of Y₁ and Y₂ may be N.

In an embodiment, Y₁ may be N; and Y₂ may be C or N.

In an embodiment, X₁ may be C or N; and X₂ may be C.

In an embodiment, Z₁ may be C, CH or N; Z₂ may be C, C-R', CH, CH₂ or N; Z₃ may be CH, CH₂, N, N-R' or S; or Z₄ may be CH or N, wherein R' may be a hydrogen atom or C₁-C₆ alkyl.

In an embodiment, when p is 0, Z₁ may be C or N, Z₂ may be C-R', CH, CH₂ or N, Z₃ may be CH, CH₂, N, N-R' or S, wherein R' may be a hydrogen atom or C₁-C₆ alkyl.

In an embodiment, when p is 1, Z₁ may be C, CH or N, Z₂ may be C or CH, Z₃ may be CH, Z₄ may be CH or N, wherein R' may be a hydrogen atom or C₁-C₆ alkyl.

In an embodiment, L₁ may be a single bond, C₁-C₆ alkyl, C₁-C₆ haloalkyl, C₁-C₆ alkyl-C=O, -O-, -NH, -N=CH-, -NH-C₁-C₆ alkyl, substituted or unsubstituted C₆-C₁₂ aryl, or 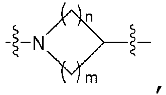 and n and m may each independently be either an integer of 1 and 2.

In an embodiment, R₁ may be a hydrogen atom, substituted or unsubstituted C₁-C₆ alkyl, C₁-C₆ alkyl-OH, - SO₂-C₁-C₆ alkyl, -N-(C₁-C₆ alkyl)₂, -C=O-R_{b}, -O-C₁-C₆ alkyl, - O-C₁-C₆ haloalkyl, substituted or unsubstituted C₃-C₆ cycloalkyl, -O-C₃-C₈ cycloalkyl, substituted or unsubstituted 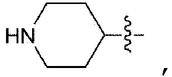 substituted or unsubstituted 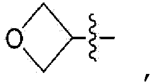 substituted or unsubstituted 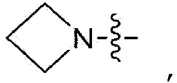 substituted or unsubstituted 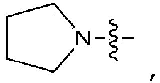 substituted or unsubstituted 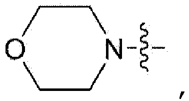 substituted or unsubstituted 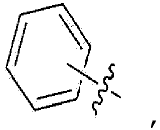 substituted or unsubstituted 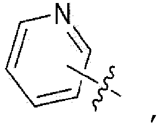 substituted or unsubstituted 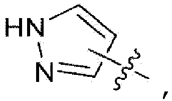 substituted or unsubstituted 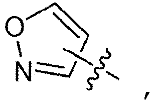 substituted or unsubstituted 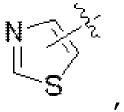 substituted or unsubstituted 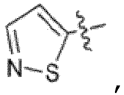 substituted or unsubstituted 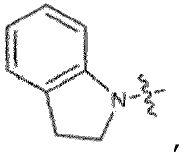 substituted or unsubstituted 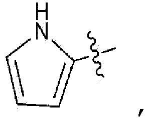 substituted or unsubstituted 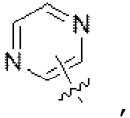 or substituted or unsubstituted 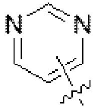 (wherein the substituted C₁-C₆ alkyl, 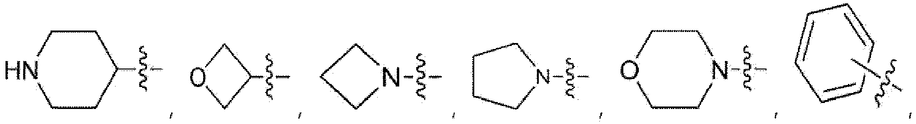 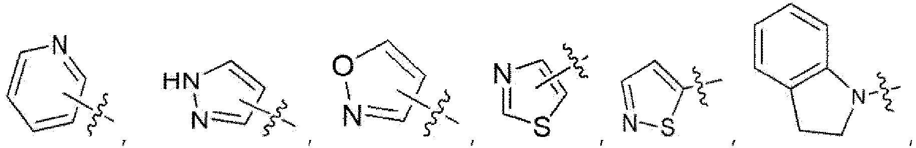 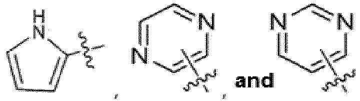 are each independently substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, C₂-C₈ heterocycloalkyl containing a heteroatom selected from oxygen, nitrogen and sulfur, C₆-C₁₂ aryl or C₃-C₁₂ heteroaryl containing a heteroatom selected from oxygen, nitrogen and sulfur), and R_{b} may be N-(C₁-C₆ alkyl)₂, O-C₁-C₆ alkyl,

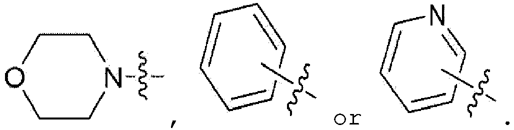

Preferably, R₁ may be a hydrogen atom, substituted or unsubstituted C₁-C₆ alkyl, C₁-C₆ alkyl-OH, -SO₂-C₁-C₆ alkyl, - N-(C₁-C₆ alkyl)₂, -C=O-N-(C₁-C₆ alkyl)₂, -C=O-O-C₁-C₆ alkyl, - O-C₁-C₆ alkyl, -O-C₁-C₆ haloalkyl,

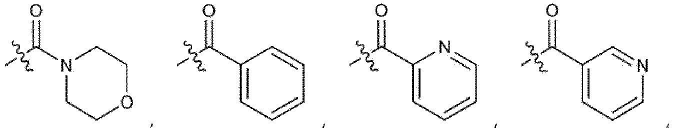

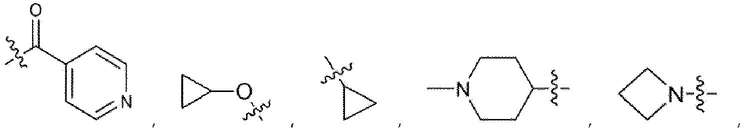

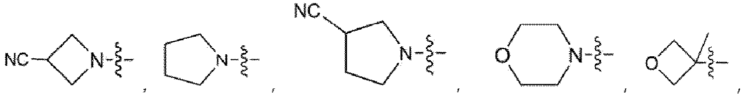

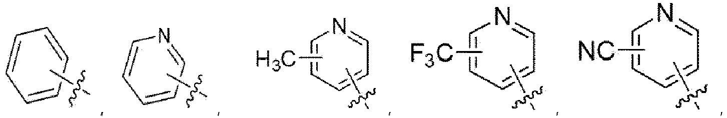

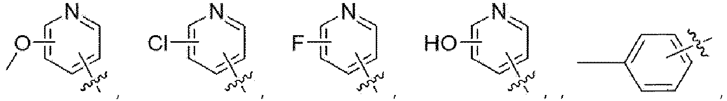

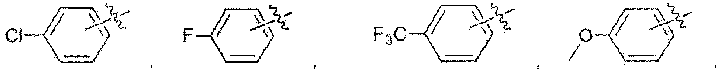

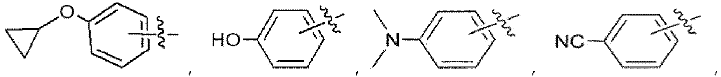

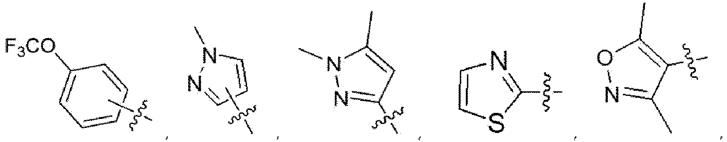

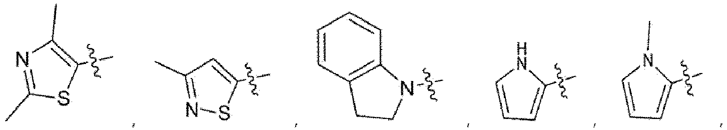

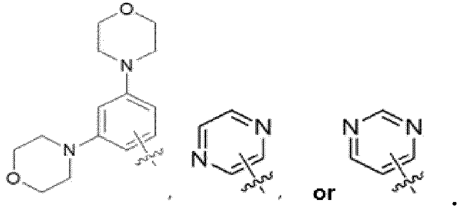

In an embodiment, L₂ may be a single bond, -NH-N=CH-, -NH-, -NH-NH-C=O-, -NH-C₁-C₆ alkyl, -O-, -O-C₁-C₆ alkyl, or

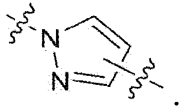

In an embodiment, L₃ may be a single bond, C₁-C₆ alkyl, -NH-N=CH-, -NH-, -NH-NH-C=O-, -NH-C₁-C₆ alkyl, -O-, -O-C₁-C₆ alkyl-, -C=O-NH-, or

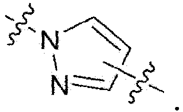

In an embodiment, R₂ may be a hydrogen atom, C₁-C₆ alkyl, C₁-C₆ haloalkyl, substituted or unsubstituted 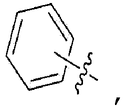 substituted or unsubstituted 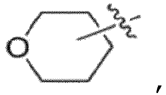 substituted or unsubstituted 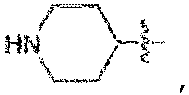 substituted or unsubstituted 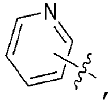 substituted or unsubstituted 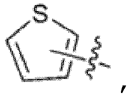 substituted or unsubstituted 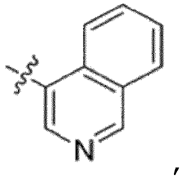 substituted or unsubstituted 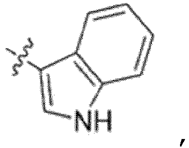 substituted or unsubstituted 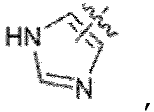 substituted or unsubstituted 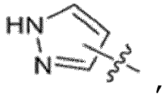 substituted or unsubstituted 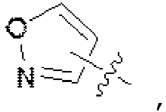 substituted or unsubstituted 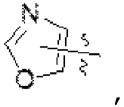 substituted or unsubstituted 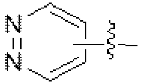 (wherein the substituted 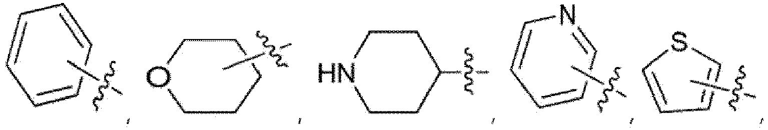 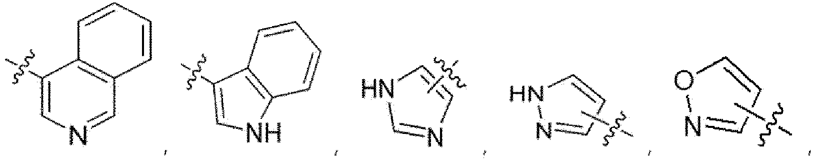 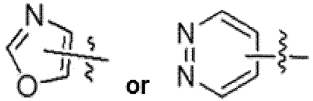 is substituted with at least one halogen atom, C₁-C₆ alkyl, C₁-C₆ haloalkyl, -O-C₁-C₆ alkyl, or CN) .

Preferably, R₂ may be a hydrogen atom, C₁-C₆ alkyl,

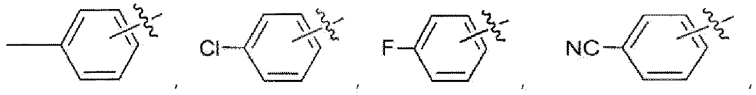

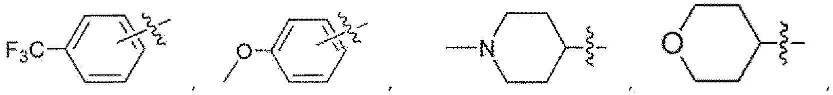

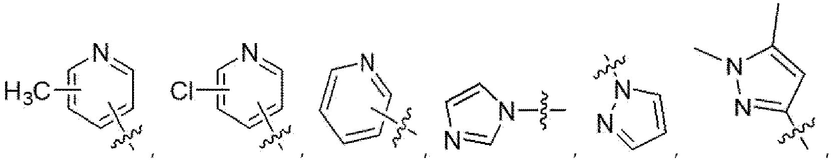

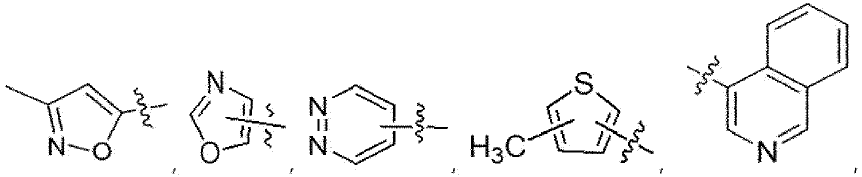

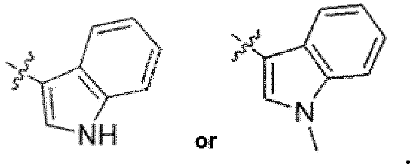

In an embodiment, L₄ may be a single bond or -NH-C₁-C₆ alkyl.

In an embodiment, 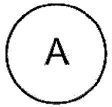 may be 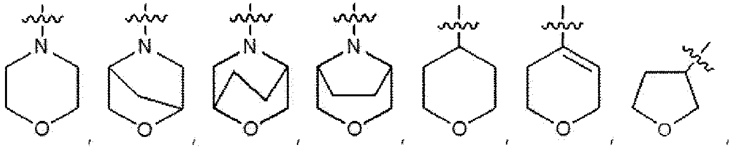 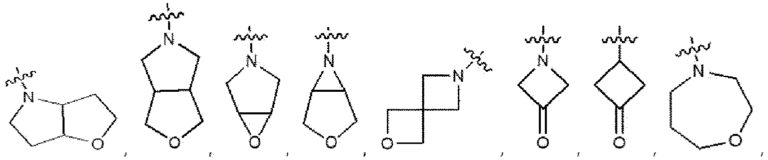 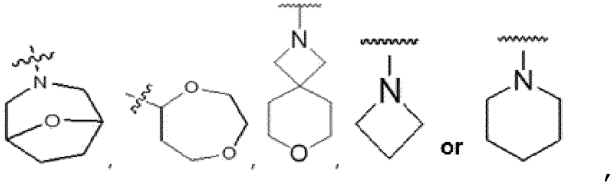 and the 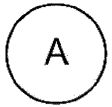 may be unsubstituted or substituted with at least one halogen, C₁-C₆ alkyl, C₁-C₆ haloalkyl, C₁-C₆ alkyl-OH, - O-C₁-C₆ alkyl, -OH, CN or NH₂.

In an embodiment, 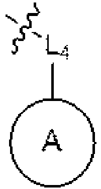 may be 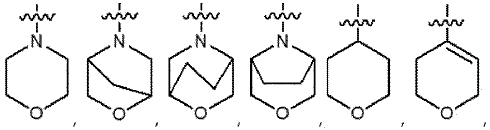 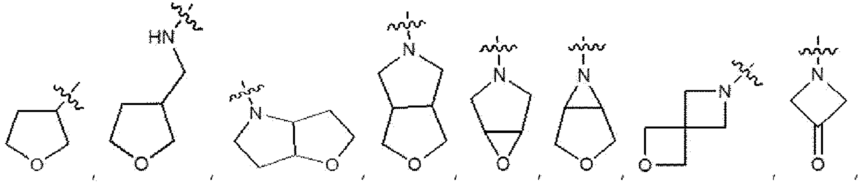 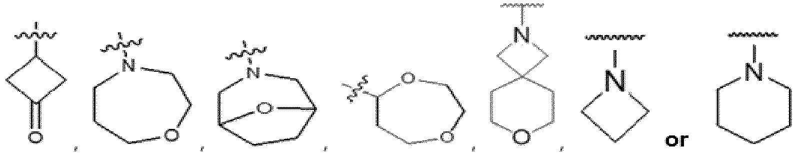 and, the 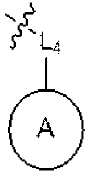 may be unsubstituted or substituted with at least one halogen, C₁-C₆ alkyl, C₁-C₆ haloalkyl, C₁-C₆ alkyl-OH, -O-C₁-C₆ alkyl, -OH, CN or NH₂.

Preferably, 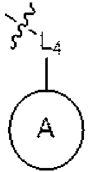 may be 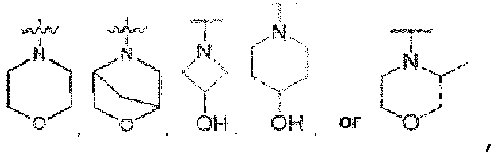 and L₄ may be a single bond.

In an embodiment, the compound represented by Chemical Formula 1 above may be a compound represented by the following Chemical Formula 2-1, an optical isomer thereof, or a pharmaceutically acceptable salt thereof:

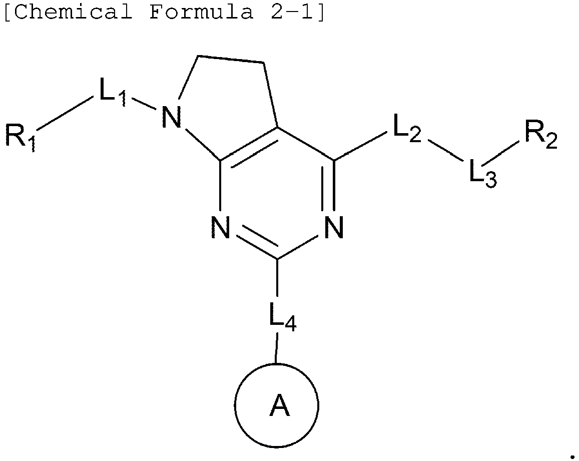

In Chemical Formula 2-1 above, L₁, L₂, L₃, L₄, R₁, R₂ and 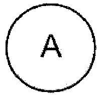 are as defined in Chemical Formula 1 above.

More specifically, in Chemical Formula 2-1 above, L₁ may be C₁-C₆ alkyl;
R₁ may be C₁-C₆ alkyl or substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 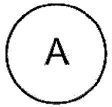 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 2-2, an optical isomer thereof, or a pharmaceutically acceptable salt thereof:

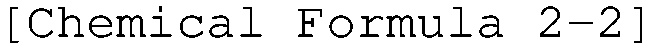

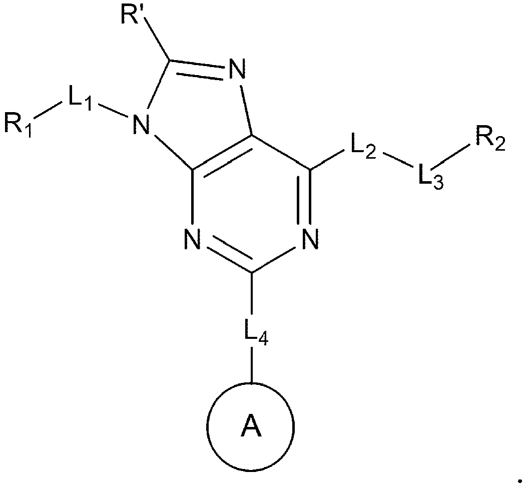

In Chemical Formula 2-2 above, L₁, L₂, L₃, L₄, R₁, R₂, R' and 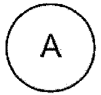 are as defined in Chemical Formula 1 above.

More specifically, R' may be a hydrogen atom or C₁-C₆ alkyl;
L₁ may be a single bond, C₁-C₆ alkyl, C₁-C₆ haloalkyl, C₁-C₆ alkyl-C=O, C₁-C₆ alkyl-Rₐ, -NH-C₁-C₆ alkyl, -N=CH-, -NH-NH-C=O-, -C=O-NH-N=CH-, -O-, -O-C₁-C₆ alkyl, substituted or unsubstituted C₆-C₁₂ aryl, or substituted or unsubstituted C₂-C₈ heterocycloalkyl containing a heteroatom selected from oxygen, nitrogen and sulfur;
Rₐ may be substituted or unsubstituted C₃-C₈ cycloalkyl or substituted or unsubstituted C₂-C₈ heterocycloalkyl containing a heteroatom selected from oxygen, nitrogen and sulfur;
R₁ may be a hydrogen atom, substituted or unsubstituted C₁-C₆ alkyl, C₁-C₆ haloalkyl, C₁-C₆ alkyl-OH, -O-C₁-C₆ alkyl, -O-C₁-C₆ haloalkyl, -O-C₃-C₈ cycloalkyl, -SO₂-C₁-C₆ alkyl, -N-(C₁-C₆ alkyl)₂, -C=O-R_{b}, substituted or unsubstituted C₃-C₈ cycloalkyl, substituted or unsubstituted C₂-C₈ heterocycloalkyl containing a heteroatom selected from oxygen, nitrogen and sulfur, substituted or unsubstituted C₂-C₈ heterocycloalkene containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted alkyl, cycloalkyl, heterocycloalkyl, heterocycloalkene, aryl, or heteroaryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
R_{b} may be C₁-C₆ alkyl, -N-(C₁-C₆ alkyl)₂, -O-C₁-C₆ alkyl, substituted or unsubstituted C₂-C₈ heterocycloalkyl containing a heteroatom selected from oxygen, nitrogen and sulfur, substituted or unsubstituted C₆-C₁₂ aryl, substituted or unsubstituted C₃-C₁₂ heteroaryl containing a heteroatom selected from oxygen, nitrogen and sulfur, wherein the substituted heterocycloalkyl, aryl and heteroaryl may be substituted with at least one halogen atom, C₁-C₆ alkyl, C₁-C₆ haloalkyl, -O-C₁-C₆ alkyl, oxo(=O), -OH, -CN or -NH₂;
L₂ and L₃ may be each independently a single bond, C₁-C₆ alkyl, -C=O-NH, -NH, -NH-C₁-C₆ alkyl, -NH-N=CH-, -NH-NH-C=O-, -O-, -O-C₁-C₆ alkyl, or substituted or unsubstituted C₃-C₁₂ heteroaryl containing a heteroatom selected from oxygen, nitrogen and sulfur, wherein the substituted heteroaryl may be substituted with at least one halogen atom, C₁-C₆ alkyl, C₁-C₆ haloalkyl, -O-C₁-C₆ alkyl, CN or NH₂;
R₂ may be a hydrogen atom, substituted or unsubstituted C₂-C₈ heterocycloalkyl containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted heterocycloalkyl, aryl and heteroaryl may be substituted with at least one halogen atom, C₁-C₆ alkyl, C₁-C₆ haloalkyl, -O-C₁-C₆ alkyl, CN or NH₂;
L₄ may be a single bond; and 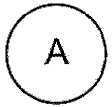 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 2-3, an optical isomer thereof, or a pharmaceutically acceptable salt thereof:

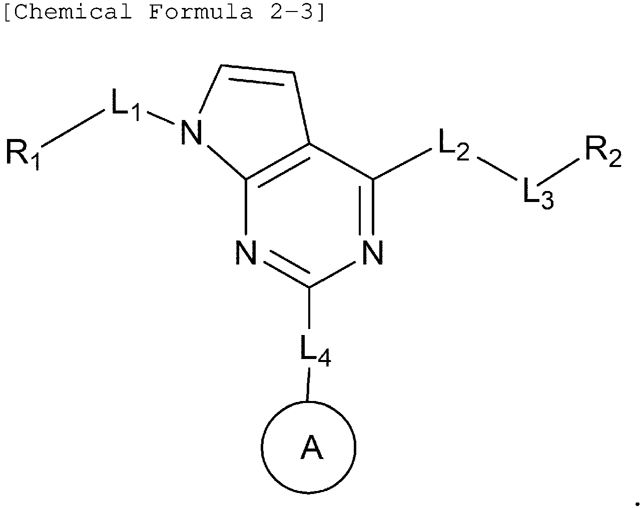

In Chemical Formula 2-3 above, L₁, L₂, L₃, L₄, R₁, R₂ and 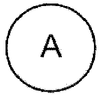 are as defined in Chemical Formula 1 above.

More specifically, L₁ may be a single bond, C₁-C₆ alkyl or C₁-C₆ haloalkyl;
R₁ may be a hydrogen atom, substituted or unsubstituted C₁-C₆ alkyl, substituted or unsubstituted C₆-C₁₂ aryl, or substituted or unsubstituted C₃-C₁₂ heteroaryl containing a heteroatom selected from oxygen, nitrogen and sulfur, wherein the substituted alkyl, aryl, or heteroaryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 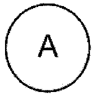 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 2-4, an optical isomer thereof, or a pharmaceutically acceptable salt thereof:

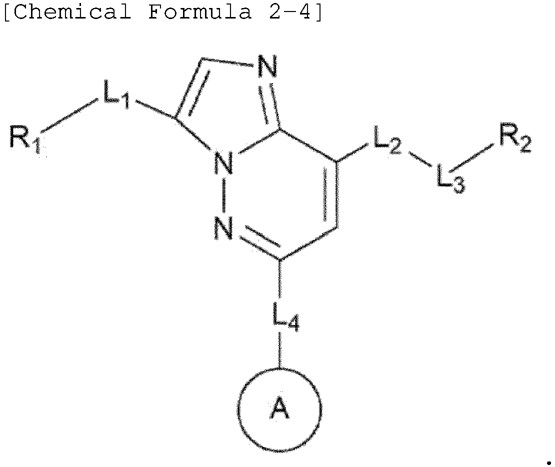

In Chemical Formula 2-4 above, L₁, L₂, L₃, L₄, R₁, R₂ and 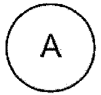 are as defined in Chemical Formula 1 above.

More specifically, L₁ may be a single bond;
R₁ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, - N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 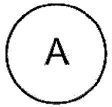 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 2-5, an optical isomer thereof, or a pharmaceutically acceptable salt thereof:

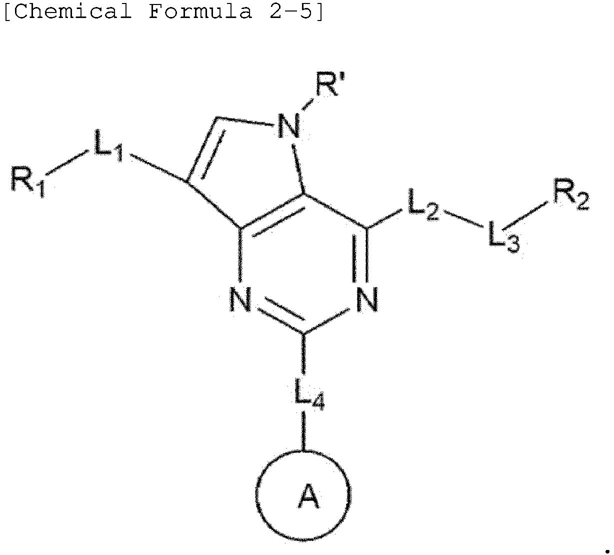

In Chemical Formula 2-5 above, L₁, L₂, L₃, L₄, R₁, R₂, R' and 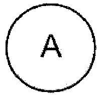 are as defined in Chemical Formula 1 above.

More specifically, in Chemical Formula 2-5 above, R' may be a hydrogen atom or C₁-C₆ alkyl;
L₁ may be a single bond;
R₁ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, - N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 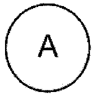 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 2-6, an optical isomer thereof, or a pharmaceutically acceptable salt thereof:

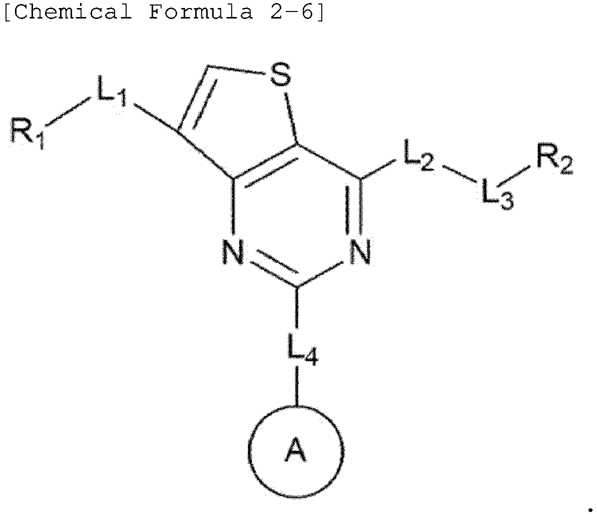

In Chemical Formula 2-6 above, L₁, L₂, L₃, L₄, R₁, R₂, and 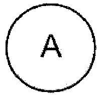 are as defined in Chemical Formula 1 above.

More specifically, L₁ may be a single bond;
R₁ may be substituted or unsubstituted C₆-C₁₂ aryl, or substituted or unsubstituted C₃-C₁₂ heteroaryl containing a heteroatom selected from oxygen, nitrogen and sulfur, wherein the substituted aryl, or heteroaryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 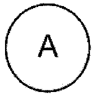 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 2-7, an optical isomer thereof, or a pharmaceutically acceptable salt thereof:

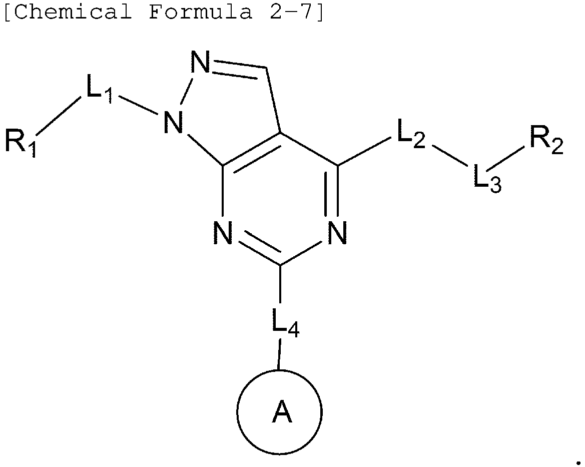

In Chemical Formula 2-7 above, L₁, L₂, L₃, L₄, R₁, R₂, and 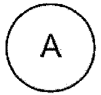 are as defined in Chemical Formula 1 above.

More specifically, L₁ may be a single bond;
R₁ may be substituted or unsubstituted C₆-C₁₂ aryl, or substituted or unsubstituted C₃-C₁₂ heteroaryl containing a heteroatom selected from oxygen, nitrogen and sulfur, wherein the substituted aryl, or heteroaryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 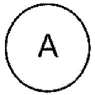 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 3-1, an optical isomer thereof, or a pharmaceutically acceptable salt thereof:

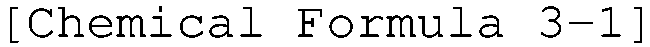

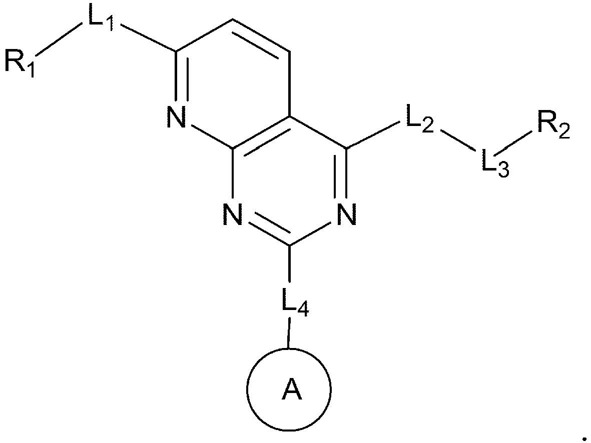

In Chemical Formula 3-1 above, L₁, L₂, L₃, L₄, R₁, R₂, and 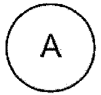 are as defined in Chemical Formula 1 above.

More specifically, L₁ may be a single bond;
R₁ may be substituted or unsubstituted C₆-C₁₂ aryl, or substituted or unsubstituted C₃-C₁₂ heteroaryl containing a heteroatom selected from oxygen, nitrogen and sulfur, wherein the substituted aryl, or heteroaryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 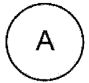 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

In an embodiment, the compound represented by Chemical Formula 1 above may be a compound represented by the following Chemical Formula 3-2, an optical isomer thereof, or a pharmaceutically acceptable salt thereof:

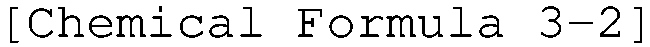

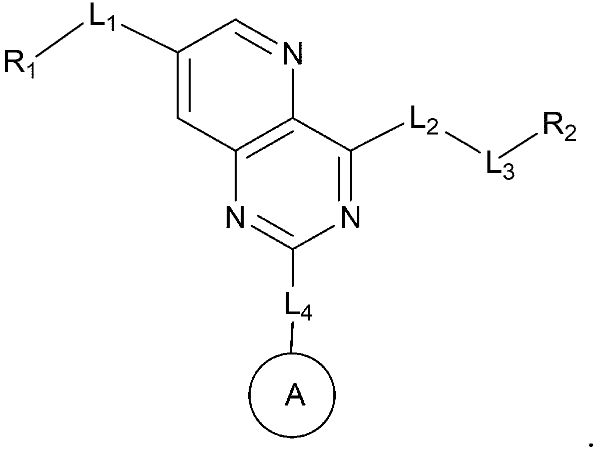

In Chemical Formula 3-2 above, L₁, L₂, L₃, L₄, R₁, R₂, and 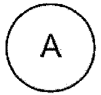 are as defined in Chemical Formula 1 above.

In an embodiment, the compound represented by Chemical Formula 1 above may be a compound represented by the following Chemical Formula 3-3, an optical isomer thereof, or a pharmaceutically acceptable salt thereof:

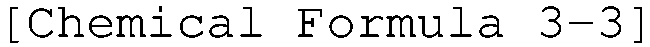

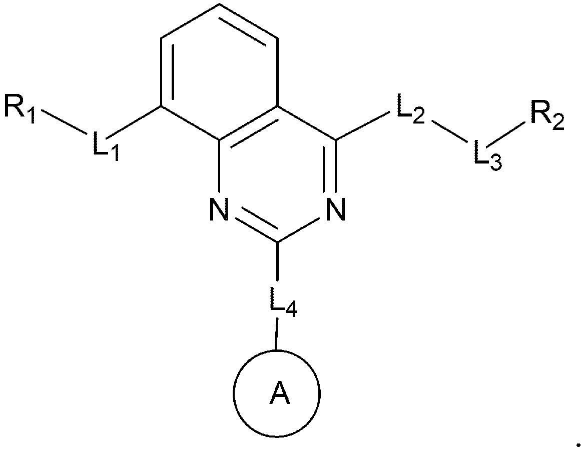

In Chemical Formula 3-3 above, L₁, L₂, L₃, L₄, R₁, R₂, and 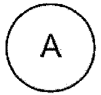 are as defined in Chemical Formula 1 above.
L₁ may be a single bond;
R₁ may be substituted or unsubstituted C₆-C₁₂ aryl, or substituted or unsubstituted C₃-C₁₂ heteroaryl containing a heteroatom selected from oxygen, nitrogen and sulfur, wherein the substituted aryl, or heteroaryl may be substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
L₂ and L₃ may each independently be a single bond or - NH-N=CH-;
R₂ may be substituted or unsubstituted C₆-C₁₂ aryl, wherein the substituted aryl may be substituted with at least one halogen atom, C₁-C₆ alkyl, C₁-C₆ haloalkyl, -O-C₁-C₆ alkyl, CN or NH₂;
L₄ may be a single bond; and 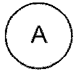 may be substituted or unsubstituted C₂-C₈ heterocycloalkyl containing a heteroatom selected from oxygen, nitrogen and sulfur, wherein the substituted heterocycloalkyl and heterocycloalkene may be substituted with at least one halogen, C₁-C₆ alkyl, C₁-C₆ haloalkyl, C₁-C₆ alkyl-OH, -O-C₁-C₆ alkyl, oxo(=O), CN or NH₂.

According to a specific example of the present disclosure, the compound represented by Chemical Formula 1 may be any one selected from the group consisting of the following compounds:
(E)-4-(4-(2-(3-methylbenzylidene)hydrazinyl)-7-phenethyl-6,7-dihydro-5H-pyrrolo[2,3-d]pyrimidin-2-yl)morpholine;
(E)-4-(7-isobutyl-4-(2-(3-methylbenzylidene)hydrazinyl)-6,7-dihydro-5H-pyrrolo[2,3-d]pyrimidin-2-yl)morpholine;
(E)-4-(7-isobutyl-4-(2-(3-methylbenzylidene)hydrazinyl)-7H-pyrrolo[2,3-d]pyrimidin-2-yl)morpholine;
(E)-4-(4-(2-(3-methylbenzylidene)hydrazinyl)-7-phenethyl-pyrrolo[2,3-d]pyrimidin-2-yl)morpholine;
(E)-4-(4-(2-(3-methylbenzylidene)hydrazinyl)-7-(2-(pyridin-2-yl)ethyl)-7H-pyrrolo[2,3-d]pyrimidin-2-yl)morpholine;
(E)-4-(7-(2,2-difluoro-2-(pyridin-2-yl)ethyl)-4-(2-(3-methylbenzylidene)hydrazinyl)-7H-pyrrolo[2,3-d]pyrimidin-2-yl)morpholine;
(E)-4-(8-(2-(3-methylbenzylidene)hydrazinyl)-3-phenylimidazo[1,2-b]pyridazin-6-yl)morpholine;
(E)-4-(4-(2-(3-methylbenzylidene)hydrazinyl)-7-phenyl-5H-pyrrolo[3,2-d]pyrimidin-2-yl)morpholine;
(E)-4-(5-methyl-4-(2-(3-methylbenzylidene)hydrazinyl)-7-phenyl-5H-pyrrolo[3,2-d]pyrimidin-2-yl)morpholine;
(E)-4-(5-isopropyl-4-(2-(3-methylbenzylidene)hydrazinyl)-7-phenyl-5H-pyrrolo[3,2-d]pyrimidin-2-yl)morpholine;
(E)-4-(4-(2-(3-methylbenzylidene)hydrazinyl)-7-phenylthieno[3,2-d]pyrimidin-2-yl)morpholine;
(E)-4-(4-(2-benzylidenehydrazinyl)-7-(1-methyl-1H-pyrazol-4-yl)thieno[3,2-d]pyrimidin-2-yl)morpholine;
(E)-4-(4-(2-(3-methylbenzylidene)hydrazinyl)-1-(pyridin-3-yl)-1H-pyrazolo[3,4-d]pyrimidin-6-yl)morpholine;
(E)-4-(4-(2-(3-methylbenzylidene)hydrazinyl)-1-phenyl-1H-pyrazolo[3,4-d]pyrimidin-6-yl)morpholine;
(E)-4-(4-(2-(3-methylbenzylidene)hydrazinyl)-1-(pyridin-2-yl)-1H-pyrazolo[3,4-d]pyrimidin-6-yl)morpholine;
(E)-4-(4-(2-(3-methylbenzylidene)hydrazinyl)-1-(pyridin-4-yl)-1H-pyrazolo[3,4-d]pyrimidin-6-yl)morpholine;
(E)-4-(9-isobutyl-6-(2-(3-methylbenzylidene)hydrazinyl)-9H-purin-2-yl)morpholine;
(E)-4-(6-(2-(3-methylbenzylidene)hydrazinyl)-9-phenethyl-9H-purin-2-yl)morpholine;
(E)-1-cyclopropyl-2-(6-(2-(3-methylbenzylidene)hydrazinyl)-2-morpholino-9H-purin-9-yl)ethan-1-one;
(E)-4-(6-(2-(3-methylbenzylidene)hydrazinyl)-9-(pyridin-2-ylmethyl)-9H-purin-2-yl)morpholine;
(E)-4-(9-(cyclopropylmethyl)-6-(2-(3-methylbenzylidene)hydrazinyl)-9H-purin-2-yl)morpholine;
(E)-2-(6-(2-(3-methylbenzylidene)hydrazinyl)-2-morpholino-9H-purin-9-yl)-1-phenylethan-1-one;
(E)-2-(6-(2-(3-methylbenzylidene)hydrazinyl)-2-morpholino-9H-purin-9-yl)-1-(pyridin-2-yl)ethan-1-one;
(E)-2-(6-(2-((1H-indol-3-yl)methylene)hydrazinyl)-2-morpholino-9H-purin-9-yl)-1-(pyridin-2-yl)ethan-1-one;
(E)-1-(4-chlorophenyl)-2-(6-(2-(3-methylbenzylidene)hydrazinyl)-2-morpholino-9H-purin-9-yl)ethan-1-one;
(E)-4-(9-(2,2-difluoro-2-(pyridin-2-yl)ethyl)-6-(2-(3-methylbenzylidene)hydrazinyl)-9H-purin-2-yl)morpholine;
(E)-2-(6-(2-(3-methylbenzylidene)hydrazinyl)-2-morpholino-9H-purin-9-yl)-1-(pyrrolidin-1-yl)ethan-1-one;
(E)-2-(6-(2-(3-methylbenzylidene)hydrazinyl)-2-morpholino-9H-purin-9-yl)-1-(pyridin-3-yl)ethan-1-one;
(E)-1-(indolin-1-yl)-2-(6-(2-(3-methylbenzylidene)hydrazinyl)-2-morpholino-9H-purin-9-yl)ethan-1-one;
(E)-1-(1-methyl-1H-pyrrol-2-yl)-2-(6-(2-(3-methylbenzylidene)hydrazinyl)-2-morpholino-9H-purin-9-yl)ethan-1-one;
(E)-1-(2-(6-(2-(3-methylbenzylidene)hydrazinyl)-2-morpholino-9H-purin-9-yl)acetyl)azetidine-3-carbonitrile;
(E)-2-(6-(2-(3-methylbenzylidene)hydrazinyl)-2-morpholino-9H-purin-9-yl)-1-(pyridin-4-yl)ethan-1-one;
(E)-1-(2-(6-(2-(3-methylbenzylidene)hydrazinyl)-2-morpholino-9H-purin-9-yl)acetyl)pyrrolidine-3-carbonitrile;
(E)-2-(6-(2-(3-methylbenzylidene)hydrazinyl)-2-morpholino-9H-purin-9-yl)-1-(1H-pyrrol-2-yl)ethan-1-one;
(E)-4-(2-(6-(2-(3-methylbenzylidene)hydrazinyl)-2-morpholino-9H-purin-9-yl)acetyl)benzonitrile;
(E)-3-(2-(6-(2-(3-methylbenzylidene)hydrazinyl)-2-morpholino-9H-purin-9-yl)acetyl)benzonitrile;
(E)-1-(2-methoxyphenyl)-2-(6-(2-(3-methylbenzylidene)hydrazinyl)-2-morpholino-9H-purin-9-yl)ethan-1-one;
(E)-1-(3-methoxyphenyl)-2-(6-(2-(3-methylbenzylidene)hydrazinyl)-2-morpholino-9H-purin-9-yl)ethan-1-one;
(E)-1-(4-methoxyphenyl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(o-tolyl)ethan-1-one;
(E)-2-(6-(2-(3-methylbenzylidene)hydrazinyl)-2-morpholino-9H-purin-9-yl)-1-(m-tolyl)ethan-1-one;
(E)-2-(6-(2-(3-methylbenzylidene)hydrazinyl)-2-morpholino-9H-purin-9-yl)-1-(p-tolyl)ethan-1-one;
(E)-2-(6-(2-(3-methylbenzylidene)hydrazinyl)-2-morpholino-9H-purin-9-yl)-1-(2-(trifluoromethyl)phenyl)ethan-1-one;
(E)-2-(6-(2-(3-methylbenzylidene)hydrazinyl)-2-morpholino-9H-purin-9-yl)-1-(3-(trifluoromethyl)phenyl)ethan-1-one;
(E)-2-(6-(2-(3-methylbenzylidene)hydrazinyl)-2-morpholino-9H-purin-9-yl)-1-(4-(trifluoromethyl)phenyl)ethan-1-one;
(E)-1-(3-fluorophenyl)-2-(6-(2-(3-methylbenzylidene)hydrazinyl)-2-morpholino-9H-purin-9-yl)ethan-1-one;
(E)-1-(4-fluorophenyl)-2-(6-(2-(3-methylbenzylidene)hydrazinyl)-2-morpholino-9H-purin-9-yl)ethan-1-one;
(E)-1-(2-chlorophenyl)-2-(6-(2-(3-methylbenzylidene)hydrazinyl)-2-morpholino-9H-purin-9-yl)ethan-1-one;
(E)-1-(3-chlorophenyl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(3-(trifluoromethoxy)phenyl)ethan-1-one;
(E)-1-(2,4-dimorpholinophenyl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(4-(trifluoromethyl)pyridin-2-yl)ethan-1-one;
(E)-1-(5-fluoropyridin-2-yl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(pyrazin-2-yl)ethan-1-one;
(E)-2-(6-(2-((5-methylthiophen-2-yl)methylene)hydrazinyl)-2-morpholino-9H-purin-9-yl)-1-(pyridin-2-yl)ethan-1-one;
(E)-3-((2-(2-morpholino-9-(2-oxo-2-(pyridin-2-yl)ethyl)-9H-purin-6-yl)hydrazinylidene)methyl)benzonitrile;
(E)-2-(2-morpholino-6-(2-(3-(trifluoromethyl)benzylidene)hydrazinyl)-9H-purin-9-yl)-1-(pyridin-2-yl)ethan-1-one;
(E)-2-(6-(2-(4-methylbenzylidene)hydrazinyl)-2-morpholino-9H-purin-9-yl)-1-(pyridin-2-yl)ethan-1-one;
(E)-2-(6-(2-(2-methylbenzylidene)hydrazinyl)-2-morpholino-9H-purin-9-yl)-1-(pyridin-2-yl)ethan-1-one;
(E)-2-(2-morpholino-6-(2-(pyridin-3-ylmethylene)hydrazinyl)-9H-purin-9-yl)-1-(pyridin-2-yl)ethan-1-one;
(E)-2-(6-(2-(3-methoxybenzylidene)hydrazinyl)-2-morpholino-9H-purin-9-yl)-1-(pyridin-2-yl)ethan-1-one;
3-methyl-N'-(2-morpholino-9-(2-oxo-2-(pyridin-2-yl)ethyl)-9H-purin-6-yl)benzohydrazide;
2-(2-morpholino-6-(3-(pyridin-3-yl)-1H-pyrazol-1-yl)-9H-purin-9-yl)-1-(pyridin-2-yl)-ethan-1-one;
2-(2-morpholino-6-(3-(m-tolyl)-1H-pyrazol-1-yl)-9H-purin-9-yl)-1-(pyridin-2-yl)ethan-1-one;
2-(2-morpholino-6-(3-(3-(trifluoromethyl)phenyl)-1H-pyrazol-1-yl)-9H-purin-9-yl)-1-(pyridin-2-yl)ethan-1-one;
2-(6-(3-(3-fluorophenyl)-1H-pyrazol-1-yl)-2-morpholino-9H-purin-9-yl)-1-(pyridin-2-yl)ethan-1-one;
3-(1-(2-morpholino-9-(2-oxo-2-(pyridin-2-yl)ethyl)-9H-purin-6-yl)-1H-pyrazol-3-yl)benzonitrile;
2-(6-(3-(3-methoxyphenyl)-1H-pyrazol-1-yl)-2-morpholino-9H-purin-9-yl)-1-(pyridin-2-yl)ethan-1-one;
2-(2-morpholino-6-(3-(tetrahydro-2H-pyran-4-yl)-1H-pyrazol-1-yl)-9H-purin-9-yl)-1-(pyridin-2-yl)ethan-1-one;
2-(6-(3-(6-methylpyridin-2-yl)-1H-pyrazol-1-yl)-2-morpholino-9H-purin-9-yl)-1-(pyridin-2-yl)ethan-1-one;
1-(2-morpholino-9-(2-oxo-2-(pyridin-2-yl)ethyl)-9H-purin-6-yl)-N-(m-tolyl)-1H-pyrazole-3-carboxamide;
(E)-1-(azetidin-1-yl)-2-(6-(2-(3-methylbenzylidene)hydrazinyl)-2-morpholino-9H-purin-9-yl)ethan-1-one;
(E)-4-(6-(2-(3-methylbenzylidene)hydrazinyl)-9-(2-(pyridin-2-yl)ethyl)-9H-purin-2-yl)morpholine;
(E)-4-(6-(2-(3-methylbenzylidene)hydrazinyl)-9-((3-methyloxetan-3-yl)methyl)-9H-purin-2-yl)morpholine;
(E)-4-(2-(6-(2-(3-methylbenzylidene)hydrazinyl)-2-morpholino-9H-purin-9-yl)ethylmorpholine;
(E)-4-(6-(2-(3-methylbenzylidene)hydrazinyl)-9-(1-methylpiperidin-4-yl)-9H-purin-2-yl)morpholine;
(E)-4-(6-(2-(3-methylbenzylidene)hydrazinyl)-9-(pyridin-4-ylmethyl)-9H-purin-2-yl)morpholine;
(E)-4-(6-(2-(3-methylbenzylidene)hydrazinyl)-9-(pyridin-3-ylmethyl)-9H-purin-2-yl)morpholine;
(E)-4-(6-(2-(isoquinolin-4-ylmethylene)hydrazinyl)-9-(2-pyridin-2-yl)ethyl)-9H-purin-2-yl)morpholine;
(E)-4-(6-(2-(3-methylbenzylidene)hydrazinyl)-9-(2-(pyridin-3-yl)ethyl)-9H-purin-2-yl)morpholine;
(E)-(3-(6-(2-(3-methylbenzylidene)hydrazinyl)-2-morpholino-9H-purin-9-yl)azetidin-1-yl) (phenyl)methanone;
(E)-3-(6-(2-(3-methylbenzylidene)hydrazinyl)-2-morpholino-9H-purin-9-yl)azetidin-1-yl(pyridin-3-yl)methanone;
methyl (E)-3-(6-(2-(3-methylbenzylidene)hydrazinyl)-2-morpholino-9H-purin-9-yl)azetidine-1-carboxylate;
(E)-N,N-dimethyl-3-(6-(2-(3-methylbenzylidene)hydrazinyl)-2-morpholino-9H-purin-9-yl)azetidine-1-carboxamide;
(E)-4-(6-(2-(3-methylbenzylidene)hydrazinyl)-9-(1-(methylsulfonyl)azetidin-3-yl)-9H-purin-2-yl)morpholine;
(E)-3-(6-(2-(3-methylbenzylidene)hydrazinyl)-2-morpholino-9H-purin-9-yl)azetidin-1-yl(morpholino)methanone;
(E)-(4-(6-(2-(3-methylbenzylidene)hydrazinyl)-2-morpholino-9H-purin-9-yl)piperidin-1-yl) (pyridin-3-yl)methanone;
(E)-(4-(6-(2-(3-methylbenzylidene)hydrazinyl)-2-morpholino-9H-purin-9-yl)piperidin-1-yl) (morpholino)methanone;
(E)-(4-(6-(2-(3-methylbenzylidene)hydrazinyl)-2-morpholino-9H-purin-9-yl)piperidin-1-yl) (phenyl)methanone;
(E)-N,N-dimethyl-4-(6-(2-(3-methylbenzylidene)hydrazinyl)-2-morpholino-9H-purin-9-yl)piperidine-1-carboxamide;
methyl (E)-4-(6-(2-(3-methylbenzylidene)hydrazinyl)-2-morpholino-9H-purin-9-yl)piperidine-1-carboxylate;
(E)-4-(6-(2-(3-methylbenzylidene)hydrazinyl)-9-(1-(methylsulfonyl)piperidin-4-yl)-9H-purin-2-yl)morpholino;
(E)-(4-(6-(2-(3-methylbenzylidene)hydrazinyl)-2-morpholino-9H-purin-9-yl)piperidin-1-yl) (pyridin-4-yl)methanone;
(E)-(4-(6-(2-(3-methylbenzylidene)hydrazinyl)-2-morpholino-9H-purin-9-yl)piperidin-1-yl) (pyridin-2-yl)methanone;
(E)-4-(9-(1-methyl-1H-pyrazol-4-yl)-6-(2-(3-methylbenzylidene)hydrazinyl)-9H-purin-2-yl)-morpholine;
(E)-4-(6-(2-(3-methylbenzylidene)hydrazinyl)-9-(pyridin-3-yl)-9H-purin-2-yl)morpholine;
(S,E)-3-methyl-4-(6-(2-(3-methylbenzylidene)hydrazinyl)-9-(pyridin-3-yl)-9H-purin-2-yl)morpholine;
(1R,4R)-5-(6-(2-(3-methylbenzylidene)hydrazinyl)-9-(pyridin-3-yl)-9H-purin-2-yl)-oxa-5-azabicyclo[2.2.1]heptane;
(E)-4-(6-(2-(3-methylbenzylidene)hydrazinyl)-9-(pyridin-2-yl)-9H-purin-2-yl)morpholine;
(E)-4-(9-(4-chlorophenyl)-6-(2-(3-methylbenzylidene)hydrazinyl)-9H-purin-2-yl)morpholine;
(E)-4-(9-(2-methoxypyridin-4-yl)-6-(2-(3-methylbenzylidene)hydrazinyl)-9H-purin-2-yl)morpholine;
(E)-4-(6-(2-(3-methylbenzylidene)hydrazinyl)-2-morpholino-9H-purin-9-yl)pyridin-2-ol;
(E)-4-(6-(2-(3-methylbenzylidene)hydrazinyl)-9-(pyridin-4-yl)-9H-purin-2-yl)morpholine;
(E)-4-(6-(2-(3-methylbenzylidene)hydrazinyl)-9-(4-(trifluoromethyl)phenyl)-9H-purin-2-yl)morpholine;
(E)-1-(6-(2-(3-methylbenzylidene)hydrazinyl)-9-(pyridin-3-yl)-9H-purin-2-yl)piperidin-4-ol;
(E)-1-(6-(2-(3-methylbenzylidene)hydrazinyl)-9-(pyridin-3-yl)-9H-purin-2-yl)azetidin-3-ol;
4-(9-(pyridin-4-yl)-6-(3-(m-tolyl)-1H-pyrazol-1-yl)-9H-purin-2-yl)morpholine;
(E)-4-(9-(5-methoxypyridin-2-yl)-6-(2-(3-methylbenzylidene)hydrazinyl)-9H-purin-2-yl)morpholine;
(E)-4-(6-(2-(3-methylbenzylidene)hydrazinyl)-9-(2-methylpyridin-3-yl)-9H-purin-2-yl)morpholine;
(E)-4-(6-(2-(3-methylbenzylidene)hydrazinyl)-9-(6-methylpyridin-3-yl)-9H-purin-2-yl)morpholine;
(E)-4-(9-(2,4-dimethylthiazol-5-yl)-6-(2-(3-methylbenzylidene)hydrazinyl)-9H-purin-2-yl)morpholine;
(E)-4-(6-(2-((1H-indol-3-yl)methylene)hydrazinyl)-9-(pyridin-4-yl)-9H-purin-2-yl)morpholine;
(E)-4-(9-(6-methoxypyridin-3-yl)-6-(2-(3-methylbenzylidene)hydrazinyl)-9H-purin-2-yl)morpholine;
(E)-5-(6-(2-(3-methylbenzylidene)hydrazinyl)-2-morpholino-9H-purin-9-yl)pyridin-2-ol;
(E)-4-(9-(2-chloropyridin-4-yl)-6-(2-(3-methylbenzylidene)hydrazinyl)-9H-purin-2-yl)morpholine;
(E)-4-(9-(5-chloropyridin-2-yl)-6-(2-(3-methylbenzylidene)hydrazinyl)-9H-purin-2-yl)morpholine;
(E)-4-(6-(2-(3-methylbenzylidene)hydrazinyl)-9-(thiazol-2-yl)-9H-purin-2-yl)morpholine;
4-(9-(pyridin-3-yl)-6-(3-(m-tolyl)-1H-pyrazol-1-yl)-9H-purin-2-yl)morpholine;
(E)-4-(6-(2-(3-methylbenzylidene)hydrazinyl)-9-phenyl-9H-purin-2-yl)morpholine;
(E)-4-(9-(4-methoxyphenyl)-6-(2-(3-methylbenzylidene)hydrazinyl)-9H-purin-2-yl)morpholine;
(E)-4-(9-(3-methoxyphenyl)-6-(2-(3-methylbenzylidene)hydrazinyl)-9H-purin-2-yl)morpholine;
(E)-4-(9-(3-chlorophenyl)-6-(2-(3-methylbenzylidene)hydrazinyl)-9H-purin-2-yl)morpholine;
(E)-N,N-dimethyl-4-(6-(2-(3-methylbenzylidene)hydrazinyl)-2-morpholino-9H-purin-9-yl)aniline;
(E)-4-(9-(2-methoxyphenyl)-6-(2-(3-methylbenzylidene)hydrazinyl)-9H-purin-2-yl)morpholine;
(E)-4-(9-(3-cyclopropoxyphenyl)-6-(2-(3-methylbenzylidene)hydrazinyl)-9H-purin-2-yl)morpholine;
(E)-3-(6-(2-(3-methylbenzylidene)hydrazinyl)-2-morpholino-9H-purin-9-yl)benzonitrile;
(E)-4-(6-(2-(3-methylbenzylidene)hydrazinyl)-9-(m-tolyl)-9H-purin-2-yl)morpholine;
(E)-4-(6-(2-(3-methylbenzylidene)hydrazinyl)-9-(3-(trifluoromethoxy)phenyl)-9H-purin-2-yl)morpholine;
(E)-4-(9-(3-fluorophenyl)-6-(2-(3-methylbenzylidene)hydrazinyl)-9H-purin-2-yl)morpholine;
(E)-4-(6-(2-(3-methylbenzylidene)hydrazinyl)-9-(3-(trifluoromethyl)phenyl)-9H-purin-2-yl)morpholine;
(E)-3-(6-(2-(3-methylbenzylidene)hydrazinyl)-2-morpholino-9H-purin-9-yl)phenol;
(E)-4-(6-(2-((1H-indol-3-yl)methylene)hydrazinyl)-9-phenyl-9H-purin-2-yl)morpholine;
(E)-4-(6-(2-((1-methyl-1H-indol-3-yl)methylene)hydrazinyl)-9-phenyl-9H-purin-2-yl)morpholine;
4-(9-(4-methoxyphenyl)-6-(3-(m-tolyl)-1H-pyrazol-1-yl)-9H-purin-2-yl)morpholine;
4-(9-(3-chlorophenyl)-6-(3-(m-tolyl)-1H-pyrazol-1-yl)-9H-purin-2-yl)morpholine;
4-(9-phenyl-6-(3-(m-tolyl)-1H-pyrazol-1-yl)-9H-purin-2-yl)morpholine;
4-(9-(3-methoxyphenyl)-6-(3-(m-tolyl)-1H-pyrazol-1-yl)-9H-purin-2-yl)morpholine;
4-(9-(3-cyclopropoxyphenyl)-6-(3-(m-tolyl)-1H-pyrazol-1-yl)-9H-purin-2-yl)morpholine;
(E)-4-(6-(2-(3-methylbenzylidene)hydrazinyl)-9-(2-(trifluoromethyl)pyridin-4-yl)-9H-purin-2-yl)morpholine;
(E)-4-(6-(2-(3-methylbenzylidene)hydrazinyl)-9-(2-methylpyridin-4-yl)-9H-purin-2-yl)morpholine;
(E)-4-(6-(2-(3-methylbenzylidene)hydrazinyl)-9-(6-methylpyridin-2-yl)-9H-purin-2-yl)morpholine;
(E)-4-(6-(2-(3-methylbenzylidene)hydrazinyl)-9-(3-methylpyridin-2-yl)-9H-purin-2-yl)morpholine;
(E)-4-(6-(2-(3-methylbenzylidene)hydrazinyl)-9-(5-methylpyridin-2-yl)-9H-purin-2-yl)morpholine;
(E)-4-(6-(2-(3-methylbenzylidene)hydrazinyl)-9-(4-methylpyridin-2-yl)-9H-purin-2-yl)morpholine;
(E)-4-(6-(2-(3-methylbenzylidene)hydrazinyl)-9-(5-methylpyridin-3-yl)-9H-purin-2-yl)morpholine;
(E)-6-(6-(2-(3-methylbenzylidene)hydrazinyl)-2-morpholino-9H-purin-9-yl)nicotinonitrile;
(E)-4-(9-(1,5-dimethyl-1H-pyrazol-3-yl)-6-(2-(3-methylbenzylidene)hydrazinyl)-9H-purin-2-yl)morpholine;
(E)-4-(6-(2-(3-methylbenzylidene)hydrazinyl)-9-(3-methylisothiazol-5-yl)-9H-purin-2-yl)morpholine;
(E)-4-(9-(5-fluoropyridin-2-yl)-6-(2-(3-methylbenzylidene)hydrazinyl)-9H-purin-2-yl)morpholine;
(E)-4-(9-(2-methoxypyridin-4-yl)-8-methyl-6-(2-(3-methylbenzylidene)hydrazinyl)-9H-purin-2-yl)morpholine;
(E)-4-(8-methyl-6-(2-(3-methylbenzylidene)hydrazinyl)-9-(2-methylpyridin-3-yl)-9H-purin-2-yl)morpholine;
(E)-4-(9-(2,4-dimethylthiazol-5-yl)-8-methyl-6-(2-(3-methylbenzylidene)hydrazinyl)-9H-purin-2-yl)morpholine;
(E)-4-(9-(1,5-dimethyl-1H-pyrazol-3-yl)-8-methyl-6-(2-(3-methylbenzylidene)hydrazinyl)-9H-purin-2-yl)morpholine;
4-(8-methyl-9-phenyl-6-(3-(m-tolyl)-1H-pyrazol-1-yl)-9H-purin-2-yl)morpholine;
(E)-4-(6-(2-(3-methylbenzylidene)hydrazinyl)-9-(pyrimidin-2-yl)-9H-purin-2-yl)-morpholine;
(E)-2-(8-methyl-6-(2-(3-methylbenzylidene)hydrazinyl)-2-morpholino-9H-purin-9-yl)-1-phenylethan-1-one
(E)-2-(8-methyl-6-(2-(3-methylbenzylidene)hydrazinyl)-2-morpholino-9H-purin-9-yl)-1-(pyridin-2-yl)ethan-1-one;
(E)-2-(8-methyl-6-(2-(3-methylbenzylidene)hydrazinyl)-2-morpholino-9H-purin-9-yl)-1-(pyridin-3-yl)ethan-1-one;
(E)-2-(8-methyl-6-(2-(3-methylbenzylidene)hydrazinyl)-2-morpholino-9H-purin-9-yl)-1-(pyridin-4-yl)ethan-1-one;
(E)-4-(9-(3-methoxyphenyl)-8-methyl-6-(2-(3-methylbenzylidene)hydrazinyl)-9H-purin-2-yl)morpholine;
(E)-4-(9-(4-methoxyphenyl)-8-methyl-6-(2-(3-methylbenzylidene)hydrazinyl)-9H-purin-2-yl)morpholine;
(E)-4-(9-(3-fluorophenyl)-8-methyl-6-(2-(3-methylbenzylidene)hydrazinyl)-9H-purin-2-yl)morpholine;
(E)-4-(9-(3-chlorophenyl)-8-methyl-6-(2-(3-methylbenzylidene)hydrazinyl)-9H-purin-2-yl)morpholine;
(E)-4-(8-methyl-6-(2-(3-methylbenzylidene)hydrazinyl)-9-(pyridin-2-yl)-9H-purin-2-yl)morpholine;
(E)-4-(8-methyl-6-(2-(3-methylbenzylidene)hydrazinyl)-9-phenyl-9H-purin-2-yl)morpholine;
(E)-9-((3-methylbenzylidene)amino)-2-morpholino-N-(pyridin-4-yl)-9H-purin-6-amine;
(E)-9-((2-methylbenzylidene)amino)-2-morpholino-N-(pyridin-4-yl)-9H-purin-6-amine;
(E)-9-((4-methylbenzylidene)amino)-2-morpholino-N-(pyridin-4-yl)-9H-purin-6-amine;
(E)-9-(benzylideneamino)-2-morpholino-N-(pyridin-4-yl)-9H-purin-6-amine;
(E)-2-morpholino-N-(pyridin-4-yl)-9-((3-(trifluoromethyl)benzylidene)amino)-9H-purin-6-amine;
(E)-9-((3-chlorobenzylidene)amino)-2-morpholino-N-(pyridin-4-yl)-9H-purin-6-amine;
(E)-9-((3-methoxybenzylidene)amino)-2-morpholino-N-(pyridin-4-yl)-9H-purin-6-amine;
(E)-9-((4-methoxybenzylidene)amino)-2-morpholino-N-(pyridin-4-yl)-9H-purin-6-amine;
(E)-2-morpholino-N-(pyridin-4-yl)-9-((pyridin-4-ylmethylene)amino)-9H-purin-6-amine;
(E)-9-((4-chlorobenzylidene)amino)-2-morpholino-N-(pyridin-4-yl)-9H-purin-6-amine;
(E)-N-(6-(1H-imidazol-1-yl)-2-morpholino-9H-purin-9-yl)-1-(m-tolyl)methanimine;
(E)-N-(2-morpholino-6-(1H-pyrazol-1-yl)-9H-purin-9-yl)-1-(m-tolyl)methanimine;
(E)-N-(4-methoxyphenyl)-9-((3-methylbenzylidene)amino)-2-morpholino-9H-purin-6-amine;
(E)-N-methyl-9-((3-methylbenzylidene)amino)-2-morpholino-9H-purin-6-amine;
(E)-9-((3-methylbenzylidene)amino)-2-morpholino-N-phenyl-9H-purin-6-amine;
(E)-9-((3-methylbenzylidene)amino)-2-morpholino-N-(p-tolyl)-9H-purin-6-amine;
(E)-9-((3-methylbenzylidene)amino)-N-(1-methylpiperidin-4-yl)-2-morpholino-9H-purin-6-amine;
(E)-N-(2-morpholino-6-(2-(pyridin-2-yl)ethoxy)-9H-purin-9-yl)-1-(m-tolyl)methanimine;
(E)-N-(3-methoxyphenyl)-9-((3-methylbenzylidene)amino)-2-morpholino-9H-purin-6-amine;
(E)-N-(3-chlorophenyl)-9-((3-methylbenzylidene)amino)-2-morpholino-9H-purin-6-amine;
(E)-N-(4-chlorophenyl)-9-((3-methylbenzylidene)amino)-2-morpholino-9H-purin-6-amine;
(E)-N-(2-methoxyphenyl)-9-((3-methylbenzylidene)amino)-2-morpholino-9H-purin-6-amine;
(E)-N-(1,5-dimethyl-1H-pyrazol-3-yl)-9-((3-methylbenzylidene)amino)-2-morpholino-9H-purin-6-amine;
(E)-N-(2-morpholino-6-(pyridin-2-yloxy)-9H-purin-9-yl)-1-(m-tolyl)methanimine;
(E)-2-morpholino-9-((pyridin-3-ylmethylene)amino)-N-(m-tolyl)-9H-purin-6-amine;
(E)-9-((3-methylbenzylidene)amino)-2-morpholino-N-(pyridin-3-yl)-9H-purin-6-amine;
(E)-9-((3-methylbenzylidene)amino)-N-(5-methylpyridin-3-yl)-2-morpholino-9H-purin-6-amine;
(E)-N-(2-chloropyridin-4-yl)-9-((3-methylbenzylidene)amino)-2-morpholino-9H-purin-6-amine;
(E)-9-((3-methylbenzylidene)amino)-N-(2-methylpyridin-3-yl)-2-morpholino-9H-purin-6-amine;
(E)-9-((3-methylbenzylidene)amino)-2-morpholino-N-(pyridin-4-ylmethyl)-9H-purin-6-amine;
(E)-N-isopropyl-9-((3-methylbenzylidene)amino)-2-morpholino-9H-purin-6-amine;
(E)-N-(2-morpholino-6-(pyridin-4-yloxy)-9H-purin-9-yl)-1-(m-tolyl)methanimine;
(E)-3-methyl-N-(9-((3-methylbenzylidene)amino)-2-morpholino-9H-purin-6-yl)isoxazol-5-amine;
(E)-9-((3-methylbenzylidene)amino)-2-morpholino-N-(pyridin-2-yl)-9H-purin-amine;
(E)-2-morpholino-9-((pyridin-3-ylmethylene)amino)-N-(pyridin-4-yl)-9H-purin-amine;
(E)-N-(9-((3-methylbenzylidene)amino)-2-morpholino-9H-purin-6-yl)oxazol-5-amine;
(E)-N,N-dimethyl-3-(6-(2-(3-methylbenzylidene)hydrazinyl)-2-morpholino-9H-purin-9-yl)aniline;
N⁹-(3-methylbenzyl)-2-morpholino-N⁶-(pyridin-4-yl)-9H-purin-6,9-diamine;
4-(9-phenyl-6-(pyridin-4-yloxy)-9H-purin-2-yl)morpholine;
(E)-N-(4-methoxyphenyl)-8-methyl-9-((3-methylbenzylidene)amino)-2-morpholino-9H-purin-6-amine;
(E)-8-methyl-9-((3-methylbenzylidene)amino)-2-morpholino-N-phenyl-9H-purin-6-amine;
(E)-8-methyl-9-((3-methylbenzylidene)amino)-2-morpholino-N-(pyridin-4-yl)-9H-purin-6-amine;
(E)-N-8-methyl-2-morpholino-(6-(2-pyridin-2-yl)ethoxy)-9H-purin-9-yl-1-(m-tolyl)methanimine;
(E)-4-(8-(1-methyl-1H-pyrazol-4-yl)-4-(2-(3-methylbenzylidene)hydrazinyl)quinazolin-2-yl)morpholine;
(E)-4-(8-(3,5-dimethylisoxazol-4-yl)-4-(2-(3-methylbenzylidene)hydrazinyl)quinazolin-2-yl)morpholine;
(E)-4-(8-(3-methoxyphenyl)-4-(2-(3-methylbenzylidene)hydrazinyl)quinazolin-2-yl)morpholine;
(E)-4-(4-(2-(3-methylbenzylidene)hydrazinyl)-8-(pyridin-4-yl)quinazolin-2-yl)morpholine;
(E)-4-(8-(4-methoxyphenyl)-4-(2-(3-methylbenzylidene)hydrazinyl)quinazolin-2-yl)morpholine;
(E)-4-(4-(2-(3-methylbenzylidene)hydrazinyl)-8-phenylquinazolin-2-yl)morpholine;
(E)-4-(4-(2-(3-methylbenzylidene)hydrazinyl)-8-(pyridin-3-yl)quinazolin-2-yl)morpholine;
(E)-4-(4-(2-(3-methylbenzylidene)hydrazinyl)-7-(pyridin-3-yl)pyrido[2,3-d]pyrimidin-2-yl)morpholine; and
(E)-4-(4-(2-(3-methylbenzylidene)hydrazinyl)-7-phenylpyrido[2,3-d]pyrimidin-2-yl)morpholine.

According to a specific example of the present disclosure, the compound represented by Chemical Formula 1 may be any one selected from the group consisting of compounds in Table 1 below:

**Table 1**

| **Example** | **Structure** | **Example** | **Structure** |
|---|---|---|---|
| 1 | 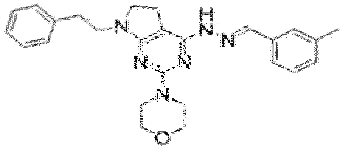 | 2 | 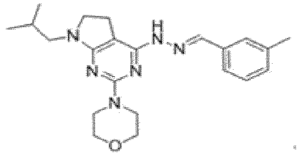 |
| 3 | 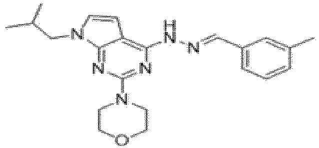 | 4 | 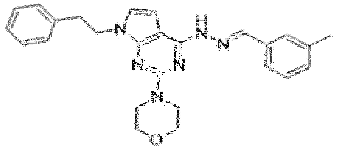 |
| 5 | 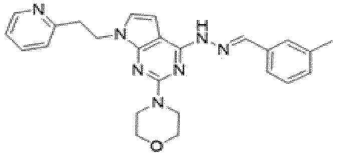 | 6 | 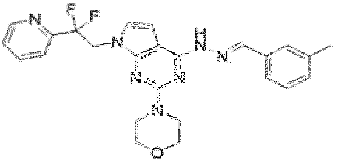 |
| 7 | 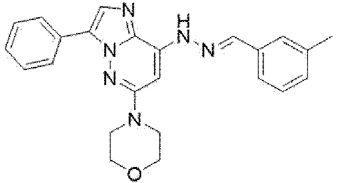 | 8 | 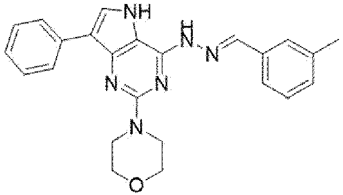 |
| 9 | 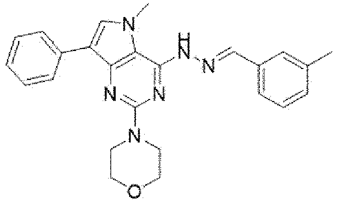 | 10 | 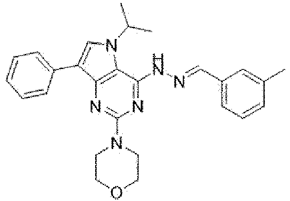 |
| 11 | 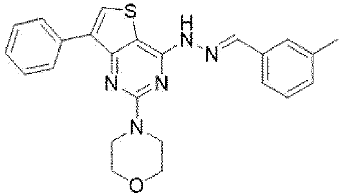 | 12 | 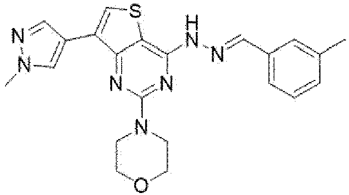 |
| 13 | 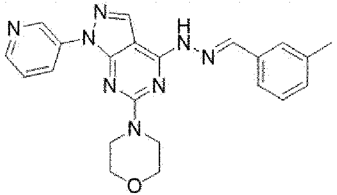 | 14 | 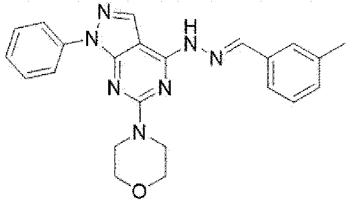 |
| 15 | 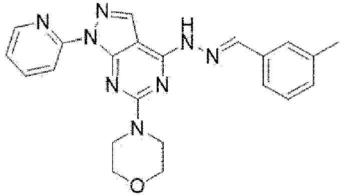 | 16 | 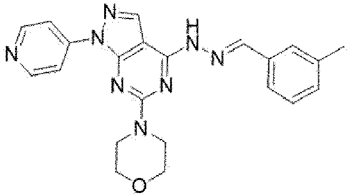 |
| 17 | 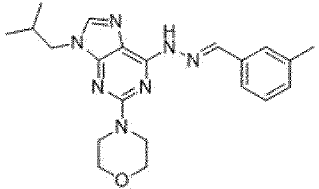 | 18 | 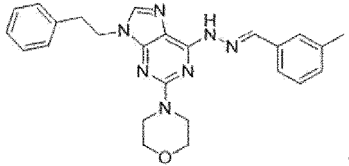 |
| 19 | 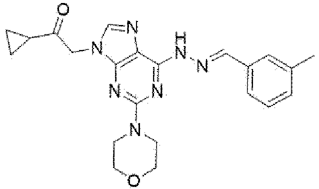 | 20 | 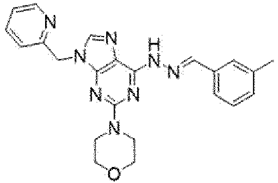 |
| 21 | 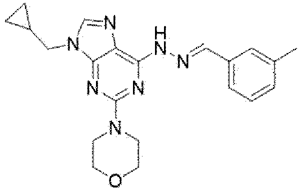 | 22 | 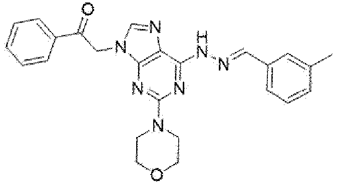 |
| 23 | 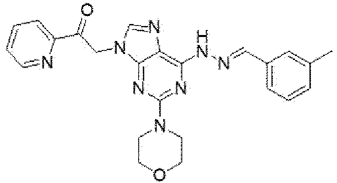 | 24 | 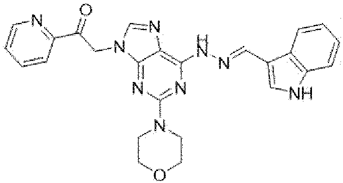 |
| 25 | 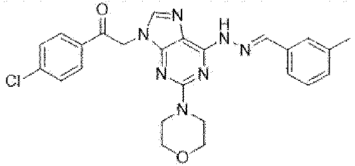 | 26 | 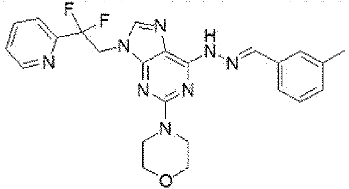 |
| 27 | 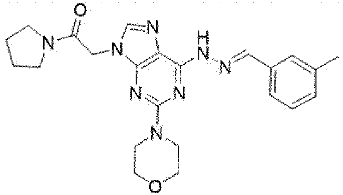 | 28 | 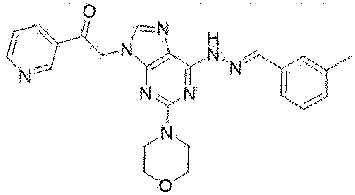 |
| 29 | 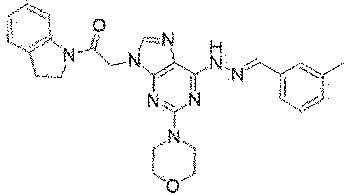 | 30 | 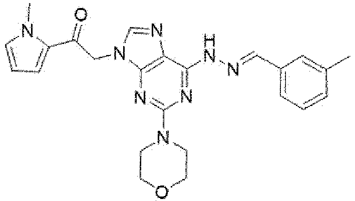 |
| 31 | 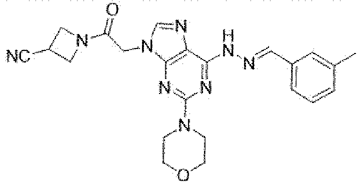 | 32 | 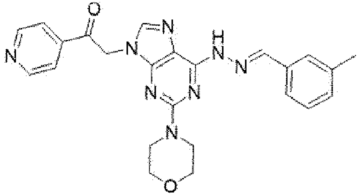 |
| 33 | 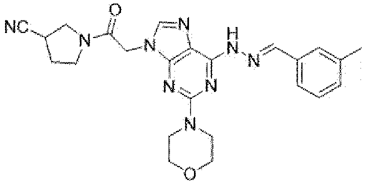 | 34 | 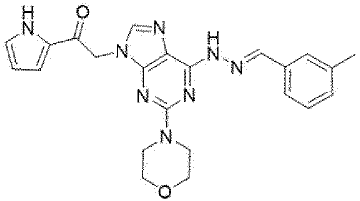 |
| 35 | 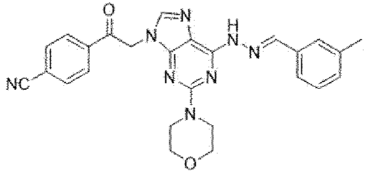 | 36 | 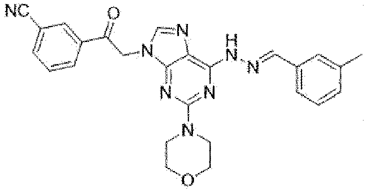 |
| 37 | 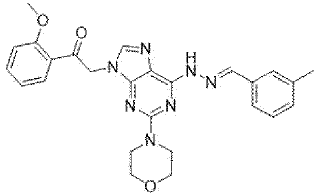 | 38 | 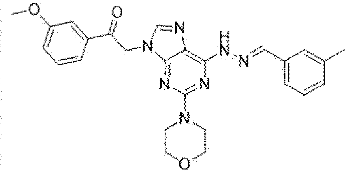 |
| 39 | 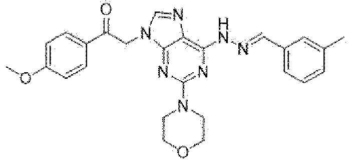 | 40 | 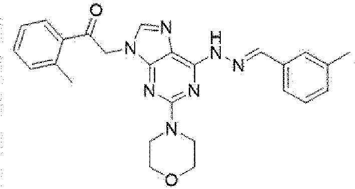 |
| 41 | 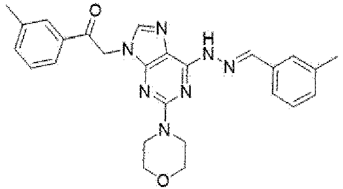 | 42 | 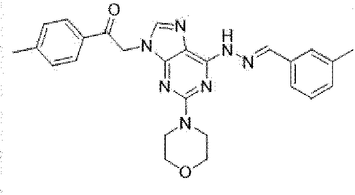 |
| 43 | 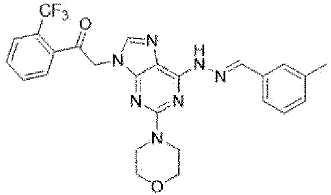 | 44 | 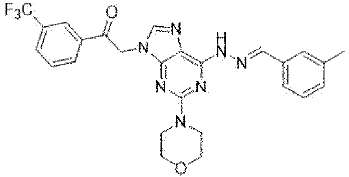 |
| 45 | 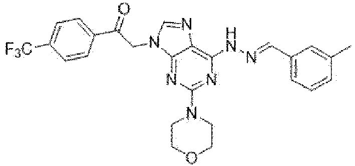 | 46 | 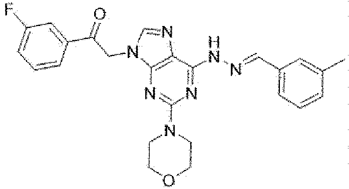 |
| 47 | 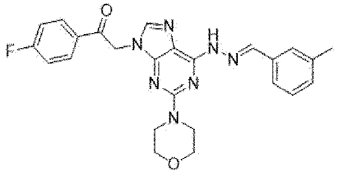 | 48 | 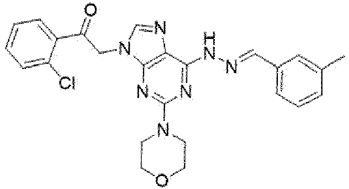 |
| 49 | 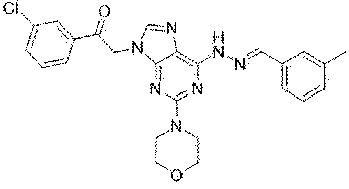 | 50 | 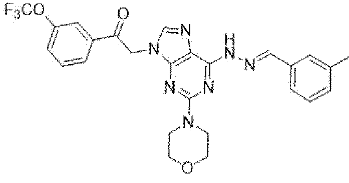 |
| 51 | 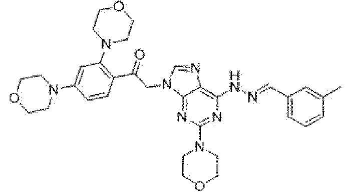 | 52 | 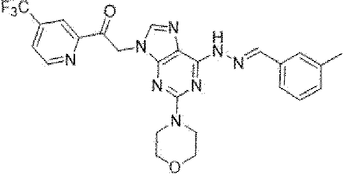 |
| 53 | 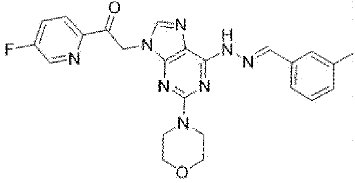 | 54 | 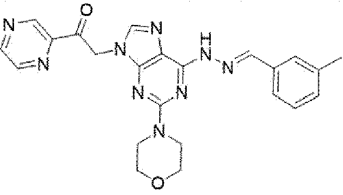 |
| 55 | 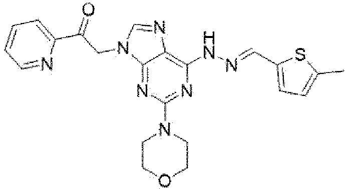 | 56 | 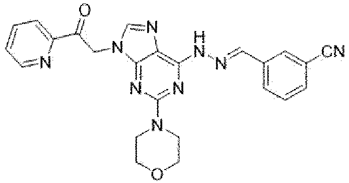 |
| 57 | 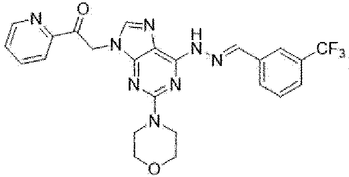 | 58 | 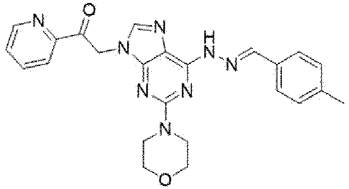 |
| 59 | 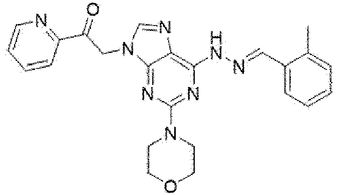 | 60 | 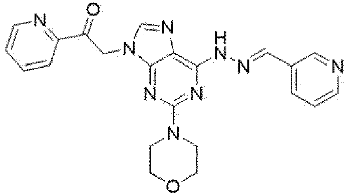 |
| 61 | 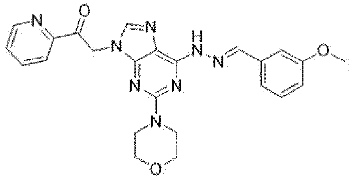 | 62 | 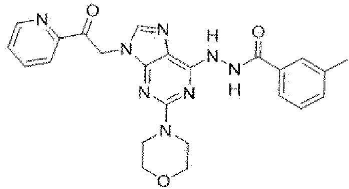 |
| 63 | 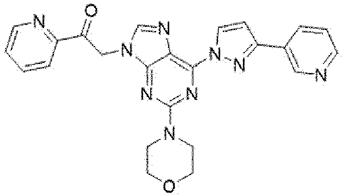 | 64 | 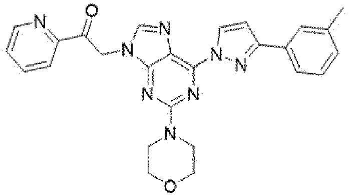 |
| 65 | 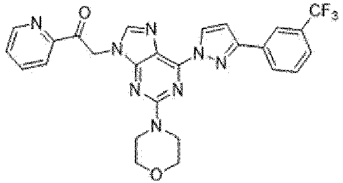 | 66 | 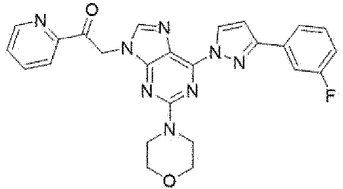 |
| 67 | 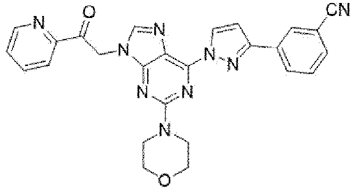 | 68 | 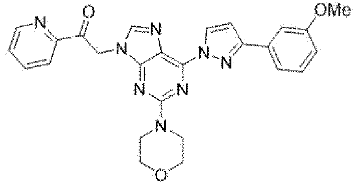 |
| 69 | 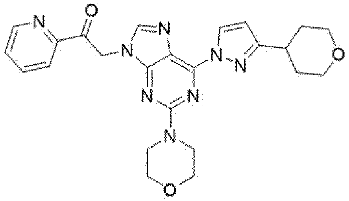 | 70 | 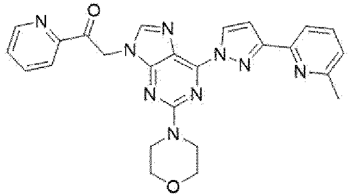 |
| 71 | 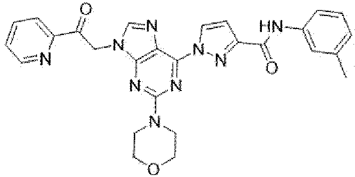 | 72 | 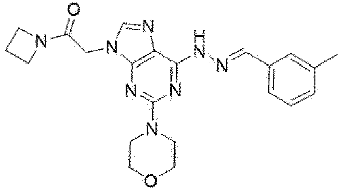 |
| 73 | 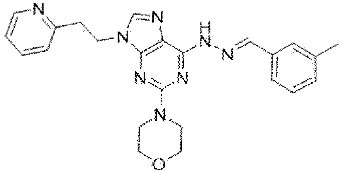 | 74 | 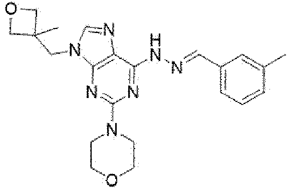 |
| 75 | 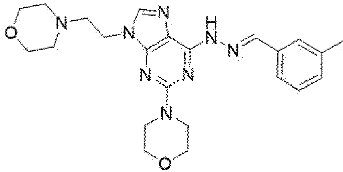 | 76 | 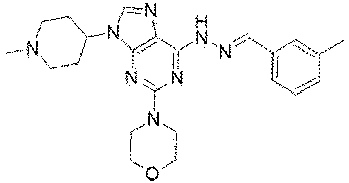 |
| 77 | 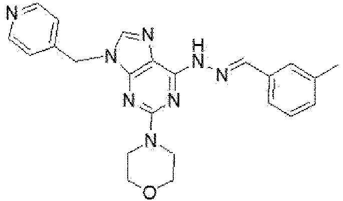 | 78 | 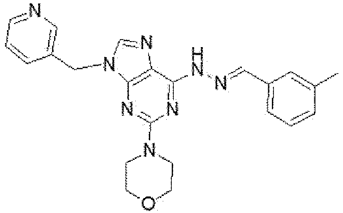 |
| 79 | 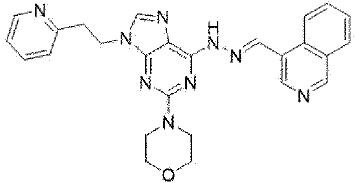 | 80 | 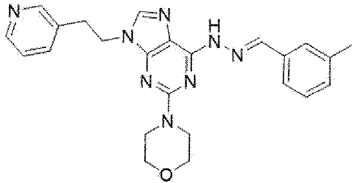 |
| 81 | 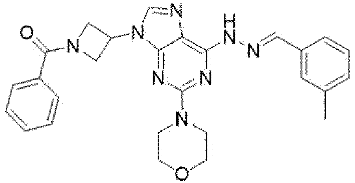 | 82 | 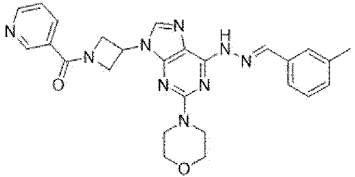 |
| 83 | 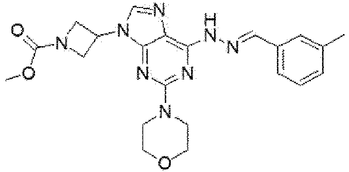 | 84 | 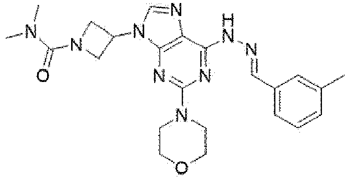 |
| 85 | 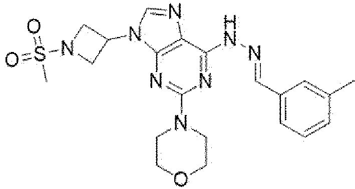 | 86 | 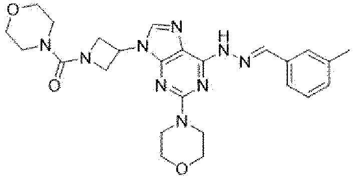 |
| 87 | 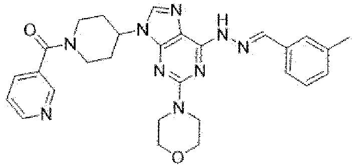 | 88 | 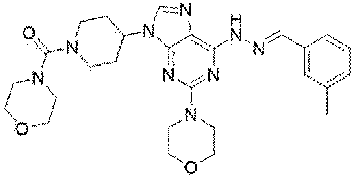 |
| 89 | 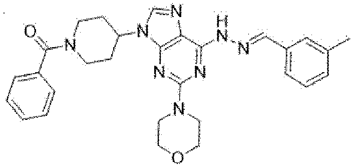 | 90 | 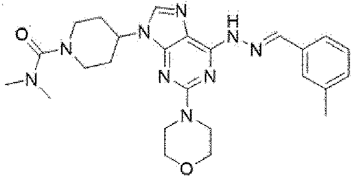 |
| 91 | 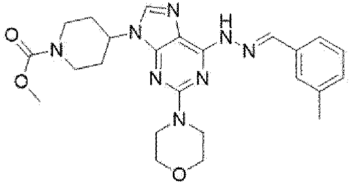 | 92 | 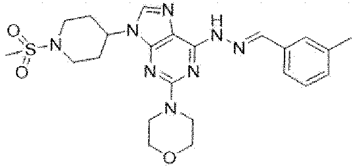 |
| 93 | 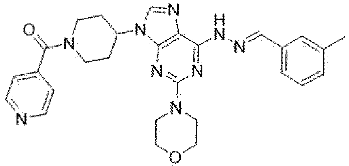 | 94 | 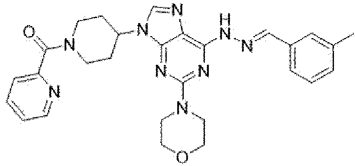 |
| 95 | 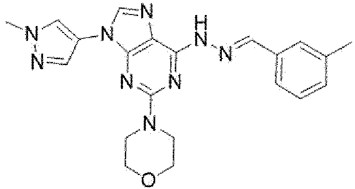 | 96 | 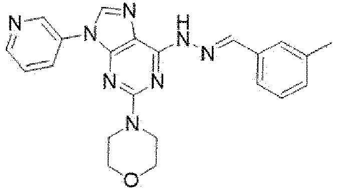 |
| 97 | 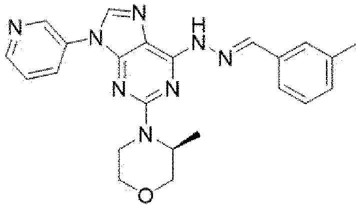 | 98 | 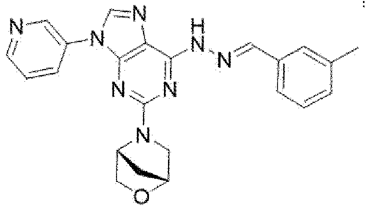 |
| 99 | 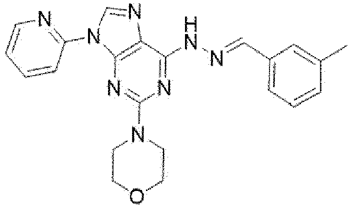 | 100 | 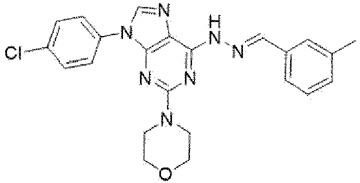 |
| 101 | 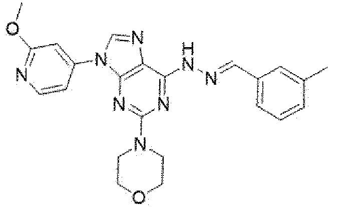 | 102 | 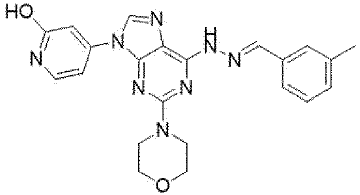 |
| 103 | 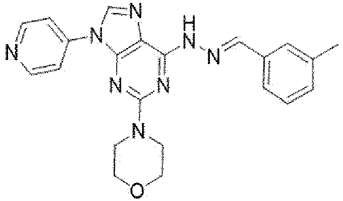 | 104 | 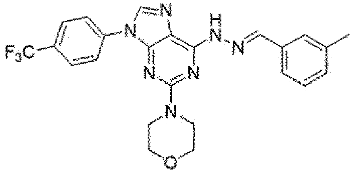 |
| 105 | 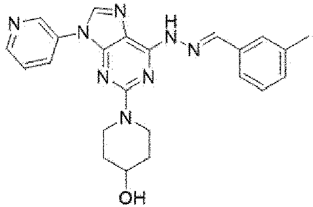 | 106 | 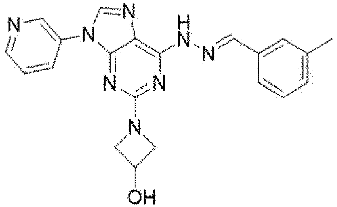 |
| 107 | 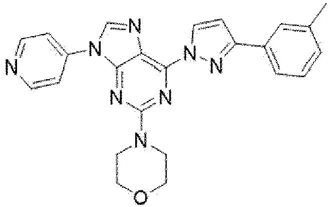 | 108 | 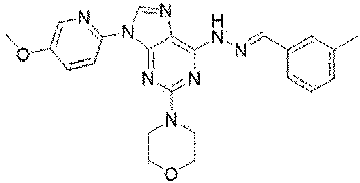 |
| 109 | 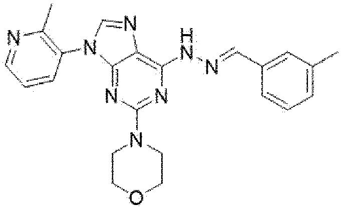 | 110 | 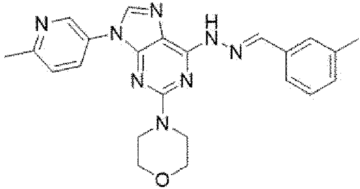 |
| 111 | 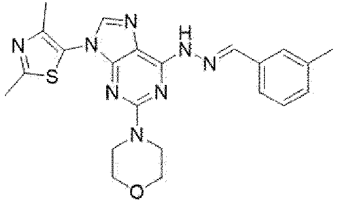 | 112 | 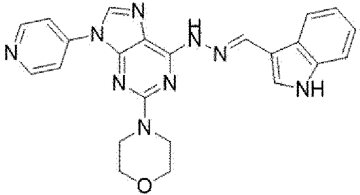 |
| 113 | 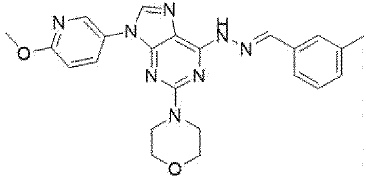 | 114 | 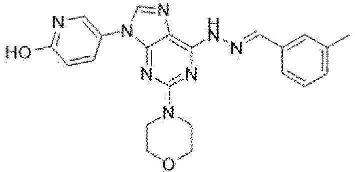 |
| 115 | 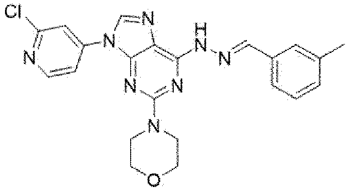 | 116 | 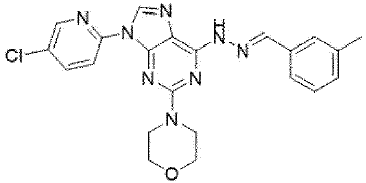 |
| 117 | 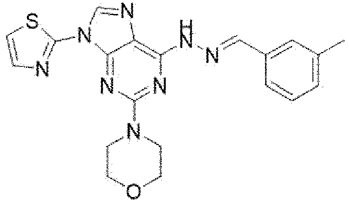 | 118 | 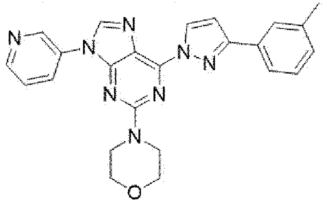 |
| 119 | 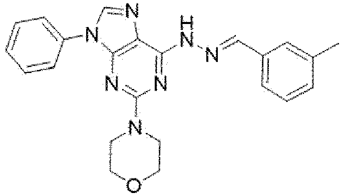 | 120 | 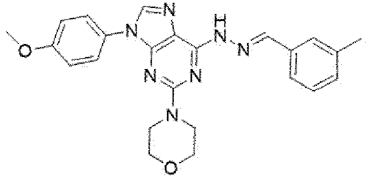 |
| 121 | 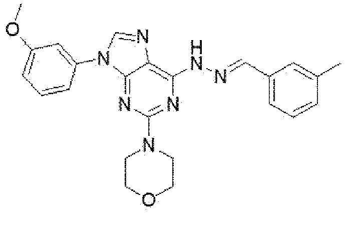 | 122 | 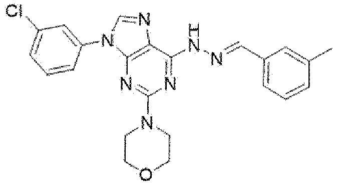 |
| 123 | 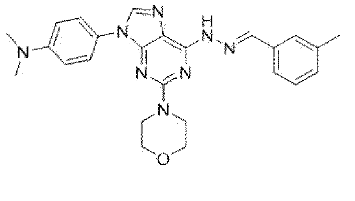 | 124 | 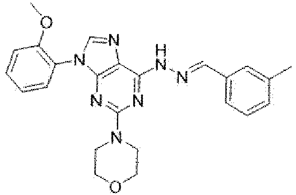 |
| 125 | 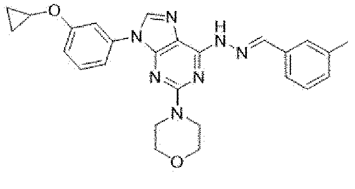 | 126 | 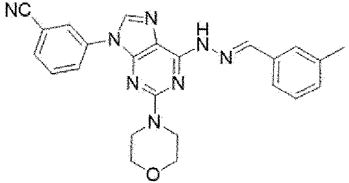 |
| 127 | 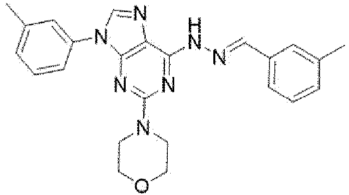 | 128 | 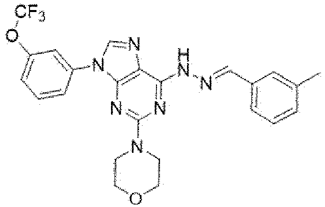 |
| 129 | 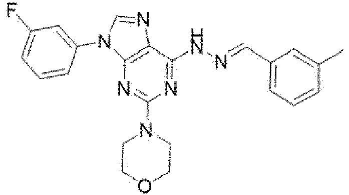 | 130 | 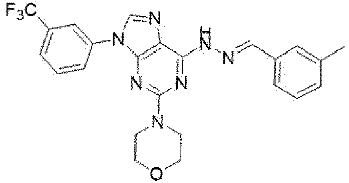 |
| 131 | 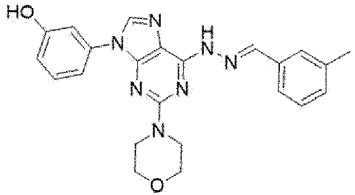 | 132 | 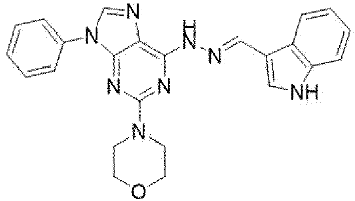 |
| 133 | 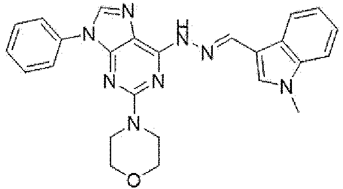 | 134 | 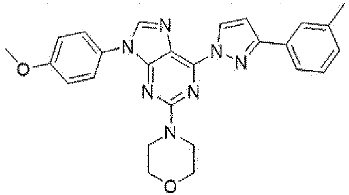 |
| 135 | 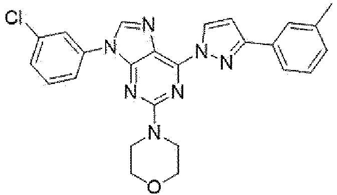 | 136 | 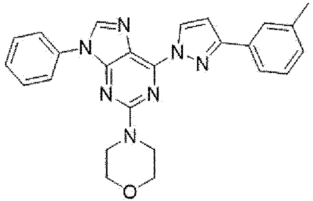 |
| 137 | 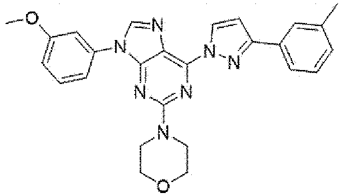 | 138 | 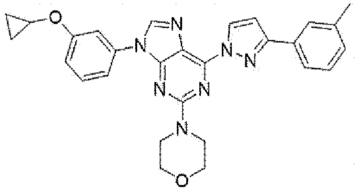 |
| 139 | 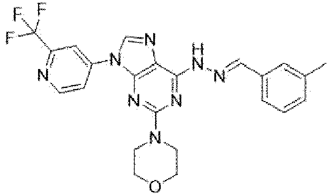 | 140 | 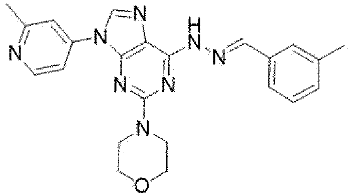 |
| 141 | 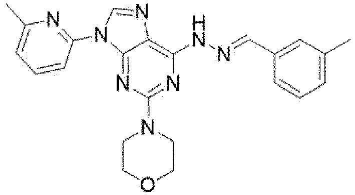 | 142 | 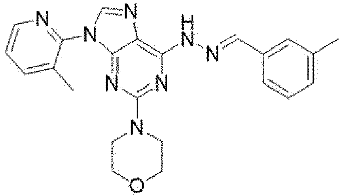 |
| 143 | 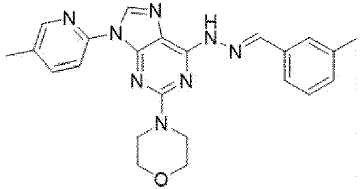 | 144 | 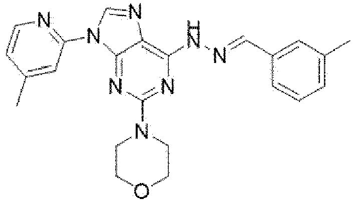 |
| 145 | 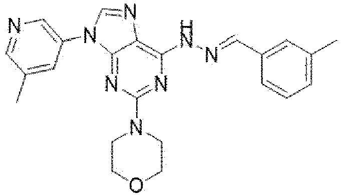 | 146 | 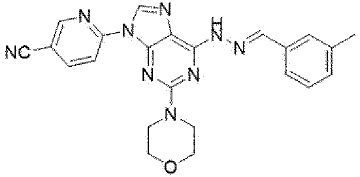 |
| 147 | 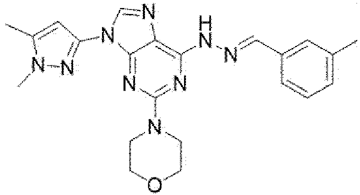 | 148 | 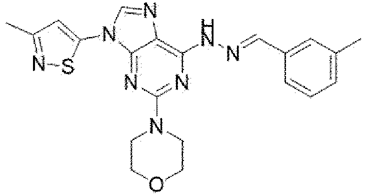 |
| 149 | 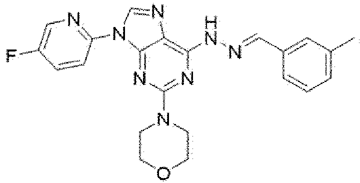 | 150 | 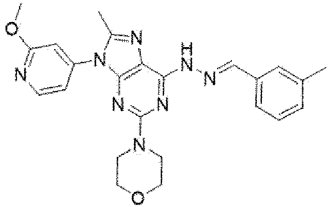 |
| 151 | 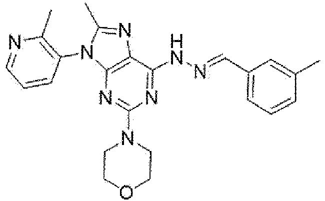 | 152 | 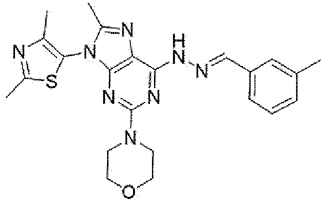 |
| 153 | 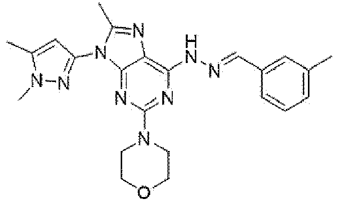 | 154 | 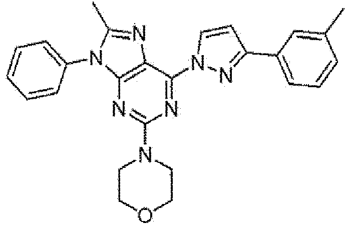 |
| 155 | 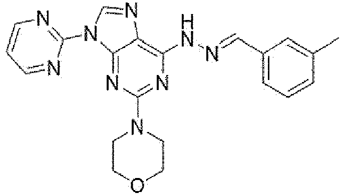 | 156 | 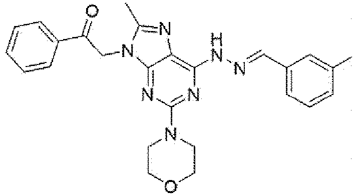 |
| 157 | 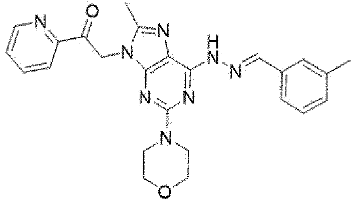 | 158 | 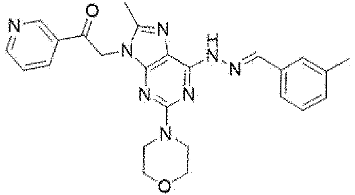 |
| 159 | 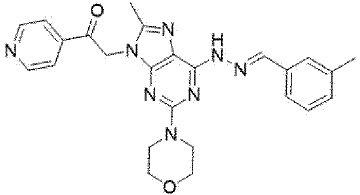 | 160 | 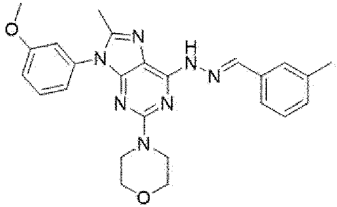 |
| 161 | 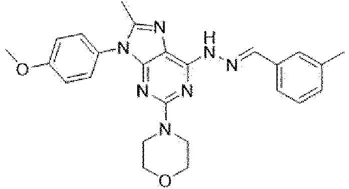 | 162 | 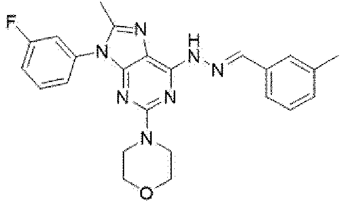 |
| 163 | 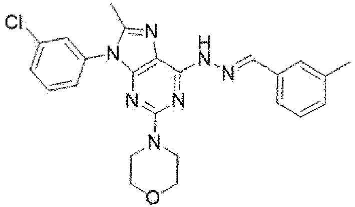 | 164 | 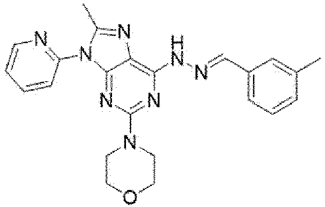 |
| 165 | 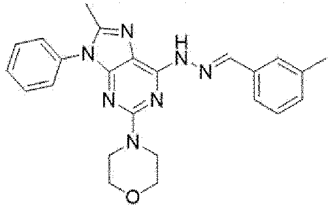 | 166 | 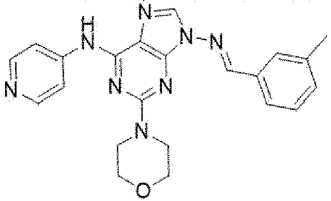 |
| 167 | 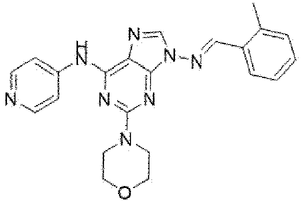 | 168 | 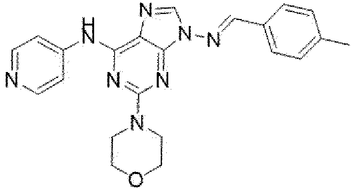 |
| 169 | 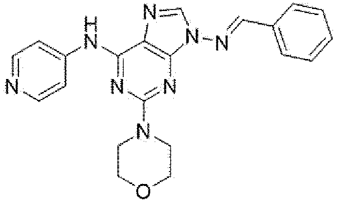 | 170 | 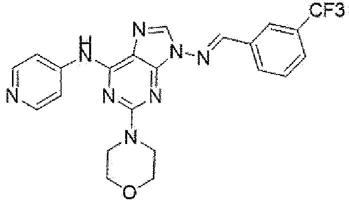 |
| 171 | 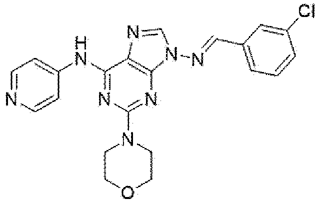 | 172 | 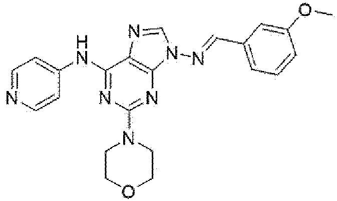 |
| 173 | 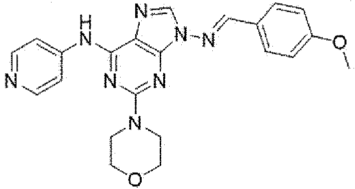 | 174 | 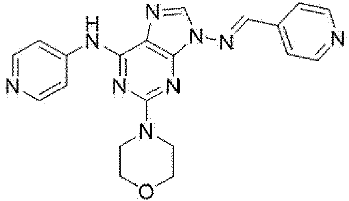 |
| 175 | 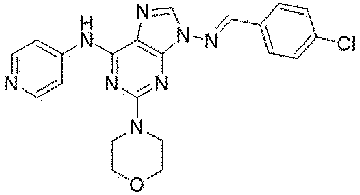 | 176 | 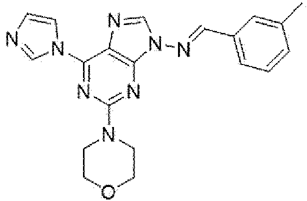 |
| 177 | 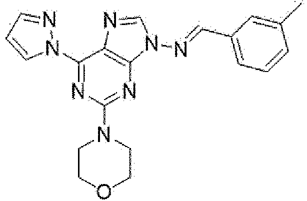 | 178 | 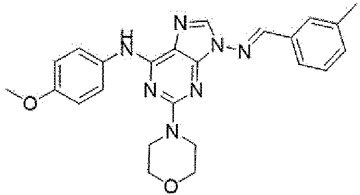 |
| 179 | 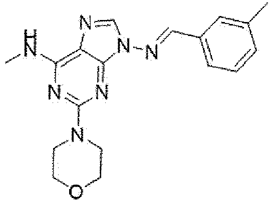 | 180 | 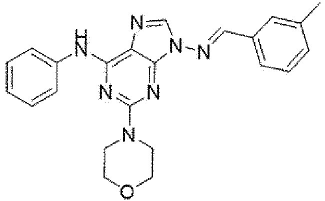 |
| 181 | 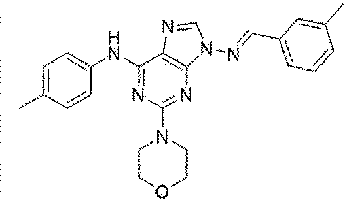 | 182 | 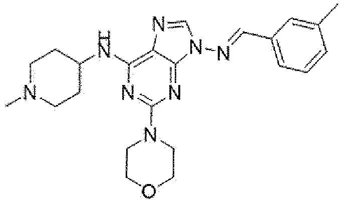 |
| 183 | 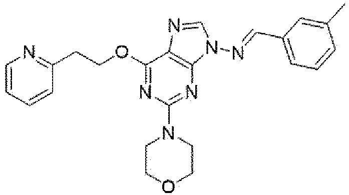 | 184 | 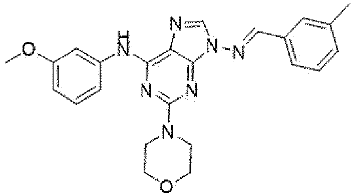 |
| 185 | 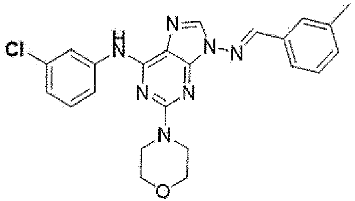 | 186 | 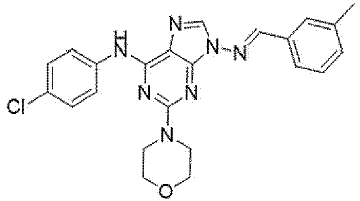 |
| 187 | 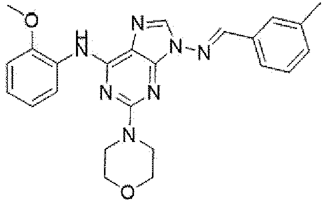 | 188 | 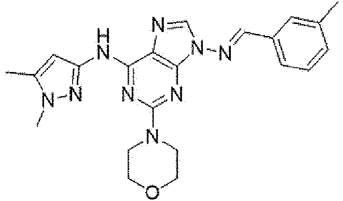 |
| 189 | 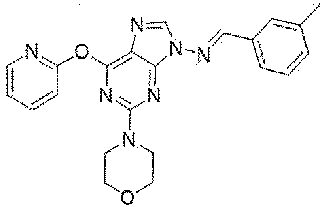 | 190 | 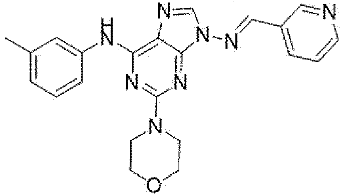 |
| 191 | 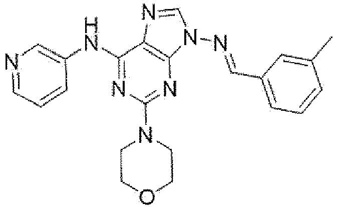 | 192 | 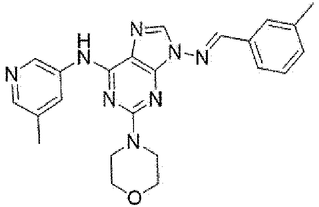 |
| 193 | 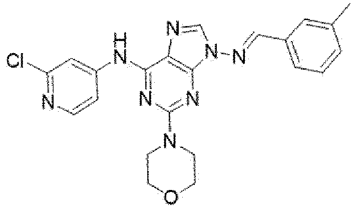 | 194 | 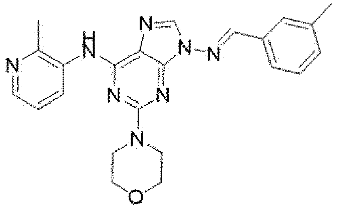 |
| 195 | 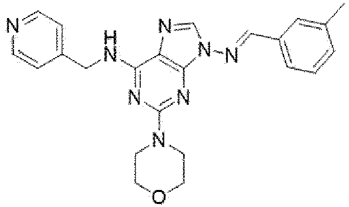 | 196 | 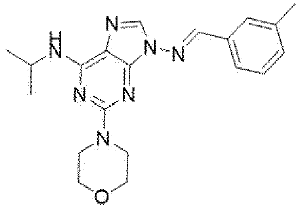 |
| 197 | 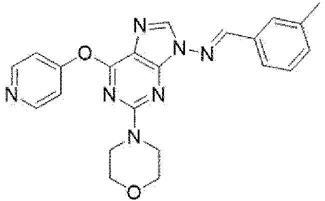 | 198 | 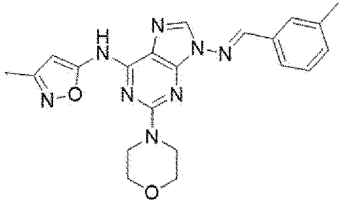 |
| 199 | 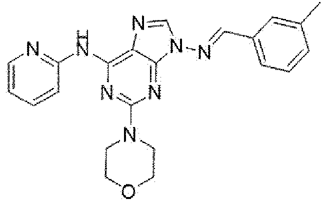 | 200 | 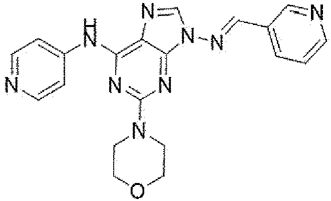 |
| 201 | 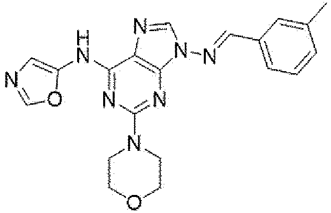 | 202 | 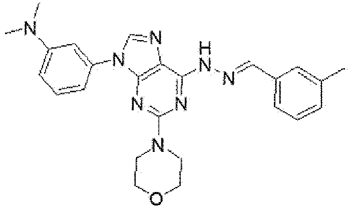 |
| 203 | 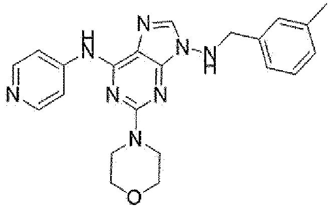 | 204 | 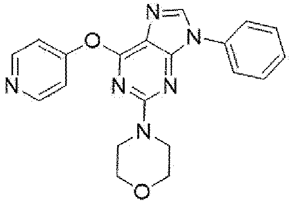 |
| 205 | 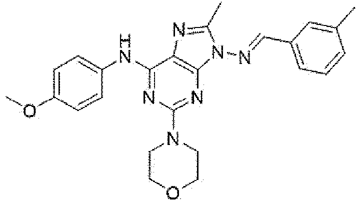 | 206 | 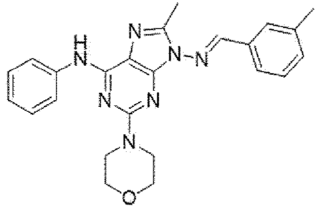 |
| 207 | 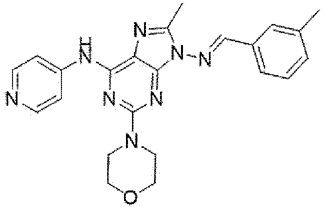 | 208 | 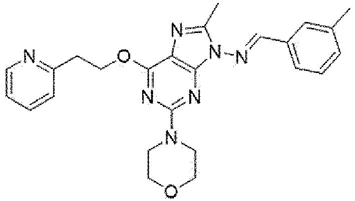 |
| 209 | 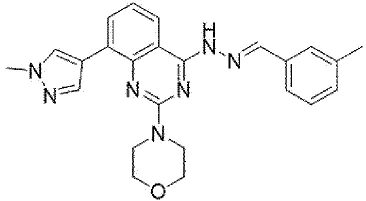 | 210 | 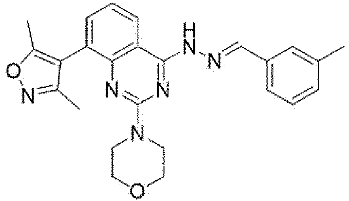 |
| 211 | 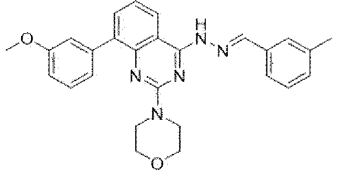 | 212 | 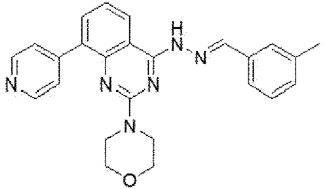 |
| 213 | 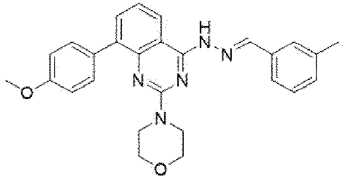 | 214 | 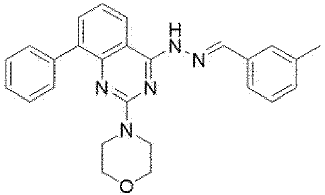 |
| 215 | 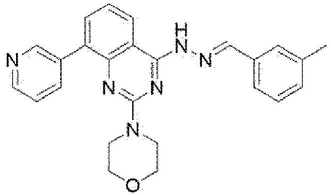 | 216 | 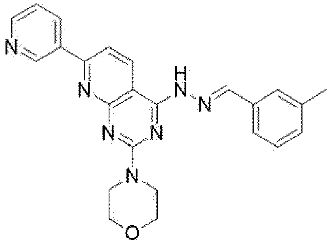 |
| 217 | 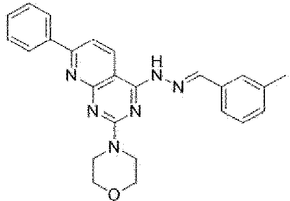 | | |

In the present disclosure, the pharmaceutically acceptable salt means a salt commonly used in the pharmaceutical industry, and may include, for example, inorganic ion salts prepared from calcium, potassium, sodium, magnesium, and the like, inorganic acid salts prepared from hydrochloric acid, nitric acid, phosphoric acid, bromic acid, iodic acid, perchloric acid, and sulfuric acid, and the like; organic acid salts prepared from acetic acid, trifluoroacetic acid, citric acid, maleic acid, succinic acid, oxalic acid, benzoic acid, tartaric acid, fumaric acid, manderic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like; sulfonic acid salts prepared from methanesulfonic acid, ethanesulfonic acid, benzenesulfonic acid, p-toluenesulfonic acid, naphthalenesulfonic acid, and the like; amino acid salts prepared from glycine, arginine, lysine, and the like; and amine salts prepared with trimethylamine, triethylamine, ammonia, pyridine, picoline, and the like, but the types of salts referred to in the present disclosure are not limited by these listed salts.

### Pharmaceutical composition

In still another general aspect, there is provided a pharmaceutical composition comprising: any one compound of Chemical Formulas 1, 2-1 to 2-7, and 3-1 to 3-3 as defined in any embodiment described herein, an optical isomer thereof, or a pharmaceutically acceptable salt thereof.

In still another general aspect, there is provided a pharmaceutical composition comprising: any one compound of Chemical Formulas 1, 2-1 to 2-7, and 3-1 to 3-3 as defined in any embodiment described herein, an optical isomer thereof, or a pharmaceutically acceptable salt thereof; and a pharmaceutically acceptable carrier.

The pharmaceutical composition of the present disclosure may include a pharmaceutically acceptable carrier, and may be formulated in the form of oral formulations such as powders, granules, tablets, capsules, suspensions, emulsions, syrups, aerosols, and the like, external preparations, suppositories or sterile injection solutions according to conventional methods, respectively.

Examples of the pharmaceutically acceptable carrier may include, but are not limited to, those commonly used, such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, etc. In addition, the pharmaceutical composition of the present disclosure may include, but is not limited to, diluents or excipients such as fillers, enhancers, binders, wetting agents, disintegrants, surfactants, and other pharmaceutically acceptable additives.

In still another general aspect, there is provided a pharmaceutical composition for preventing or treating cancer, comprising: any one compound selected from Chemical Formulas 1, 2-1 to 2-7, and 3-1 to 3-3 as defined in any embodiment described herein, an optical isomer thereof, or a pharmaceutically acceptable salt thereof.

The cancer is selected from the group consisting of, but is not limited to, hematological malignancies, solid tumors, sarcoma, angiosarcoma, fibrosarcoma, rhabdomyosarcoma, liposarcoma, myxoma, rhabdomyosarcoma, fibroma, lipoma, teratoma, lung cancer, non-small cell lung cancer, breast cancer, bronchial carcinoma squamous cell, undifferentiated small cell, undifferentiated large cell, adenocarcinoma, alveolar bronchial carcinoma, bronchial adenoma, lymphoma, cartilaginous hamartoma, mesothelioma, gastric cancer, esophageal cancer, squamous cell carcinoma, , leiomyosarcoma, stomach cancer, colon cancer, pancreatic cancer, lymphoma, ductal adenocarcinoma, insulinoma, glucagonoma, gastrinoma, carcinoid tumor, vipoma, small intestinal cancer, Kaposi sarcoma, leiomyoma, hemangioma, neurofibroma, tubular adenoma, villous adenoma, hamartoma, genitourinary cancer, kidney cancer, Wilms' tumor (nephroblastoma), brain tumor, leukemia, bladder cancer, urethral cancer, gallbladder cancer, transitional cell carcinoma, prostate cancer, testicular cancer, seminoma, embryonal carcinoma, teratocarcinoma, choriocarcinoma, stromal cell carcinoma, fibroadenoma, adenomatoid tumor, liver cancer, hepatoma, hepatocellular carcinoma, cholangiocarcinoma, hepatoblastoma, hepatocellular adenoma, bone cancer, osteogenic sarcoma (osteosarcoma), malignant fibrous histiocytosis, chondrosarcoma, Ewing's sarcoma, malignant lymphoma (reticular cell sarcoma), multiple myeloma, malignant giant cell tumor, chordoma, osteocartilaginous exostoses, benign chondrosis, chondroblastoma, chondromyxofibroma, osteoid giant cell tumor, nervous system cancer, skull cancer, osteoma, granuloma, xanthoma, osteitis deformans, meningeal cancer, meningiomas, meningiosarcomas, gliomatosis, brain cancer, astrocytoma, medulloblastoma, glioma, ependymoma, germinoma (pinealoma), glioblastoma multiforme, oligodendroglioma, schwannoma, retinoblastoma, congenital tumors, spinal cord cancer, meningioma, gynecological cancers, uterine cancer, endometrial carcinoma, cervical cancer, cervical carcinoma, pre-tumor cervical dysplasia, ovarian cancer, ovarian carcinoma, serous cystadenocarcinoma, mucinous cystadenocarcinoma, colorectal cancer, rectal cancer, unclassified carcinoma, granulosa-theca cell tumor, Sertoli Leydig cell tumor, dysgerminoma, malignant teratoma, vulvar cancer, intraepithelial carcinoma, melanoma, vaginal cancer, clear cell carcinoma, staphylococcal sarcoma, embryonal rhabdomyosarcoma, fallopian tube cancer, hematologic cancer, lymphoblastic leukemia, acute myeloid leukemia (AML), chronic myeloid leukemia (CML), acute lymphoblastic leukemia (ALL), chronic lymphoblastic leukemia, chronic lymphocytic leukemia, myeloproliferative disorders, myelodysplastic syndrome (MDS), solitary myeloma, Hodgkin's lymphoma, non-Hodgkin's lymphoma (malignant lymphoma), Waldenström macroglobulinemia, skin cancer, head and neck cancer, malignant melanoma, basal cell carcinoma, colorectal cancer, moles dysplastic nevi, colorectal carcinoma, osteosarcoma, dermatofibroma, keloid, endocrine carcinoma and neuroblastoma. In some embodiments, the cancer comprises lymphoblastic leukemia, lymphoma, colorectal cancer, glioblastoma multiforme (GBM), medulloblastoma, colorectal carcinoma, osteosarcoma, pancreatic cancer, etc.

The cancer may include a metastatic cancer.

In another general aspect, there is provided a pharmaceutical composition for preventing or treating inflammatory diseases, comprising: any one compound of Chemical Formulas 1, 2-1 to 2-7, and 3-1 to 3-3 as defined in any embodiment described herein, an optical isomer thereof, or a pharmaceutically acceptable salt thereof.

The above inflammatory diseases include, but are not limited to, sepsis, acute sepsis, alopecia, hair loss syndrome, gout, arthritis, rheumatoid arthritis, sclerosis, inflammatory bowel disease, ankylosing spondylitis (AS), antiphospholipid antibody syndrome (APS), myositis, scleroderma, Sjogren's syndrome, systemic lupus erythematosus, vasculitis, familial Mediterranean fever, neonatal-onset multisystem inflammatory disease, Behcet's disease, dermatitis, type 1 diabetes, autoimmune diseases, psoriasis, psoriatic arthritis, dermatitis herpetiformis, pemphigus vulgaris, vitiligo, multiple sclerosis, systemic sclerosis, Addison's disease, Graves' disease, Hashimoto's disease, myasthenia gravis, Guillain-Barre syndrome, autoimmune uveitis, autoimmune hemolytic anemia, Wegener's granulomatosis, pernicious anemia, autoimmune thrombocytopenia, temporal arteritis, celiac disease, chronic inflammation, rheumatism, encephalomyelitis, postinfectious cerebellitis, neuromyelitis optica, Devic's disease, encephalitis, metabolic encephalopathy, asthma, periodontitis, ulcerative colitis, interstitial fibrosis of the lung, myelofibrosis, liver fibrosis, myocarditis, primary biliary cirrhosis, Crohn's disease, sinusitis, orchitis, polymyositis, dermatomyositis, autoimmune oophoritis, autoimmune adrenalitis, systemic lupus erythematosus, scleroderma, peptic ulcer, etc.

In still another general aspect, there is provided a pharmaceutical composition for preventing or treating lysosomal storage diseases (LSDs), comprising: any one compound of Chemical Formulas 1, 2-1 to 2-7, and 3-1 to 3-3 as defined in any embodiment described herein, an optical isomer thereof, or a pharmaceutically acceptable salt thereof.

The lysosomal storage diseases include, but are not limited to, α-mannosidosis, aspartylglucosaminuria, β-mannosidosis, cystinosis, α-N-acetylgalactosaminidase deficiency, Schindler disease, aspartoacylase or aminoacylase deficiency, Canavan disease, multiple sulfatase deficiency or MSD, steroid sulfatase deficiency, cholesteryl ester storage disease, Wolman disease, Fabry disease, Farber disease, Gaucher disease types I, II and III, Krabbe disease and its infantile-onset and late-onset variants, Niemann-Pick disease types A/B and C, fucosidosis, galactosialidosis, GM1 gangliosidosis of infantile, late infantile/juvenile and adult/chronic forms, activator deficiency, Sandhoff disease and its variants, GM2 gangliosidosis including Tay-Sachs disease, glycogen storage disease, glycogen storage disease type I or Von Gierke disease, glycogen storage disease type II or pompe disease, glycogen storage disease type IIb or Danon disease, glycogen storage disease type V or McArdle disease, glycogen storage disease type VII or Tarui disease, metachromatic leukodystrophy and all its variants, including those due to activator deficiency, neuronal ceroid lipofuscinosis (NCL) from NCL1 to NCL10 and all its variants, mucopolysaccharidosis type I, sialidosis and its variants, including the infantile and Salla disease forms and juvenile forms, mucopolysaccharidosis type II, I-cell disease, mucopolysaccharidosis type IIIA or α/β type, pseudo-Hurler polydystrophy, mucopolysaccharidosis type IIIC or γ type, mucopolysaccharidosis type IV, mucopolysaccharidosis type I, Hurler, Scheie and Hurler-Scheie syndromes, mucopolysaccharidosis type II, Hunter syndrome, mucopolysaccharidosis type III, Sanfilippo syndrome type A/MPS III A, Sanfilippo syndrome type B/MPS III B, Sanfilippo syndrome type C/MPS III C and Sanfilippo syndrome type D/MPS III D, mucopolysaccharidosis type IV, Morquio types A/MPS IVA and Morquio types B/MPS IVB, mucopolysaccharidosis type VI, Maroteaux-Lamy disease, mucopolysaccharidosis type VII, Sly syndrome, mucopolysaccharidosis type IX due to hyaluronidase deficiency or pycnodysostosis.

In still another general aspect, there is provided a pharmaceutical composition for preventing or treating neurological diseases, comprising: any one compound of Chemical Formulas 1, 2-1 to 2-7, and 3-1 to 3-3 as defined in any embodiment described herein, an optical isomer thereof, or a pharmaceutically acceptable salt thereof.

The neurological diseases include, but are not limited to, amyotrophic lateral sclerosis (ALS), primary lateral sclerosis (PLS), Charcot-Marie-Tooth (CMT; including type 4J (CMT4J)), Yunis-Varon syndrome, autophagy, congenital brain lesions, polymicrogyria, temporo-occipital polymicrogyria, Pick's disease, Parkinson's disease, Parkinson's disease with Lewy bodies, dementia with Lewy bodies, Lewy body disease, fronto-temporal dementia, diseases of neuronal nuclear inclusions of polyglutamine and intranuclear inclusion bodies, disease of Marinesco and Hiranobodies, Tauopathy Alzheimer's disease, neurodegeneration, spongiform neurodegeneration, peripheral neuropathy, leukoencephalopathy, motorneuropathy, sensoryneuropathy, inclusion body disease, progressive supranuclearpalsy, corticobasal syndrome, chronic traumatic encephalopathy, traumatic braininjury (TBI), cerebralischemia, Guillain-Barre Syndrome, chronic inflammatory demyelinating polyneuropathy, multiple sclerosis, Niemann-Pick C disease, Tay-Sachs disease, Mucolipidosis type IV, Mucolipidosis type V, neuropathy, Huntington's disease, apsychiatric disorder, attention-deficit/hyperactivity disorder (ADHD), schizophrenia, mood disorder, major depressive disorder, depression, bipolar disorder I, bipolar disorder II, frontotemporal dementia (FTD), orchronictraumatic encephalopathy, Fabry's disorder, Gaucher's disorder, and progressive muscle atrophy, etc.

As used herein, the term "prevention" means any act of inhibiting or delaying the onset of cancer, inflammation formation, lysosomal accumulation, or neurological disease by administration of the composition.

As used herein, the term "treatment" refers to all activities in which the symptoms of the disease are improved or beneficially changed by administration of the composition.

The composition of the present disclosure may be administered orally or parenterally (for example, intravenous, subcutaneous, intraperitoneal or topical application) according to the desired method, and the dosage varies depending on the patient's weight, age, sex, health condition, diet, administration time, administration method, excretion rate, and type and severity of the disease.

With respect to the administration route, the pharmaceutical composition of the present disclosure may be administered to mammals such as rats, mice, livestock, and humans through any conventional route as long as it can reach the target tissue, and for example, may be administered by intradermal injection, intravein injection, intraperitoneal injection, intravitreal injection, intrathecal, inner ear, abdominal cavity or vein, muscle, subcutaneous, intrauterine epidural, sublingual or intracerebrovascular injection, subcutaneous injection using an osmotic pump, but the present disclosure is not limited thereto.

The pharmaceutical composition of the present disclosure may comprise 0.001 to 95 wt%, preferably 0.01 to 80 wt%, of the compound represented by Chemical Formula 1, an optical isomer thereof, or a pharmaceutically acceptable salt thereof, based on the total weight of the composition.

When the pharmaceutical composition of the present disclosure is formulated as an oral solid preparation, it includes tablets, pills, powders, granules, capsules, etc., and these solid preparations may include at least one excipient, for example, starch, calcium carbonate, sucrose or lactose, gelatin, etc., and may include, but are not limited to, lubricants, such as magnesium stearate and talc.

When the pharmaceutical composition of the present disclosure is formulated as an oral liquid preparation, it includes suspensions, oral solutions, emulsions, syrups, etc., and may include, but is not limited to, diluents such as water or liquid paraffin, wetting agents, sweeteners, flavoring agents, preservatives, etc.

When the pharmaceutical composition of the present disclosure is formulated for parenteral use, it includes sterile aqueous solutions, non-aqueous solvents, suspensions, emulsions, lyophilized preparations, and suppositories. Non-aqueous solvents and suspensions include, but are not limited to, propylene glycol, polyethylene glycol, vegetable oils such as olive oil, and injectable esters such as ethyl oleate, etc. The suppository base may include, but is not limited to, witepsol, macrogol, Tween 61, cacao butter, laurin butter, glycerogelatin, etc.

In still another general aspect, there is provided a PIKfyve inhibitor, comprising: any one compound of Chemical Formulas 1, 2-1 to 2-7, and 3-1 to 3-3 as defined in any embodiment described herein, an optical isomer thereof, or a pharmaceutically acceptable salt thereof.

In another general aspect, there is provided a method of treating cancer, comprising: administering to a subject in need thereof a therapeutically effective amount of any one compound of Chemical Formulas 1, 2-1 to 2-7, and 3-1 to 3-3 as defined in any embodiment described herein, an optical isomer thereof, or a pharmaceutically acceptable salt thereof.

As used herein, the term "any one compound of Chemical Formulas 1, 2-1 to 2-7, and 3-1 to 3-3, an optical isomer thereof, or a pharmaceutically acceptable salt thereof" or "cancer" is as defined above.

In still another general aspect, there is provided a method for treating inflammatory diseases, comprising: administering to a subject in need thereof a therapeutically effective amount of any one compound of Chemical Formulas 1, 2-1 to 2-7, and 3-1 to 3-3 as defined in any embodiment described herein, an optical isomer thereof, or a pharmaceutically acceptable salt thereof.

As used herein, the term "any one compound of Chemical Formulas 1, 2-1 to 2-7, and 3-1 to 3-3, an optical isomer thereof, or a pharmaceutically acceptable salt thereof" or "inflammatory disease" is as described above.

In still another general aspect, there is provided a method for treating lysosomal storage diseases (LSDs), comprising: administering to a subject in need thereof a therapeutically effective amount of any one compound of Chemical Formulas 1, 2-1 to 2-7, and 3-1 to 3-3 as defined in any embodiment described herein, an optical isomer thereof, or a pharmaceutically acceptable salt thereof.

As used herein, the term "any one compound of Chemical Formulas 1, 2-1 to 2-7, and 3-1 to 3-3, an optical isomer thereof, or a pharmaceutically acceptable salt thereof" or "lysosomal storage disease (LSD)" is as described above.

In still another general aspect, there is provided a method for treating neurological diseases, comprising: administering to a subject in need thereof a therapeutically effective amount of any one compound of Chemical Formulas 1, 2-1 to 2-7, and 3-1 to 3-3 as defined in any embodiment described herein, an optical isomer thereof, or a pharmaceutically acceptable salt thereof.

As used herein, the term "any one compound of Chemical Formulas 1, 2-1 to 2-7, and 3-1 to 3-3, an optical isomer thereof, or a pharmaceutically acceptable salt thereof" or "neurological disease" is as described above.

The term "subject" of the present disclosure means any animal that has developed or may develop cancer, and typically may be any animal capable of exhibiting a beneficial effect by treatment with the compound represented by Chemical Formula 1 of the present disclosure, an optical isomer thereof, or a pharmaceutically acceptable salt thereof, but includes, without limitation, any subject that exhibits symptoms of cancer or is at risk of exhibiting such symptoms. As described above, the pharmaceutical composition of the present disclosure may be administered to a subject to effectively prevent or treat any of the diseases described above. The pharmaceutical composition of the present disclosure may be administered as an individual therapeutic agent, or in combination with conventional therapeutic agents for cancer, inflammatory diseases, or lysosomal storage diseases, and may be administered sequentially or concurrently with conventional therapeutic agents.

As used herein, the term "therapeutically effective amount" means an amount sufficient to prevent or treat a disease at a reasonable benefit/risk ratio applicable to the prevention or treatment of a medical condition, and refers to an amount of any one compound of Chemical Formulas 1, 2-1 to 2-7, and 3-1 to 3-3, an optical isomer thereof, or a pharmaceutically acceptable salt thereof that is effective for the prevention or treatment of the disease. The effective dose level may be determined depending on factors including the severity of the disease, the activity of the drug, the patient's age, weight, health, sex, the patient's sensitivity to the drug, the administration time, the route of administration, and excretion rate, of the composition of the present disclosure used, the treatment period, drugs used in combination or concurrently with the composition of the present disclosure used, and other factors well known in the medical field. For example, any one compound of Chemical Formulas 1, 2-1 to 2-7, and 3-1 to 3-3, or a pharmaceutically acceptable salt thereof may be administered at 0.0001 to 100 mg/kg per day, and the dosage may be administered once a day or in multiple divided doses.

The term "administration" of the present disclosure means introducing a predetermined substance into a patient by an appropriate method, and the composition may be administered through any general route as long as the composition is able to reach the target tissue. In addition, the pharmaceutical composition of the present disclosure may be administered by any device capable of transporting an active substance to a target tissue. For example, the administration may be oral administration, intrathecal administration, intraperitoneal administration, intravenous administration, intramuscular administration, subcutaneous administration, intradermal administration, topical administration, intranasal administration, intrapulmonary administration, intrarectal administration, inner ear administration, endometrial administration, sublingual administration, or intracerebrovascular injection, but is not limited thereto. Solid preparations for oral administration include tablets, pills, powders, granules, capsules, etc., and preparations for parenteral administration include sterile aqueous solutions, non-aqueous solutions, suspensions, emulsions, lyophilized preparations, and suppositories.

The pharmaceutical composition of the present disclosure may be administered as an individual therapeutic agent or in combination with other therapeutic agents, or may be administered sequentially or simultaneously with conventional therapeutic agents. The composition may be administered in an amount capable of obtaining the maximum effect with the minimum amount without side effects in consideration of all of the above factors, which may be easily determined by those skilled in the art.

The treatment method of the present disclosure includes not only treating the disease itself before the onset of symptoms, but also inhibiting or avoiding symptoms thereof by administering any one compound of Chemical Formulas 1, 2-1 to 2-7, and 3-1 to 3-3, an optical isomer thereof, or a pharmaceutically acceptable salt thereof. In the management of diseases, a prophylactic or therapeutic dose of a particular active ingredient will vary depending on the nature and severity of the disease or condition and the route by which the active ingredient is administered. The dose and frequency of dose will vary depending on the individual patient's age, weight and response. A suitable dosage regimen may be readily selected by those skilled in the art who take these factors for granted. In addition, the treatment method of the present disclosure may further comprise administration of a therapeutically effective amount of an additional active agent to help treat a disease together with any one compound of Chemical Formulas 1, 2-1 to 2-7, and 3-1 to 3-3, an optical isomer thereof, or a pharmaceutically acceptable salt thereof, wherein the additional active agent may exhibit a synergistic or adjuvant effect together with the compound represented by Chemical Formula 1 above, an optical isomer thereof, or a pharmaceutically acceptable salt thereof.

As used herein, the term "improvement" refers to any action that at least reduces a parameter associated with the condition being treated, for example, the severity of a symptom.

In addition, the present disclosure provides use of any one compound of Chemical Formulas 1, 2-1 to 2-7, and 3-1 to 3-3, an optical isomer thereof, or a pharmaceutically acceptable salt thereof, for the manufacture of a medicament for treating cancer.

As used herein, the term "any one compound of Chemical Formulas 1, 2-1 to 2-7, and 3-1 to 3-3" or "lysosomal storage disease (LSD)" is as described above.

In addition, the present disclosure provides use of any one compound of Chemical Formulas 1, 2-1 to 2-7, and 3-1 to 3-3, an optical isomer thereof, or a pharmaceutically acceptable salt thereof, for the manufacture of a medicament for treating inflammatory diseases.

Further, the present disclosure provides use of any one compound of Chemical Formulas 1, 2-1 to 2-7, and 3-1 to 3-3, an optical isomer thereof, or a pharmaceutically acceptable salt thereof, for the manufacture of a medicament for treating lysosomal storage diseases (LSDs).

In addition, the present disclosure provides use of any one compound of Chemical Formulas 1, 2-1 to 2-7, and 3-1 to 3-3, an optical isomer thereof, or a pharmaceutically acceptable salt thereof, for the manufacture of a medicament for treating neurological diseases.

### [Advantageous Effects]

The novel compound of the present disclosure not only has excellent activity for PIKfyve inhibition, but also has excellent metabolic stability and in vivo stability, and is therefore useful as a therapeutic agent for cancer, inflammatory diseases, lysosomal storage diseases, or neurological diseases associated with PIKfyve activity.

### [Description of Drawings]

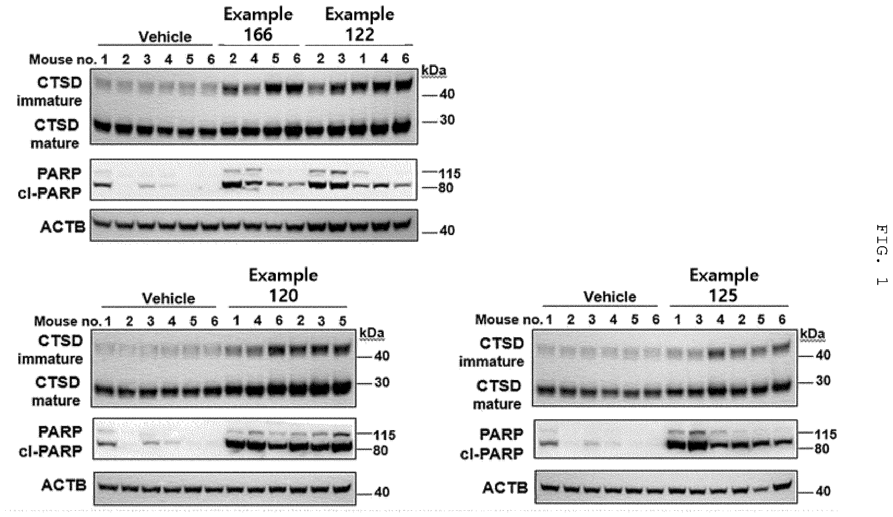

FIG. 1 shows the results of pharmacodynamic analysis of compounds of the present disclosure in tumor tissue.

### [Best Mode]

Hereinafter, the present disclosure will be described in detail with reference to embodiments for better understanding. However, the exemplary embodiments according to the present disclosure may be modified in various different forms, and the scope of the present disclosure should not be construed as being limited to the following Examples. Examples of the present disclosure are provided to more completely explain the present disclosure to those skilled in the art.

The reagents and solvents described below were purchased from Sigma-Aldrich, TCI, and other suppliers, unless otherwise specified. HPLC analysis was conducted using the Alliance HPLC system from Waters, and silica gel for column chromatography was utilized with the Flash purification system from Biotage. ¹H NMR data were measured at 400 MHz using the AscendTM system from Bruker, and mass spectra were obtained using the Masslynx MS system from Waters.

The ¹H nuclear magnetic resonance (NMR) spectra were all consistent with the chemical structures of the compounds according to Examples in the present disclosure.

The characteristic chemical shifts (*δ*) are given in parts-per-million (ppm) for the residual proton signal in the deuterated solvent (CDCl₃: 7.27 ppm; CD₃OD: 3.31 ppm; DMSO-d₆: 2.50 ppm) and are reported with the conventional abbreviations for the designation of the major peaks: For example, S, single; D, double; T, triple; Q, quadruple; M, multiple; BR, broad.

### <Example>

### Example 1. Synthesis of (E)-4-(4-(2-(3-methylbenzylidene)hydrazinyl)-7-phenethyl-6,7-dihydro-5H-pyrrolo[2,3-d]pyrimidin-2-yl)morpholine

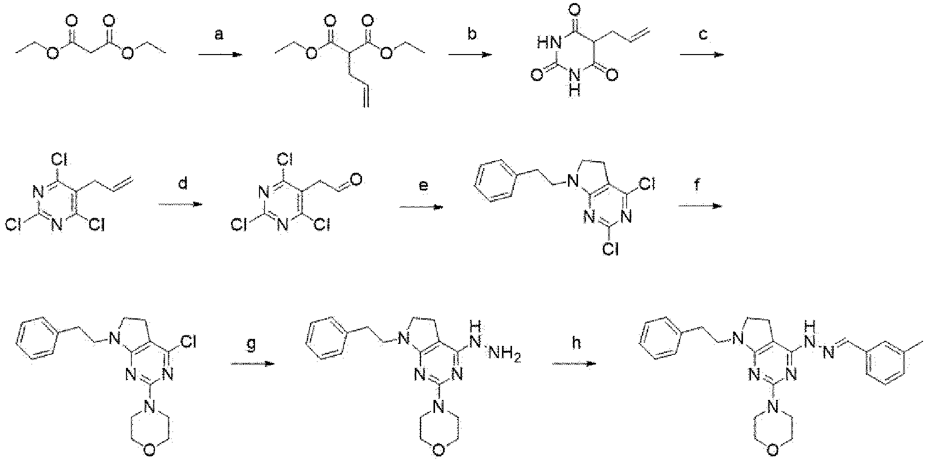

### (Step a) Synthesis of diethyl 2-allylmalonate

Diethyl malonate (4.7 mL) was added to 21% sodium ethoxide (17.6 mL). After confirming that the solid melted at a temperature of 45°C, allyl bromide (2.6 mL) was added and stirred at 60°C for 22 hours. Then, the reaction solution was concentrated in a reduced pressure evaporator, and subjected to silica chromatography under ethyl acetate/hexane conditions to obtian the target compound diethyl 2-allylmalonate (1.6 g) as a white solid in a yield of 26%. MS: m/z = 201 (M+1, ESI+).

### (Step b) Synthesis of 5-allylpyrimidine-2,4,6(1H,3H,5H)-trione

Diethyl 2-allylmalonate (1.6 g) was dissolved in ethanol (4.0 mL), then urea (479.9 mg) and 21% sodium ethoxide (3.0 mL) were slowly added, and the reaction solution was stirred at 100°C for 18 hours. The reaction solution was then allowed to cool to room temperature and the resulting solid was filtered to obtain the target compound 5-allylpyrimidine-2,4,6(1H,3H,5H)-trione (730.0 mg) in a yield of 54%. MS: m/z = 169 (M+1, ESI+).

### (Step c) Synthesis of 5-allyl-2,4,6-trichloropyrimidine

5-Allylpyrimidine-2,4,6(1H,3H,5H)-trione (526.0 mg) was dissolved in phosphoryl chloride (3.2 mL), N,N-dimethylaniline (0.6 mL) was added, and the reaction solution was stirred at 120°C for 26 hours. Then, the reaction solution was cooled to room temperature and concentrated. Next, distilled water was added, and the reaction mixture was extracted with ethyl acetate followed by silica chromatography under ethyl acetate/hexane conditions to obtain the target compound 5-allyl-2,4,6-trichloropyrimidine (440.0 mg) in a yield of 45%. MS: m/z = 224 (M+1, ESI+).

### (Step d) Synthesis of 2-(2,4,6-trichloropyrimidin-5-yl)acetaldehyde

5-Allyl-2,4,6-trichloropyrimidine (440.0 mg) was dissolved in acetone (5.0 mL) and distilled water (5.0 mL), then potassium osmate(IV) dihydrate (36.3 mg) and sodium periodate (1.7 g) were added and stirred at room temperature for 5 hours. After removing the solid produced by filtration, the solution was concentrated using a vacuum concentrator. Then, distilled water was added and the reaction mixture was extracted with ethyl acetate. Next, the reaction mixture was subjected to silica chromatography under ethyl acetate/hexanes to obtain the target compound 2-(2,4,6-trichloropyrimidin-5-yl)acetaldehyde (330.0 mg) in a yield of 74%. MS: m/z = 226 (M+1, ESI+).

### (Step e) Synthesis of 2,4-dichloro-7-phenethyl-6,7-dihydro-5H-pyrrolo[2,3-d]pyrimidine

To 2-(2,4,6-trichloropyrimidin-5-yl)acetaldehyde (200.0 mg), 2-phenylethanamine (122.6 µL), acetic acid (112.7 µL), and sodium cyanoborohydride (78.0 mg) were added, and the mixture was stirred at room temperature for 25 hours. The reaction solution was concentrated using a vacuum concentrator. The concentrated product was subjected to silica chromatography under ethyl acetate/hexane conditions to obtain the target compound 2,4-dichloro-7-phenethyl-6,7-dihydro-5H-pyrrolo[2,3-d]pyrimidine (150.0 mg) in a yield of 57%. MS: m/z = 295 (M+1, ESI+).

### (Step f) Synthesis of 4-(4-chloro-7-phenethyl-6,7-dihydro-5H-pyrrolo[2,3-d]pyrimidin-2-yl)morpholine

2, 4-Dichloro-7-phenethyl-6, 7-dihydro-5H-pyrrolo[2, 3-d]pyrimidine (150.0 mg) was dissolved in tetrahydrofuran (14.0 mL), then morpholine (0.2 mL) was added, and the mixture was stirred at 50°C for 14 hours. Then, the reaction solution was concentrated using a vacuum evaporator, and the target compound 4-(4-chloro-7-phenethyl-6,7-dihydro-5H-pyrrolo[2,3-d]pyrimidin-2-yl)morpholine (180.0 mg) was obtained in a yield of 100% without separate purification. MS: m/z = 346 (M+1, ESI+).

### (Step g) Synthesis of 4-(4-hydrazinyl-7-phenethyl-6,7-dihydro-5H-pyrrolo[2,3-d]pyrimidin-2-yl)morpholine

To 4-(4-chloro-7-phenethyl-6,7-dihydro-5H-pyrrolo[2,3-d]pyrimidin-2-yl)morpholine (180.0 mg), N-methyl-2-pyrrolidone (2.0 mL), hydrazine monohydrate (127.2 µL), and N,N-diisopropylethylamine (181.8 µL) were added, and the mixture was stirred in a microwave at 120°C for 12 hours. The reaction solution was concentrated using a vacuum evaporator and used in the next reaction without separate purification. MS: m/z = 342 (M+1, ESI+).

### (Step h) Synthesis of (E)-4-(4-(2-(3-methylbenzylidene)hydrazinyl)-7-phenethyl-6,7-dihydro-5H-pyrrolo[2,3-d]pyrimidin-2-yl)morpholine

4-(4-Hydrazinyl-7-phenethyl-6,7-dihydro-5H-pyrrolo[2,3-d]pyrimidin-2-yl)morpholine (253.0 mg) was dissolved in methanol (3.0 mL), followed by addition of acetic acid (5 drops) and 3-methylbenzaldehyde (131.2 µL), and stirred at room temperature for 19 hours. The reaction solution was concentrated using a vacuum evaporator, followed by preparative thin layer chromatography to obtain the target compound (E)-4-(4-(2-(3-methylbenzylidene)hydrazinyl)-7-phenethyl-6,7-dihydro-5H-pyrrolo[2,3-d]pyrimidin-2-yl)morpholine (17.0 mg) in a yield of 5%.

¹H NMR (400 MHz, CDCl₃) *δ* 10.4 (s, 1H), 7.91 (s, 1H), 7.37 (d, *J* = 8.0 Hz, 1H), 7.33 - 7.26 (m, 6H), 7.21 - 7.17 (m, 1H), 7.12 (d, *J* = 7.6 Hz, 1H), 3.67 (s, 8H), 3.60 - 3.46 (m, 4H), 3.13 (t, *J* = 8.6 Hz, 2H), 2.84 (t, *J* = 7.4 Hz, 2H), 2.32 (s, 3H). MS: m/z = 443 (M+1, ESI+).

### Example 2. Synthesis of (E)-4-(7-isobutyl-4-(2-(3-methylbenzylidene)hydrazinyl)-6,7-dihydro-5H-pyrrolo[2,3-d]pyrimidin-2-yl)morpholine

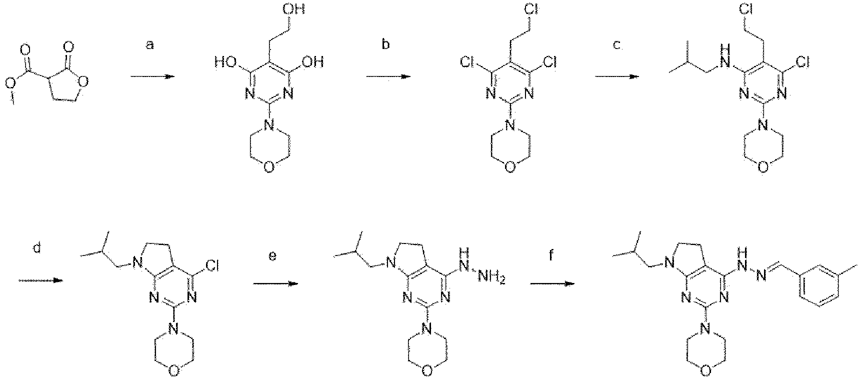

### (Step a) Synthesis of 5-(2-hydroxyethyl)-2-morpholinopyrimidine-4,6-diol

Methyl 2-oxotetrahydrofuran-3-carboxylate (5.0 g) was dissolved in methanol (40.0 mL), then morpholine-4-carboxamide (5.0 g) and 25% sodium methoxide (12.0 mL) were added, and the mixture was stirred at 90°C for 2 hours. The reaction solution was concentrated using a vacuum evaporator and used in the next reaction without separate purification. MS: m/z = 242 (M+1, ESI+).

### (Step b) Synthesis of 4-(4,6-dichloro-5-(2-chloroethyl)pyrimidin-2-yl)morpholine

Phosphoryl chloride (33.8 mL) was added to 5-(2-hydroxyethyl)-2-morpholinopyrimidine-4,6-diol (5.0 g), and the mixture was stirred at 100°C for 10 hours. The reaction was terminated by adding distilled water, and then the reaction mixture was extracted with ethyl acetate. The extracted product was subjected to silica chromatography under ethyl acetate/hexane conditions to obtain the target compound 4-(4,6-dichloro-5-(2-chloroethyl)pyrimidin-2-yl)morpholine (2.1 g) in a yield of 34%. MS: m/z = 296 (M+1, ESI+) .

### (Step c) Synthesis of 6-chloro-5-(2-chloroethyl)-N-isobutyl-2-morpholinopyrimidin-4-amine

4-(4,6-Dichloro-5-(2-chloroethyl)pyrimidin-2-yl)morpholine (200.0 mg) was dissolved in acetonitrile (12.0 mL), then 1-cyclopropylethenone (155.4 µL) and N,N-diisopropylethylamine (293.7 µL) were added, and the mixture was stirred at 120°C for 72 hours. The reaction solution was concentrated using a vacuum evaporator, followed by silica chromatography under ethyl acetate/hexane conditions to obtain the target compound 6-chloro-5-(2-chloroethyl)-N-isobutyl-2-morpholinopyrimidin-4-amine (150.0 mg) in a yield of 66%. MS: m/z = 333 (M+1, ESI+).

### (Step d) Synthesis of 4-(4-chloro-7-isobutyl-6,7-dihydro-5H-pyrrolo[2,3-d]pyrimidin-2-yl)morpholine

6-Chloro-5-(2-chloroethyl)-N-isobutyl-2-morpholinopyrimidin-4-amine (150.0 mg) was dissolved in acetonitrile (7.5 mL), then sodium iodide (134.9 mg) and cesium carbonate (433.6 mg) were added, and the mixtue was stirred at 120°C for 17 hours. The reaction solution was concentrated using a vacuum concentrator. The concentrated product was subjected to silica chromatography under ethyl acetate/hexane conditions to obtain the target compound 4-(4-chloro-7-isobutyl-6,7-dihydro-5H-pyrrolo[2,3-d]pyrimidin-2-yl)morpholine (125.0 mg) in a yield of 94%. MS: m/z = 297 (M+1, ESI+).

### (Step e) Synthesis of 4-(4-hydrazinyl-7-isobutyl-6,7-dihydro-5H-pyrrolo[2,3-d]pyrimidin-2-yl)morpholine

4-(4-Chloro-7-isobutyl-6,7-dihydro-5H-pyrrolo[2,3-d]pyrimidin-2-yl)morpholine (125.0 mg) was dissolved in N-methyl-2-pyrrolidone (3.0 mL), followed by addition of hydrazine monohydrate (102.6 µL) and N,N-diisopropylethylamine (146.7 µL), and the mixture was stirred in a microwave at 120°C for 17 hours. The reaction solution was concentrated using a vacuum evaporator and used in the next reaction. MS: m/z = 293 (M+1, ESI+).

### (Step f) Synthesis of (E)-4-(7-isobutyl-4-(2-(3-methylbenzylidene)hydrazinyl)-6,7-dihydro-5H-pyrrolo[2,3-d]pyrimidin-2-yl)morpholine

4-(4-Hydrazinyl-7-isobutyl-6,7-dihydro-5H-pyrrolo[2,3-d]pyrimidin-2-yl)morpholine (100.0 mg) was dissolved in methanol (1.1 mL), followed by addition of acetic acid (10 drops) and 3-methylbenzaldehyde (60.4 µL), and the reaction mixture was stirred at room temperature for 15 hours. The reaction solution was concentrated using a vacuum evaporator, followed by silica chromatography under ethyl acetate/hexane conditions to obtain the target compound (E)-4-(7-isobutyl-4-(2-(3-methylbenzylidene)hydrazinyl)-6,7-dihydro-5H-pyrrolo[2,3-d]pyrimidin-2-yl)morpholine (36.0 mg) in a yield of 27%.

¹H NMR (400 MHz, CDCl₃) *δ* 10.4 (s, 1H), 7.91 (s, 1H), 7.37 (d, *J* = 7.6 Hz, 1H), 7.33 (s, 1H), 7.27 (t, *J* = 7.6 Hz, 1H), 7.12 (d, *J* = 7.2 Hz, 1H), 3.66 - 3.60 (m, 8H), 3.49 (t, *J* = 8.6 Hz, 2H), 3.17 (t, *J* = 8.6 Hz, 2H), 3.09 (d, *J* = 7.2 Hz, 2H), 2.32 (s, 3H), 0.88 - 0.84 (m, 6H). MS: m/z = 395 (M+1, ESI+).

### Example 3. Synthesis of (E)-4- (7-isobutyl-4-(2-(3-methylbenzylidene)hydrazinyl)-7H-pyrrolo[2,3-d]pyrimidin-2-yl)morpholine

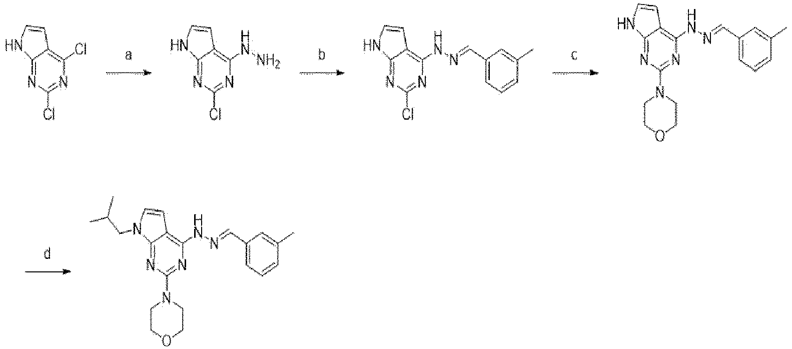

### (Step a) Synthesis of 2-chloro-4-hydrazinyl-7H-pyrrolo[2,3-d]pyrimidine

2, 4-Dichloro-7H-pyrrolo[2, 3-d] pyrimidine (10 g) and hydrazine monohydrate (5.2 mL) were added to tetrahydrofuran (106.4 mL) to prepare a suspension, and the mixture was stirred at 70°C for 3.5 hours. The reaction solution was filtered with dichloromethane to obtain the target compound 2-chloro-4-hydrazinyl-7H-pyrrolo[2,3-d]pyrimidine (10.4 g) as a yellow solid in a yield of 99%. MS: m/z = 188 (M+1, ESI+) .

### (Step b) Synthesis of (E)-2-chloro-4-(2-(3-methylbenzylidene)hydrazinyl)-7H-pyrrolo[2,3-d]pyrimidine

2-Chloro-4-hydrazinyl-7H-pyrrolo[2,3-d]pyrimidine (10.4 g) and 3-methylbenzaldehyde (6.8 mL) were dissolved in ethanol (568 mL) to prepare a suspension, and a catalytic equivalent of acetic acid was added, followed by stirring at 60°C for 1 hour. Distilled water was added to the reaction solution, then extracted with ethyl acetate, and concentrated. Then, the target compound (E)-2-chloro-4-(2-(3-methylbenzylidene)hydrazinyl)-7H-pyrrolo[2,3-d]pyrimidine (12.7 g) as a yellow powder was obtained in a yield of 78%. MS: m/z = 286 (M+1, ESI+).

### (Step c) Synthesis of (E) -4- (4- (2- (3-methylbenzylidene)hydrazinyl)-7H-pyrrolo[2,3-d]pyrimidin-2-yl)morpholine

(E)-2-Chloro-4-(2-(3-methylbenzylidene)hydrazinyl)-7H-pyrrolo[2,3-d]pyrimidine (5.7 g) was dissolved in morpholine (100.0 mL) and stirred at 90°C for 17 hours. The reaction solution was concentrated under reduced pressure, followed by silica chromatography under ethyl acetate/benzene conditions to obtain the target compound (E)-4-(4-(2-(3-methylbenzylidene)hydrazinyl)-7H-pyrrolo[2,3-d]pyrimidin-2-yl)morpholine (2.5 g) as a white solid in a yield of 39%. MS: m/z = 337 (M+1, ESI+).

### (Step d) Synthesis of (E)-4-(7-isobutyl-4-(2-(3-methylbenzylidene)hydrazinyl)-7H-pyrrolo[2,3-d]pyrimidin-2-yl)morpholine

(E)-4-(4-(2-(3-Methylbenzylidene)hydrazinyl)-7H-pyrrolo[2,3-d]pyrimidin-2-yl)morpholine (114.0 mg), 1-iodo-2-methylpropane (39.0 µL), and cesium carbonate (132.5 mg) were dissolved in dimethylformaldehyde (0.47 mL), and the mixture was stirred at 50°C for 17 hours. The reaction solution was concentrated under reduced pressure, followed by silica chromatography under ethyl acetate/benzene conditions to obtain the target compound (E)-4-(7-isobutyl-4-(2-(3-methylbenzylidene)hydrazinyl)-7H-pyrrolo[2,3-d]pyrimidin-2-yl)morpholine (38 mg) as a white solid in a yield of 29%.

¹H NMR (CDCl₃): *δ* 8.40 (s, 1H), 7.83 (s, 1H), 7.61 (d, *J* = 7.6 Hz, 1H), 7.58 (s, 1H), 7.36 (t, *J* = 7.6 Hz, 1H), 7.20 (d, *J* = 7.6 Hz, 1H), 7.14-7.13 (m, 1H), 6.89-6.88 (m, 1H), 4.29 (d, *J* = 3.8 Hz, 2H), 3.85-3.79 (m, 8 H), 2.45 (s, 3H), 2.33-2.26 (m, 1H), 1.05 (s, 3H), 1.03 (s, 3H). MS: m/z = 393 (M+1, ESI+).

### Example 4. Synthesis of (E)-4-(4-(2-(3-methylbenzylidene)hydrazinyl)-7-phenethyl-pyrrolo[2,3-d]pyrimidin-2-yl)morpholine

The (E)-4-(4-(2-(3-methylbenzylidene)hydrazinyl)-7H-pyrrolo[2,3-d]pyrimidin-2-yl)morpholine (660.0 mg) prepared in the same manner as in (Step a) to (Step c) of Example 3, (2-bromoethyl)benzene (265.0 µL), and cesium carbonate (767 mg) were dissolved in dimethylformaldehyde (98 mL), and then the mixture was stirred at 60°C for 18 hours. Saturated ammonium chloride solution was added to the reaction solution, then extracted with ethyl acetate, and concentrated. The reaction mixture was subjected to silica chromatography under hexane/ethyl acetate conditions to obtain the target compound (E)-4-(4-(2-(3-methylbenzylidene)hydrazinyl)-7-phenethyl-pyrrolo[2,3-d]pyrimidin-2-yl)morpholine (25.0 mg) an off-white solid in a yield of 2.9%.

¹H NMR (DMSO-*d*₆): *δ* 11.18 (s, 1H), 8.13 (s, 1H), 7.65-7.62 (m, 2H), 7.42-7.22 (m, 7H), 6.97-6.95 (m, 1H), 6.88-6.87 (m, 1H), 4.57 (t, *J* = 7.8 Hz, 2H), 3.71-3.66 (m, 8H), 2.94 (t, *J* = 7.8 Hz, 2H), 2.40 (s, 3H). MS: m/z = 441 (M+1, ESI+).

### Example 5. Synthesis of (E)-4-(4-(2-(3-methylbenzylidene)hydrazinyl)-7-(2-(pyridin-2-yl)ethyl)-7H-pyrrolo[2,3-d]pyrimidin-2-yl)morpholine

The target compound (E)-4-(4-(4-(2-(3-methylbenzylidene)hydrazinyl)-7-(2-(pyridin-2-yl)ethyl)-7H-pyrrolo[2,3-d]pyrimidin-2-yl)morpholine as a yellow solid was obtained in the same manner as in Example 73 below, except using 2,4-dichloro-7H-pyrrolo[2,3-d]pyrimidin-2-yl in (Step a).

¹H NMR (DMSO-*d*₆) *δ* 11.10 (s, 1H), 8.52-8.51 (m, 1H), 8.09 (s, 1H), 7.66 (td, *J* = 7.8, 1.8 Hz, 1H), 7.50 (d, *J* = 7.6 Hz, 1H), 7.46 (s, 1H), 7.33 (t, *J* = 7.6 Hz, 1H), 7.24-7.17 (m, 3H), 6.87 (d, *J* = 3.2 Hz, 1H), 6.69 (d, *J* = 3.6 Hz, 1H), 4.43 (t, *J* = 7.2 Hz, 2H), 3.75-3.62 (m, 8H), 3.23 (t, *J* = 7.2 Hz, 2H), 2.37 (s, 3H). MS: m/z = 442 (M+1, ESI+).

### Example 6. Synthesis of (E)-4-(7-(2,2-difluoro-2-(pyridin-2-yl)ethyl)-4-(2-(3-methylbenzylidene)hydrazinyl)-7H-pyrrolo[2,3-d]pyrimidin-2-yl)morpholine

The target compound (E)-4-(7-(2,2-difluoro-2-(pyridin-2-yl)ethyl)-4-(2-(3-methylbenzylidene)hydrazinyl)-7H-pyrrolo[2,3-d]pyrimidin-2-yl)morpholine as an off-white solid was obtained in the same manner as in Example 73 below, except using 2,4-dichloro-7H-pyrrolo[2,3-d]pyrimidine, 2,2-difluoro-2-(pyridin-2-yl) in (Step a).

¹H NMR (DMSO-*d*₆) *δ* 11.15 (s, 1H), 8.75-8.74 (m, 1H), 8.10 (s, 1H), 7.93 (td, *J* = 7.8, 1.8 Hz, 1H), 7.59-7.56 (m, 2H), 7.33 (d, *J* = 8 Hz, 1H), 7.47 (s, 1H), 7.35 (t, *J* = 7.6 Hz, 1H), 7.21 (d, *J* = 7.2 Hz, 1H), 6.88 (d, *J* = 3.6 Hz, 1H), 6.79 (d, *J* = 3.2 Hz, 1H), 4.97 (t, *J* = 14.4 Hz, 2H), 3.66-3.61 (m, 8H), 2.37 (s, 3H); MS: m/z = 478 (M+1, ESI+).

### Example 7. Synthesis of (E)-4-(8-(2-(3-methylbenzylidene)hydrazinyl)-3-phenylimidazo[1,2-b]pyridazin-6-yl)morpholine

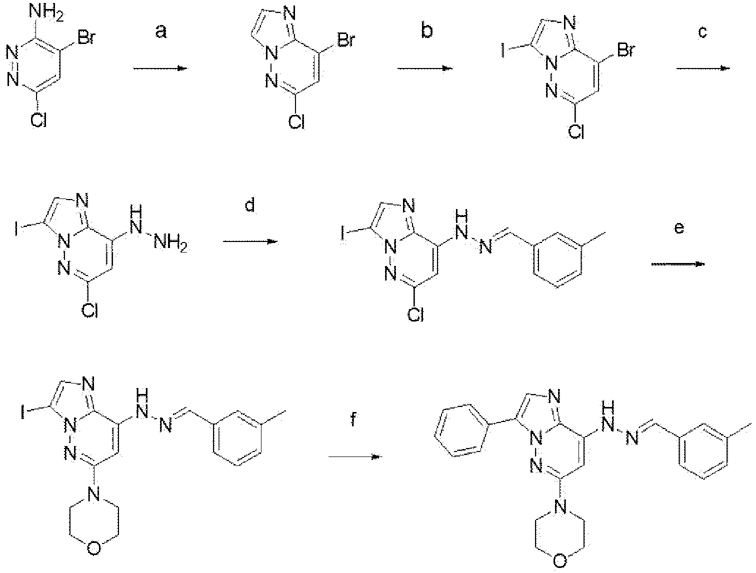

### (Step a) Synthesis of 8-bromo-6-chloroimidazo[1,2-b]pyridazine

2-Chloroacetaldehyde (18.83 g) and ethanol (100 mL) were added to 4-bromo-6-chloropyridazin-3-amine (5 g), and the mixture was stirred at 85°C for 8 hours. The target compound 8-bromo-6-chloroimidazo[1,2-b]pyridazine (4.7 g) was synthesized as a white solid through filtration in a yield of 84.3%. MS: m/z = 233.0 (M+1, ESI+).

### (Step b) Synthesis of 8-bromo-6-chloro-3-iodoimidazo[1,2-b]pyridazine

To 8-bromo-6-chloroimidazo[1,2-b]pyridazine (3 g), N-iodosuccinimide (3.6 g), trifluoroacetic acid (3.64 g), and chloroform (50 mL) were added, and the mixture was stirred at 25°C for 2 hours. Distilled water was added to the reaction solution and extracted with dichloromethane. After washing the organic solvent with a saturated NaCl solution, sodium sulfate was added, and the reaction mixture was filtered and concentrated. Then, the target compound 8-bromo-6-chloro-3-iodoimidazo[1,2-b]pyridazine (5.4 g) was synthesized as a white solid through silica chromatography. MS: m/z = 359.0(M+1, ESI+)

### (Step c) Synthesis of 6-chloro-8-hydrazinyl-3-iodoimidazo[1,2-b]pyridazine

Tetrahydrofuran (50 mL) and hydrazine monohydrate (7.41 g) were added to 8-bromo-6-chloro-3-iodoimidazo[1,2-b]pyridazine (5.3 g), and the mixture was stirred at 25°C for 1 hour. After adding distilled water, the resulting solid was filtered and concentrated under reduced pressure to synthesize the target compound 6-chloro-8-hydrazinyl-3-iodoimidazo[1,2-b]pyridazine (3.4 g) as a white solid in a yield of 74.6%. MS: m/z = 309.1(M+1, ESI+).

### (Step d) Synthesis of (E)-6-chloro-3-iodo-8-(2-(3-methylbenzylidene)hydrazinyl)imidazo[1,2-b]pyridazine

Acetic acid (0.5 mL), 3-methylbenzaldehyde (1.59 g), and methanol (40 mL) were added to 6-chloro-8-hydrazinyl-3-iodoimidazo[1,2-b]pyridazine (3.4 g), and the mixture was stirred at 70°C for 15 minutes. Distilled water was added to the reaction solution and extracted with dichloromethane. After washing the organic solvent with a saturated NaCl solution, sodium sulfate was added, and the reaction mixture was filtered and concentrated. The concentrated product was subjected to silica chromatography to obtain the target compound (E)-6-chloro-3-iodo-8-(2-(3-methylbenzylidene)hydrazinyl)imidazo[1,2-b]pyridazine (4.3 g) as a yellow solid in a yield of 95%. MS: m/z =411.1(M+1, ESI+).

### (Step e) Synthesis of (E)-4-(3-iodo-8-(2-(3-methylbenzylidene)hydrazinyl)imidazo[1,2-b]pyridazin-6-yl)morpholine

To (E)-6-chloro-3-iodo-8-(2-(3-methylbenzylidene)hydrazinyl)imidazo[1,2-b]pyridazine (2.8 g), acetic acid (0.5 mL), morpholine (5.94 g), and isopropyl alcohol (30 mL) were added, and the mixture was stirred at 170°C for 3 hours. Distilled water was added to the reaction solution and extracted with ethyl acetate. After washing the organic solvent with a saturated NaCl solution, sodium sulfate was added, and the reaction mixture was filtered and concentrated. The concentrated product was subjected to silica chromatography under benzene/ethyl acetate conditions to obtain the target compound (E)-4-(3-iodo-8-(2-(3-methylbenzylidene)hydrazinyl)imidazo[1,2-b]pyridazin-6-yl)morpholine (270 mg) as a white solid in a yield of 8.57%. MS: m/z =463.1(M+1, ESI+).

### (Step f) Synthesis of (E)-4-(8-(2-(3-methylbenzylidene)hydrazinyl)-3-phenylimidazo[1,2-b]pyridazin-6-yl)morpholine

To (E)-4-(3-iodo-8-(2-(3-methylbenzylidene)hydrazinyl)imidazo[1,2-b]pyridazin-6-yl)morpholine (186 mg), phenylboronic acid (186 mg), Pd(dppf)Cl₂ (37 mg), cesium carbonate (413 mg), dimethylformamide (5 mL), and distilled water (1 mL) were added, and the mixture was stirred at 120°C for 2 hours. After filtering the reaction solution, distilled water was added and the reaction mixture was extracted with dichloromethane. After washing the organic solvent with a saturated NaCl solution, sodium sulfate was added, and the reaction mixture was filtered and concentrated. The concentrated product was subjected to silica chromatography under dichloromethane/methanol conditions to synthesize the target compound (E)-4-(8-(2-(3-methylbenzylidene)hydrazinyl)-3-phenylimidazo[1,2-b]pyridazin-6-yl)morpholine (16.18 mg) as a white solid in a yield of 7.55%.

¹H NMR (400 MHz, DMSO-*d*₆) *δ* 11.37 (s, 1H), 8.44 (s, 1H), 8.17 (d, *J* = 7.6 Hz, 2H), 7.91 (s, 1H), 7.60 (d, *J* = 7.6 Hz, 1H), 7.56 (s, 1H), 7.48 (t, *J* = 7.6 Hz, 3H), 7.33 (q, *J* = 7.0 Hz, 2H), 7.22 (d, *J* = 7.7 Hz, 1H), 6.71 (s, 1H), 3.79 (s, 4H), 3.48 (s, 4H), 2.37 (s, 3H). MS: m/z =413.3(M+1, ESI+).

### Example 8. Synthesis of (E)-4-(4-(2-(3-methylbenzylidene)hydrazinyl)-7-phenyl-5H-pyrrolo[3,2-d]pyrimidin-2-yl)morpholine

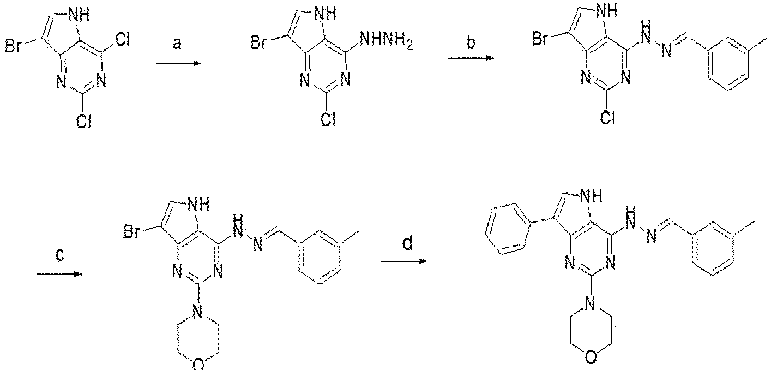

### (Step a) Synthesis of 7-bromo-2-chloro-4-hydrazinyl-5H-pyrrolo[3,2-d]pyrimidine

7-Bromo-2,4-dichloro-5H-pyrrolo[3,2-d]pyrimidine (700.0 mg) was dissolved in tetrahydrofuran (10.0 mL), hydrazine monohydrate (0.5 mL) was added, and the mixture was stirred at room temperature for 1 hour. After adding distilled water, the target compound 7-bromo-2-chloro-4-hydrazinyl-5H-pyrrolo[3,2-d]pyrimidine (620.0 mg) was obtained as a white solid in a yield of 90.0%. MS: m/z = 261.8 (M+1, ESI+).

### (Step b) Synthesis of (E)-7-bromo-2-chloro-4-(2-(3-methylbenzylidene)hydrazinyl)-5H-pyrrolo[3,2-d]pyrimidine

7-Bromo-2-chloro-4-hydrazinyl-5H-pyrrolo[3,2-d]pyrimidine (300.0 mg) was dissolved in methanol (10.0 mL), and acetic acid (0.2 mL) and 3-methylbenzaldehyde (275.0 mg) were added. The mixture was stirred at 70°C for 30 minutes. The reaction solution was concentrated under reduced pressure, followed by silica chromatography under ethyl acetate/benzene conditions to obtain the target compound (E)-7-bromo-2-chloro-4-(2-(3-methylbenzylidene)hydrazinyl)-5H-pyrrolo[3,2-d]pyrimidine (350.0 mg) as a white solid in a yield of 84.0%. MS: m/z = 364.3 (M+1, ESI+).

### (Step c) Synthesis of (E)-4-(7-bromo-4-(2-(3-methylbenzylidene)hydrazinyl)-5H-pyrrolo[3,2-d]pyrimidin-2-yl)morpholine

(E)-7-Bromo-2-chloro-4-(2-(3-methylbenzylidene)hydrazinyl)-5H-pyrrolo[3,2-d]pyrimidine (350.0 mg) was dissolved in isopropyl alcohol (50.0 mL), and morpholine (1.0 mL) and acetic acid (0.2 mL) were added. The mixture was stirred at 120°C for 50 hours. The reaction solution was concentrated under reduced pressure, followed by silica chromatography under ethyl acetate/benzene conditions to obtain the target compound (E)-4-(7-bromo-4-(2-(3-methylbenzylidene)hydrazinyl)-5H-pyrrolo[3,2-d]pyrimidin-2-yl)morpholine (260.0 mg) as a white solid in a yield of 65.0%. MS: m /z = 416.4 (M+1, ESI+).

### (Step d) Synthesis of (E) -4- (4- (2- (3-methylbenzylidene)hydrazinyl)-7-phenyl-5H-pyrrolo[3,2-d]pyrimidin-2-yl)morpholine

(E)-4-(7-Bromo-4-(2-(3-methylbenzylidene)hydrazinyl)-5H-pyrrolo[3,2-d]pyrimidin-2-yl)morpholine (260.0 mg) was dissolved in 1,4-dioxane (5.0 mL), and phenylboronic acid (153.0 mg), potassium carbonate (260.0 mg), distilled water (1.0 mL), and Pd(dppf)Cl₂ (50.0 mg) were added. The mixture was stirred at 100°C for 12 hours. The formed solid was filtered and dried to obtain the target compound (E)-4-(4-(2-(3-methylbenzylidene)hydrazinyl)-7-phenyl-5H-pyrrolo[3,2-d]pyrimidin-2-yl)morpholine (30.0 mg) as a yellow solid in a yield of 12.0%.

¹H NMR (400 MHz, DMSO-*d*₆) *δ* 11.23 (s, 1H), 10.40 (s, 1H), 8.21 - 8.17 (m, 3H), 7.91 (s, 1H), 7.72 (d, *J* = 7.6 Hz, 1H), 7.63 (s, 1H), 7.37 (q, *J* = 7.5 Hz, 3H), 7.25 - 7.12 (m, 2H), 3.71 (s, 8H), 2.39 (s, 3H); MS: m/z = 413.2 (M+1, ESI+).

### Example 9. Synthesis of (E)-4-(5-methyl-4-(2-(3-methylbenzylidene)hydrazinyl)-7-phenyl-5H-pyrrolo[3,2-d]pyrimidin-2-yl)morpholine

The target compound (E)-4-(5-methyl-4-(2-(3-methylbenzylidene)hydrazinyl)-7-phenyl-5H-pyrrolo[3,2-d]pyrimidin-2-yl)morpholine as a white solid was obtained in the same manner as in Example 8 above, except that 7-bromo-2,4-dichloro-5-methyl-5H-pyrrolo[3,2-d]pyrimidine was used instead of 7-bromo-2,4-dichloro-5H-pyrrolo[3,2-d]pyrimidine in (Step a) .

¹H NMR (400 MHz, DMSO-*d*₆) *δ* 10.50 (s, 1H), 8.25 (s, 1H), 8.14 (d, *J* = 7.6 Hz, 2H), 7.95 (s, 1H), 7.48 (d, *J* = 6.5 Hz, 2H), 7.39 - 7.32 (m, 3H), 7.21 (d, *J* = 7.5 Hz, 1H), 7.15 (t, *J* = 7.2 Hz, 1H), 4.02 (s, 3H), 3.72 (s, 8H), 2.36 (s, 3 H); MS: m/z = 427.2 (M+1, ESI+).

### Example 10. Synthesis of (E)-4-(5-isopropyl-4-(2-(3-methylbenzylidene)hydrazinyl)-7-phenyl-5H-pyrrolo[3,2-d]pyrimidin-2-yl)morpholine

The target compound (E)-4-(5-isopropyl-4-(2-(3-methylbenzylidene)hydrazinyl)-7-phenyl-5H-pyrrolo[3,2-d]pyrimidin-2-yl)morpholine as a white solid was obtained in the same manner as in Example 8 above, except that 7-bromo-2,4-dichloro-5-isopropyl-5H-pyrrolo[3,2-d]pyrimidine was used instead of 7-bromo-2,4-dichloro-5H-pyrrolo[3,2-d]pyrimidine in (Step a).

¹H NMR (400 MHz, DMSO-*d*₆) *δ* 10.48 (s, 1H), 8.21 (dd, *J* = 18.7, 12.5 Hz, 3H), 7.46 (d, *J* = 19.5 Hz, 2H), 7.35 (dt, *J* = 14.8, 7.2 Hz, 3H), 7.23 - 7.10 (m, 2H), 5.38 (s, 1H), 3.72 (s, 8H), 2.35 (s, 3H), 1.43 (d, *J* = 5.5 Hz, 6H); MS: m/z = 455.2 (M+1, ESI+).

### Example 11. Synthesis of (E)-4-(4-(2-(3-methylbenzylidene)hydrazinyl)-7-phenylthieno[3,2-d]pyrimidin-2-yl)morpholine

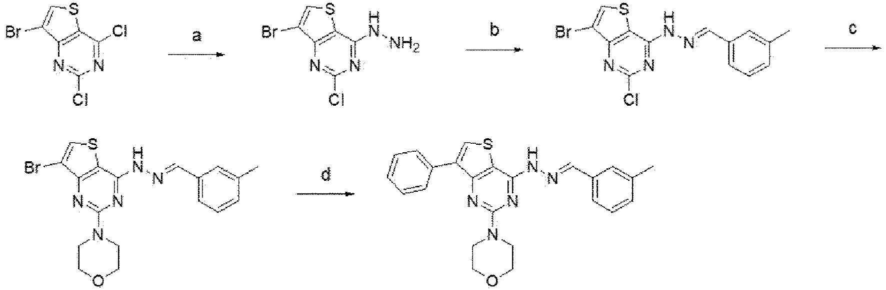

### (Step a) Synthesis of 7-bromo-2-chloro-4-hydrazinylthieno[3,2-d]pyrimidine

7-Bromo-2,4-dichlorothieno[3,2-d]pyrimidine (940.0 mg) was dissolved in isopropyl alcohol (11.0 mL), then hydrazine monohydrate (212.2 mg) was added, and the mixture was stirred at 50°C for 17 hours. The formed solid was filtered and dried to obtain the target compound 7-bromo-2-chloro-4-hydrazinylthieno[3,2-d]pyrimidine (550.0 mg) as a white solid with a yield of 59.4%. MS: m/z = 279 (M+1, ESI+).

### (Step b) Synthesis of (E)-7-bromo-2-chloro-4-(2-(3-methylbenzylidene)hydrazinyl)thieno[3,2-d]pyrimidine

7-Bromo-2-chloro-4-hydrazinylthienol[3,2-d]pyrimidine (550.0 mg) was dissolved in methanol (6.6 mL), then m-tolualdehyde (354.6 mg) was added, and the mixture was stirred at room temperature for 2.5 hours. The formed solid was filtered and dried to obtain the target compound (E)-7-bromo-2-chloro-4-(2-(3-methylbenzylidene)hydrazinyl)thieno[3,2-d]pyrimidine (400.0 mg) as a white solid with a yield of 53.3%. MS: m/z = 381 (M+1, ESI+).

### (Step c) Synthesis of (E)-4-(7-bromo-4-(2-(3-methylbenzylidene)hydrazinyl)thieno[3,2-d]pyrimidin-2-yl)morpholine

(E)-7-Bromo-2-chloro-4-(2-(3-methylbenzylidene)hydrazinyl)thieno[3,2-d]pyrimidine (100.0 mg) was dissolved in N-methyl-2-pyrrolidone (1.1 mL), and morpholine (68.5 µL) and diisopropylethylamine (136.9 µL) were added. The reaction mixture was stirred at 200°C for 4 hours using a microwave. After the reaction solution was concentrated under reduced pressure, distilled water was added, and the reaction mixture was extracted with ethyl acetate, and concentrated. Then, the target compound (E)-4-(7-bromo-4-(2-(3-methylbenzylidene)hydrazinyl)thieno[3,2-d]pyrimidin-2-yl)morpholine (80.0 mg) was obtained as a white solid in a yield of 70.6% through silica chromatography under dichloromethane/methanol conditions. MS: m/z = 432 (M+1, ESI+).

### (Step d) Synthesis of (E) -4- (4- (2- (3-methylbenzylidene)hydrazinyl)-7-phenylthieno[3,2-d]pyrimidin-2-yl)morpholine

(E)-4-(7-bromo-4-(2-(3-methylbenzylidene)hydrazinyl)thieno[3,2-d]pyrimidin-2-yl)morpholine (80.0 mg) was dissolved in 1,4-dioxane (5.0 mL), and then Pd(dppf)Cl₂ (6.8 mg), phenylboronic acid (112.8 mg), sodium carbonate (49.0 mg), and distilled water (1.7 mL) were added. The reaction mixture was stirred at 140°C for 14 hours using a microwave. The reaction solution was concentrated under reduced pressure, followed by silica chromatography under ethyl acetate/benzene conditions to obtain the target compound (E)-4-(4-(2-(3-methylbenzylidene)hydrazinyl)-7-phenylthieno[3,2-d]pyrimidin-2-yl)morpholine (20.0 mg) as a white solid in a yield of 25.2%.

¹H NMR (DMSO-*d*₆) : *δ* 11.80 (s, 1H), 8.40 (s, 1H), 8.15 (s, 1H), 8.09 (d, *J* = 7.2 Hz, 2H), 7.66 (d, *J* = 8.0 Hz, 1H), 7.60 (s, 1H), 7.46 (t, *J* = 7.6 Hz, 2H), 7.39 (t, *J* = 7.8 Hz, 1H), 7.33 (t, *J* = 7.4 Hz, 1H), 7.25 (d, *J* = 7.6 Hz, 1H), 3.72 (d, *J* = 6.4 Hz, 1H), 2.39 (s, 3H); MS: m/z = 430 (M+1, ESI+)

### Example 12. Synthesis of (E)-4-(4-(2-benzylidenehydrazinyl)-7-(1-methyl-1H-pyrazol-4-yl)thieno[3,2-d]pyrimidin-2-yl)morpholine

The target compound (E)-4-(4-(2-benzylidenehydrazinyl)-7-(1-methyl-1H-pyrazol-4-yl)thieno[3,2-d]pyrimidin-2-yl)morpholine was obtained in the same manner as in Example 11 above, except that (1-methylpyrazol-4-yl)boronic acid was used instead of phenylboronic acid in (Step d).

¹H NMR (400 MHz, DMSO-*d*₆) *δ* 11.75 (s, 1H), 8.37 (s, 1H), 8.18 (s, 1H), 8.14 (s, 1H), 8.11 (s, 1H), 7.64 (d, *J* = 7.6 Hz, 1H), 7.59 (s, 1H), 7.39 (t, *J* = 7.6 Hz, 1H), 7.24 (d, *J* = 7.6 Hz, 1H), 3.91 (s, 3H), 3.74 (d, *J* = 5.2 Hz, 8H), 2.38 (s, 3H); MS: m/z =434(M+1, ESI+).

### Example 13. Synthesis of (E)-4-(4-(2-(3-methylbenzylidene)hydrazinyl)-1-(pyridin-3-yl)-1H-pyrazolo[3,4-d]pyrimidin-6-yl)morpholine

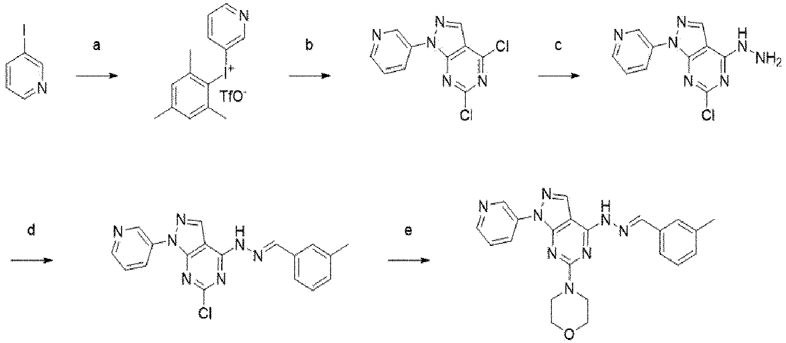

### (Step a) Synthesis of mesityl (pyridin-3-yl) iodonium trifluoromethane sulfonate

Dichloromethane (15 mL), 3-chlorobenzoperoxoic acid (1.26 g), and trifluoromethanesulfonic acid (2.93 g) were added to 3-iodopyridine (1 g) at 0°C, and the mixture was stirred at 25°C for 2 hours. After adding mesitylene (0.65 g) at 0°C, the mixture was stirred at 25°C for 2 hours. Distilled water was added to the reaction solution, then extracted with ethyl acetate, and concentrated. The concentrated product was subjected to silica chromatography under dichloromethane/methanol conditions to synthesize the target compound mesityl (pyridin-3-yl) iodonium trifluoromethanesulfonate (1 g) as a yellow solid in a yield of 63%. MS: m/z = 324.0 (M+1-TfO⁻, ESI+).

### (Step b) Synthesis of 4,6-dichloro-1-(pyridin-3-yl)-1H-pyrazolo[3,4-d]pyrimidine

To mesityl (pyridin-3-yl) iodonium trifluoromethanesulfonate (0.9 g), 4,6-dichloro-1H-pyrazolo[3,4-d]pyrimidine (0.262 g), triethylamine (0.211 g), copper bromide (0.04 g), and dichloromethane (15 mL) were added, and the mixture was stirred at 60°C for 2 hours. Distilled water was added to the reaction solution, then extracted with ethyl acetate, and concentrated. The concentrated product was subjected to silica chromatography under benzene/ethyl acetate conditions to synthesize the target compound 4,6-dichloro-1-(pyridin-3-yl)-1H-pyrazolo[3,4-d]pyrimidine (300 mg) as a yellow solid in a yield of 53%. MS: m/z = 266.0 (M+1, ESI+).

### (Step c) Synthesis of 6-chloro-4-hydrazinyl-1-(pyridin-3-yl)-1H-pyrazolo[3,4-d]pyrimidine

To 4,6-dichloro-1-(pyridin-3-yl)-1H-pyrazolo[3,4-d]pyrimidine (300 mg), hydrazine monohydrate (0.15 g) and tetrahydrofuran (1 mL) were added, and the mixture was stirred at room temperature for 3 hours. The reaction solution was concentrated under reduced pressure, and the resulting solid was filtered to synthesize the target compound 6-chloro-4-hydrazinyl-1-(pyridin-3-yl)-1H-pyrazolo[3,4-d]pyrimidine (110 mg) in a yield of 70.35%. MS: m/z = 262.1(M+1, ESI+).

### (Step d) Synthesis of (E)-6-chloro-4-(2-(3-methylbenzylidene)hydrazinyl)-1-(pyridin-3-yl)-1H-pyrazolo[3,4-d]pyrimidine

To 6-chloro-4-hydrazinyl-1-(pyridin-3-yl)-1H-pyrazolo[3,4-d]pyrimidine (110 mg), 3-methylbenzaldehyde (61 mg), acetic acid (3 µg), and methanol (5 mL) were added, and the mixture was stirred at 70°C for 3 hours. The reaction solution was concentrated under reduced pressure to synthesize the target compound (E)-6-chloro-4-(2-(3-methylbenzylidene)hydrazinyl)-1-(pyridin-3-yl)-1H-pyrazolo[3,4-d]pyrimidine (101 mg) in a yield of 66.04%. MS: m/z = 364.1(M+1, ESI+).

### (Step e) Synthesis of (E)-4-(4-(2-(3-methylbenzylidene)hydrazinyl)-1-(pyridin-3-yl)-1H-pyrazolo[3,4-d]pyrimidin-6-yl)morpholine

To (E)-6-chloro-4-(2-(3-methylbenzylidene)hydrazinyl)-1-(pyridin-3-yl)-1H-pyrazolo[3,4-d]pyrimidine (101 mg), morpholine (121 mg), acetic acid (2 µg), and isopropyl alcohol (5 mL) were added, and the mixture was stirred at 120°C for 2 hours. The resulting product was subjected to preparative HPLC to synthesize the target compound (E)-4-(4-(2-(3-methylbenzylidene)hydrazinyl)-1-(pyridin-3-yl)-1H-pyrazolo[3,4-d]pyrimidin-6-yl)morpholine (6.4 mg) as a yellow solid in a yield of 5.56%.

¹H NMR (400 MHz, DMSO-*d*₆) *δ* 11.92 - 11.65 (m, 1H), 9.52 - 9.39 (m, 1H), 8.67 - 8.56 (m, 1H), 8.54 - 8.46 (m, 1H), 8.43 (s, 1H), 8.21 (s, 1H), 7.68 - 7.52 (m, 3H), 7.43 - 7.33 (m, 1H), 7.29 - 7.22 (m, 1H), 3.80 (s, 4H), 3.70 (s, 4H), 2.39 (s, 3H); MS: m/z = 414.19 (M+1, ESI+).

### Example 14. Synthesis of (E)-4-(4-(2-(3-methylbenzylidene)hydrazinyl)-1-phenyl-1H-pyrazolo[3,4-d]pyrimidin-6-yl)morpholine

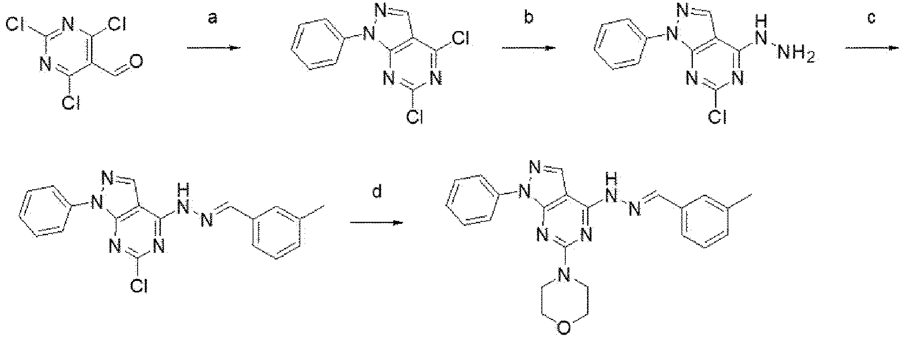

### (Step a) Synthesis of 4,6-dichloro-1-phenyl-1H-pyrazolo[3,4-d]pyrimidine

Ethanol (20 mL), phenylhydrazine (308 mg), and triethylamine (866 mg) were added to 2,4,6-trichloropyrimidine-5-carbaldehyde (600 mg) at -78°C, and stirred for 30 minutes. The reaction solution was additionally stirred at 0°C for 3 hours. Distilled water was added to the reaction solution and extracted with dichloromethane. After washing the organic solvent with a saturated NaCl solution, sodium sulfate was added, and the reaction mixture was filtered and concentrated. The concentrated product was subjected to silica chromatography under benzene/ethyl acetate conditions to synthesize the target compound 4,6-dichloro-1-phenyl-1H-pyrazolo[3,4-d]pyrimidine (500 mg) as a white solid in a yield of 66.28%.

¹H NMR (400 MHz, DMSO-*d*₆) *δ* 8.85 (s, 1H), 8.08 (d, *J* = 8.0 Hz, 2H), 7.68 (t, *J* = 7.6 Hz, 2H), 7.52 (t, *J* = 7.2 Hz, 1H). MS: m/z = 265.1 (M+1, ESI+).

### (Step b) Synthesis of 6-chloro-4-hydrazinyl-1-phenyl-1H-pyrazolo[3,4-d]pyrimidine

The synthesis was performed in the same manner as in (Step c) of Example 13 using 4,6-dichloro-1-phenyl-1H-pyrazolo[3,4-d]pyrimidine to obtain the target compound 6-chloro-4-hydrazinyl-1-phenyl-1H-pyrazolo[3,4-d]pyrimidine (420 mg) as a white solid in a yield of 94.77%. MS: m/z = 261.2(M+1, ESI+).

### (Step c) Synthesis of (E)-6-chloro-4-(2-(3-methylbenzylidene)hydrazinyl)-1-phenyl-1H-pyrazolo[3,4-d]pyrimidine

The target compound (E)-6-chloro-4-(2-(3-methylbenzylidene)hydrazinyl)-1-phenyl-1H-pyrazolo[3,4-d]pyrimidine was synthesized as a white solid (400 mg) in a yield of 68.40% in the same manner as in (Step d) of Example 13 using 6-chloro-4-hydrazinyl-1-phenyl-1H-pyrazolo[3,4-d]pyrimidine. MS: m/z = 363.1(M+1, ESI+).

### (Step d) Synthesis of (E)-4-(4-(2-(3-methylbenzylidene)hydrazinyl)-1-phenyl-1H-pyrazolo[3,4-d]pyrimidin-6-yl)morpholine

The target compound (E)-4-(4-(2-(3-methylbenzylidene)hydrazinyl)-1-phenyl-1H-pyrazolo[3,4-d]pyrimidin-6-yl)morpholine (100 mg) was synthesized as a white solid in a yield of 21.91% in the same manner as in (Step e) of Example 13 using (E)-6-chloro-4-(2-(3-methylbenzylidene)hydrazinyl)-1-phenyl-1H-pyrazolo[3,4-d]pyrimidine.

¹H NMR (400 MHz, DMSO-*d*₆) *δ* 11.77 (s, 1H), 8.40 (s, 1H), 8.30 - 8.19 (m, 3H), 7.63 - 7.56 (m, 2H), 7.53 (t, *J* = 8.0 Hz, 2H), 7.39 (t, *J* = 7.6 Hz, 1H), 7.32 - 7.22 (m, 2H), 3.87 - 3.76 (m, 4H), 3.75 - 3.64 (m, 4H), 2.40 (s, 3H); MS: m/z = 414.3 (M+1, ESI+).

### Example 15. Synthesis of (E)-4-(4-(2-(3-methylbenzylidene)hydrazinyl)-1-(pyridin-2-yl)-1H-pyrazolo[3,4-d]pyrimidin-6-yl)morpholine

The target compound (E)-4-(4-(2-(3-methylbenzylidene)hydrazinyl)-1-(pyridin-2-yl)-1H-pyrazolo[3,4-d]pyrimidin-6-yl)morpholine (35 mg) was synthesized as a yellow solid in a yield of 30.7% in the same manner as in Example 14, except that 2-hydrazinylpyridine was used instead of phenylhydrazine in (Step a) .

¹H NMR (400 MHz, DMSO-*d*₆) *δ* 11.99 (s, 1H), 9.34 (s, 1H), 8.57 (d, *J* = 4.4 Hz, 1H), 8.25 (s, 1H), 8.11 - 8.04 (m, 2H), 7.63 (s, 1H), 7.54 (d, *J* = 7.6 Hz, 1H), 7.47 - 7.38 (m, 2H), 7.29 (d, *J* = 7.2 Hz, 1H), 3.82 - 3.74 (m, 4H), 3.72 - 3.64 (m, 4H), 2.43 (s, 3H); MS: m/z = 415.3 (M+1, ESI+).

### Example 16. Synthesis of (E)-4-(4-(2-(3-methylbenzylidene)hydrazinyl)-1-(pyridin-4-yl)-1H-pyrazolo[3,4-d]pyrimidin-6-yl)morpholine

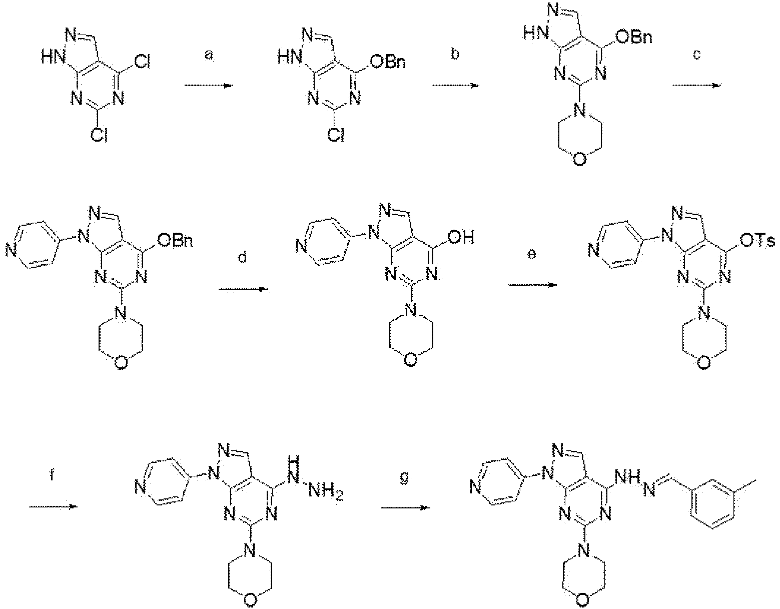

### (Step a) Synthesis of 4-(benzyloxy)-6-chloro-1H-pyrazolo[3,4-d]pyrimidine

Tetrahydrofuran (10 mL) and sodium hydride (267 mg) were added to phenylmethanol (720 mg), and the mixture was stirred at room temperature for 1 hour. 4,6-Dichloro-1H-pyrazolo[3,4-d]pyrimidine (1.05 g) dissolved in tetrahydrofuran (5 mL) was added and stirred at 70°C overnight. Distilled water was added to the reaction solution and extracted with ethyl acetate. After washing the organic solvent with a saturated NaCl solution, sodium sulfate was added, and the reaction mixture was filtered and concentrated. The concentrated product was subjected to silica chromatography under benzene/ethyl acetate conditions to synthesize the target compound 4-(benzyloxy)-6-chloro-1H-pyrazolo[3,4-d]pyrimidine (895 mg) as a yellow solid in a yield of 61.96%.

¹H NMR (400 MHz, DMSO) *δ* 7.92 (s, 1H), 7.49 (d, J = 7.2 Hz, 2H), 7.39 (t, J = 7.2 Hz, 2H), 7.36 - 7.25 (m, 2H), 5.47 (s, 2H). MS: m/z = 261.1(M+1, ESI+).

### (Step b) Synthesis of 4-(4-(benzyloxy)-1H-pyrazolo[3,4-d]pyrimidin-6-yl)morpholine

To 4-(benzyloxy)-6-chloro-1H-pyrazolo[3,4-d]pyrimidine (850 mg), isopropyl alcohol (20 mL), acetic acid (196 mg), and morpholine (1.42 g) were added, and the mixture was stirred at 120°C overnight. Distilled water was added to the reaction solution and extracted with ethyl acetate. After washing the organic solvent with a saturated NaCl solution, sodium sulfate was added, and the reaction mixture was filtered and concentrated. The concentrated product was subjected to silica chromatography under dichloromethane/methanol conditions to synthesize the target compound 4-(4-(benzyloxy)-1H-pyrazolo[3,4-d]pyrimidin-6-yl)morpholine (900 mg) as a yellow solid in a yield of 88.5%.

¹H NMR (400 MHz, DMSO-*d*6) *δ* 13.12 (s, 1H), 7.94 (s, 1H), 7.57 - 7.53 (m, 2H), 7.48 - 7.36 (m, 4H), 5.60 (s, 2H), 3.82 - 3.78 (m, 4H), 3.73 - 3.70 (m, 4H). MS: m/z = 312.1 (M+1, ESI+) .

### (Step c) Synthesis of 4-(4-(benzyloxy)-1-(pyridin-4-yl)-1H-pyrazolo[3,4-d]pyrimidin-6-yl)morpholine

To 4-(4-(benzyloxy)-1H-pyrazolo[3,4-d]pyrimidin-6-yl)morpholine (700 mg), triethylamine (338 mg), dichloromethane (20 mL), and mesityl(pyridin-4-yl)iodonium trifluoromethanesulfonate (1.39 g), and copper bromide (65 mg) were added and stirred at 60°C overnight. Distilled water was added to the reaction solution and extracted with dichloromethane. After washing the organic solvent with a saturated NaCl solution, sodium sulfate was added, and the reaction mixture was filtered and concentrated. The target compound 4-(4-(benzyloxy)-1-(pyridin-4-yl)-1H-pyrazolo[3,4-d]pyrimidin-6-yl)morpholine (350 mg) was synthesized as a brown solid in a yield of 40.08% through silica chromatography under dichloromethane/methanol conditions. MS: m/z = 389.1(M+1, ESI+).

### (Step d) Synthesis of 6-morpholino-1-(pyridin-4-yl)-1H-pyrazolo[3,4-d]pyrimidin-4-ol

To 4-(4-(benzyloxy)-1-(pyridin-4-yl)-1H-pyrazolo[3,4-d]pyrimidin-6-yl)morpholine (300 mg), dichloromethane (2 mL) and trifluoroacetic acid (2 mL) were added and stirred at room temperature for 4 hours. The reaction solution was concentrated under reduced pressure to synthesize the target compound 6-morpholino-1-(pyridin-4-yl)-1H-pyrazolo[3,4-d]pyrimidin-4-ol (230 mg) as a gray solid in a yield of 99.8%. MS: m/z = 299.0 (M+1, ESI+).

### (Step e) Synthesis of 6-morpholino-1-(pyridin-4-yl)-1H-pyrazolo[3,4-d]pyrimidin-4-yl 4-methylbenzenesulfonate

To 6-morpholino-1-(pyridin-4-yl)-1H-pyrazolo[3,4-d]pyrimidin-4-ol (230 mg), dichloromethane (10 mL), triethylamine (390 mg), and 4-toluenesulfonyl chloride (221 mg) were added, and the mixture was stirred at room temperature for 2 hours. Distilled water was added to the reaction solution and extracted with dichloromethane. After washing the organic solvent with a saturated NaCl solution, sodium sulfate was added, then the resulting mixture was filtered and concentrated to obtain the target compound 6-morpholino-1-(pyridin-4-yl)-1H-pyrazolo[3,4-d]pyrimidin-4-yl 4-methylbenzenesulfonate (265 mg) as a brown solid in a yield of 75.95%. MS: m/z = 453.0 (M+1, ESI+).

### (Step f) Synthesis of 4-(4-hydrazinyl-1-(pyridin-4-yl)-1H-pyrazolo[3,4-d]pyrimidin-6-yl)morpholine

To 6-morpholino-1-(pyridin-4-yl)-1H-pyrazolo[3,4-d]pyrimidin-4-yl 4-methylbenzenesulfonate (265 mg), tetrahydrofuran (5 mL) and hydrazine monohydrate (147 mg) were added, and the mixture was stirred at room temperature for 20 minutes. The reaction solution was concentrated under reduced pressure to synthesize the target compound 4-(4-hydrazinyl-1-(pyridin-4-yl)-1H-pyrazolo[3,4-d]pyrimidin-6-yl)morpholine (186 mg) as a yellow solid. MS: m/z = 313.1 (M+1, ESI+).

### (Step g) Synthesis of (E) -4- (4- (2- (3-methylbenzylidene)hydrazinyl)-1-(pyridin-4-yl)-1H-pyrazolo[3,4-d]pyrimidin-6-yl)morpholine

To 4-(4-hydrazinyl-1-(pyridin-4-yl)-1H-pyrazolo[3,4-d]pyrimidin-6-yl)morpholine (206 mg), methanol (5 mL) was added, followed by addition of acetic acid (12 mg) and 3-methylbenzaldehyde (158 mg), and the mixture was stirred at 70°C for 30 minutes. After concentrating the reaction solution under reduced pressure, the target compound (E)-4-(4-(2-(3-methylbenzylidene)hydrazinyl)-1-(pyridin-4-yl)-1H-pyrazolo[3,4-d]pyrimidin-6-yl)morpholine (40 mg) was synthesized as a yellow solid in a yield of 14.63% through silica chromatography under dichloromethane/methanol conditions.

¹H NMR (400 MHz, DMSO-*d*₆) *δ* 11.80 (s, 1H), 8.67 (s, 2H), 8.42 (s, 1H), 8.37 - 8.25 (m, 2H), 8.20 (s, 1H), 7.61 - 7.50 (m, 2H), 7.41 - 7.33 (m, 1H), 7.29 - 7.21 (m, 1H), 3.87 - 3.77 (m, 4H), 3.76 - 3.67 (m, 4H), 2.39 (s, 3H); MS: m/z = 415.3 (M+1, ESI+).

### Example 17. Synthesis of (E)-4-(9-isobutyl-6-(2-(3-methylbenzylidene)hydrazinyl)-9H-purin-2-yl)morpholine

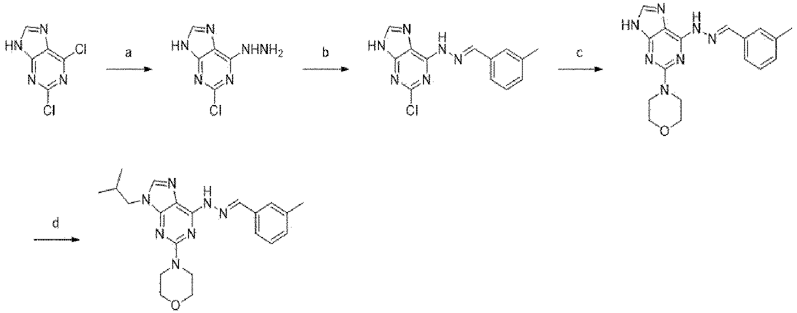

### (Step a) Synthesis of 2-chloro-6-hydrazinyl-9H-purine

2,6-Dichloro-9H-purine (1.0 g) and hydrazine monohydrate (515.8 µL) were dissolved in distilled water (10.6 mL) and stirred at 110°C for 1 hour. The reaction solution was filtered with distilled water to obtain the target compound 2-chloro-6-hydrazinyl-9H-purine (950 mg) as a yellow powder in a yield of 97%. MS: m/z = 185 (M+1, ESI+)

### (Step b) Synthesis of (E)-2-chloro-6-(2-(3-methylbenzylidene)hydrazinyl)-9H-purine

2-Chloro-6-hydrazinyl-9H-purine (950 mg) and 3-methylbenzaldehyde (620 µL) were dissolved in ethanol (52 mL), then a catalytic equivalent of acetic acid was added, and the mixture was stirred at 60°C for 1 hour. The reaction solution was filtered with ethyl acetate to obtain the target compound (E)-2-chloro-6-(2-(3-methylbenzylidene)hydrazinyl)-9H-purine (1.2 g) as a white powder in a yield of 79%. MS: m/z = 287 (M+1, ESI+)

### (Step c) Synthesis of (E)-4-(6-(2-(3-methylbenzylidene)hydrazinyl)-9H-purin-2-yl)morpholine

(E)-2-Chloro-6-(2-(3-methylbenzylidene)hydrazinyl)-9H-purine (1.2 g) was dissolved in morpholine (20.4 mL), and the mixture was stirred at 90°C for 16 hours. After the reaction solution was distilled under reduced pressure, the target compound (E)-4-(6-(2-(3-methylbenzylidene)hydrazinyl)-9H-purin-2-yl)morpholine (205 mg) was obtained as a white solid in a yield of 15% through silica chromatography under ethyl acetate/dichloromethane conditions. MS: m/z = 338 (M+1, ESI+)

### (Step d) Synthesis of (E)-4-(9-isobutyl-6-(2-(3-methylbenzylidene)hydrazinyl)-9H-purin-2-yl)morpholine

(E)-4-(6-(2-(3-Methylbenzylidene)hydrazinyl)-9H-purin-2-yl)morpholine (205 mg), 1-iodo-2-methylpropane (70 µL), and cesium carbonate (237.6 mg) were dissolved in dimethylformaldehyde (0.9 mL), and the mixture was stirred at 50°C for 17 hours. Saturated ammonium chloride solution was added to the reaction solution, extracted with ethyl acetate, and concentrated. The reaction mixture was subjected to silica chromatography under ethyl acetate/methanol conditions to obtain the target compound (E)-4-(9-isobutyl-6-(2-(3-methylbenzylidene)hydrazinyl)-9H-purin-2-yl)morpholine (43 mg) as an off-white solid in a yield of 18%.

¹H NMR (400 MHz, CDCl₃) *δ* 8.79 (s, 1H), 7.96 (s, 1H), 7.62-7.57 (m, 3H), 7.33 (t, *J* = 3.8 Hz, 1H), 7.23 (d, *J* = 14 Hz, 1H), 3.89-3.82 (m, 10H), 2.42 (s, 3H), 2.31-2.24 (m, 1H), 0.99 (s, 3H), 0.97 (s, 3H). MS: m/z = 394 (M+1, ESI+).

### Example 18. Synthesis of (E)-4-(6-(2-(3-methylbenzylidene)hydrazinyl)-9-phenethyl-9H-purin-2-yl)morpholine

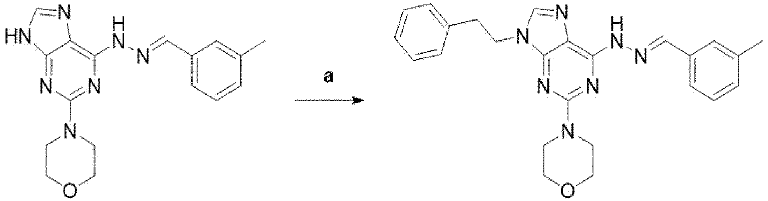

### (Step a) Synthesis of (E)-4-(6-(2-(3-methylbenzylidene)hydrazinyl)-9-phenethyl-9H-purin-2-yl)morpholine

The (E)-4-(6-(2-(3-methylbenzylidene)hydrazinyl)-9H-purin-2-yl)morpholine (100.0 mg) prepared in the same manner as in (step a) to (step c) of Example 17, (2-bromoethyl)benzene (33.4 µL), and cesium carbonate (96.6 mg) were dissolved in dimethylformaldehyde (0.35 mL) and stirred at 100°C for 18 hours. Saturated ammonium chloride solution was added to the reaction solution, extracted with ethyl acetate, and concentrated. The reaction mixture was subjected to silica chromatography under ethyl acetate/methanol conditions to obtain the target compound (E)-4-(6-(2-(3-methylbenzylidene)hydrazinyl)-9-phenethyl-9H-purin-2-yl)morpholine (56.6 mg) as an off-white solid in a yield of 51%.

¹H NMR (400 MHz, CDCl₃) *δ* 8.90 (s, 1H), 7.96 (s, 1H), 7.61 (s, 1H), 7.58 (d, *J* = 8 Hz, 2H), 7.34-7.27 (m, 2H), 7.26-7.23 (m, 1H), 7.21 (d, *J* = 7.6 hz, 1H), 7.13-7.11 (m, 2H), 4.33 (t, *J* = 7.2 Hz, 2H), 3.93-3.91 (m, 4H), 3.86-3.84 (m, 4H), 3.18 (t, *J* = 7.2 hz, 2H), 2.42 (s, 3H); MS: m/z = 442 (M+1, ESI+).

### Example 19. Synthesis of (E)-1-cyclopropyl-2-(6-(2-(3-methylbenzylidene)hydrazinyl)-2-morpholino-9H-purin-9-yl)ethan-1-one

The target compound (E)-1-cyclopropyl-2-(6-(2-(3-methylbenzylidene)hydrazinyl)-2-morpholino-9H-purin-9-yl)ethan-1-one was obtained as a yellow solid in the same manner as in Example 35 below, except that 2-bromo-1-cyclopropylethan-1-one was used instead of 4-(2-bromoacetyl)benzonitrile in (Step a).

¹H NMR (400 MHz, DMSO-*d*₆) *δ* 11.32 (s, 1H), 8.25 (s, 1H), 7.84 (s, 1H), 7.55 (d, *J* = 7.6 Hz, 1H), 7.49 (s, 1H), 7.32 (t, *J* = 7.6 Hz, 1H), 7.19 (d, *J* = 7.6 Hz, 1H), 5.20 (s, 2H), 3.68 (d, *J* = 3.2 Hz, 8H), 2.35 (s, 3H), 2.24 - 2.15 (m, 1H), 1.06 - 0.92 (m, 4H); MS: m/z = 420.2 (M+1, ESI+).

### Example 20. Synthesis of (E)-4-(6-(2-(3-methylbenzylidene)hydrazinyl)-9-(pyridin-2-ylmethyl)-9H-purin-2-yl)morpholine

The target compound (E)-4-(6-(2-(3-methylbenzylidene)hydrazinyl)-9-(pyridin-2-ylmethyl)-9H-purin-2-yl)morpholine was obtained as a white solid in the same manner as in Example 35 below, except that pyridin-2-ylethanesulfonate was used instead of 4-(2-bromoacetyl)benzonitrile in (Step a).

¹H NMR (400 MHz, DMSO-*d*₆) *δ* 11.38 (s, 1H), 8.58 (d, *J* = 4.4 Hz, 1H), 8.32 (s, 1H), 8.09 (s, 1H), 7.84 (td, *J* = 7.7, 1.4 Hz, 1H), 7.69 - 7.45 (m, 2H), 7.38 - 7.16 (m, 4H), 5.44 (s, 2H), 3.72 (d, *J* = 3.6 Hz, 10H), 2.41 (s, 3H); MS: m/z = 429.1 (M+1, ESI+).

### Example 21. Synthesis of (E)-4-(9-(cyclopropylmethyl)-6-(2-(3-methylbenzylidene)hydrazinyl)-9H-purin-2-yl)morpholine

The target compound (E)-4-(9-(cyclopropylmethyl)-6-(2-(3-methylbenzylidene)hydrazinyl)-9H-purin-2-yl)morpholine was obtained as a yellow solid in the same manner as in Example 35 below, except that (bromomethyl)cyclopropane was used instead of 4-(2-bromoacetyl)benzonitrile in (Step a).

¹H NMR (400 MHz, DMSO-*d*₆) *δ* 11.30 (s, 1H), 8.25 (s, 1H), 7.99 (s, 1H), 7.55 (d, *J* = 7.8 Hz, 1H), 7.49 (s, 1H), 7.32 (t, *J* = 7.6 Hz, 1H), 7.19 (d, *J* = 7.6 Hz, 1H), 3.90 (d, *J* = 7.1 Hz, 2H), 3.70 (d, *J* = 7.6 Hz, 8H), 2.35 (s, 3H), 1.28 (s, 1H), 0.52 (d, *J* = 7.4 Hz, 2H), 0.44 (d, *J* = 3.8 Hz, 2H); MS: m/z = 392.4 (M+1, ESI+).

### Example 22. Synthesis of (E)-2-(6-(2-(3-methylbenzylidene)hydrazinyl)-2-morpholino-9H-purin-9-yl)-1-phenylethan-1-one

The target compound (E)-2- (6-(2-(3-methylbenzylidene)hydrazinyl)-2-morpholino-9H-purin-9-yl)-1-phenylethan-1-one was obtained as a white solid in the same manner as in Example 35 below, except that 2-bromo-1-phenylethan-1-one was used instead of 4-(2-bromoacetyl)benzonitrile in (Step a).

¹H NMR (400 MHz, DMSO-*d*₆) *δ* 11.35 (s, 1H), 8.28 (s, 1H), 8.13 (d, *J* = 7.4 Hz, 2H), 7.91 (s, 1H), 7.75 (t, *J* = 7.4 Hz, 1H), 7.67 - 7.54 (m, 3H), 7.50 (s, 1H), 7.33 (t, *J* = 7.6 Hz, 1H), 7.19 (d, *J* = 7.4 Hz, 1H), 5.75 (s, 2H), 3.64 (d, *J* = 5.1 Hz, 9H), 2.36 (s, 3H); MS: m/z = 456.2 (M+1, ESI+) .

### Example 23. Synthesis of (E)-2-(6-(2-(3-methylbenzylidene)hydrazinyl)-2-morpholino-9H-purin-9-yl)-1-(pyridin-2-yl)ethan-1-one

The target compound (E)-2-(6-(2-(3-methylbenzylidene)hydrazinyl)-2-morpholino-9H-purin-9-yl)-1-(pyridin-2-yl)ethan-1-one was obtained as a white solid in the same manner as in Example 35 below, except that 2-bromo-1-(pyridin-2-yl)ethan-1-one was used instead of 4-(2-bromoacetyl)benzonitrile in (Step a).

¹H NMR (400 MHz, DMSO-*d*₆) *δ* 11.34 (s, 1H), 8.85 (d, *J* = 4.5 Hz, 1H), 8.28 (s, 1H), 8.06 (dd, *J* = 21.7, 7.6 Hz, 2H), 7.92 (s, 1H), 7.79 (s, 1H), 7.60 - 7.45 (m, 2H), 7.33 (s, 1H), 7.20 (d, *J* = 7.4 Hz, 1H), 5.84 (s, 2H), 3.63 (s, 8H), 2.34 (d, *J* = 13.4 Hz, 3H); MS: m/z = 457.2 (M+1, ESI+).

### Example 24. Synthesis of (E)-2-(6-(2-((1H-indol-3-yl)methylene)hydrazinyl)-2-morpholino-9H-purin-9-yl)-1-(pyridin-2-yl)ethan-1-one

The target compound (E)-2-(6-(2-((1H-indol-3-yl)methylene)hydrazinyl)-2-morpholino-9H-purin-9-yl)-1-(pyridin-2-yl)ethan-1-one was obtained as a green solid in the same manner as in Example 35 above, except that 2-bromo-1-(pyridin-2-yl)ethan-1-one was used instead of 4-(2-bromoacetyl)benzonitrile in (Step a) and 1H-indole-3-carbaldehyde was used instead of 3-methylbenzaldehyde in (Step c).

¹H NMR (400 MHz, DMSO-*d*₆) *δ* 11.40 (s, 1H), 11.08 (s, 1H), 8.85 (d, *J* = 4.4 Hz, 1H), 8.47 (s, 1H), 8.14 - 8.00 (m, 3H), 7.91 (s, 1H), 7.81 - 7.76 (m, 1H), 7.70 (s, 1H), 7.41 (d, J = 8.0 Hz, 1H), 7.18 (t, J = 7.4 Hz, 1H), 7.11 (t, J = 7.4 Hz, 1H), 5.84 (s, 2H), 3.67 (d, J = 11.3 Hz, 8H); MS: m/z = 482.1 (M+1, ESI+).

### Example 25. Synthesis of (E)-1-(4-chlorophenyl)-2-(6-(2-(3-methylbenzylidene)hydrazinyl)-2-morpholino-9H-purin-9-yl)ethan-1-one

The target compound (E)-1-(4-chlorophenyl)-2-(6-(2-(3-methylbenzylidene)hydrazinyl)-2-morpholino-9H-purin-9-yl)ethan-1-one was obtained as a yellow solid in the same manner as in Example 35 below, except that 2-bromo-1-(4-fluorophenyl)ethan-1-one was used instead of 4-(2-bromoacetyl)benzonitrile in (Step a).

¹H NMR (400 MHz, DMSO-*d*₆) *δ* 11.37 (s, 1H), 8.27 (s, 1H), 8.15 (dd, *J* = 6.8, 1.6 Hz), 7.91 (s, 1H), 7.76-7.70 (m, 2H), 7.58 (d, *J* = 7.6 Hz, 1H), 7.53 (s, 1H), 7.34 (t, *J* = 7.6 Hz, 1H), 7.20 (d, *J* = 7.6 Hz, 1H), 5.75 (s, 2H), 3.75-3.60 (m, 8H), 2.37 (s, 3H); MS: m/z = 490 (M+1, ESI+).

### Example 26. Synthesis of (E)-4-(9-(2,2-difluoro-2-(pyridin-2-yl)ethyl)-6-(2-(3-methylbenzylidene)hydrazinyl)-9H-purin-2-yl)morpholine

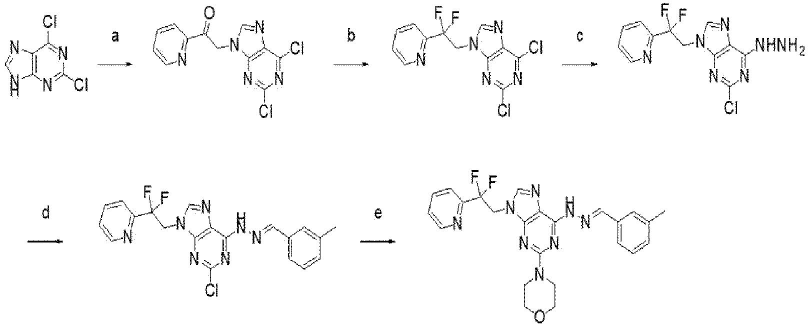

### (Step a) Synthesis of 2-(2,6-dichloro-9H-purin-9-yl)-1-(pyridin-2-yl)ethan-1-one

2,6-Dichloro-9H-purine (1.0 g) was dissolved in dimethylformamide (20.0 mL), then 2-(bromoacetyl)pyridine hydrobromide (1.5 g) and potassium carbonate (2.2 g) were added, and the mixture was stirred at room temperature for 2 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 2-(2,6-dichloro-9H-purin-9-yl)-1-(pyridin-2-yl)ethan-1-one (780.0 mg) as a yellow solid in a yield of 48.0%. MS: m/z = 308.1(M+1, ESI+)

### (Step b) Synthesis of 2,6-dichloro-9-(2,2-difluoro-2-(pyridin-2-yl)ethyl)-9H-purine

2-(2,6-Dichloro-9H-purin-9-yl)-1-(pyridin-2-yl)ethan-1-one (670.0 mg) was dissolved in dichloromethane (10.0 mL), and then diethylaminosulfur trifluoride (782.0 mg) was added at 0°C. The reaction solution was stirred at 0°C for 1 hour, and then distilled water was added. The reaction mixture was extracted with ethyl acetate, and concentrated. Then, the concentrated product was subjected to silica chromatography under ethyl acetate/benzene conditions to obtain the target compound 2,6-dichloro-9-(2,2-difluoro-2-(pyridin-2-yl)ethyl)-9H-purine (460.0 mg) as a yellow solid in a yield of 64.1%. MS: m /z = 330.1(M+1, ESI+)

The synthesis of (Step c) to (Step e) was performed in the same manner as in (Step b) to (Step d) of Example 35 to obtain the target compound (E)-4-(9-(2,2-difluoro-2-(pyridin-2-yl)ethyl)-6-(2-(3-methylbenzylidene)hydrazinyl)-9H-purin-2-yl)morpholine as a yellow solid.

¹H NMR (400 MHz, DMSO-*d*₆) *δ* 11.35 (s, 1H), 8.73 (s, 1H), 8.27 (s, 1H), 7.90 (s, 2H), 7.64 - 7.50 (m, 4H), 7.31 (s, 1H), 7.18 (s, 1H), 5.02 (s, 2H), 3.66 (s, 8H), 2.33 (s, 3H); MS: m/z = 479.3 (M+1, ESI+).

### Example 27. Synthesis of (E)-2-(6-(2-(3-methylbenzylidene)hydrazinyl)-2-morpholino-9H-purin-9-yl)-1-(pyrrolidin-1-yl)ethan-1-one

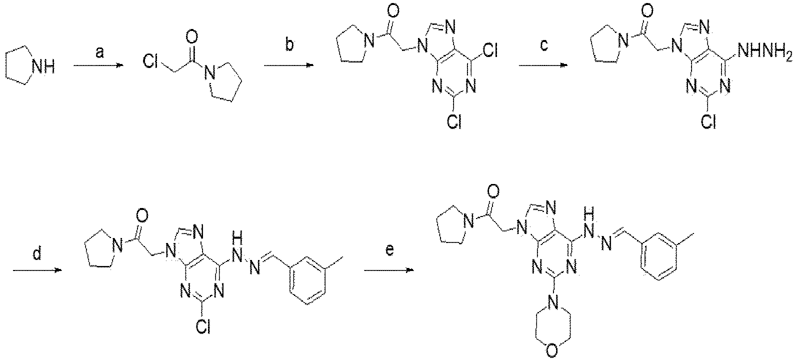

### (Step a) Synthesis of 2-chloro-1-(pyrrolidin-1-yl)ethan-1-one

Pyrrolidine (2.0 g) and triethylamine (8.52 g) were dissolved in dichloromethane (60 mL), then 2-chloroacetyl chloride (4.76 g) was added at 0°C, and the mixture was stirred for 30 minutes. After the reaction solution was concentrated under reduced pressure, distilled water was added, and the reaction mixture was extracted with dichloromethane and concentrated. Then, the target compound 2-chloro-1-(pyrrolidin-1-yl)ethan-1-one (4.23 g) as a white solid was used in the next reaction without separate purification. MS: m /z = 149.1 (M+2, ESI+).

The synthesis of (Step b) to (Step e) was performed in the same manner as (Step a) to (Step d) of Example 35 to obtain the target compound (E)-2-(6-(2-(3-methylbenzylidene)hydrazinyl)-2-morpholino-9H-purin-9-yl)-1-(pyrrolidin-1-yl)ethan-1-one as a white solid.

¹H NMR (400 MHz, DMSO-*d*₆) *δ* 11.31 (s, 1H), 8.27 (s, 1H), 7.84 (s, 1H), 7.57 (d, J = 8.0 Hz, 1H), 7.50 (s, 1H), 7.32 (t, *J* = 8.0 Hz, 1H), 7.18 (d, *J* = 8.0 Hz, 1H), 4.91 (s, 2H), 3.69 (dd, *J* = 15.4, 4.5 Hz, 8H), 3.58 (t, *J* = 6.8 Hz, 2H), 3.32 (t, *J* = 6.8 Hz, 2H), 2.34 (s, 3H), 1.99-1.90 (m, 2H), 1.82 - 1.76 (m, 2H); MS: m/z = 449.1 (M+1, ESI+).

### Example 28. Synthesis of (E)-2-(6-(2-(3-methylbenzylidene)hydrazinyl)-2-morpholino-9H-purin-9-yl)-1-(pyridin-3-yl)ethan-1-one

The target compound (E)-2-(6-(2-(3-methylbenzylidene)hydrazinyl)-2-morpholino-9H-purin-9-yl)-1-(pyridin-3-yl)ethan-1-one was obtained as a white solid in the same manner as in Example 35 below, except that 2-bromo-1-(pyridin-3-yl)ethan-1-one hydrobromide was used instead of 4-(2-bromoacetyl)benzonitrile in (Step a).

¹H NMR (400 MHz, DMSO-*d*₆) *δ* 11.35 (s, 1H), 9.29 (s, 1H), 8.89 (d, *J* = 4.0 Hz, 1H), 8.45 (d, *J* = 8.0 Hz, 1H), 8.27 (s, 1H), 7.91 (s, 1H), 7.66 (dd, *J* = 8.0, 4.0 Hz, 1H), 7.57 (d, *J* = 8.0 Hz, 1H), 7.50 (s, 1H), 7.33 (t, J = 8.0 Hz, 1H), 7.19 (d, *J* = 4.0 Hz, 1H), 5.79 (s, 2H), 3.64 (d, *J* = 8.0 Hz, 8H), 2.36 (s, 3H); MS: m/z = 457.9 (M+1, ESI+).

### Example 29. Synthesis of (E)-1-(indolin-1-yl)-2-(6-(2-(3-methylbenzylidene)hydrazinyl)-2-morpholino-9H-purin-9-yl)ethan-1-one

### (Step a) Synthesis of 2-chloro-1-(indolin-1-yl)ethan-1-one

Indoline (3.0 g) and triethylamine (3.06 g) were dissolved in dichloromethane (40 mL), and 2-chloroacetyl chloride (3.13 g) was added. After replacing the air inside the reactor with N₂, the reaction product was stirred at room temperature for 3 hours. After the reaction solution was concentrated under reduced pressure, distilled water was added, and the reaction mixture was extracted with dichloromethane and concentrated. Then, the concentrated product was subjected to silica chromatography under ethyl acetate/benzene conditions to obtain the target compound 2-chloro-1-(indolin-1-yl)ethan-1-one (4.0 g) as a yellow solid in a yield of 81%. MS: m/z = 196.0 (M+1, ESI+).

The synthesis of (Step b) to (Step e) was performed in the same manner as (Step a) to (Step d) of Example 35 to obtain the target compound (E)-1-(indolin-1-yl)-2-(6-(2-(3-methylbenzylidene)hydrazinyl)-2-morpholino-9H-purin-9-yl)ethan-1-one as a white solid.

¹H NMR (400 MHz, DMSO-*d*₆) *δ* 11.32 (s, 1H), 8.27 (s, 1H), 7.97 (d, J = 8.0 Hz, 1H), 7.90 (s, 1H), 7.56 (d, *J =* 8.0 Hz, 1H), 7.50 (s, 1H), 7.35 - 7.28 (m, 2H), 7.17 (dd, J = 16.4, 8.0 Hz, 2H), 7.04 (t, J = 7.2 Hz, 1H), 5.13 (s, 2H), 4.30 (t, J = 8.4 Hz, 2H), 3.74 - 3.68 (m, 4H), 3.68 - 3.62 (m, 4H), 3.26 (t, J = 8.4 Hz, 2H), 2.36 (s, 3H); MS: m/z = 497.2 (M+1, ESI+).

### Example 30. Synthesis of (E)-1-(1-methyl-1H-pyrrol-2-yl)-2-(6-(2-(3-methylbenzylidene)hydrazinyl)-2-morpholino-9H-purin-9-yl)ethan-1-one

### (Step a) Synthesis of 2-chloro-1-(1-methyl-1H-pyrrol-2-yl)ethan-1-one

2-Chloroacetyl chloride (4.18 g) was added to a mixed solution of 1-methyl-1H-pyrrole (3.0 g) and zinc oxide (752 mg) at 0°C, and the mixture was stirred at room temperature for 5 hours. After the reaction solution was concentrated under reduced pressure, distilled water was added, and the reaction mixture was extracted with dichloromethane and concentrated. Then, the concentrated product was subjected to silica chromatography under ethyl acetate/benzene conditions to obtain the target compound 2-chloro-1-(1-methyl-1H-pyrrol-2-yl)ethan-1-one (1.90 g) as a purple solid in a yield of 32%. MS: m /z = 158.1 (M+1, ESI+).

The synthesis of (Step b) to (Step e) was performed in the same manner as in (Step a) to (Step d) of Example 35 to obtain the target compound (E)-1-(1-methyl-1H-pyrrol-2-yl)-2-(6-(2-(3-methylbenzylidene)hydrazinyl)-2-morpholino-9H-purin-9-yl)ethan-1-one as a white solid.

¹H NMR (400 MHz, DMSO-*d*₆) *δ* 11.32 (s, 1H), 8.27 (s, 1H), 7.89 (s, 1H), 7.56 (d, *J* = 7.6 Hz, 1H), 7.50 (s, 1H), 7.39 (d, *J =* 4.0 Hz, 1H), 7.33 (t, *J =* 7.6 Hz, 1H), 7.25 (s, 1H), 7.19 (d, *J =* 7.6 Hz, 1H), 6.24 - 6.22 (m, 1H), 5.45 (s, 2H), 3.84 (s, 3H), 3.66 - 3.65 (m, 8H), 2.36 (s, 3H); MS: m/z = 459.2 (M+1, ESI+).

### Example 31. Synthesis of (E)-1-(2-(6-(2-(3-methylbenzylidene)hydrazinyl)-2-morpholino-9H-purin-9-yl)acetyl)azetidine-3-carbonitrile

### (Step a) Synthesis of 1-(2-chloroacetyl)azetidine-3-carbonitrile

To a mixed solution of azetidine-3-carbonitrile hydrochloride (2.00 g) and triethylamine (5.16 g) in dichloromethane (50 mL), 2-chloroacetyl chloride (2.86 g) was added. The reaction solution was stirred at 0°C for 2 hours. After the reaction solution was concentrated under reduced pressure, distilled water was added, and the reaction mixture was extracted with dichloromethane and concentrated. Then, the concentrated product was subjected to silica chromatography under ethyl acetate/benzene conditions to obtain the target compound 1-(2-chloroacetyl)azetidine-3-carbonitrile (2.0 g) as a yellow solid in a yield of 77%. MS: m/z = 159.1 (M+1, ESI+).

The synthesis of (Step b) to (Step e) was performed in the same manner as in (Step a) to (Step d) of Example 35 to obtain the target compound (E)-1-(2-(6-(2-(3-methylbenzylidene)hydrazinyl)-2-morpholino-9H-purin-9-yl)acetyl)azetidine-3-carbonitrile as a white solid.

¹H NMR (400 MHz, DMSO-*d*₆) *δ* 11.32 (s, 1H), 8.25 (s, 1H), 7.84 (s, 1H), 7.55 (d, *J* = 7.4 Hz, 1H), 7.48 (s, 1H), 7.32 (t, *J* = 7.3 Hz, 1H), 7.19 (d, *J* = 7.2 Hz, 1H), 4.76 (s, 2H), 4.57-4.52 (m, 2H), 4.21 (t, *J* = 8.0 Hz, 1H), 4.11-4.09 (m, 1H), 3.89-3.88 (m, 1H), 3.70 (d, *J* = 8.0 Hz, 8H), 2.35 (s, 3H); m/z = 460.2 (M+1, ESI+).

### Example 32. Synthesis of (E)-2-(6-(2-(3-methylbenzylidene)hydrazinyl)-2-morpholino-9H-purin-9-yl)-1-(pyridin-4-yl)ethan-1-one

The target compound (E)-2-(6-(2-(3-methylbenzylidene)hydrazinyl)-2-morpholino-9H-purin-9-yl)-1-(pyridin-4-yl)ethan-1-one as a white solid was obtained in the same manner as in Example 35 below, except that 2-bromo-1-(pyridin-4-yl)ethan-1-one was used instead of 4-(2-bromoacetyl)benzonitrile in (Step a).

¹H NMR (400 MHz, DMSO-*d*₆) *δ* 11.35 (s, 1H), 8.90 (d, *J* = 4.8 Hz, 2H), 8.28 (s, 1H), 7.98 (d, J = 4.4 Hz, 2H), 7.91 (s, 1H), 7.58 (d, *J* = 7.6 Hz, 1H), 7.51 (s, 1H), 7.33 (t, *J* = 7.6 Hz, 1H), 7.20 (d, *J* = 7.6 Hz, 1H), 5.78 (s, 2H), 3.65 - 3.64 (m, 8H), 2.36 (s, 3H); MS: m/z = 457.1 (M+1, ESI+).

### Example 33. Synthesis of (E)-1-(2-(6-(2-(3-methylbenzylidene)hydrazinyl)-2-morpholino-9H-purin-9-yl)acetyl)pyrrolidine-3-carbonitrile

### (Step a) Synthesis of pyrrolidine-3-carbonitrile

To a reaction solution of tert-butyl 3-cyanopyrrolidine-1-carboxylate (3.0 g) dissolved in dichloromethane (20 mL), trifluoroacetic acid (7 mL) was added, and the mixture was stirred at room temperature for 3 hours. The reaction solution was concentrated under reduced pressure to obtain the target compound pyrrolidine-3-carbonitrile (1.45 g) as a brown oil with a yield of 99%. MS: m/z = 97.2(M+1, ESI+).

### (Step b) Synthesis of 1-(2-chloroacetyl)pyrrolidine-3-carbonitrile

To a mixed solution of pyrrolidine-3-carbonitrile (1.30 g) and triethylamine (5.47 g) in dichloromethane (20 mL), 2-chloroacetyl chloride (1.68 g) was added. After replacing the air inside the reactor with N₂, the reaction product was stirred at room temperature for 3 hours. After the reaction solution was concentrated under reduced pressure, distilled water was added, and the reaction mixture was extracted with dichloromethane and concentrated. Then, the concentrated product was subjected to silica chromatography under ethyl acetate/benzene conditions to obtain the target compound 1-(2-chloroacetyl)pyrrolidine-3-carbonitrile (2.0 g) as a white solid in a yield of 86%. MS: m/z = 173.1(M+1, ESI+).

The synthesis of (Step c) to (Step f) was performed in the same manner as in (Step a) to (Step d) of Example 35 to obtain the target compound (E)-1-(2-(6-(2-(3-methylbenzylidene)hydrazinyl)-2-morpholino-9H-purin-9-yl)acetyl)pyrrolidine-3-carbonitrile as a white solid.

¹H NMR (400 MHz, DMSO-*d*₆) *δ* 11.30 (s, 1H), 8.26 (s, 1H), 7.84 (d, *J* = 2.8 Hz, 1H), 7.56 (d, *J* = 7.6 Hz, 1H), 7.49 (s, 1H), 7.32 (t, *J =* 7.6 Hz, 1H), 7.19 (d, J = 7.6 Hz, 1H), 5.08 - 4.86 (m, 2H), 4.04 - 3.85 (m, 1H), 3.77 - 3.41 (m, 12H), 2.41 - 2.06 (m, 5H); MS: m/z = 474.3 (M+1, ESI+).

### Example 34. Synthesis of (E)-2-(6-(2-(3-methylbenzylidene)hydrazinyl)-2-morpholino-9H-purin-9-yl)-1-(1H-pyrrol-2-yl)ethan-1-one

### (Step a) Synthesis of 2-chloro-1-(1H-pyrrol-2-yl)ethan-1-one

2-Chloroacetyl chloride (5.05 g) was added to a mixed solution of 1H-pyrrole (3.0 g) and zinc oxide (910 mg) at 0°C, and the mixture was stirred at room temperature for 5 hours. After the reaction solution was concentrated under reduced pressure, distilled water was added, and the reaction mixture was extracted with dichloromethane and concentrated. Then, the concentrated product was subjected to silica chromatography under ethyl acetate/benzene conditions to obtain the target compound 2-chloro-1-(1H-pyrrol-2-yl)ethan-1-one (600 mg) as a purple solid in a yield of 9%. MS: m /z = 144.1 (M+1, ESI+).

The synthesis of (Step b) to (Step e) was performed in the same manner as in (Step a) to (Step d) of Example 35 to obtain the target compound (E)-2-(6-(2-(3-methylbenzylidene)hydrazinyl)-2-morpholino-9H-purin-9-yl)-1-(1H-pyrrol-2-yl)ethan-1-one as a white solid.

¹H NMR (400 MHz, DMSO-*d*₆) *δ* 12.04 (s, 1H), 11.32 (s, 1H), 8.27 (s, 1H), 7.91 (s, 1H), 7.57 (d, *J* = 7.6 Hz, 1H), 7.50 (s, 1H), 7.33 (t, *J* = 7.6 Hz, 1H), 7.26 (s, 1H), 7.22 - 7.17 (m, 2H), 6.36 - 6.26 (m, 1H), 5.45 (s, 2H), 3.69 - 3.60 (m, 8H), 2.36 (s, 3H); MS: m/z = 445.3 (M+1, ESI+).

### Example 35. Synthesis of (E)-4-(2-(6-(2-(3-methylbenzylidene)hydrazinyl)-2-morpholino-9H-purin-9-yl)acetyl)benzonitrile

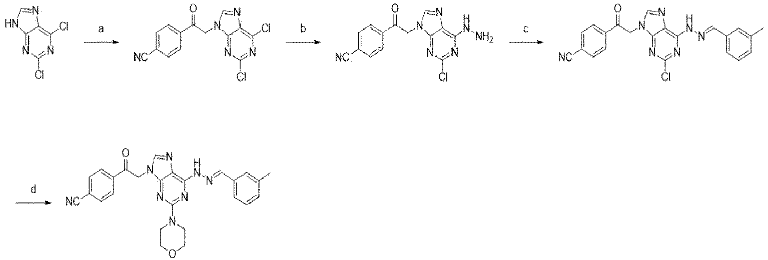

### (Step a) Synthesis of 4-(2-(2,6-dichloro-9H-purin-9-yl)acetyl)benzonitrile

2,6-Dichloro-9H-purine (2.0 g) and 4-(2-bromoacetyl)benzonitrile (2.85 g) were dissolved in dichloromethane (52.9 mL), triethylamine (4.42 mL) was added, and the mixture was stirred at 60°C for 2 hours. After the reaction solution was concentrated under reduced pressure, distilled water was added, and the reaction mixture was extracted with dichloromethane and concentrated. Then, the concentrated product was subjected to silica chromatography under ethyl acetate/hexane to obtain the target compound 4-(2-(2,6-dichloro-9H-purin-9-yl)acetyl)benzonitrile (2.11 g) as a white solid in a yield of 60.0%.

¹H NMR (400 MHz, DMSO-*d*₆) *δ* 8.66 (s, 1H), 8.30 - 8.25 (m, 2H), 8.16 - 8.10 (m, 2H), 6.13 (s, 2H).

### (Step b) Synthesis of 4-(2-(2-chloro-6-hydrazinyl-9H-purin-9-yl)acetyl)benzonitrile

4-(2-(2,6-dichloro-9H-purin-9-yl)acetyl)benzonitrile (2.11 g) was dissolved in tetrahydrofuran (12.7 mL), hydrazine monohydrate (619 µL) was added, and the mixture was stirred at 70°C for 1 hour. After the reaction solution was concentrated under reduced pressure, distilled water was added, and the reaction mixture was extracted with ethyl acetate, and concentrated. Then, the next reaction was performed without further purification.

### (Step c) Synthesis of (E)-4-(2-(2-chloro-6-(2-(3-methylbenzylidene)hydrazinyl)-9H-purin-9-yl)acetyl)benzonitrile

4-(2-(2-Chloro-6-hydrazinyl-9H-purin-9-yl)acetyl)benzonitrile (1.54 g) and 3-methylbenzaldehyde (1.54 mL) were dissolved in ethanol (47 mL) and stirred at 60°C for 3 hours. The reaction solution was concentrated under reduced pressure, followed by silica chromatography under ethyl acetate/hexane conditions to obtain the target compound (E)-4-(2-(2-chloro-6-(2-(3-methylbenzylidene)hydrazinyl)-9H-purin-9-yl)acetyl)benzonitrile (0.93 g) as a white solid in a yield of 46%.

¹H NMR (400 MHz, DMSO-*d*₆) *δ* 12.16 (s, 1H), 8.30 - 8.25 (m, 4H), 8.16 - 8.13 (m, 2H), 7.65 - 7. 55 (m, 2H), 7.37 (t, *J* = 8.0 Hz, 1H), 7.26 (d, *J* = 7.2 Hz, 1H), 6.01 (s, 2H), 2.38 (s, 3H).

### (Step d) Synthesis of (E)-4-(2-(6-(2-(3-methylbenzylidene)hydrazinyl)-2-morpholino-9H-purin-9-yl)acetyl)benzonitrile

(E)-4-(2-(2-chloro-6-(2-(3-methylbenzylidene)hydrazinyl)-9H-purin-9-yl)acetyl)benzonitrile (0.93 g) was dissolved in morpholine (9.3 mL) and stirred at 90°C for 5.5 hours. The reaction solution was concentrated under reduced pressure, followed by silica chromatography under ethyl acetate/hexane conditions to obtain the target compound (E)-4-(2-(6-(2-(3-methylbenzylidene)hydrazinyl)-2-morpholino-9H-purin-9-yl)acetyl)benzonitrile (150 mg) as an off-white solid in a yield of 14.4%.

¹H NMR (400 MHz, DMSO-*d*₆) *δ* 11.37 (s, 1H), 8.29 - 8.27 (m, 3H), 8.20 - 8.10 (m, 2H), 7.91 (s, 1H), 7.58 (d, J = 8.6 Hz, 1H), 7.51 (s, 1H), 7.34 (t, J = 7.6 Hz, 1H), 7.20 (d, J = 7.6 Hz, 1H), 5.80 (s, 2H), 3.70 - 3.60 (m, 8H), 2.36 (s, 3H); MS: m/z = 481 (M+1, ESI+).

### Example 36. Synthesis of (E)-3-(2-(6-(2-(3-methylbenzylidene)hydrazinyl)-2-morpholino-9H-purin-9-yl)acetyl)benzonitrile

The target compound (E)-3-(2-(6-(2-(3-methylbenzylidene)hydrazinyl)-2-morpholino-9H-purin-9-yl)acetyl)benzonitrile was obtained as an off-white solid in the same manner as in Example 35 above, except that 3-(2-bromoacetyl)benzonitrile was used instead of 4-(2-bromoacetyl)benzonitrile in (Step a).

¹H NMR (400 MHz, DMSO-*d*₆) *δ* 11.37 (s, 1H), 8.63 (s, 1H), 8.37 (dt, *J =* 1.4, 8.0 Hz, 1H), 8.27 (s, 1H), 8.21 (dt, *J* = 1.2, 7.6 Hz, 1H), 7.90 (s, 1H), 7.83 (t, *J =* 7.8 Hz, 1H), 7.57 (t, *J =* 7.6 Hz, 1H), 7.50 (s, 1H), 7.33 (t, *J =* 7.8 Hz, 1H), 7.19 (d, *J* = 7.6 Hz, 1H), 5.80 (s, 2H), 3.65 - 3.63 (m, 8H), 2.36 (s, 3H); MS: m/z = 481 (M+1, ESI+).

### Example 37. Synthesis of (E)-1-(2-methoxyphenyl)-2-(6-(2-(3-methylbenzylidene)hydrazinyl)-2-morpholino-9H-purin-9-yl) ethan-1-one

The target compound (E)-1-(2-methoxyphenyl)-2-(6-(2-(3-methylbenzylidene)hydrazinyl)-2-morpholino-9H-purin-9-yl)ethan-1-one was obtained as a yellow solid in the same manner as in Example 35 below, except that 2-bromo-1-(2-methoxyphenyl)ethan-1-one was used instead of 4-(2-bromoacetyl)benzonitrile in (Step a).

¹H NMR (400 MHz, DMSO-*d*₆) *δ* 11.33 (s, 1H), 8.27 (s, 1H), 7.91 (s, 1H), 7.76 (dd, *J* = 1.8, 7.8 Hz, 1H), 7.68 - 7.64 (m, 1H), 7.56 (d, *J* = 7.7 Hz, 1H), 7.50 (s, 1H), 7.33 (t, *J* = 7.6 Hz, 1H), 7.29 (d, *J* = 8.2 Hz, 1H), 7.19 (d, *J* = 7.5 Hz, 1H), 7.13 - 7.09 (m, 1H), 5.50 (s, 2H), 4.01 (s, 3H), 3.64 (s, 8H), 2.35 (s, 3H); MS: m/z = 486 (M+1, ESI+).

### Example 38. Synthesis of (E)-1-(3-methoxyphenyl)-2-(6-(2-(3-methylbenzylidene)hydrazinyl)-2-morpholino-9H-purin-9-yl)ethan-1-one

The target compound (E)-1-(3-methoxyphenyl)-2-(6-(2-(3-methylbenzylidene)hydrazinyl)-2-morpholino-9H-purin-9-yl)ethan-1-one was obtained as an off-white solid in the same manner as in Example 35 above, except that 2-bromo-1-(3-methoxyphenyl)ethan-1-one was used instead of 4-(2-bromoacetyl)benzonitrile in (Step a).

¹H NMR (400 MHz, DMSO-*d*₆) *δ* 11.36 (s, 1H), 8.27 (s, 1H), 7.90 (s, 1H), 7.73 (d, *J* = 8.0 Hz, 2H), 7.60 - 7.50 (m, 4H) 7.35 - 7.30 (m, 2H), 7.19 (d, J = 7.6 Hz, 1H), 5.74 (s, 2H), 3.85 (s, 3H), 3.64 - 3.63 (m, 8H), 2.36 (s, 3H); MS: m/z = 486 (M+1, ESI+).

### Example 39. Synthesis of (E)-1-(4-methoxyphenyl)-2-(6-(2-(3-methylbenzylidene)hydrazinyl)-2-morpholino-9H-purin-9-yl)ethan-1-one

The target compound (E)-1-(4-methoxyphenyl)-2-(6-(2-(3-methylbenzylidene)hydrazinyl)-2-morpholino-9H-purin-9-yl)ethan-1-one was obtained as a white solid in the same manner as in Example 35 above, except that 2-bromo-1-(3-methoxyphenyl)ethan-1-one was used instead of 4-(2-bromoacetyl)benzonitrile in (Step a).

¹H NMR (400 MHz, DMSO-*d*₆) *δ* 11.36 (s, 1H), 8.28 (s, 1H), 8.12 - 8.05 (m, 2H), 7.90 (s, 1H), 7.58 (d, *J* = 7.6 Hz, 1H), 7.51 (s, 1H), 7.34 (t, *J* = 7.6 Hz, 1H), 7.20 (d, *J =* 7.6 Hz, 1H), 7.20 - 7.10 (m, 2H), 5.69 (s, 2H), 3.89 (s, 3H), 3.85 - 3.55 (m, 8H), 2.36 (s, 3H); MS: m/z = 486 (M+1, ESI+).

### Example 40. Synthesis of (E)-2-(6-(2-(3-methylbenzylidene)hydrazinyl)-2-morpholino-9H-purin-9-yl)-1-(o-tolyl)ethan-1-one

The target compound (E)-2-(6-(2-(3-methylbenzylidene)hydrazinyl)-2-morpholino-9H-purin-9-yl)-1-(o-tolyl)ethan-1-one as a white solid was obtained in the same manner as in Example 35 above, except that 2-bromo-1-(o-tolyl)ethan-1-one was used instead of 4-(2-bromoacetyl)benzonitrile in (Step a).

¹H NMR (400 MHz, DMSO-*d*₆) *δ* 11.36 (s, 1H), 8.27 (s, 1H), 8.03 (d, *J* = 7.2 Hz, 1H), 7.93 (s, 1H), 7.59 - 7.45 (m, 3H), 7.45 - 7.30 (m, 3H), 7.20 (d, *J =* 7.6 Hz, 1H), 5.59 (s, 2H), 3.70 - 3.62 (m, 8H), 2.44 (s, 3H), 2.37 (s, 3H); MS: m/z = 470 (M+1, ESI+).

### Example 41. Synthesis of (E)-2-(6-(2-(3-methylbenzylidene)hydrazinyl)-2-morpholino-9H-purin-9-yl)-1-(m-tolyl)ethan-1-one

The target compound (E)-2-(6-(2-(3-methylbenzylidene)hydrazinyl)-2-morpholino-9H-purin-9-yl)-1-(m-tolyl)ethan-1-one as an off-white solid was obtained in the same manner as in Example 35 above, except that 2-bromo-1-(m-tolyl)ethan-1-one was used instead of 4-(2-bromoacetyl)benzonitrile in (Step a).

¹H NMR (400 MHz, DMSO-*d*₆) *δ* 11.35 (s, 1H), 8.27 (s, 1H), 7.94 - 7.90 (m, 3H), 7.56 (d, J = 8.4 Hz, 2H), 7.50 (t, *J* = 7.4 Hz, 2H), 7.33 (t, *J* = 7.6 Hz, 1H), 7.19 (d, *J* = 7.6 Hz, 1H), 5.72 (s, 2H), 3.64 - 3.63 (m, 8H), 2.43 (s, 3H), 2.36 (s, 3H); MS: m/z = 470 (M+1, ESI+).

### Example 42. Synthesis of (E)-2-(6-(2-(3-methylbenzylidene)hydrazinyl)-2-morpholino-9H-purin-9-yl)-1-(p-tolyl)ethan-1-one

The target compound (E)-2-(6-(2-(3-methylbenzylidene)hydrazinyl)-2-morpholino-9H-purin-9-yl)-1-(p-tolyl)ethan-1-one as an off-white solid was obtained in the same manner as in Example 35 above, except that 2-bromo-1-(p-tolyl)ethan-1-one was used instead of 4-(2-bromoacetyl)benzonitrile in (Step a).

¹H NMR (400 MHz, DMSO-*d*₆) *δ* 11.36 (s, 1H), 8.28 (s, 1H), 8.03 (d, *J* = 8.4 Hz, 1H), 7.58 (d, *J* = 7.2 Hz, 1H), 7.51 (s, 1H), 7.43 (d, *J* = 8.4 Hz, 1H), 7.34 (t, *J* = 7.2, 1H), 7.20 (d, *J* = 7.6 Hz, 1H), 5.72 (s, 2H), 3.38 - 3.60 (m, 8H), 2.43 (s, 3H), 2.37 (s, 3H); MS: m/z = 470 (M+1, ESI+).

### Example 43. Synthesis of (E)-2-(6-(2-(3-methylbenzylidene)hydrazinyl)-2-morpholino-9H-purin-9-yl)-1-(2-(trifluoromethyl)phenyl)ethan-1-one

The target compound (E)-2-(6-(2-(3-methylbenzylidene)hydrazinyl)-2-morpholino-9H-purin-9-yl)-1-(2-(trifluoromethyl)phenyl)ethan-1-one as an off-white solid was obtained in the same manner as in Example 35 above, except that 2-bromo-1-(2-(trifluoromethyl)phenyl)ethan-1-one was used instead of 4- (2-bromoacetyl) benzonitrile in (Step a).

¹H NMR (400 MHz, DMSO-*d*₆) *δ* 11.37 (s, 1H), 8.26 (s, 1H), 8.10 (d, *J* = 6.8 Hz, 1H), 7.94 - 7.89 (m, 3H), 7.82 (t, *J =* 7.4 Hz, 1H), 7.57 (d, *J* = 7.6 Hz, 1H) 7.50 (s, 1H), 7.33 (t, *J =* 7.6 Hz, 1H), 7.20 (d, *J =* 7.6 Hz, 1H), 5.59 (s, 2H), 3.71 - 3.66 (m, 8H), 2.36 (s, 3H); MS: m/z = 524 (M+1, ESI+).

### Example 44. Synthesis of (E)-2-(6-(2-(3-methylbenzylidene)hydrazinyl)-2-morpholino-9H-purin-9-yl)-1-(3-(trifluoromethyl)phenyl)ethan-1-one

The target compound (E)-2-(6-(2-(3-methylbenzylidene)hydrazinyl)-2-morpholino-9H-purin-9-yl)-1-(3-(trifluoromethyl)phenyl)ethan-1-one as a yellow solid was obtained in the same manner as in Example 35 above, except that 2-bromo-1-(3-(trifluoromethyl)phenyl)ethan-1-one was used instead of 4- (2-bromoacetyl) benzonitrile in (Step a).

¹H NMR (400 MHz, DMSO-*d*₆) *δ* 11.34 (s, 1H), 9.14 (d, J = 5.0 Hz, 1H), 8.26 (d, *J* = 11.1 Hz, 2H), 8.20 (s, 1H), 7.92 (s, 1H), 7.57 (d, *J* = 8.0 Hz, 1H), 7.50 (s, 1H), 7.32 (d, *J* = 7.6 Hz, 1H), 7.20 (d, *J* = 7.5 Hz, 1H), 5.86 (s, 2H), 3.63 (s, 8H), 2.36 (s, 3H); MS: m/z = 524 (M+1, ESI+).

### Example 45. Synthesis of (E)-2-(6-(2-(3-methylbenzylidene)hydrazinyl)-2-morpholino-9H-purin-9-yl)-1-(4-(trifluoromethyl)phenyl)ethan-1-one

The target compound (E)-2-(6-(2-(3-methylbenzylidene)hydrazinyl)-2-morpholino-9H-purin-9-yl)-1-(4-(trifluoromethyl)phenyl)ethan-1-one as a yellow solid was obtained in the same manner as in Example 35 above, except that 2-bromo-1-(4-(trifluoromethyl)phenyl)ethan-1-one was used instead of 4- (2-bromoacetyl) benzonitrile in (Step a).

¹H NMR (400 MHz, DMSO-*d*₆) *δ* 11.36 (s, 1H), 8.32 (d, J = 8.4 Hz, 1H), 8.28 (s, 1H), 8.01 (d, *J* = 8.4 Hz, 1H), 7.92 (s, 1H), 7.58 (d, *J* = 7.6 Hz, 1H), 7.51 (s, 1H), 7.34 (t, *J* = 7.6 Hz, 1H), 7.20 (d, *J* = 7.6 Hz, 1H), 5.81 (s, 2H), 3.70 - 3.60 (m, 8H), 2.37 (s, 3H); MS: m/z = 524 (M+1, ESI+).

### Example 46. Synthesis of (E)-1-(3-fluorophenyl)-2-(6-(2-(3-methylbenzylidene)hydrazinyl)-2-morpholino-9H-purin-9-yl)ethan-1-one

The target compound (E)-1-(3-fluorophenyl)-2-(6-(2-(3-methylbenzylidene)hydrazinyl)-2-morpholino-9H-purin-9-yl)ethan-1-one as a pink solid was obtained in the same manner as in Example 35 above, except that 2-bromo-1-(3-fluorophenyl)ethan-1-one was used instead of 4-(2-bromoacetyl)benzonitrile in (Step a).

¹H NMR (400 MHz, DMSO-*d*₆) *δ* 11.38 (s, 1H), 8.28 (s, 1H), 7.99 (d, *J* = 7.6 Hz, 1H), 7.96 - 7.90 (m, 2H), 7.73 - 7.55 (m, 3H), 7.51 (s, 1H), 7.34 (t, *J* = 7.6 Hz, 1H), 7.20 (d, *J =* 7.6 Hz, 1H), 5.77 (s, 2H), 3.70 - 3.60 (m, 8H), 2.37 (s, 3H); MS: m/z = 474 (M+1, ESI+).

### Example 47. Synthesis of (E)-1-(4-fluorophenyl)-2-(6-(2-(3-methylbenzylidene)hydrazinyl)-2-morpholino-9H-purin-9-yl)ethan-1-one

The target compound (E)-1-(4-fluorophenyl)-2-(6-(2-(3-methylbenzylidene)hydrazinyl)-2-morpholino-9H-purin-9-yl)ethan-1-one as a white solid was obtained in the same manner as in Example 35 above, except that 2-bromo-1-(4-fluorophenyl)ethan-1-one was used instead of 4-(2-bromoacetyl)benzonitrile in (Step a).

¹H NMR (400 MHz, DMSO-*d*₆) *δ* 11.37 (s, 1H), 8.28 - 8.20 (m, 3H), 7.91 (s, 1H), 7.58 (d, *J* = 8.0 Hz, 1H), 7.51 - 7.40 (m, 3H), 7.34 (t, *J =* 7.6 Hz, 1H), 7.20 (d, *J* = 7.6 Hz, 1H), 5.75 (s, 2H), 3.70 - 3.60 (m, 8H), 2.37 (s, 3H); MS: m/z = 474 (M+1, ESI+).

### Example 48. Synthesis of (E)-1-(2-chlorophenyl)-2-(6-(2-(3-methylbenzylidene)hydrazinyl)-2-morpholino-9H-purin-9-yl) ethan-1-one

The target compound (E)-1-(2-chlorophenyl)-2-(6-(2-(3-methylbenzylidene)hydrazinyl)-2-morpholino-9H-purin-9-yl)ethan-1-one was obtained as a white solid in the same manner as in Example 35 above, except that 2-bromo-1-(2-chlorophenyl)ethan-1-one was used instead of 4-(2-bromoacetyl)benzonitrile in (Step a).

¹H NMR (400 MHz, DMSO-*d*₆) *δ* 11.36 (s, 1H), 8.26 (s, 1H), 7.94 (s, 1H), 7.93 (d, 1H), 7.64-7.60 (m, 2H), 7.58 (m, 2H), 7.50 (s, 1H), 7.35-7.33 (t, 1H), 7.33 (d, 1H), 3.68-3.66 (m, 8H), 2.36 (s, 3H); MS: m/z = 491 (M+1, ESI+).

### Example 49. Synthesis of (E)-1-(3-chlorophenyl)-2-(6-(2-(3-methylbenzylidene)hydrazinyl)-2-morpholino-9H-purin-9-yl) ethan-1-one

The target compound (E)-1-(3-chlorophenyl)-2-(6-(2-(3-methylbenzylidene)hydrazinyl)-2-morpholino-9H-purin-9-yl)ethan-1-one was obtained as a yellow solid in the same manner as in Example 35 above, except that 2-bromo-1-(3-chlorophenyl)ethan-1-one was used instead of 4-(2-bromoacetyl)benzonitrile in (Step a).

¹H NMR (400 MHz, DMSO-*d*₆) *δ* 11.36 (s, 1H), 8.27 (s, 1H), 8.15 (t, J = 1.8 Hz, 1H), 8.07 (d, J = 8.0 Hz, 1H), 7.89 (s, 1H), 7.83 - 7.81 (m, 1H), 7.66 (t, J = 8.0 Hz, 1H), 7.57 (d, J = 7.6 Hz, 1H), 7.50 (s, 1H), 7.33 (t, J = 7.8 Hz, 1H), 7.19 (d, J = 7.6 Hz, 1H), 5.76 (s, 2H), 3.65 - 3.63 (m, 8H), 2.36 (s, 3H); MS: m/z = 491 (M+1, ESI+).

### Example 50. Synthesis of (E)-2-(6-(2-(3-methylbenzylidene)hydrazinyl)-2-morpholino-9H-purin-9-yl)-1-(3-(trifluoromethoxy)phenyl)ethan-1-one

The target compound (E)-2-(6-(2-(3-methylbenzylidene)hydrazinyl)-2-morpholino-9H-purin-9-yl)-1-(3-(trifluoromethoxy)phenyl)ethan-1-one as an off-white solid was obtained in the same manner as in Example 35 above, except that 2-bromo-1-(3-(trifluoromethoxy)phenyl)ethan-1-one was used instead of 4- (2-bromoacetyl) benzonitrile in (Step a) .

¹H NMR (400 MHz, DMSO-*d*₆) *δ* 11.38 (s, 1H), 8.27 (s, 1H), 8.21 - 8.15 (m, 1H), 8.04 (s, 1H), 7.79 (d, *J* = 4.4 Hz, 2H), 7.58 (d, *J* = 7.6 Hz, 1H), 7.51 (s, 1H), 7.34 (t, *J* = 7.6 Hz, 1H), 7.20 (d, *J* = 7.6 Hz, 1H), 5.79 (s, 2H), 3.69 - 3.60 (m, 8H), 2.37 (s, 3H); MS: m/z = 540 (M+1, ESI+).

### Example 51. Synthesis of (E)-1-(2,4-dimorpholinophenyl)-2-(6-(2-(3-methylbenzylidene)hydrazinyl)-2-morpholino-9H-purin-9-yl)ethan-1-one

The target compound (E)-1-(2,4-dimorpholinophenyl)-2-(6-(2-(3-methylbenzylidene)hydrazinyl)-2-morpholino-9H-purin-9-yl)ethan-1-one as beige solid was obtained in the same manner as in Example 35 above, except that 2-bromo-1-(2,4-difluorophenyl)ethan-1-one was used instead of 4-(2-bromoacetyl)benzonitrile in (Step a).

¹H NMR (400 MHz, DMSO-*d*₆) : *δ* 11.34 (s, 1H), 8.27 (s, 1H), 7.90 (s, 1H), 7.63 (d, J = 8.8 Hz, 1H), 7.57 (d, *J =* 7.6 Hz, 1H), 7.50 (s, 1H), 7.34 (t, J = 7.6 Hz, 1H), 7.20 (d, J = 7.2 Hz, 1H), 6.73 (dd, J = 9.2, 2.4 Hz, 1H), 6.65 - 6.60 (m, 1H), 5.65 (s, 2H), 3.81 (t, J = 4.0 Hz, 4H), 3.75 (t, J = 4.4 Hz, 4H), 3.35 - 3.30 (m, 4H), 3.01 (t, J = 4.4 Hz, 4H), 2.36 (s, 3H); MS: m/z = 626 (M+1, ESI+).

### Example 52. Synthesis of (E)-2-(6-(2-(3-methylbenzylidene)hydrazinyl)-2-morpholino-9H-purin-9-yl)-1-(4-(trifluoromethyl)pyridin-2-yl)ethan-1-one

The target compound (E)-2-(6-(2-(3-methylbenzylidene)hydrazinyl)-2-morpholino-9H-purin-9-yl)-1-(4-(trifluoromethyl)pyridin-2-yl)ethan-1-one as a yellow solid was obtained in the same manner as in Example 35 above, except that 2-bromo-1-(4-(trifluoromethyl)pyridin-2-yl)ethan-1-one was used instead of 4-(2-bromoacetyl)benzonitrile in (Step a).

¹H NMR (400 MHz, DMSO-*d*₆) *δ* 11.34 (s, 1H), 9.14 (d, J = 5.0 Hz, 1H), 8.26 (d, J = 11.1 Hz, 2H), 8.20 (s, 1H), 7.92 (s, 1H), 7.57 (d, *J* = 8.0 Hz, 1H), 7.50 (s, 1H), 7.32 (d, *J* = 7.6 Hz, 1H), 7.20 (d, *J* = 7.5 Hz, 1H), 5.86 (s, 2H), 3.63 (s, 8H), 2.36 (s, 3H); MS: m/z = 525.2 (M+1, ESI+).

### Example 53. Synthesis of (E)-1-(5-fluoropyridin-2-yl)-2-(6-(2-(3-methylbenzylidene)hydrazinyl)-2-morpholino-9H-purin-9-yl)ethan-1-one

The target compound (E)-1-(5-fluoropyridin-2-yl)-2-(6-(2-(3-methylbenzylidene)hydrazinyl)-2-morpholino-9H-purin-9-yl)ethan-1-one as a yellow solid was obtained in the same manner as in Example 35 above, except that 2-bromo-1-(5-fluoropyridin-2-yl)ethan-1-one was used instead of 4-(2-bromoacetyl)benzonitrile in (Step a).

¹H NMR (400 MHz, DMSO-*d*₆) *δ* 11.34 (s, 1H), 8.85 (d, J = 2.6 Hz, 1H), 8.28 (s, 1H), 8.14 (dd, *J* = 8.6, 4.7 Hz, 1H), 8.00 (td, *J* = 8.6, 2.8 Hz, 1H), 7.92 (s, 1H), 7.57 (d, *J* = 7.4 Hz, 1H), 7.50 (s, 1H), 7.32 (t, *J* = 7.5 Hz, 1H), 7.19 (d, *J* = 7.0 Hz, 1H), 5.81 (s, 2H), 3.63 (s, 8H), 2.35 (s, 3H); MS: m/z = 475.2 (M+1, ESI+).

### Example 54. Synthesis of (E)-2-(6-(2-(3-methylbenzylidene)hydrazinyl)-2-morpholino-9H-purin-9-yl)-1-(pyrazin-2-yl)ethan-1-one

The target compound (E)-2-(6-(2-(3-methylbenzylidene)hydrazinyl)-2-morpholino-9H-purin-9-yl)-1-(pyrazin-2-yl)ethan-1-one as a yellow solid was obtained in the same manner as in Example 35 above, except that 2-bromo-1-(pyrazin-2-yl)ethan-1-one was used instead of 4-(2-bromoacetyl)benzonitrile in (Step a).

¹H NMR (400 MHz, DMSO-*d*₆) *δ* 11.34 (s, 1H), 9.20 (d, *J* = 1.2 Hz, 1H), 9.02 (d, *J* = 2.4 Hz, 1H), 8.97 - 8.85 (m, 1H), 8.27 (s, 1H), 7.92 (s, 1H), 7.57 (d, *J* = 7.6 Hz, 1H), 7.50 (s, 1H), 7.33 (t, *J =* 7.6 Hz, 1H), 7.20 (d, *J =* 7.6 Hz, 1H), 5.82 (s, 2H), 3.68 - 3.60 (m, 8H), 2.36 (s, 3H); MS: m/z = 458.2 (M+1, ESI+).

### Example 55. Synthesis of (E)-2-(6-(2-((5-methylthiophen-2-yl)methylene)hydrazinyl)-2-morpholino-9H-purin-9-yl)-1-(pyridin-2-yl)ethan-1-one

The target compound (E)-2-(6-(2-((5-methylthiophen-2-yl)methylene)hydrazinyl)-2-morpholino-9H-purin-9-yl)-1-(pyridin-2-yl)ethan-1-one as a brown solid was obtained in the same manner as in Example 35 above, except that 2-bromo-1-(pyridin-2-yl)ethan-1-one was used instead of 4-(2-bromoacetyl)benzonitrile in (Step a) and 5-methylthiophene-2-carbaldehyde was used instead of 3-methylbenzaldehyde in (Step c).

¹H NMR (400 MHz, DMSO-*d*₆) *δ* 11.31 (s, 1H), 8.87 - 8.84 (m, 1H), 8.43 (s, 1H), 8.15 - 8.05 (m, 2H), 7.90 (s, 1H), 7.82 - 7.78 (m, 1H), 7.13 (d, *J* = 3.6 Hz, 1H), 6.82 - 6.80 (m, 1H), 5.84 (s, 2H), 3.70 - 3.58 (m, 8H), 2.47 (s, 3H); MS: m/z = 463 (M+1, ESI+).

### Example 56. Synthesis of (E)-3-((2-(2-morpholino-9-(2-oxo-2-(pyridin-2-yl)ethyl)-9H-purin-6-yl)hydrazinylidene)methyl)benzonitrile

The target compound (E)-3-((2-(2-morpholino-9-(2-oxo-2-(pyridin-2-yl)ethyl)-9H-purin-6-yl)hydrazinylidene)methyl)benzonitrile as a brown solid was obtained in the same manner as in Example 35 above, except that 2-bromo-1-(pyridin-2-yl)ethan-1-one was used instead of 4-(2-bromoacetyl)benzonitrile in (Step a) and 3-formylbenzonitrile was used instead of 3-methylbenzaldehyde in (Step c).

¹H NMR (400 MHz, DMSO-*d*₆) *δ* 11.63 (s, 1H), 8.89 - 8.80 (m, 1H), 8.31 (s, 1H), 8.15 - 8.05 (m, 4H), 7.97 (s, 1H), 7.86 - 7.75 (m, 2H), 7.67 (t, *J =* 8.0 Hz, 1H), 5.86 (s, 2H), 3.64 (s, 8H); MS: m/z = 468 (M+1, ESI+).

### Example 57. Synthesis of (E)-2-(2-morpholino-6-(2-(3-(trifluoromethyl)benzylidene)hydrazinyl)-9H-purin-9-yl)-1-(pyridin-2-yl)ethan-1-one

The target compound (E)-2-(2-morpholino-6-(2-(3-(trifluoromethyl)benzylidene)hydrazinyl)-9H-purin-9-yl)-1-(pyridin-2-yl)ethan-1-one as a yellow solid was obtained in the same manner as in Example 35 above, except that 2-bromo-1-(pyridin-2-yl)ethan-1-one was used instead of 4-(2-bromoacetyl)benzonitrile in (Step a) and 3-(trifluoromethyl)benzaldehyde was used instead of 3-methylbenzaldehyde in (Step c).

¹H NMR (400 MHz, DMSO-*d*₆) *δ* 11.62 (s, 1H), 8.86 - 8.84 (m, 1H), 8.35 (s, 1H), 8.10 - 8.03 (m, 4H), 7.95 (s, 1H), 7.81 - 7.77 (m, 1H), 7.74 - 7.68 (m, 2H), 5.85 (s, 2H), 3.63 (s, 8H); MS: m/z = 511 (M+1, ESI+).

### Example 58. Synthesis of (E)-2-(6-(2-(4-methylbenzylidene)hydrazinyl)-2-morpholino-9H-purin-9-yl)-1-(pyridin-2-yl)ethan-1-one

The target compound (E)-2-(6-(2-(4-methylbenzylidene)hydrazinyl)-2-morpholino-9H-purin-9-yl)-1-(pyridin-2-yl)ethan-1-one as a yellow solid was obtained in the same manner as in Example 35 above, except that 2-bromo-1-(pyridin-2-yl)ethan-1-one was used instead of 4-(2-bromoacetyl)benzonitrile in (Step a) and 4-methylbenzaldehyde was used instead of 3-methylbenzaldehyde in (Step c).

¹H NMR (400 MHz, DMSO-*d*₆) *δ* 11.32 (s, 1H), 8.86 - 8.84 (m, 1H), 8.27 (s, 1H), 8.10 - 8.08 (m, 1H), 8.05 - 8.02 (m, 1H), 7.92 (s, 1H), 7.81 - 7.77 (m, 1H), 7.62 (d, *J =* 8.0 Hz, 2H), 7.26 (d, *J* = 8.0 Hz, 2H), 5.84 (s, 2H), 3.62 (s, 8H), 2.34 (s, 3H); MS: m/z = 457 (M+1, ESI+).

### Example 59. Synthesis of (E)-2-(6-(2-(2-methylbenzylidene)hydrazinyl)-2-morpholino-9H-purin-9-yl)-1-(pyridin-2-yl)ethan-1-one

The target compound (E)-2-(6-(2-(2-methylbenzylidene)hydrazinyl)-2-morpholino-9H-purin-9-yl)-1-(pyridin-2-yl)ethan-1-one as a yellow solid was obtained in the same manner as in Example 35 above, except that 2-bromo-1-(pyridin-2-yl)ethan-1-one was used instead of 4-(2-bromoacetyl)benzonitrile in (Step a) and 2-methylbenzaldehyde was used instead of 3-methylbenzaldehyde in (Step c).

¹H NMR (400 MHz, DMSO-*d*₆) *δ* 11.35 (s, 1H), 8.86 - 8.84 (m, 1H), 8.62 (s, 1H), 8.10 (td, *J* = 1.7, 7.6 Hz, 1H), 8.03 (dt, *J* = 1.1, 7.8 Hz, 1H), 7.92 (s, 1H), 7.91 - 7.88 (m, 1H), 7.80 - 7.77 (m, 1H), 7.29 - 7.22 (m, 3H), 5.84 (s, 2H), 3.62 (s, 8H), 2.48 (s, 3H); MS: m/z = 457 (M+1, ESI+).

### Example 60. Synthesis of (E)-2-(2-morpholino-6-(2-(pyridin-3-ylmethylene)hydrazinyl)-9H-purin-9-yl)-1-(pyridin-2-yl)ethan-1-one

The target compound (E)-2-(2-morpholino-6-(2-(pyridin-3-ylmethylene)hydrazinyl)-9H-purin-9-yl)-1-(pyridin-2-yl)ethan-1-one as a brown solid was obtained in the same manner as in Example 35 above, except that 2-bromo-1-(pyridin-2-yl)ethan-1-one was used instead of 4-(2-bromoacetyl)benzonitrile in (Step a) and nicotinaldehyde was used instead of 3-methylbenzaldehyde in (Step c).

¹H NMR (400 MHz, DMSO-*d*₆) *δ* 11.60 (s, 1H), 8.88 (d, J = 1.7 Hz, 1H), 8.86 - 8.84 (m, 1H), 8.55 (dd, *J* = 1.6, 4.8 Hz, 1H), 8.32 (s, 1H), 8.15 (dt, *J* = 1.8, 8.0 Hz, 1H), 8.10 (td, *J* = 1.7, 7.6 Hz, 1H), 7.95 (s, 1H), 8.05 - 8.02 (m, 1H), 7.81 - 7.77 (m, 1H), 7.47 (q, *J* = 4.2 Hz, 1H), 5.85 (s, 2H), 3.63 (s, 8H); MS: m/z = 444 (M+1, ESI+).

### Example 61. Synthesis of (E)-2-(6-(2-(3-methoxybenzylidene)hydrazinyl)-2-morpholino-9H-purin-9-yl)-1-(pyridin-2-yl)ethan-1-one

The target compound (E)-2-(6-(2-(3-methoxybenzylidene)hydrazinyl)-2-morpholino-9H-purin-9-yl)-1-(pyridin-2-yl)ethan-1-one as a brown solid was obtained in the same manner as in Example 35 above, except that 2-bromo-1-(pyridin-2-yl)ethan-1-one was used instead of 4-(2-bromoacetyl)benzonitrile in (Step a) and 3-methoxybenzaldehyde was used instead of 3-methylbenzaldehyde in (Step c).

¹H NMR (400 MHz, DMSO-*d*₆) *δ* 11.43 (s, 1H), 8.85 (dt, J = 1.2, 4.6 Hz, 1H), 8.25 (s, 1H), 8.10 (td, J = 1.7, 7.7 Hz, 1H), 8.04 - 8.02 (m, 1H), 7.93 (s, 1H), 7.81 - 7.77 (m, 1H), 7.36 (t, *J* = 7.7 Hz, 1H), 7.32 - 7.29 (m, 2H), 6.97 - 6.94 (m, 1H), 5.84 (s, 2H), 3.81 (s, 3H), 3.62 (s, 8H); MS: m/z = 473 (M+1, ESI+).

### Example 62. Synthesis of 3-methyl-N'-(2-morpholino-9-(2-oxo-2-(pyridin-2-yl)ethyl)-9H-purin-6-yl)benzohydrazide

The synthesis was performed in the same manner as in Example 35 above, except that 2-bromo-1-(pyridin-2-yl)ethan-1-one was used instead of 4-(2-bromoacetyl)benzonitrile in (Step a), and (Step c) was performed as follows.

### (Step c) Synthesis of N'-(2-chloro-9-(2-oxo-2-(pyridin-2-yl)ethyl)-9H-purin-6-yl)-3-methylbenzohydrazide

To 2-(2-chloro-6-hydrazinyl-9H-purin-9-yl)-1-(pyridin-2-yl)ethan-1-one (320 mg), 3-methylbenzoic acid (172 mg), N,N-diisopropylethylamine (409 mg) and dimethylformamide (10 mg) were added, followed by addition of HATU (602 mg). After stirring at 25°C for 2 hours, distilled water and ethyl acetate were added and the organic solvent was extracted. Brine was added to the organic solution to extract the organic solvent, then sodium sulfate was added, and the mixture was filtered and concentrated under reduced pressure. The concentrated product was subjected to silica chromatography to synthesize the target compound N'-(2-chloro-9-(2-oxo-2-(pyridin-2-yl)ethyl)-9H-purin-6-yl)-3-methylbenzohydrazide (520 mg) as a yellow solid. MS: m/z =422.1(M+1, ESI+).

Then, the target compound 3-methyl-N'-(2-morpholino-9-(2-oxo-2-(pyridin-2-yl)ethyl)-9H-purin-6-yl)benzohydrazide as a yellow solid was obtained in the same manner as in (Step d) of Example 35.

¹H NMR (400 MHz, DMSO-*d*₆) *δ* 10.32 (s, 1H), 9.51 (s, 1H), 8.83 (d, *J* = 4.7 Hz, 1H), 8.16 (s, 1H), 8.09 (d, *J =* 1.5 Hz, 1H), 8.03 (s, 1H), 7.88 - 7.76 (m, 2H), 7.74 (s, 2H), 7.42 - 7.36 (m, 2H), 5.81 (s, 2H), 3.52 (s, 8H), 2.38 (s, 3H); MS: m/z = 473.2 (M+1, ESI+).

### Example 63. Synthesis of 2-(2-morpholino-6-(3-(pyridin-3-yl)-1H-pyrazol-1-yl)-9H-purin-9-yl)-1-(pyridin-2-yl)-ethan-1-one

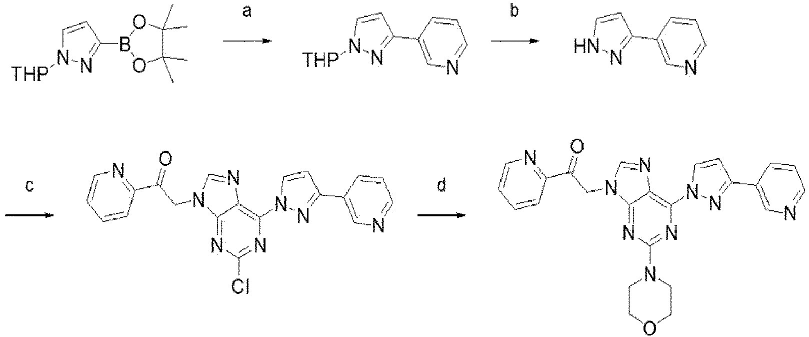

### (Step a) Synthesis of 3-(1-(tetrahydro-2H-pyran-2-yl)-1H-pyrazol-3-yl)pyridine

To 1-(tetrahydro-2H-pyran-2-yl)-3-(4,4,5,5-tetramethyl-1,3,2-dioxaborolan-2-yl)-1H-pyrazole (9.0 g), 3-bromopyridine (4.27 g), Pd(dppf)Cl₂ (1.98 g), potassium carbonate (9.32 g), 1,4-dioxane (30 mL), and distilled water (6 mL) were added, and the mixture was stirred at 120°C for 8 hours. The reaction solution was filtered, and distilled water and ethyl acetate were added to extract the organic solvent. Brine was added to the extracted organic solvent, the organic layer was extracted, and sodium sulfate was added thereto. The resulting product was subjected to silica chromatography under benzene/ethyl acetate conditions to synthesize the target compound 3-(1-(tetrahydro-2H-pyran-2-yl)-1H-pyrazol-3-yl)pyridine (6.5 g) as a white solid. MS: m/z =230.3(M+1, ESI+).

### (Step b) Synthesis of 3-(1H-pyrazol-3-yl)pyridine

To 3-(1-(tetrahydro-2H-pyran-2-yl)-1H-pyrazol-3-yl)pyridine (6.5 g), 4 M hydrochloric acid/1,4-dioxane (35 mL) using 1,4-dioxane as a solvent was added, and the mixture was stirred at 25°C for 1 hour. The reaction solution was filtered to synthesize the target compound 3-(1H-pyrazol-3-yl)pyridine (5.2 g) as a white solid. MS: m/z =146.1(M+1, ESI+).

### (Step c) Synthesis of 2-(2-chloro-6-(3-(pyridin-3-yl)-1H-pyrazol-1-yl)-9H-purin-9-yl)-1-(pyridin-2-yl)ethan-1-one

The target compound 2-(2-chloro-6-(3-(pyridin-3-yl)-1H-pyrazol-1-yl)-9H-purin-9-yl)-1-(pyridin-2-yl)ethan-1-one (320 mg) was synthesized as a yellow solid in a yield of 2.58% in the same manner as in (Step a) of Example 62, except using 3-(1H-pyrazol-3-yl)pyridine. MS: m/z =417.1(M+1, ESI+).

### (Step d) Synthesis of 2-(2-morpholino-6-(3-(pyridin-3-yl)-1H-pyrazol-1-yl)-9H-purin-9-yl)-1-(pyridin-2-yl)-ethan-1-one

The target compound 2-(2-morpholino-6-(3-(pyridin-3-yl)-1H-pyrazol-1-yl)-9H-purin-9-yl)-1-(pyridin-2-yl)ethan-1-one was synthesized as a yellow solid (178.5 mg) in a yield of 49.64% in the same manner as in (Step d) of Example 62, except using 2-(2-chloro-6-(3-(pyridin-3-yl)-1H-pyrazol-1-yl)-9H-purin-9-yl)-1-(pyridin-2-yl)ethan-1-one.

¹H NMR (400 MHz, DMSO-*d*₆) *δ* 9.24 - 9.21 (m, 2H), 8.86 (d, *J* = 4.2 Hz, 1H), 8.62 (dd, *J* = 4.8, 1.6 Hz, 1H), 8.38 (d, *J* = 8.0 Hz, 1H), 8.27 (s, 1H), 8.13 - 8.04 (m, 2H), 7.80 (s, 1H), 7.56 - 7.52 (m, 1H), 7.33 (d, J = 2.7 Hz, 1H), 5.96 (s, 2H), 3.75 (d, J = 4.3 Hz, 4H), 3.68 (d, J = 4.6 Hz, 4H). MS: m/z = 468.2 (M+1, ESI+).

### Example 64. Synthesis of 2-(2-morpholino-6-(3-(m-tolyl)-1H-pyrazol-1-yl)-9H-purin-9-yl)-1-(pyridin-2-yl)ethan-1-one

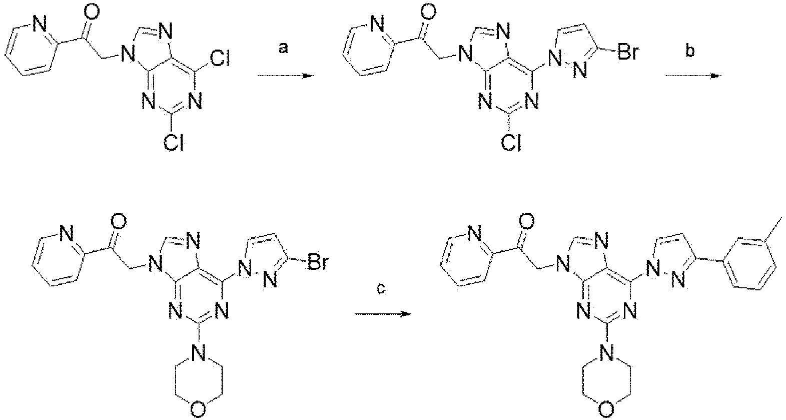

### (Step a) Synthesis of 2-(6-(3-bromo-1H-pyrazol-1-yl)-2-chloro-9H-purin-9-yl)-1-(pyridin-2-yl)ethan-1-one

3-Bromo-1H-pyrazole (570 mg) and sodium hydride (195 mg) were added to tetrahydrofuran (20 mL) and stirred at 0°C for 30 minutes. Then, 2-(2,6-dichloro-9H-purin-9-yl)-1-(pyridin-2-yl)ethan-1-one (1.0 g) was added and stirred at 0°C for 1 hour. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 2-(6-(3-bromo-1H-pyrazol-1-yl)-2-chloro-9H-purin-9-yl)-1-(pyridin-2-yl)ethan-1-one (600 mg) as a yellow solid in a yield of 44%. MS: m/z = 419.1 (M+1, ESI+).

### (Step b) Synthesis of 2-(6-(3-bromo-1H-pyrazol-1-yl)-2-morpholino-9H-purin-9-yl)-1-(pyridin-2-yl)ethan-1-one

2-(6-(3-Bromo-1H-pyrazol-1-yl)-2-chloro-9H-purin-9-yl)-1-(pyridin-2-yl)ethan-1-one (600 mg) and morpholine (3.0 mL) were dissolved in isopropyl alcohol (20 mL), acetic acid (0.2 mL) was added, and the mixture was stirred at 120°C for 12 hours. After the reaction was completed, the reaction solution was diluted with distilled water (10 mL) and the resulting solid was filtered to obtain the target compound 2-(6-(3-bromo-1H-pyrazol-1-yl)-2-morpholino-9H-purin-9-yl)-1-(pyridin-2-yl)ethan-1-one (400 mg) as a yellow solid in a yield of 66%. MS: m /z = 469.1 (M+1, ESI+).

### (Step c) Synthesis of 2-(2-morpholino-6-(3-(m-tolyl)-1H-pyrazol-1-yl)-9H-purin-9-yl)-1-(pyridin-2-yl)ethan-1-one

2-(6-(3-Bromo-1H-pyrazol-1-yl)-2-morpholino-9H-purin-9-yl)-1-(pyridin-2-yl)ethan-1-one (450 mg), m-tolylboronic acid (450 mg), Pd(dppf)Cl₂ (450 mg), and cesium carbonate (450 mg) were dissolved in 1,4-dioxane (10 mL) and distilled water (2 mL), and the mixture was stirred at 120°C for 3 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 2-(2-morpholino-6-(3-(m-tolyl)-1H-pyrazol-1-yl)-9H-purin-9-yl)-1-(pyridin-2-yl)ethan-1-one (94 mg) as a white solid in a yield of 14%.

¹H NMR (400 MHz, DMSO-*d*₆) *δ* 9.18 (d, *J* = 2.6 Hz, 1H), 8.86 (d, *J* = 4.1 Hz, 1H), 8.26 (s, 1H), 8.10 (d, *J* = 7.7 Hz, 1H), 8.05 (d, *J* = 7.2 Hz, 1H), 7.82 (d, *J* = 9.9 Hz, 3H), 7.38 (d, *J* = 7.5 Hz, 1H), 7.23 (d, *J* = 8.1 Hz, 1H), 7.19 (d, *J* = 2.6 Hz, 1H), 5.96 (s, 2H), 3.75 (s, 4H), 3.67 (s, 4H), 2.41 (s, 3H); MS: m/z = 481.5 (M+1, ESI+).

### Example 65. Synthesis of 2-(2-morpholino-6-(3-(3-(trifluoromethyl)phenyl)-1H-pyrazol-1-yl)-9H-purin-9-yl)-1-(pyridin-2-yl)ethan-1-one

The target compound 2-(2-morpholino-6-(3-(3-(trifluoromethyl)phenyl)-1H-pyrazol-1-yl)-9H-purin-9-yl)-1-(pyridin-2-yl)ethan-1-one (30 mg) was synthesized as a white solid in a yield of 8.76% in the same manner as in Example 64 above, except that (3-trifluorophenyl)boronic acid was used instead of m-tolylboronic acid in (Step c).

¹H NMR (400 MHz, DMSO-*d*₆) *δ* 9.21 (d, *J* = 2.8 Hz, 1H), 8.86 (d, *J* = 4.4 Hz, 1H), 8.37 - 8.31 (m, 2H), 8.28 (s, 1H), 8.14 - 8.08 (m, 1H), 8.08 - 8.02 (m, 1H), 7.82 - 7.74 (m, 3H), 7.39 (d, *J* = 2.8 Hz, 1H), 5.96 (s, 2H), 3.78 - 3.72 (m, 4H), 3.71 - 3.64 (m, 4H); MS: m/z = 535.2 (M+1, ESI+).

### Example 66. Synthesis of 2-(6-(3-(3-fluorophenyl)-1H-pyrazol-1-yl)-2-morpholino-9H-purin-9-yl)-1-(pyridin-2-yl)ethan-1-one

The target compound 2-(6-(3-(3-fluorophenyl)-1H-pyrazol-1-yl)-2-morpholino-9H-purin-9-yl)-1-(pyridin-2-yl)ethan-1-one (50 mg) was synthesized as a white solid in a yield of 16.1% in the same manner as in Example 64 above, except that (3-fluorophenyl)boronic acid was used instead of m-tolylboronic acid in (Step c).

¹H NMR (400 MHz, DMSO-*d*₆) *δ* 9.18 (d, *J* = 2.6 Hz, 1H), 8.85 (d, *J* = 4.4 Hz, 1H), 8.26 (s, 1H), 8.07 (dd, J = 19.3, 7.5 Hz, 2H), 7.88 (d, *J =* 7.7 Hz, 1H), 7.80 (t, J = 10.5 Hz, 2H), 7.55 (dd, J = 14.2, 7.8 Hz, 1H), 7.25 (dd, J = 9.2, 5.7 Hz, 2H), 5.95 (s, 2H), 3.74 (s, 4H), 3.67 (d, J = 3.9 Hz, 4H). MS: m/z = 485.2 M+1, ESI+).

### Example 67. Synthesis of 3-(1-(2-morpholino-9-(2-oxo-2-(pyridin-2-yl)ethyl)-9H-purin-6-yl)-1H-pyrazol-3-yl)benzonitrile

The target compound 3-(1-(2-morpholino-9-(2-oxo-2-(pyridin-2-yl)ethyl)-9H-purin-6-yl)-1H-pyrazol-3-yl)benzonitrile (25.3 mg) was synthesized as a white solid in a yield of 8.17% in the same manner as in Example 64 above, except that (3-cyanophenyl)boronic acid was used instead of m-tolylboronic acid in (Step c).

¹H NMR (400 MHz, DMSO-*d*₆) *δ* 9.20 (s, 1H), 8.85 (s, 1H), 8.43 (s, 1H), 8.37 (d, *J* = 7.4 Hz, 1H), 8.27 (s, 1H), 8.07 (dd, *J* = 19.2, 7.1 Hz, 2H), 7.88 (d, *J* = 7.2 Hz, 1H), 7.80 (s, 1H), 7.72 (t, *J* = 7.4 Hz, 1H), 7.35 (s, 1H), 5.95 (s, 2H), 3.74 (s, 4H), 3.67 (s, 4H). MS: m/z = 492.2 (M+1, ESI+).

### Example 68. Synthesis of 2-(6-(3-(3-methoxyphenyl)-1H-pyrazol-1-yl)-2-morpholino-9H-purin-9-yl)-1-(pyridin-2-yl)ethan-1-one

The target compound 2-(6-(3-(3-methoxyphenyl)-1H-pyrazol-1-yl)-2-morpholino-9H-purin-9-yl)-1-(pyridin-2-yl)ethan-1-one (18 mg) was synthesized as a white solid in a yield of 5.66% in the same manner as in Example 64 above, except that (3-methoxyphenyl)boronic acid was used instead of m-tolylboronic acid in (Step c).

¹H NMR (400 MHz, DMSO-*d*₆) *δ* 9.18 (d, *J* = 2.8 Hz, 1H), 8.86 (d, *J* = 4.4 Hz, 1H), 8.25 (s, 1H), 8.14 - 8.08 (m, 1H), 8.05 (d, *J* = 7.6 Hz, 1H), 7.84 - 7.77 (m, 1H), 7.61 (d, *J* = 8.0 Hz, 1H), 7.57 - 7.53 (m, 1H), 7.42 (t, J = 8.0 Hz, 1H), 7.23 (d, *J* = 2.4 Hz, 1H), 7.00 (dd, *J* = 8.0, 2.0 Hz, 1H), 5.96 (s, 2H), 3.85 (s, 3H), 3.78 - 3.72 (m, 4H), 3.71 - 3.64 (m, 4H); MS: m/z = 497.2 (M+1, ESI+).

### Example 69. Synthesis of 2-(2-morpholino-6-(3-(tetrahydro-2H-pyran-4-yl)-1H-pyrazol-1-yl)-9H-purin-9-yl)-1-(pyridin-2-yl)ethan-1-one

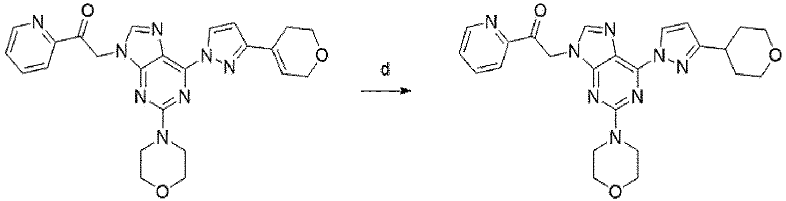

The target compound 2-(6-(3-(3,6-dihydro-2H-pyran-4-yl)-1H-pyrazol-1-yl)-2-morpholino-9H-purin-9yl)-1-(pyridin-2-yl)ethan-1-one (170 mg) was synthesized as a yellow solid in a yield of 56.28% in the same manner as in Example 64 above, except that 2-(3,6-dihydro-2H-pyran-4-yl)-4,4,5,5-tetramethyl-1, 3, 2-dioxaborolane was used in (Step c). MS: m/z = 473.2(M+1, ESI+).

### (Step d) Synthesis of 2-(2-morpholino-6-(3-(tetrahydro-2H-pyran-4-yl)-1H-pyrazol-1-yl)-9H-purin-9-yl)-1-(pyridin-2-yl)ethan-1-one

To 2-(6-(3-(3,6-dihydro-2H-pyran-4-yl)-1H-pyrazol-1-yl)-2-morpholino-9H-purin-9yl)-1-(pyridin-2-yl)ethan-1-one (150 mg), ethyl acetate and palladium carbonate (10%, 70 mg) were added, and the mixture was stirred at room temperature for 2 hours. The reaction solution was filtered and concentrated under reduced pressure. The target compound 2-(2-morpholino-6-(3-(tetrahydro-2H-pyran-4-yl)-1H-pyrazol-1-yl)-9H-purin-9-yl)-1-(pyridin-2-yl)ethan-1-one (13 mg) was synthesized as a white solid in a yield of 8.63% through silica chromatography under dichloromethane/methanol conditions.

¹H NMR (400 MHz, DMSO-*d*₆) *δ* 9.06 (d, *J* = 2.8 Hz, 1H), 8.52 (d, *J* = 4.8 Hz, 1H), 8.13 (s, 1H), 7.77 (t, *J* = 7.6 Hz, 1H), 7.43 (d, J = 8.0 Hz, 1H), 7.33 - 7.26 (m, 1H), 6.88 (d, *J* = 2.8 Hz, 1H), 6.51 (s, 1H), 6.00 (s, 1H), 5.15 - 5.02 (m, 1H), 4.55 (dd, *J* = 14.0, 4.4 Hz, 1H), 4.34 (dd, *J* = 14.0, 7.6 Hz, 1H), 4.26 (d, *J* = 2.8 Hz, 2H), 3.83 (t, *J* = 5.6 Hz, 2H), 3.71 (d, *J* = 4.8 Hz, 8H), 2.58 (s, 2H); MS: m/z = 475.2(M+1, ESI+).

### Example 70. Synthesis of 2-(6-(3-(6-methylpyridin-2-yl)-1H-pyrazol-1-yl)-2-morpholino-9H-purin-9-yl)-1-(pyridin-2-yl)ethan-1-one

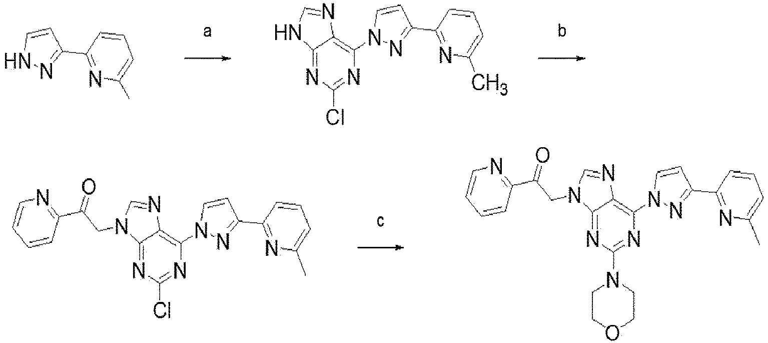

### (Step a) Synthesis of 2-chloro-6-(3-(6-methylpyridin-2-yl)-1H-pyrazol-1-yl)-9H-purine

Dimethylformamide (5 mL) was added to 2-methyl-6-(1H-pyrazol-3-yl)pyridine (0.5 g), and sodium hydride (0.5 g) was then added at 0°C. After adding 2,6-dichloro-9H-purine (0.6 g), the mixture was stirred at 50°C for 48 hours. After adding distilled water and ethyl acetate, the organic solvent was extracted. After concentrating the resulting solution under reduced pressure, the target compound 2-chloro-6-(3-(6-methylpyridin-2-yl)-1H-pyrazol-1-yl)-9H-purine (0.8 g) was synthesized as a yellow solid in a yield of 81.7% through silica chromatography under dichloromethane/methanol conditions. MS: m/z = 312.0 (M+1, ESI+).

### (Step b) Synthesis of 2-(2-chloro-6-(3-(6-methylpyridin-2-yl)-1H-pyrazol-1-yl)-9H-purin-9-yl)-1-(pyridin-2-yl)ethan-1-one

To 2-chloro-6-(3-(6-methylpyridin-2-yl)-1H-pyrazol-1-yl)-9H-purine (0.8 g), dichloromethane (5 mL), 2-bromo-1-(pyridin-2-yl)ethan-1-one (1.03 g), and triethylamine (1.04 g) were added, and the mixture was stirred at 25°C for 3 hours. After adding distilled water and ethyl acetate, the organic solvent was extracted. After concentrating the resulting solution under reduced pressure, the target compound 2-(2-chloro-6-(3-(6-methylpyridin-2-yl)-1H-pyrazol-1-yl)-9H-purin-9-yl)-1-(pyridin-2-yl)ethan-1-one (280 mg) was synthesized as a yellow solid in a yield of 25.3% through silica chromatography under dichloromethane/methanol conditions. MS: m/z = 431.0 (M+1, ESI+).

### (Step c) Synthesis of 2-(6-(3-(6-methylpyridin-2-yl)-1H-pyrazol-1-yl)-2-morpholino-9H-purin-9-yl)-1-(pyridin-2-yl)ethan-1-one

To 2-(2-chloro-6-(3-(6-methylpyridin-2-yl)-1H-pyrazol-1-yl)-9H-purin-9-yl)-1-(pyridin-2-yl)ethan-1-one (0.28 g), isopropyl alcohol (3 mL), morpholine (0.28 g), and acetic acid (4 µg) were added, and the mixture was stirred at 120°C for 2 hours. The resulting product was subjected to preparative HPLC to synthesize the target compound 2-(6-(3-(6-methylpyridin-2-yl)-1H-pyrazol-1-yl)-2-morpholino-9H-purin-9-yl)-1-(pyridin-2-yl)ethan-1-one (7 mg) in a yield of 2.2%.

¹H NMR (400 MHz, DMSO-*d*₆) *δ* 9.20 (d, J = 2.6 Hz, 1H), 8.86 (d, J = 4.6 Hz, 1H), 8.27 (s, 1H), 8.10 (t, J = 7.6 Hz, 1H), 8.05 (d, J = 7.6 Hz, 1H), 7.99 (d, J = 7.7 Hz, 1H), 7.86 - 7.77 (m, 2H), 7.29 (d, J = 7.6 Hz, 1H), 7.21 (d, J = 2.6 Hz, 1H), 5.96 (s, 2H), 3.75 (d, J = 4.0 Hz, 4H), 3.67 (d, J = 4.3 Hz, 4H), 2.56 (s, 3H); MS: m/z = 482.4 (M+1, ESI+).

### Example 71. Synthesis of 1-(2-morpholino-9-(2-oxo-2-(pyridin-2-yl)ethyl)-9H-purin-6-yl)-N-(m-tolyl)-1H-pyrazole-3-carboxamide

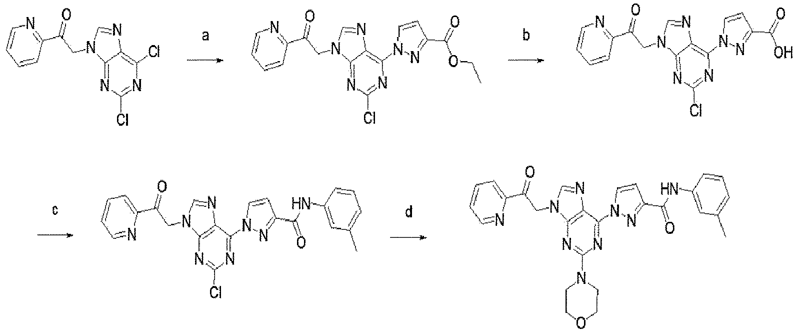

### (Step a) Synthesis of ethyl-1-(2-chloro-9-(2-oxo-2-(pyridin-2-yl)ethyl)-9H-purin-6-yl)-1H-pyrazole-3-carboxylate

Tetrahydrofuran (20 mL) and sodium hydride (208 mg) were added to 1H-pyrazole-3-carboxylate (546 mg) at 0°C, and stirred for 30 minutes. 2-(2,6-Dichloro-9H-purin-9-yl)-1-(pyridin-2-yl)ethan-1-one (1.0 g) was added, and the mixture was stirred at 25°C for 2 hours. Distilled water and dichloromethane were added to the reaction solution to extract the organic solvent. After adding NaCl solution to the organic solvent, the organic layer was extracted, and then sodium sulfate was added and filtered. The filtered product was subjected to silica chromatography to synthesize the target compound ethyl-1-(2-chloro-9-(2-oxo-2-(pyridin-2-yl)ethyl)-9H-purin-6-yl)-1H-pyrazole-3-carboxylate (320 mg) as a yellow solid in a yield of 24%. MS: m/z =411.1(M+1, ESI+) .

### (Step b) Synthesis of 1-(2-chloro-9-(2-oxo-2-(pyridin-2-yl)ethyl)-9H-purin-6-yl)-1H-pyrazole-3-carboxylic acid

To ethyl-1-(2-chloro-9-(2-oxo-2-(pyridin-2-yl)ethyl)-9H-purin-6-yl)-1H-pyrazole-3-carboxylate (300 mg), 12N HCl (5 mL) was added, and the mixture was stirred at 100°C for 1 hour. The reaction solution was concentrated under reduced pressure to synthesize the target compound 1-(2-chloro-9-(2-oxo-2-(pyridin-2-yl)ethyl)-9H-purin-6-yl)-1H-pyrazole-3-carboxylic acid (280 mg) as a yellow solid. MS: m/z =384.1 (M+1, ESI+).

### (Step c) Synthesis of 1-(2-chloro-9-(2-oxo-2-(pyridin-2-yl)ethyl)-9H-purin-6-yl)-N-(m-tolyl)-1H-pyrazole-3-carboxamide

To 1-(2-chloro-9-(2-oxo-2-(pyridin-2-yl)ethyl)-9H-purin-6-yl)-1H-pyrazole-3-carboxylic acid (260 mg), m-Toluidine (145 mg), N,N-diisopropylethylamine (438 mg), dimethylformamide (10 mL), and HATU (516 mg) were added, and the mixture was stirred at 25°C for 3 hours. After adding distilled water and ethyl acetate, the organic solvent was extracted. After adding NaCl solution to the organic solvent, the organic layer was extracted. Sodium sulfate was added, and the mixture was filtered and concentrated under reduced pressure. The concentrated product was subjected to silica chromatography to synthesize the target compound 1-(2-chloro-9-(2-oxo-2-(pyridin-2-yl)ethyl)-9H-purin-6-yl)-N-(m-tolyl)-1H-pyrazole-3-carboxamide (230 mg) as a white solid in a yield of 72.7%. MS: m/z =473.1(M+1, ESI+).

### (Step d) Synthesis of 1-(2-morpholino-9-(2-oxo-2-(pyridin-2-yl)ethyl)-9H-purin-6-yl)-N-(m-tolyl)-1H-pyrazole-3-carboxamide

To 1-(2-chloro-9-(2-oxo-2-(pyridin-2-yl)ethyl)-9H-purin-6-yl)-N-(m-tolyl)-1H-pyrazole-3-carboxamide (230 mg), acetic acid (0.05 mL), morpholine (424 mg), and isopropyl alcohol (15 mL) were added, and the mixture was stirred at 120°C for 8 hours. After adding distilled water and ethyl acetate, the organic solvent was extracted. After adding NaCl solution to the organic solvent, the organic layer was extracted. Sodium sulfate was added, and the mixture was filtered and concentrated under reduced pressure. The concentrated product was subjected to silica chromatography under dichloromethane/methanol conditions to synthesize the target compound 1-(2-morpholino-9-(2-oxo-2-(pyridin-2-yl)ethyl)-9H-purin-6-yl)-N-(m-tolyl)-1H-pyrazole-3-carboxamide (68.7 mg) in a yield of 27.3%.

¹H NMR (400 MHz, DMSO-*d*₆) *δ* 10.26 (s, 1H), 9.23 (d, J = 2.7 Hz, 1H), 8.86 (d, J = 4.7 Hz, 1H), 8.31 (s, 1H), 8.08 (dd, *J* = 20.9, 7.4 Hz, 2H), 7.81 (s, 1H), 7.67 (s, 1H), 7.62 (d, *J* = 7.5 Hz, 1H), 7.25 (t, *J* = 7.8 Hz, 1H), 7.15 (d, *J* = 2.7 Hz, 1H), 6.95 (d, *J* = 7.3 Hz, 1H), 5.97 (s, 2H), 3.77 (s, 4H), 3.67 (d, *J* = 5.1 Hz, 4H), 2.33 (s, 3H); MS: m/z = 525.2 (M+1, ESI+).

### Example 72. Synthesis of (E) -1- (azetidin-1-yl) -2- (6-(2-(3-methylbenzylidene)hydrazinyl)-2-morpholino-9H-purin-9-yl) ethan-1-one

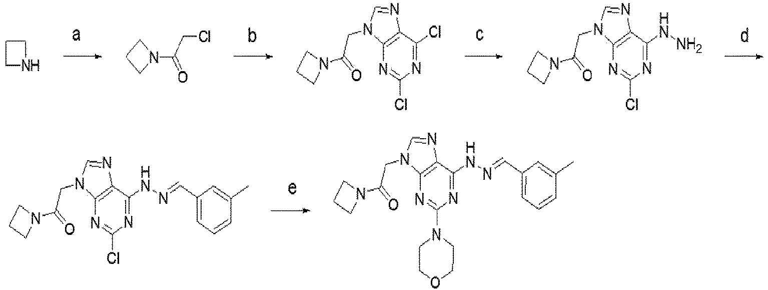

### (Step a) Synthesis of 1-(azetidin-1-yl)-2-chloroethan-1-one

After dissolving azetidine (2.0 g) and triethylamine (5.31 g) in dichloromethane (40 mL), 2-chloroacetyl chloride (2.97 g) was added at 0°C, and the mixture was stirred for 30 minutes. After the reaction solution was concentrated under reduced pressure, distilled water was added, and the reaction mixture was extracted with dichloromethane and concentrated. Then, the concentrated product was subjected to silica chromatography under ethyl acetate/benzene conditions to obtain the target compound 1-(azetidin-1-yl)-2-chloroethan-1-one (4.23 g) as a yellow solid, which was used in the following reaction without further purification. MS: m /z = 134.1 (M+2, ESI+).

The synthesis of (Step b) to (Step e) was performed in the same manner as in (Step a) to (Step d) of Example 35 to obtain the target compound (E)-1-(azetidin-1-yl)-2-(6-(2-(3-methylbenzylidene)hydrazinyl)-2-morpholino-9H-purin-9-yl)ethan-1-one as a white solid.

¹H NMR (400 MHz, DMSO-*d*₆) *δ* 11.31 (s, 1H), 8.25 (s, 1H), 7.85 (s, 1H), 7.54 (s, 1H), 7.49 (s, 1H), 7.31 (d, J = 7.6 Hz, 1H), 7.20 (s, 1H), 4.72 (s, 2H), 4.28 (t, J = 7.7 Hz, 2H), 3.92 (t, J = 7.7 Hz, 2H), 3.73 - 3.67 (m, 8H), 2.35 (s, 3H), 2.30 - 2.24 (m, 2H); MS: m/z = 435.1 (M+1, ESI+).

### Example 73. Synthesis of (E)-4-(6-(2-(3-methylbenzylidene)hydrazinyl)-9-(2-(pyridin-2-yl)ethyl)-9H-purin-2-yl)morpholine

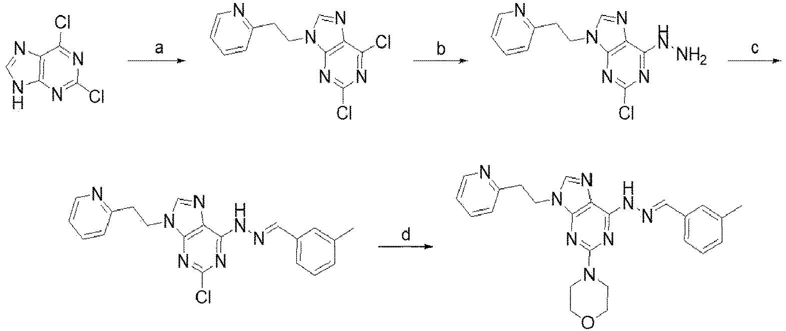

### (Step a) Synthesis of 2,6-dichloro-9-(2-(pyridin-2-yl)ethyl)-9H-purine

2,6-Dichloro-9H-purine (1.0 g), 2-(pyridin-2-yl)ethan-1-ol (723 mg), and triphenylphosphine (2.08 g) were dissolved in tetrahydrofuran (20 mL), and after cooling, diisopropyl azodicarboxylate (1.6 g) was added at 0°C. The reaction solution was stirred at 50°C for 2 hours, distilled water was added, and the resulting compound was extracted with ethyl acetate. After concentrating the organic layer under reduced pressure, the target compound 2,6-dichloro-9-(2-(pyridin-2-yl)ethyl)-9H-purine (1.1 g) was obtained as a white solid in a yield of 70% through silica chromatography under ethyl acetate/benzene conditions. MS: m/z = 294.0 (M+1, ESI+) .

### (Step b) Synthesis of 2-chloro-6-hydrazinyl-9-(2-(pyridin-2-yl)ethyl)-9H-purine

2,6-Dichloro-9-(2-(pyridin-2-yl)ethyl)-9H-purine (1.0 g) and hydrazine monohydrate (10 mL) were dissolved in isopropyl alcohol (10 mL), and the mixture was stirred at room temperature for 4 hours. After concentrating the reaction solution under reduced pressure, the target compound 2-chloro-6-hydrazinyl-9-(2-(pyridin-2-yl)ethyl)-9H-purine (700 mg) was obtained as a white solid in a yield of 65% through silica chromatography under dichloromethane/methanol conditions. MS: m/z = 290.1 (M+1, ESI+) .

### (Step c) Synthesis of (E)-2-chloro-6-(2-(3-methylbenzylidene)hydrazinyl)-9-(2-(pyridin-2-yl)ethyl)-9H-purine

2-Chloro-6-hydrazinyl-9-(2-(pyridin-2-yl)ethyl)-9H-purine (700 mg), 3-methylbenzaldehyde (348 mg), and acetic acid (0.1 mL) were dissolved in methanol (10 mL), and the mixture was stirred at 80°C for 1 hour. After concentrating the reaction solution under reduced pressure, the target compound (E)-2-chloro-6-(2-(3-methylbenzylidene)hydrazinyl)-9-(2-(pyridin-2-yl)ethyl)-9H-purine (800 mg) was obtained as a white solid in a yield of 85% through silica chromatography under dichloromethane/methanol conditions. MS: m/z = 392.2 (M+1, ESI+).

### (Step d) Synthesis of (E)-4-(6-(2-(3-methylbenzylidene)hydrazinyl)-9-(2-(pyridin-2-yl)ethyl)-9H-purin-2-yl)morpholine

(E)-2-Chloro-6-(2-(3-methylbenzylidene)hydrazinyl)-9-(2-(pyridin-2-yl)ethyl)-9H-purine (100 mg) and morpholine (111 mg) were dissolved in isopropyl alcohol (1.0 mL), then acetic acid (0.1 µL) was added, and the mixture was stirred at 120°C for 18 hours. Distilled water was added to the reaction solution, and the compound was extracted into the organic layer using ethyl acetate. After concentrating the organic layer under reduced pressure, the target compound (E)-4-(6-(2-(3-methylbenzylidene)hydrazinyl)-9-(2-(pyridin-2-yl)ethyl)-9H-purin-2-yl)morpholine (25 mg) was obtained as a white solid in a yield of 22% through silica chromatography under dichloromethane/methanol conditions.

¹H NMR (400 MHz, CDCl₃) *δ* 8.96 (s, 1H), 8.58 (d, J = 4.8 Hz, 1H), 7.93 (s, 1H), 7.58-7.48 (m, 3H), 7.34-7.27 (m, 2H), 7.19-7.12 (m, 2H), 6.94 (d, *J* = 7.7 Hz, 1H), 4.54 (t, *J* = 6.7 Hz, 2H), 3.92-3.85 (m, 4H), 3.85-3.80 (m, 4H), 3.34 (t, *J* = 6.8 Hz, 2H), 2.39 (s, 3H). MS: m/z = 443.0 (M+1, ESI+) .

### Example 74. Synthesis of (E)-4-(6-(2-(3-methylbenzylidene)hydrazinyl)-9-((3-methyloxetan-3-yl)methyl)-9H-purin-2-yl)morpholine

The target compound (E)-4-(6-(2-(3-methylbenzylidene)hydrazinyl)-9-((3-methyloxetan-3-yl)methyl)-9H-purin-2-yl)morpholine as a white solid was obtained in the same manner as in Example 73 above, except that (3-methyloxetan-3-yl)methanol was used instead of 2-(pyridin-2-yl)ethan-1-ol in (Step a).

¹H NMR (400 MHz, DMSO-d₆) *δ* 11.32 (s, 1H), 8.25 (s, 1H), 7.95 (s, 1H), 7.55 (d, *J* = 7.8 Hz, 1H), 7.49 (s, 1H), 7.31 (d, *J* = 7.6 Hz, 1H), 7.19 (s, 1H), 4.69 (d, *J* = 5.9 Hz, 2H), 4.30 (s, 2H), 4.25 (d, *J* = 5.9 Hz, 2H), 3.69 (d, *J =* 4.0 Hz, 8H), 2.35 (s, 3H), 1.18 (s, 3H); MS: m/z = 422.3 (M+1, ESI+)

### Example 75. Synthesis of (E)-4-(2-(6-(2-(3-methylbenzylidene)hydrazinyl)-2-morpholino-9H-purin-9-yl)ethylmorpholine

The target compound (E)-4-(2-(6-(2-(3-methylbenzylidene)hydrazinyl)-2-morpholino-9H-purin-9-yl)ethylmorpholine as a yellow solid was obtained in the same manner as in Example 73 above, except that 2-morpholine ethan-1-ol was used instead of 2-(pyridin-2-yl)ethan-1-ol in (Step a).

¹H NMR (400 MHz, DMSO-d₆) *δ* 11.28 (s, 1H), 8.25 (s, 1H), 7.94 (s, 1H), 7.55 (d, *J* = 7.6 Hz, 1H), 7.49 (s, 1H), 7.32 (t, *J* = 7.6 Hz, 1H), 7.19 (d, *J* = 7.6 Hz, 1H), 4.17 (t, *J* = 6.4 Hz, 2H), 3.69 (d, *J* = 4.4 Hz, 8H), 3.56 - 3.49 (m, 4H), 2.69 (t, *J* = 6.4 Hz, 2H), 2.45 (s, 4H), 2.35 (s, 3H); MS: m/z = 451.2 (M+1, ESI+).

### Example 76. Synthesis of (E)-4-(6-(2-(3-methylbenzylidene)hydrazinyl)-9-(1-methylpiperidin-4-yl)-9H-purin-2-yl)morpholine

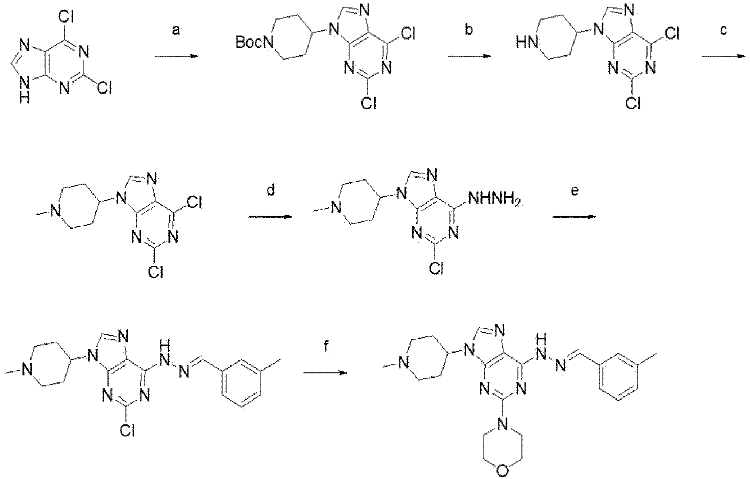

### (Step a) Synthesis of tert-butyl 4-(2,6-dichloro-9H-purin-9-yl)piperidine-1-carboxylate

2,6-Dichloro-9H-purine (10.0 g), tert-butyl 4-hydroxypiperidine-1-carboxylate (16.0 g), and triphenylphosphine (15.0 g) were dissolved in tetrahydrofuran (120.0 mL), and then diisopropyl azodicarboxylate (11.3 g) was added at room temperature. The reaction solution was stirred at 70°C for 6 hours, distilled water was added, and the resulting compound was extracted with ethyl acetate. After concentrating the organic layer under reduced pressure, the target compound tert-butyl 4-(2,6-dichloro-9H-purin-9-yl)piperidine-1-carboxylate (9.5 g) was obtained as a yellow solid in a yield of 49% through silica chromatography under ethyl acetate/benzene conditions. MS: m/z = 372.2 (M+1, ESI+).

### (Step b) Synthesis of 2, 6-dichloro-9-(piperidin-4-yl)-9H-purine

To tert-butyl 4-(2,6-dichloro-9H-purin-9-yl)piperidine-1-carboxylate (9.5 g), 4 M hydrochloric acid/1,4-dioxane (50.0 mL) using 1,4-dioxane as a solvent was added, and the mixture was stirred at room temperature for 0.5 hours. After the reaction solution was concentrated under reduced pressure, the hydrogen ion concentration index (pH) was adjusted to 8 with sodium bicarbonate. After extraction with dichloromethane, the organic layer was concentrated under reduced pressure, followed by silica chromatography under ethyl acetate/benzene conditions, to obtain the target compound 2,6-dichloro-9-(piperidin-4-yl)-9H-purine (6.5 g) as a yellow solid in a yield of 94%. MS: m/z = 272.1 (M+1, ESI+).

### (Step c) Synthesis of 2,6-dichloro-9-(1-methylpiperidin-4-yl)-9H-purine

2,6-Dichloro-9-(piperidin-4-yl)-9H-purine (1.0 g) and formaldehyde (330.0 mg) were dissolved in methanol (20.0 mL), and acetic acid (0.1 mL) was added. Sodium cyanoborohydride (350.0 mg) was added to the reaction solution at 0°C and stirred at room temperature for 2 hours. Then, distilled water was added and the reaction mixture was extracted with ethyl acetate. After concentrating the organic layer under reduced pressure, the target compound 2,6-dichloro-9-(1-methylpiperidin-4-yl)-9H-purine (560.0 mg) was obtained as a white solid in a yield of 53% through silica chromatography under ethyl acetate/benzene conditions. MS: m/z = 286.2 (M+1, ESI+).

The synthesis of (Step d) to (Step f) was performed in the same manner as in (Step b) to (Step d) of Example 73 to obtain the target compound (E)-4-(6-(2-(3-methylbenzylidene)hydrazinyl)-9-(1-methylpiperidin-4-yl)-9H-purin-2-yl)morpholine as a white solid.

¹H NMR (400 MHz, DMSO-d₆) *δ* 11.29 (s, 1H), 8.25 (s, 1H), 8.03 (s, 1H), 7.54 (d, *J* = 7.7 Hz, 1H), 7.48 (s, 1H), 7.32 (t, *J =* 7.6 Hz, 1H), 7.18 (d, *J* = 7.8 Hz, 1H), 4.22 (s, 1H), 3.70 (s, 8H), 2.90 (d, *J* = 11.6 Hz, 2H), 2.35 (s, 3H), 2.22 (s, 3H), 2.17 (s, 2H), 2.05 (t, *J* = 10.8 Hz, 2H), 1.92 (d, *J* = 10.3 Hz, 2H); MS: m/z = 435.2 (M+1, ESI+).

### Example 77. Synthesis of (E)-4-(6-(2-(3-methylbenzylidene)hydrazinyl)-9-(pyridin-4-ylmethyl)-9H-purin-2-yl)morpholine

The target compound (E)-4-(6-(2-(3-methylbenzylidene)hydrazinyl)-9-(pyridin-4-ylmethyl)-9H-purin-2-yl)morpholine was obtained as a white solid in the same manner as in Example 73 above, except that pyridin-4-ylethanol was used instead of 2-(pyridin-2-yl)ethan-1-ol in (Step a).

¹H NMR (400 MHz, DMSO-*d*₆) *δ* 10.90 (s, 1H), 8.44 - 8.31 (m, 3H), 8.06 (s, 1H), 7.34 (d, *J* = 8.9 Hz, 3H), 7.22 (d, *J* = 6.6 Hz, 1H), 6.69 (d, *J* = 4.3 Hz, 2H), 5.94 (s, 2H), 3.65 (s, 8H), 2.34 (s, 3H); MS: m/z = 429.2 (M+1, ESI+).

### Example 78. Synthesis of (E)-4-(6-(2-(3-methylbenzylidene)hydrazinyl)-9-(pyridin-3-ylmethyl)-9H-purin-2-yl)morpholine

The target compound (E)-4-(6-(2-(3-methylbenzylidene)hydrazinyl)-9-(pyridin-3-ylmethyl)-9H-purin-2-yl)morpholine was obtained as a white solid in the same manner as in Example 73 above, except that pyridin-3-ylethanol was used instead of 2-(pyridin-2-yl)ethan-1-ol in (Step a).

¹H NMR (400 MHz, DMSO-*d*₆) *δ* 10.93 (s, 1H), 8.40 (d, J = 15.1 Hz, 2H), 8.13 (d, *J* = 8.2 Hz, 2H), 7.37 (d, *J* = 26.1 Hz, 3H), 7.21 (d, *J* = 16.4 Hz, 3H), 5.92 (s, 2H), 3.64 (s, 8H), 2.33 (s, 3H); MS: m/z = 429.2 (M+1, ESI+).

### Example 79. Synthesis of (E)-4-(6-(2-(isoquinolin-4-ylmethylene)hydrazinyl)-9-(2-pyridin-2-yl)ethyl)-9H-purin-2-yl)morpholine

The target compound (E)-4-(6-(2-(isoquinolin-4-ylmethylene)hydrazinyl)-9-(2-pyridin-2-yl)ethyl)-9H-purin-2-yl)morpholine was obtained as a white solid in the same manner as in Example 73 above, except that isoquinoline-4-carbaldehyde was used instead of 3-methylbenzaldehyde in (Step c).

¹H NMR (400 MHz, DMSO-*d*₆) *δ* 11.77 (s, 1H), 9.39 (d, *J* =8.3 Hz, 1H), 8.95 (d, *J* = 4.5 Hz, 1H), 8.81 (s, 1H), 8.52 (d, *J* = 4.3 Hz, 1H), 8.08 (d, *J* = 8.3 Hz, 1H), 7.88 (s, 1H),7.82 (t, *J* = 7.3 Hz, 1H), 7.74 (d, *J* = 4.5 Hz, 1H), 7.67 (t, *J* = 7.6 Hz, 2H), 7.22 (dd, *J* = 10.2, 5.5 Hz, 2H), 4.49 (t, *J* =7.0 Hz, 2H), 3.76 (dd, *J* = 16.1, 4.1 Hz, 8H), 3.32 (t, *J* =7.0 Hz, 4H). MS: m/z = 480.2 (M+1, ESI+).

### Example 80. Synthesis of (E)-4-(6-(2-(3-methylbenzylidene)hydrazinyl)-9-(2-(pyridin-3-yl)ethyl)-9H-purin-2-yl)morpholine

The target compound (E)-4-(6-(2-(3-methylbenzylidene)hydrazinyl)-9-(2-(pyridin-3-yl)ethyl)-9H-purin-2-yl)morpholine as a white solid was obtained in the same manner as in Example 73 above, except that 2-(pyridin-3-yl)ethan-1-ol was used instead of 2-(pyridin-2-yl)ethan-1-ol in (Step a).

¹H NMR (400 MHz, DMSO-*d*₆) *δ* 11.25 (s, 1H), 8.39 (d, *J* = 4.8 Hz, 1H), 8.33 (s, 1H), 8.23 (s, 1H), 7.78 (s, 1H), 7.55 (t, *J* = 6.0 Hz, 2H), 7.48 (s, 1H), 7.32 (t, *J* = 7.6 Hz, 1H), 7.27 (dd, *J* = 7.6, 4.8 Hz, 1H), 7.18 (d, *J* = 7.2 Hz, 1H), 4.34 (t, *J* = 6.8 Hz, 2H), 3.69 (m, 8H), 3.17 (t, *J* = 6.8 Hz, 2H), 2.35 (s, 3H); MS: m/z = 443.2 (M+1, ESI+).

### Example 81. Synthesis of (E)-(3-(6-(2-(3-methylbenzylidene)hydrazinyl)-2-morpholino-9H-purin-9-yl)azetidin-1-yl)(phenyl)methanone

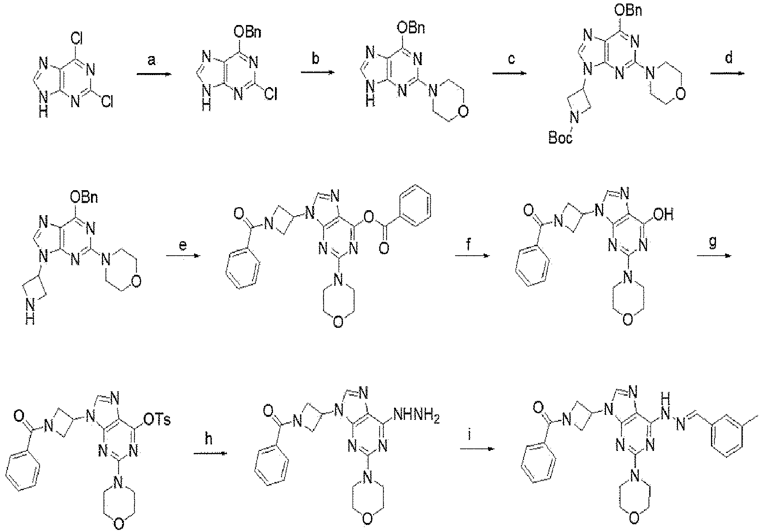

### (Step a) Synthesis of 6-(benzyloxy)-2-chloro-9H-purine

Benzyl alcohol (3.43 g) and sodium hydride (1.27 g) were dissolved in tetrahydrofuran (100 mL) and stirred at room temperature for 30 minutes. Then, 2,6-dichloro-9H-purine (5.0 g) was added and stirred at 70°C for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 6-(benzyloxy)-2-chloro-9H-purine (4.6 g) as a white solid in a yield of 66.70%. MS: m/z = 261.1 (M+1, ESI+).

### (Step b) Synthesis of 4-(6-(benzyloxy)-9H-purin-2-yl)morpholine

6-(Benzyloxy)-2-chloro-9H-purine (4.5 g) was dissolved in isopropyl alcohol (60 mL), then acetic acid (1.04 g) and morpholine (7.52 g) were added. The mixture was stirred at 120°C for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 4-(6-(benzyloxy)-9H-purin-2-yl)morpholine (2.9 g) as a white solid in a yield of 53.96%. MS: m/z = 312.1(M+1, ESI+).

### (Step c) Synthesis of tert-butyl 3-(6-(benzyloxy)-2-morpholino-9H-purin-9-yl)azetidine-1-carboxylate

4-(6-(Benzyloxy)-9H-purin-2-yl)morpholine (3.0 g) and cesium carbonate (6.07 g) were dissolved in dimethylformamide (30 mL), then tert-butyl 3-iodoazetidine-1-carboxylate (3.43 g) was added, and the mixture was stirred at 40°C for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound tert-butyl 3-(6-(benzyloxy)-2-morpholino-9H-purin-9-yl)azetidine-1-carboxylate (1.36 g) as a white solid in a yield of 31.30 %. MS: m/z = 467.2(M+1, ESI+).

### (Step d) Synthesis of 4-(9-(azetidin-3-yl)-6-(benzyloxy)-9H-purin-2-yl) morpholine

To tert-butyl 3-(6-(benzyloxy)-2-morpholino-9H-purin-9-yl)azetidine-1-carboxylate (850 mg), 4 M hydrochloric acid/1,4-dioxane (2.0 mL) using 1,4-dioxane as a solvent was added, and the mixture was stirred at room temperature for 3 hours. After the reaction solution was concentrated under reduced pressure, the hydrogen ion concentration index (pH) was adjusted to 8 with sodium bicarbonate. After extraction with dichloromethane, the organic layer was concentrated under reduced pressure, followed by silica chromatography under ethyl acetate/benzene conditions, to obtain the target compound 4-(9-(azetidin-3-yl)-6-(benzyloxy)-9H-purin-2-yl) morpholine (600 mg) as a white solid in a yield of 89.87%. MS: m/z = 367.2 (M+1, ESI+)

### (Step e) Synthesis of 9-(1-benzoylazetidin-3-yl)-2-morpholino-9H-purin-6-yl benzoate

4-(9-(Azetidin-3-yl)-6-(benzyloxy)-9H-purin-2-yl)morpholine (500 mg), triethylamine (552 mg), and benzoyl chloride (288 mg) were added to dichloromethane (10 mL), and the mixture was stirred at room temperature for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 9-(1-benzoylazetidin-3-yl)-2-morpholino-9H-purin-6-yl benzoate (300 mg) as a white solid in a yield of 45.38 %. MS: m/z = 485.5(M+1, ESI+)

### (Step f) Synthesis of 3-(6-hydroxy-2-morpholino-9H-purin-9-yl)azetidin-1-yl) (phenyl)methanone

9-(1-Benzoylazetidin-3-yl)-2-morpholino-9H-purin-6-yl benzoate (300 mg) was dissolved in tetrahydrofuran (1.0 mL) and methanol (1.0 mL), and lithium hydroxide (78 mg) was then added. The mixture was stirred at room temperature for 2 hours. After the reaction solution was concentrated under reduced pressure, 0.5 N aqueous hydrogen chloride solution was added, and the resulting product was extracted with ethyl acetate. Then, the organic layer was concentrated under reduced pressure to obtain the target compound 3-(6-hydroxy-2-morpholino-9H-purin-9-yl)azetidin-1-yl) (phenyl)methanone (210 mg) as a yellow solid with a yield of 89.16%. MS: m/z = 381.2 (M+1, ESI+).

### (Step g) Synthesis of 9-(1-benzoylazetidin-3-yl)-2-morpholino-9H-purin-6-yl-4-methylbenzenesulfonate

3-(6-Hydroxy-2-morpholino-9H-purin-9-yl)azetidin-1-yl) (phenyl)methanone (200 mg), triethylamine (552 mg), and toluenesulfonyl chloride (200 mg) were added to dichloromethane (5 mL), and the mixture was stirred at room temperature for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 9-(1-benzoylazetidin-3-yl)-2-morpholino-9H-purin-6-yl-4-methylbenzenesulfonate (160 mg) as a white solid in a yield of 56.93 %. MS: m/z = 535.3(M+1, ESI+)

### (Step h) Synthesis of 3-(6-hydrazinyl-2-morpholino-9H-purin-9-yl)azetidin-1-yl) (phenyl)methanone

9-(1-Benzoylazetidin-3-yl)-2-morpholino-9H-purin-6-yl-4-methylbenzenesulfonate (100 mg) and hydrazine monohydrate (50 mg) were dissolved in dichloromethane (10 mL), and the mixture was stirred at room temperature for 30 minutes. After the reaction solution was concentrated under reduced pressure, distilled water was added to form a solid. The formed solid was filtered and dried to obtain the target compound 3-(6-hydrazinyl-2-morpholino-9H-purin-9-yl)azetidin-1-yl) (phenyl)methanone (105 mg) as a white solid with a yield of 94.87%. MS: m/z = 395.2 (M+1, ESI+).

### (Step i) Synthesis of (E)-(3-(6-(2-(3-methylbenzylidene)hydrazinyl)-2-morpholino-9H-purin-9-yl)azetidin-1-yl) (phenyl)methanone

3-(6-Hydrazinyl-2-morpholino-9H-purin-9-yl)azetidin-1-yl) (phenyl)methanone (105 mg), 3-methylbenzaldehyde (64 mg), and acetic acid (2 mg) were dissolved in methanol (3 mL), and the mixture was stirred at 70°C for 30 minutes. The reaction solution was concentrated under reduced pressure, followed by silica chromatography under ethyl acetate/benzene conditions, to obtain the target compound (E)-(3-(6-(2-(3-methylbenzylidene)hydrazinyl)-2-morpholino-9H-purin-9-yl)azetidin-1-yl) (phenyl)methanone (30 mg) as a white solid in a yield of 22.69 %.

¹H NMR (400 MHz, DMSO-*d*₆) *δ* 11.34 (s, 1H), 8.27 (s, 1H), 8.08 (s, 1H), 7.73 - 7.67 (m, 2H), 7.58 - 7.48 (m, 5H), 7.32 (t, *J* = 7.6 Hz, 1H), 7.18 (d, *J* = 7.2 Hz, 1H), 5.44 - 5.34 (m, 1H), 4.85 (s, 2H), 4.71 - 4.62 (m, 1H), 4.51 (t, J = 8.4 Hz, 1H), 3.64 (s, 8H), 2.35 (s, 3H); MS: m/z = 497.3 (M+1, ESI+).

### Example 82. Synthesis of (E)-3-(6-(2-(3-methylbenzylidene)hydrazinyl)-2-morpholino-9H-purin-9-yl)azetidin-1-yl(pyridin-3-yl)methanone

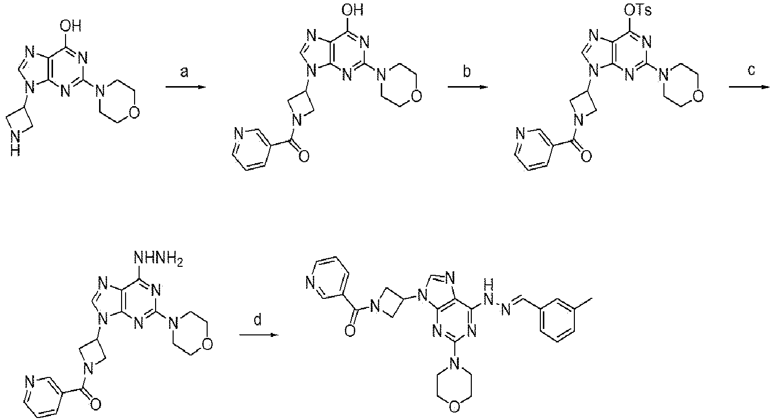

### (Step a) Synthesis of (3-(6-hydroxy-2-morpholino-9H-purin-9-yl)azetidin-1-yl) (pyridin-3-yl)methanone

9-(Azetidin-3-yl)-2-morpholino-9H-purin-6-ol (404 mg) and nicotinic acid (204 mg) were dissolved in dichloromethane (6.0 mL), then 1,1'-carbonyldiimidazole (78 mg) was added, and the mixture was stirred at room temperature for 1 hour. Triethylamine (252 mg) and dichloromethane (6.0 mL) were added to the reaction solution and stirred at room temperature for an additional 2 hours. The reaction solution was extracted three times using distilled water and dichloromethane, and washed with brine, then sodium sulfate was added, followed by filtration and concentration under reduced pressure. Then, the concentrated product was subjected to silica chromatography under ethyl acetate/petroleum ether conditions to obtain the target compound (3-(6-hydroxy-2-morpholino-9H-purin-9-yl)azetidin-1-yl) (pyridin-3-yl)methanone (210 mg) as a yellow solid with a yield of 89.16%. MS: m/z = 382.2 (M+1, ESI+)

### (Step b) Synthesis of 2-morpholino-9-(1-nicotinoylazetidin-3-yl)-9H-purin-6-yl 4-methylbenzenesulfonate

(3-(6-Hydroxy-2-morpholino-9H-purin-9-yl)azetidin-1-yl) (pyridin-3-yl)methanone (420 mg), triethylamine (446 mg), and toluenesulfonyl chloride (420 mg) were added to dichloromethane (10 mL), and the mixture was stirred at room temperature for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound 2-morpholino-9-(1-nicotinoylazetidin-3-yl)-9H-purin-6-yl 4-methylbenzenesulfonate (400 mg) as a white solid in a yield of 67.82 %. MS: m/z = 536.2(M+1, ESI+)

### (Step c) Synthesis of 3-(6-hydrazinyl-2-morpholino-9H-purin-9-yl)azetidin-1-yl) (pyridin-3-yl)methanone

2-Morpholino-9-(1-nicotinoylazetidin-3-yl)-9H-purin-6-yl 4-methylbenzenesulfonate (400 mg) and hydrazine monohydrate (187 mg) were dissolved in dichloromethane (10 mL), and the mixture was stirred at room temperature for 2 hours. After the reaction solution was concentrated under reduced pressure, distilled water was added to form a solid. The formed solid was filtered and dried to obtain the target compound 3-(6-hydrazinyl-2-morpholino-9H-purin-9-yl)azetidin-1-yl) (pyridin-3-yl)methanone (200 mg) as a white solid with a yield of 67.72%. MS: m/z = 396.2 (M+1, ESI+).

### (Step d) Synthesis of (E)-3-(6-(2-(3-methylbenzylidene)hydrazinyl)-2-morpholino-9H-purin-9-yl)azetidin-1-yl(pyridin-3-yl)methanone

3-(6-Hydrazinyl-2-morpholino-9H-purin-9-yl)azetidin-1-yl) (pyridin-3-yl)methanone (180 mg), 3-methylbenzaldehyde (109 mg), and acetic acid (8 mg) were dissolved in methanol (5 mL), and the mixture was stirred at 70°C for 30 minutes. The reaction solution was concentrated under reduced pressure, followed by silica chromatography under ethyl acetate/benzene conditions, to obtain the target compound (E)-3-(6-(2-(3-methylbenzylidene)hydrazinyl)-2-morpholino-9H-purin-9-yl)azetidin-1-yl(pyridin-3-yl)methanone (185 mg) as a yellow solid in a yield of 81.68 %.

¹H NMR (400 MHz, DMSO-*d*₆) *δ* 11.33 (s, 1H), 8.88 (s, 1H), 8.73 (d, *J* = 4.4 Hz, 1H), 8.26 (s, 1H), 8.14 - 8.06 (m, 2H), 7.59 - 7.52 (m, 2H), 7.49 (s, 1H), 7.32 (t, *J* = 7.6 Hz, 1H), 7.19 (d, *J =* 7.6 Hz, 1H), 5.45 - 5.35 (m, 1H), 4.89 (d, *J* = 6.8 Hz, 2H), 4.70 - 4.61 (m, 1H), 4.54 (t, J = 9.2 Hz, 1H), 3.64 (s, 8H), 2.35 (s, 3H); MS: m/z = 498.2 (M+1, ESI+).

### Example 83. Synthesis of methyl (E)-3-(6-(2-(3-methylbenzylidene)hydrazinyl)-2-morpholino-9H-purin-9-yl)azetidine-1-carboxylate

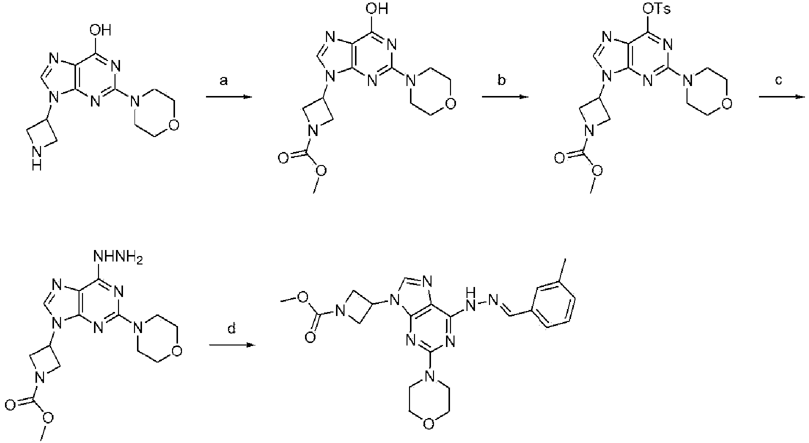

### (Step a) Synthesis of methyl 3-(6-hydroxy-2-morpholino-9H-purin-9-yl)azetidine-1-carboxylate

9-(Azetidin-3-yl)-2-morpholino-9H-purin-6-ol (650 mg), triethylamine (878 mg), and methyl carbonochloridate (360 mg) were added to dichloromethane (10 mL) and stirred at room temperature for 2 hours. After the reaction solution was concentrated under reduced pressure, distilled water was added, and the reaction mixture was extracted with ethyl acetate, and concentrated. Then, the concentrated product was subjected to silica chromatography under dichloromethane/methanol conditions to obtain the target compound methyl 3-(6-hydroxy-2-morpholino-9H-purin-9-yl)azetidine-1-carboxylate (700 mg) as a white solid in a yield of 89.7 %. MS: m/z = 335.2(M+1, ESI+)

### (Step b) Synthesis of methyl 3-(2-morpholino-6-(tosyloxy)-9H-purin-9-yl)azetidine-1-carboxylate

Methyl 3-(6-hydroxy-2-morpholino-9H-purin-9-yl)azetidine-1-carboxylate (700 mg), triethylamine (1.13 g), and hypochlorous acid (634 mg) were added to dichloromethane (20 mL), and the mixture was stirred at room temperature for 24 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound methyl 3-(2-morpholino-6-(tosyloxy)-9H-purin-9-yl)azetidine-1-carboxylate (800 mg) as a white solid in a yield of 80.1 %. MS: m/z = 489.1(M+1, ESI+)

### (Step c) Synthesis of methyl 3-(6-hydrazinyl-2-morpholino-9H-purin-9-yl)azetidine-1-carboxylate

Methyl 3-(2-morpholino-6-(tosyloxy)-9H-purin-9-yl)azetidine-1-carboxylate (800 mg) and hydrazine monohydrate (300 mg) were dissolved in tetrahydrofuran (10 mL), and the mixture was stirred at room temperature for 0.5 hours. After the reaction solution was concentrated under reduced pressure, distilled water was added to form a solid. The formed solid was filtered and dried to obtain the target compound methyl 3-(6-hydrazinyl-2-morpholino-9H-purin-9-yl)azetidine-1-carboxylate (150 mg) as a white solid with a yield of 70.4%. MS: m/z = 349.2 (M+1, ESI+).

### (Step d) Synthesis of methyl (E) -3- (6- (2- (3-methylbenzylidene)hydrazinyl)-2-morpholino-9H-purin-9-yl)azetidine-1-carboxylate

Methyl 3-(6-hydrazinyl-2-morpholino-9H-purin-9-yl)azetidine-1-carboxylate (150 mg), 3-methylbenzaldehyde (78 mg), and acetic acid (8 mg) were dissolved in methanol (15 mL), and the mixture was stirred at 70°C for 30 minutes. The reaction solution was concentrated under reduced pressure, followed by silica chromatography under ethyl acetate/benzene conditions, to obtain the target compound methyl (E)-3-(6-(2-(3-methylbenzylidene)hydrazinyl)-2-morpholino-9H-purin-9-yl)azetidine-1-carboxylate (65 mg) as a yellow solid in a yield of 32.6 %.

¹H NMR (400 MHz, DMSO-*d*₆) *δ* 11.36 (s, 1H), 8.25 (s, 1H), 8.05 (s, 1H), 7.55 (d, *J* = 7.6 Hz, 1H), 7.48 (s, 1H), 7.32 (t, *J* = 7.6 Hz, 1H), 7.19 (d, *J* = 7.4 Hz, 1H), 5.33 - 5.25 (m, 1H), 4.52 (s, 2H), 4.35 (s, 2H), 3.68 (s, 8H), 3.60 (s, 3H), 2.35 (s, 3H); MS: m/z = 451.2 (M+1, ESI+).

### Example 84. Synthesis of (E)-N,N-dimethyl-3-(6-(2-(3-methylbenzylidene)hydrazinyl)-2-morpholino-9H-purin-9-yl)azetidine-1-carboxamide

The target compound (E)-N,N-dimethyl-3-(6-(2-(3-methylbenzylidene)hydrazinyl)-2-morpholino-9H-purin-9-yl)azetidine-1-carboxamide as a yellow solid was obtained in the same manner as in Example 83 above, except that dimethyl carbamoyl chloride was used instead of methyl carbonochloridate in (Step a).

¹H NMR (400 MHz, DMSO-*d*₆) *δ* 11.41 (s, 1H), 8.26 (s, 1H), 8.15 (s, 1H), 7.57 (d, *J* = 7.6 Hz, 1H), 7.50 (s, 1H), 7.32 (t, *J =* 7.6 Hz, 1H), 7.19 (d, *J* = 7.4 Hz, 1H), 5.24 (s, 1H), 4.54 (dd, *J* = 8.4, 6.3 Hz, 2H), 4.29 (t, *J* = 8.4 Hz, 2H), 3.68 (d, *J* = 5.0 Hz, 8H), 2.80 (s, 6H), 2.35 (s, 3H); MS: m/z = 464.2 (M+1, ESI+).

### Example 85. Synthesis of (E)-4-(6-(2-(3-methylbenzylidene)hydrazinyl)-9-(1-(methylsulfonyl)azetidin-3-yl)-9H-purin-2-yl)morpholine

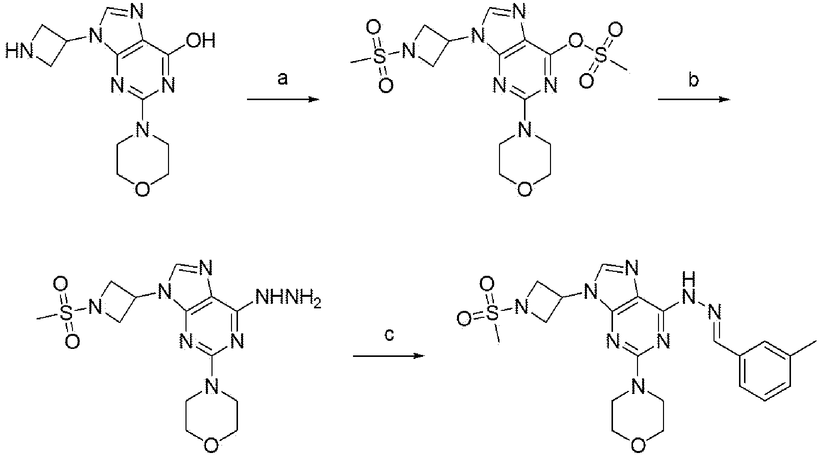

### (Step a) Synthesis of 9-(1-(methylsulfonyl)azetidin-3-yl)-2-morpholino-9H-purin-6-yl methanesulfonate

9-(Azetidin-3-yl)-2-morpholino-9H-purin-6-ol (800 mg), triethylamine (886 mg), and methanesulfonyl chloride (980 mg) were added to dichloromethane (20 mL), and the mixture was stirred at room temperature for 2 hours. After the reaction solution was concentrated under reduced pressure, distilled water was added, and the reaction mixture was extracted with ethyl acetate, and concentrated. Then, the concentrated product was subjected to silica chromatography under dichloromethane/methanol conditions to obtain the target compound 9-(1-(methylsulfonyl)azetidin-3-yl)-2-morpholino-9H-purin-6-yl methanesulfonate (600 mg) as a yellow solid in a yield of 48.0%. MS: m/z = 433.2(M+1, ESI+)

The synthesis of (Step b) to (Step c) was performed in the same manner as in (Step c) to (Step d) of Example 83 to obtain the target compound (E)-4-(6-(2-(3-methylbenzylidene)hydrazinyl)-9-(1-(methylsulfonyl)azetidin-3-yl)-9H-purin-2-yl)morpholine as a yellow solid.

¹H NMR (400 MHz, DMSO-*d*₆) *δ* 11.37 (s, 1H), 8.26 (s, 1H), 8.13 (s, 1H), 7.55 (d, *J* = 7.6 Hz, 1H), 7.49 (s, 1H), 7.32 (t, *J =* 7.6 Hz, 1H), 7.19 (d, *J =* 7.5 Hz, 1H), 5.31 (t, *J* = 7.4 Hz, 1H), 4.52 (t, *J* = 7.7 Hz, 2H), 4.30 (t, *J* = 8.4 Hz, 2H), 3.75 (s, 4H), 3.68 (d, *J* = 3.6 Hz, 4H), 3.14 (s, 3H), 2.35 (s, 3H); MS: m/z = 471.2 (M+1, ESI+).

### Example 86. Synthesis of (E)-3-(6-(2-(3-methylbenzylidene)hydrazinyl)-2-morpholino-9H-purin-9-yl)azetidin-1-yl(morpholino)methanone

The target compound (E)-3-(6-(2-(3-methylbenzylidene)hydrazinyl)-2-morpholino-9H-purin-9-yl)azetidin-1-yl(morpholino)methanone as a yellow solid was obtained in the same manner as in Example 83 above, except that morpholine-4-carbonyl chloride was used instead of methyl carbonochloridate in (Step a).

¹H NMR (400 MHz, DMSO-*d*₆) *δ* 11.36 (s, 1H), 8.26 (s, 1H), 8.06 (s, 1H), 7.56 (d, *J* = 7.6 Hz, 1H), 7.50 (s, 1H), 7.33 (t, *J* = 7.6 Hz, 1H), 7.19 (d, *J* = 7.6 Hz, 1H), 5.29 - 5.21 (m, 1H), 4.57 (t, *J* = 7.2 Hz, 2H), 4.32 (t, *J* = 8.4 Hz, 2H), 3.72 - 3.65 (m, 8H), 3.58 - 3.53 (m, 4H), 3.28 - 3.24 (m, 4H), 2.35 (s, 3H); MS: m/z = 506.2 (M+1, ESI+).

### Example 87. Synthesis of (E)-(4-(6-(2-(3-methylbenzylidene)hydrazinyl)-2-morpholino-9H-purin-9-yl)piperidin-1-yl)(pyridin-3-yl)methanone

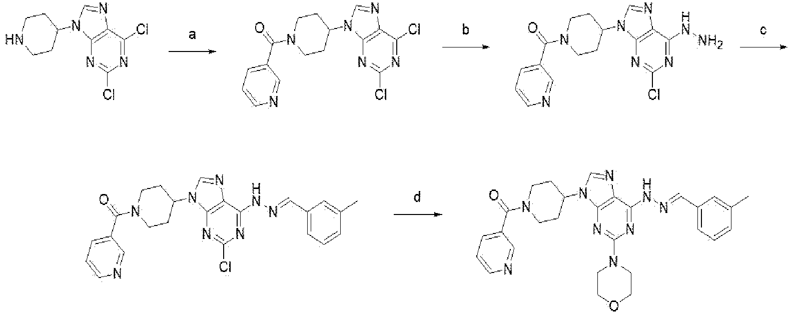

### (Step a) Synthesis of (4-(2,6-dichloro-9H-purin-9-yl)piperidin-1-yl) (pyridin-3-yl)methanone

2,6-Dichloro-9-(piperidin-4-yl)-9H-purine (900.0 mg) was dissolved in dichloromethane (15.0 mL), then nicotinic acid (300.0 mg), triethylamine (370.0 mg), and 1,1'-carbonyldiimidazole (591.0 mg) were added, and the mixture was stirred at room temperature for 2 hours. After the reaction solution was concentrated under reduced pressure, distilled water was added, and the reaction mixture was extracted with ethyl acetate, and concentrated. Then, the concentrated product was subjected to silica chromatography under ethyl acetate/benzene conditions to obtain the target compound (4-(2,6-dichloro-9H-purin-9-yl)piperidin-1-yl) (pyridin-3-yl)methanone (330.0 mg) as a white solid in a yield of 24.0%. MS: m /z = 378.1(M+1, ESI+).

### (Step b) Synthesis of (4-(2-chloro-6-hydrazinyl-9H-purin-9-yl)piperidin-1-yl) (pyridin-3-yl)methanone

(4-(2,6-Dichloro-9H-purin-9-yl)piperidin-1-yl) (pyridin-3-yl)methanone (330.0 mg) was dissolved in tetrahydrofuran (10.0 mL), and then hydrazine monohydrate (221.0 mg) was added. The reaction mixture was stirred at room temperature for 30 minutes. After concentrating the organic solvent under reduced pressure, ethyl acetate was added. The resulting solid was filtered to obtain the target compound (4-(2-chloro-6-hydrazinyl-9H-purin-9-yl)piperidin-1-yl) (pyridin-3-yl)methanone (150.0 mg) as a yellow solid in a yield of 45.9%. MS: m/z = 374.2 (M+1, ESI+).

### (Step c) Synthesis of (E)-(4-(2-chloro-6-(2-(3-methylbenzylidene)hydrazinyl)-9H-purin-9-yl)piperidin-1-yl) (pyridin-3-yl)methanone

(4-(2-Chloro-6-hydrazinyl-9H-purin-9-yl)piperidin-1-yl) (pyridin-3-yl)methanone (150.0 mg) was dissolved in methanol (10.0 mL), and then 3-methylbenzaldehyde (60.0 mg) and acetic acid (0.1 mL) were added. The reaction solution was stirred at 70°C for 30 minutes. The reaction solution was concentrated under reduced pressure, followed by silica chromatography under ethyl acetate/benzene conditions, to obtain the target compound (E)-(4-(2-chloro-6-(2-(3-methylbenzylidene)hydrazinyl)-9H-purin-9-yl)piperidin-1-yl) (pyridin-3-yl)methanone (100.0 mg) as a yellow solid in a yield of 52.6%. MS: m/z = 476.2 (M+1, ESI+).

### (Step d) Synthesis of (E)-(4-(6-(2-(3-methylbenzylidene)hydrazinyl)-2-morpholino-9H-purin-9-yl)piperidin-1-yl) (pyridin-3-yl)methanone

(E)-(4-(2-chloro-6-(2-(3-methylbenzylidene)hydrazinyl)-9H-purin-9-yl)piperidin-1-yl) (pyridin-3-yl)methanone (100.0 mg) was dissolved in isopropyl alcohol (15.0 mL), followed by the addition of morpholine (1.0 mL) and acetic acid (0.2 mL). The reaction solution was stirred at 120°C for 12 hours. The reaction solution was concentrated under reduced pressure, followed by silica chromatography under dichloromethane/methanol conditions, to obtain the target compound (E)-(4-(6-(2-(3-methylbenzylidene)hydrazinyl)-2-morpholino-9H-purin-9-yl)piperidin-1-yl) (pyridin-3-yl)methanone (25.0 mg) as a white solid in a yield of 22.6%.

¹H NMR (400 MHz, DMSO-*d*₆) *δ* 11.31 (s, 1H), 8.25 (s, 1H), 7.85 (s, 1H), 7.54 (s, 1H), 7.49 (s, 1H), 7.31 (d, *J* = 7.6 Hz, 1H), 7.20 (s, 1H), 4.72 (s, 2H), 4.28 (t, *J* = 7.7 Hz, 2H), 3.92 (t, *J* = 7.7 Hz, 2H), 3.73 - 3.67 (m, 8H), 2.35 (s, 3H), 2.30 - 2.24 (m, 2H); MS: m/z = 526.3 (M+1, ESI+).

### Example 88. Synthesis of (E)-(4-(6-(2-(3-methylbenzylidene)hydrazinyl)-2-morpholino-9H-purin-9-yl)piperidin-1-yl)(morpholino)methanone

The target compound (E)-(4-(6-(2-(3-methylbenzylidene)hydrazinyl)-2-morpholino-9H-purin-9-yl)piperidin-1-yl) (morpholino)methanone as a yellow solid was obtained in the same manner as in Example 87 above, except using morpholine-4-carbonyl chloride in (Step a).

¹H NMR (400 MHz, DMSO-*d*₆) *δ* 11.30 (s, 1H), 8.26 (s, 1H), 8.04 (s, 1H), 7.55 (d, *J* = 7.7 Hz, 1H), 7.48 (s, 1H), 7.32 (t, *J* = 7.6 Hz, 1H), 7.18 (d, *J* = 7.5 Hz, 1H), 4.45 (s, 1H), 3.77 - 3.69 (m, 10H), 3.59 (t, *J* = 4.0 Hz, 1H), 3.17 - 3.15 (m, 4H), 2.94 (t, *J* = 12.0 Hz, 2H), 2.34 (s, 3H), 2.12 - 2.06 (m, 2H), 1.97 (d, *J* = 9.8 Hz, 2H); MS: m/z = 534.2 (M+1, ESI+).

### Example 89. Synthesis of (E)-(4-(6-(2-(3-methylbenzylidene)hydrazinyl)-2-morpholino-9H-purin-9-yl)piperidin-1-yl)(phenyl)methanone

The target compound (E)-(4-(6-(2-(3-methylbenzylidene)hydrazinyl)-2-morpholino-9H-purin-9-yl)piperidin-1-yl) (phenyl)methanone as a white solid was obtained in the same manner as in Example 87 above, except using benzoyl chloride in (Step a).

¹H NMR (400 MHz, DMSO-*d*₆) *δ* 11.31 (s, 1H), 8.25 (s, 1H), 8.10 (s, 1H), 7.54 (d, *J* = 7.7 Hz, 1H), 7.47 (t, *J =* 5.6 Hz, 6H), 7.32 (t, *J* = 7.6 Hz, 1H), 7.19 (d, *J* = 7.4 Hz, 1H), 4.59 (s, 1H), 3.71 (d, *J* = 8.7 Hz, 10H), 3.41 - 3.40 (m, 2H), 2.35 (s, 3H), 2.13 - 1.97 (m, 4H); MS: m/z = 525.2 (M+1, ESI+).

### Example 90. Synthesis of (E)-N,N-dimethyl-4-(6-(2-(3-methylbenzylidene)hydrazinyl)-2-morpholino-9H-purin-9-yl)piperidine-1-carboxamide

The target compound (E)-N,N-dimethyl-4-(6-(2-(3-methylbenzylidene)hydrazinyl)-2-morpholino-9H-purin-9-yl)piperidine-1-carboxamide as a yellow solid was obtained in the same manner as in Example 87 above, except using dimethylcarbamic chloride in (Step a).

¹H NMR (400 MHz, DMSO-*d*₆) *δ* 11.31 (s, 1H), 8.26 (s, 1H), 8.04 (s, 1H), 7.55 (d, *J* = 7.6 Hz, 1H), 7.49 (s, 1H), 7.31 (t, *J* = 7.6 Hz, 1H), 7.17 (d, *J* = 7.5 Hz, 1H), 4.43 - 4.40 (m, 1H), 3.71 - 3.68 (m, 10H), 2.87 (t, *J* = 12.3 Hz, 2H), 2.76 (s, 6H), 2.34 (s, 3H), 2.12 (dd, *J* = 12.0, 3.1 Hz, 1H), 2.07 (s, 1H), 1.96 (d, *J* = 10.2 Hz, 2H); MS: m/z = 492.3 (M+1, ESI+).

### Example 91. Synthesis of methyl (E)-4-(6-(2-(3-methylbenzylidene)hydrazinyl)-2-morpholino-9H-purin-9-yl)piperidine-1-carboxylate

The target compound methyl (E)-4-(6-(2-(3-methylbenzylidene)hydrazinyl)-2-morpholino-9H-purin-9-yl)piperidine-1-carboxylate as a white solid was obtained in the same manner as in Example 87 above, except using methyl carbonochloridate in (Step a).

¹H NMR (400 MHz, DMSO-*d*₆) *δ* 11.30 (s, 1H), 8.25 (s, 1H), 8.04 (s, 1H), 7.54 (d, *J* = 7.5 Hz, 1H), 7.48 (s, 1H), 7.32 (t, *J* = 7.6 Hz, 1H), 7.18 (d, *J* = 7.5 Hz, 1H), 4.54 - 4.43 (m, 1H), 4.13 (s, 2H), 3.70 (d, *J* = 4.9 Hz, 8H), 3.63 (s, 3H), 2.99 (s, 2H), 2.35 (d, *J* = 6.6 Hz, 3H), 2.05 - 1.94 (m, 4H); MS: m/z = 479.3 (M+1, ESI+).

### Example 92. Synthesis of (E)-4-(6-(2-(3-methylbenzylidene)hydrazinyl)-9-(1-(methylsulfonyl)piperidin-4-yl)-9H-purin-2-yl)morpholino

The target compound (E)-4-(6-(2-(3-methylbenzylidene)hydrazinyl)-9-(1-(methylsulfonyl)piperidin-4-yl)-9H-purin-2-yl)morpholino as a white solid was obtained in the same manner as in Example 87 above, except using methanesulfonyl chloride in (Step a).

¹H NMR (400 MHz, DMSO-*d*₆) *δ* 11.31 (s, 1H), 8.25 (s, 1H), 8.07 (s, 1H), 7.55 (d, *J* = 7.6 Hz, 1H), 7.49 (s, 1H), 7.32 (t, *J* = 7.6 Hz, 1H), 7.19 (d, *J* = 7.4 Hz, 1H), 4.42 (s, 1H), 3.70 (d, *J* = 5.9 Hz, 10H), 2.98 (d, *J* = 11.9 Hz, 2H), 2.94 (s, 3H), 2.35 (s, 3H), 2.18 (d, *J* = 9.2 Hz, 2H), 2.09 (d, *J* = 10.2 Hz, 2H); MS: m/z = 499.2 (M+1, ESI+).

### Example 93. Synthesis of (E)-(4-(6-(2-(3-methylbenzylidene)hydrazinyl)-2-morpholino-9H-purin-9-yl)piperidin-1-yl)(pyridin-4-yl)methanone

The target compound (E)-(4-(6-(2-(3-methylbenzylidene)hydrazinyl)-2-morpholino-9H-purin-9-yl)piperidin-1-yl) (pyridin-4-yl)methanone as a white solid was obtained in the same manner as in Example 87 above, except using isonicotinoyl chloride in (Step a).

¹H NMR (400 MHz, DMSO-*d*₆) *δ* 11.44 (s, 1H), 8.42 (s, 1H), 8.29 (s, 1H), 7.99 (d, *J* = 8.8 Hz, 2H), 7.64 (d, *J* = 8.8 Hz, 2H), 7.57 (d, *J* = 7.6 Hz, 1H), 7.51 (s, 1H), 7.33 (t, *J* = 7.6 Hz, 1H), 7.20 (d, *J* = 7.4 Hz, 1H), 3.70 (dd, *J* = 14.6, 3.7 Hz, 8H), 2.35 (s, 3H); MS: m/z = 526.2 (M+1, ESI+) .

### Example 94. Synthesis of (E)-(4-(6-(2-(3-methylbenzylidene)hydrazinyl)-2-morpholino-9H-purin-9-yl)piperidin-1-yl)(pyridin-2-yl)methanone

Synthesis was performed in the same manner as in Example 87 above, but (Step a) was performed as follows.

### (Step a) Synthesis of (4-(2,6-dichloro-9H-purin-9-yl)piperidin-1-yl) (pyridin-2-yl)methanone

2,6-Dichloro-9-(piperidin-4-yl)-9H-purine (1.0 g) was dissolved in acetonitrile (10 mL), then picolinic acid (679 mg), N-methylimidazole (905 mg), and N,N,N',N'-tetramethylchloroformamidinium hexafluorophosphate (996 mg) were added, and the mixture was stirred at room temperature for 3 hours. The reaction solution was extracted with ethyl acetate (30 mL) and concentrated. Then, the concentrated product was subjected to silica chromatography under ethyl acetate/petroleum ether conditions to obtain the target compound (4-(2,6-dichloro-9H-purin-9-yl)piperidin-1-yl) (pyridin-2-yl)methanone (660 mg) as a pale yellow solid in a yield of 43%. MS: m /z = 377.0 (M+1, ESI+).

Then, the synthesis was performed in the same manner as in (Step b) to (Step d) of Example 87 to obtain the target compound (E)-(4-(6-(2-(3-methylbenzylidene)hydrazinyl)-2-morpholino-9H-purin-9-yl)piperidin-1-yl) (pyridin-2-yl)methanone as a yellow solid.

¹H NMR (400 MHz, DMSO-*d*₆) *δ* 11.30 (s, 1H), 8.61 (d, *J* = 4.4 Hz, 1H), 8.25 (s, 1H), 8.08 (s, 1H), 7.96 (d, *J* = 1.6 Hz, 1H), 7.61 (d, *J* = 7.8 Hz, 1H), 7.54 (d, *J* = 7.6 Hz, 1H), 7.49 (d, *J* = 6.2 Hz, 2H), 7.32 (t, *J* = 7.6 Hz, 1H), 7.18 (d, *J* = 7.5 Hz, 1H), 4.71 - 4.56 (m, 2H), 3.89 - 3.82 (m, 1H), 3.75 - 3.64 (m, 9H), 3.05 - 2.96 (m, 1H), 2.35 (s, 3H), 2.13 (d, *J* = 3.6 Hz, 3H), 1.99 - 1.92 (m, 1H), 1.35 - 1.21 (m, 1H); MS: m/z = 526.4 (M+1, ESI+).

### Example 95. Synthesis of (E)-4-(9-(1-methyl-1H-pyrazol-4-yl)-6-(2-(3-methylbenzylidene)hydrazinyl)-9H-purin-2-yl)-morpholine

The target compound (E)-4-(9-(1-methyl-1H-pyrazol-4-yl)-6-(2-(3-methylbenzylidene)hydrazinyl)-9H-purin-2-yl)-morpholine as a white solid was obtained in the same manner as in Example 96 below, except that 4-iodo-1-methyl-1H-pyrazole was used instead of 3-iodopyridine in (Step a).

¹H NMR (400 MHz, DMSO-*d*₆) *δ* 11.41 (s, 1H), 8.35 - 8.24 (m, 3H), 8.02 (s, 1H), 7.57 (d, *J* = 8.0 Hz, 1H), 7.51 (s, 1H), 7.33 (t, *J* = 8.0 Hz, 1H), 7.20 (d, *J* = 8.0 Hz, 1H), 3.94 (s, 3H), 3.73 (dd, *J* = 4.0 Hz, *J* = 20.0 Hz, 8H), 2.36 (s, 3H); MS: m/z = 418.2 (M+1, ESI+).

### Example 96. Synthesis of (E)-4-(6-(2-(3-methylbenzylidene)hydrazinyl)-9-(pyridin-3-yl)-9H-purin-2-yl)morpholine

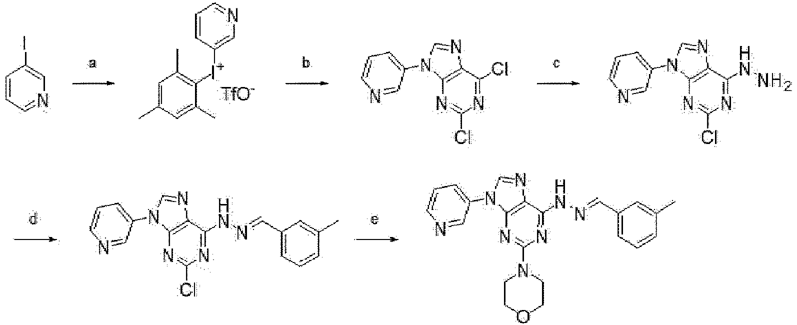

### (Step a) Synthesis of mesityl (pyridin-3-yl)iodonium trifluoromethane sulfonate

3-Iodopyridine (3.0 g) and m-chloroperoxybenzoic acid (3.8 g) were dissolved in dichloromethane (100.0 mL), then trifluoromethanesulfonic acid (8.8 g) was added at 0°C, and the mixture was stirred at room temperature for 2 hours. After adding mesitylene (1.9 g) at 0°C, the mixture was stirred at room temperature for 24 hours. After adding distilled water, the reaction mixture was extracted with dichloromethane and concentrated under reduced pressure. The concentrated product was subjected to silica chromatography under dichloromethane/methanol conditions to synthesize the target compound mesityl (pyridin-3-yl)iodonium trifluoromethanesulfonate (4.9 g) as a yellow solid in a yield of 71%. MS: m/z = 323.9 (M-TfO-, ESI+).

### (Step b) Synthesis of 2,6-dichloro-9-(pyridin-3-yl)-9H-purine

Mesityl(pyridin-3-yl)iodonium trifluoromethanesulfonate (4.8 g), triethylamine (1.54 g), 2,6-dichloro-9H-purine (1.4 g), and copper bromide (291.0 mg) were dissolved in dichloromethane (100.0 mL), and the reaction mixture was stirred at 60°C for 24 hours. After adding distilled water, the reaction mixture was extracted with dichloromethane and concentrated under reduced pressure. The resulting product was subjected to silica chromatography under dichloromethane/methanol conditions to obtain the target compound 2,6-dichloro-9-(pyridin-3-yl)-9H-purine (540.0 mg) as a yellow solid in a yield of 28%. MS: m/z = 265.8(M+1, ESI+).

### (Step c) Synthesis of 2-chloro-6-hydrazinyl-9-(pyridin-3-yl)-9H-purine

2,6-Dichloro-9-(pyridin-3-yl)-9H-purine (540.0 mg) was dissolved in tetrahydrofuran (15.0 mL), then hydrazine monohydrate (406.0 mg) was added, and the mixture was stirred at room temperature for 20 minutes. After concentrating the reaction solution under reduced pressure, distilled water was added, and the resulting yellow solid was filtered to obtain the target compound 2-chloro-6-hydrazinyl-9-(pyridin-3-yl)-9H-purine (500.0 mg) in a yield of 94%. MS: m/z = 261.9 (M+1, ESI+)

### (Step d) Synthesis of (E)-2-chloro-6-(2-(3-methylbenzylidene)hydrazinyl)-9-(pyridin-3-yl)-9H-purine

2-Chloro-6-hydrazinyl-9-(pyridin-3-yl)-9H-purine (500.0 mg), 3-methylbenzaldehyde (459.0 mg), and acetic acid (11.0 mg) were dissolved in methanol (10.0 mL), and the mixture was stirred at 70°C for 20 minutes. After concentrating the reaction solution under reduced pressure, the target compound (E)-2-chloro-6-(2-(3-methylbenzylidene)hydrazinyl)-9-(pyridin-3-yl)-9H-purine (400.0 mg) was obtained as a yellow solid in a yield of 58% through silica chromatography under dichloromethane/methanol conditions. MS: m/z = 364.2 (M+1, ESI+)

### (Step e) Synthesis of (E)-4-(6-(2-(3-methylbenzylidene)hydrazinyl)-9-(pyridin-3-yl)-9H-purin-2-yl)morpholine

(E)-2-Chloro-6-(2-(3-methylbenzylidene)hydrazinyl)-9-(pyridin-3-yl)-9H-purine (350.0 mg), morpholine (419.0 mg), and acetic acid (58.0 mg) were dissolved in isopropyl alcohol (6.0 mL), and the mixture was stirred at 120°C for 24 hours. After adding distilled water, the reaction mixture was extracted with ethyl acetate, and the organic layer solvent was concentrated under reduced pressure. The target compound (E)-4-(6-(2-(3-methylbenzylidene)hydrazinyl)-9-(pyridin-3-yl)-9H-purin-2-yl)morpholine (55.0 mg) was obtained as a white solid in a yield of 13.1% through silica chromatography under dichloromethane/methanol conditions.

¹H NMR (400 MHz, DMSO-*d*₆) *δ* 11.49 (s, 1H), 9.18 (d, *J* = 2.4 Hz, 1H), 8.61 (d, *J* = 4.8 Hz, 1H), 8.50 (s, 1H), 8.41 - 8.36 (m, 1H), 8.30 (s, 1H), 7.64 (dd, *J* = 8.4, 4.8 Hz, 1H), 7.58 (d, *J* = 7.6 Hz, 1H), 7.52 (s, 1H), 7.34 (t, *J* = 7.6 Hz, 1H), 7.21 (d, *J* = 7.2 Hz, 1H), 3.74 - 3.69 (m, 8H), 2.36 (s, 3H); MS: m/z = 415.2 (M+1, ESI+).

### Example 97. Synthesis of (S,E)-3-methyl-4-(6-(2-(3-methylbenzylidene)hydrazinyl)-9-(pyridin-3-yl)-9H-purin-2-yl)morpholine

The target compound (S,E)-3-methyl-4-(6-(2-(3-methylbenzylidene)hydrazinyl)-9-(pyridin-3-yl)-9H-purin-2-yl)morpholine was obtained as a yellow solid in the same manner as in Example 96 above, except that (S)-3-methylmorpholine was used instead of morpholine in (Step e).

¹H NMR (400 MHz, DMSO-*d*₆) *δ* 11.48 (s, 1H), 9.20 (d, *J* = 2.8 Hz, 1H), 8.62 - 8.61 (m, 1H), 8.50 (s, 1H), 8.40 - 8.37 (m, 1H), 8.30 (s, 1H), 7.66 - 7.63 (m, 1H), 7.59 (d, *J* = 7.6 Hz, 1H), 7.52 (s, 1H), 7.35 (t, *J* = 7.6 Hz, 1H), 7.21 (d, *J* = 7.2 Hz, 1H), 4.67 (d, *J* = 6.8 Hz, 1H), 4.30 (d, *J* = 14.4 Hz, 1H), 3.96 - 3.93 (m, 1H), 3.74 (d, *J* = 10.8 Hz, 1H), 3.63 - 3.59 (m, 1H), 3.4 (s, 1H), 3.2 - 3.1 (m, 1H), 2.37 (s, 3H), 1.2 (s, 3H); MS: m/z = 429 (M+1, ESI+).

### Example 98. Synthesis of (1R,4R)-5-(6-(2-(3-methylbenzylidene)hydrazinyl)-9-(pyridin-3-yl)-9H-purin-2-yl)-oxa-5-azabicyclo[2.2.1]heptane

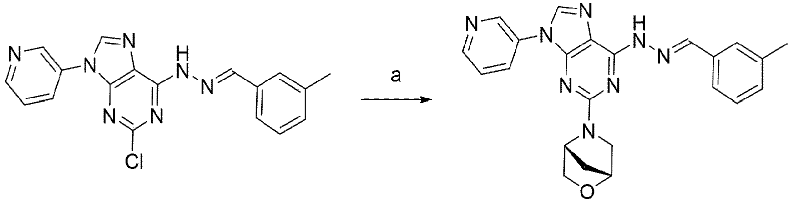

The target compound (1R,4R)-5-(6-(2-(3-methylbenzylidene)hydrazinyl)-9-(pyridin-3-yl)-9H-purin-2-yl)-oxa-5-azabicyclo[2.2.1]heptane was obtained as a yellow solid in the same manner as in Example 96 above, except that (1R,4R)-2-oxa-5-azabicyclo[2.2.1]heptane was used instead of morpholine in (Step e).

¹H NMR (400 MHz, DMSO-*d*₆) *δ* 11.46 (s, 1H), 9.21 (d, *J* = 2.4 Hz, 1H), 8.62 (d, *J* = 1.2 Hz, 1H), 8.48 (s, 1H), 8.43 - 8.41 (m, 1H), 8.30 (s, 1H), 7.65 - 7.62 (m, 1H), 7.60 (d, *J* = 14.4 Hz, 1H), 7.53 (s, 1H), 7.35 (t, *J* = 7.6 Hz, 1H), 7.33 - 7.2 (m, 1H), 4.90 (s, 1H), 4.66 (s, 1H), 3.82 - 3.74 (m, 1H), 3.74 - 3.72 (m, 1H), 3.52 - 3.49 (m, 2H), 2.37 (s, 3H), 1.91 - 1.82 (m, 2H); MS: m/z = 427 (M+1, ESI+).

### Example 99. Synthesis of (E)-4-(6-(2-(3-methylbenzylidene)hydrazinyl)-9-(pyridin-2-yl)-9H-purin-2-yl)morpholine

The target compound (E)-4-(6-(2-(3-methylbenzylidene)hydrazinyl)-9-(pyridin-2-yl)-9H-purin-2-yl)morpholine was obtained as a yellow solid in the same manner as in Example 96 above, except that 2-iodopyridine was used instead of 3-iodopyridine in (Step a).

¹H NMR (400 MHz, DMSO-*d*₆) *δ* 11.49 (s, 1H), 8.71 (s, 1H), 8.59 - 8.57 (m, 2H), 8.31 (s, 1H), 8.09 (t, *J* = 7.6 Hz, 1H), 7.58 (d, *J* = 6.8 Hz, 1H), 7.51 (s, 1H), 7.42 (t, *J =* 5.2 Hz, 1H), 7.34 (t, *J* = 7.2 Hz, 1H), 7.21 (d, *J* = 8.0 Hz, 1H), 3.76 (d, *J* = 26.8 Hz, 8H), 2.36 (s, 3H); MS: m/z = 415.2 (M+1, ESI+).

### Example 100. Synthesis of (E)-4-(9-(4-chlorophenyl)-6-(2-(3-methylbenzylidene)hydrazinyl)-9H-purin-2-yl)morpholine

The target compound (E)-4-(9-(4-chlorophenyl)-6-(2-(3-methylbenzylidene)hydrazinyl)-9H-purin-2-yl)morpholine was obtained as a white solid in the same manner as in Example 96 above, except that 1-chloro-4-iodobenzene was used instead of 3-iodopyridine in (Step a).

¹H NMR (400 MHz, DMSO-*d*₆) *δ* 11.44 (s, 1H), 8.42 (s, 1H), 8.29 (s, 1H), 7.99 (d, J = 8.8 Hz, 2H), 7.64 (d, J = 8.8 Hz, 2H), 7.57 (d, J = 7.6 Hz, 1H), 7.51 (s, 1H), 7.33 (t, J = 7.6 Hz, 1H), 7.20 (d, J = 7.4 Hz, 1H), 3.70 (dd, J = 14.6, 3.7 Hz, 8H), 2.35 (s, 3H); MS: m/z = 448.2 (M+1, ESI+) .

### Example 101. Synthesis of (E)-4-(9-(2-methoxypyridin-4-yl)-6-(2-(3-methylbenzylidene)hydrazinyl)-9H-purin-2-yl)morpholine

The target compound (E)-4-(9-(2-methoxypyridin-4-yl)-6-(2-(3-methylbenzylidene)hydrazinyl)-9H-purin-2-yl)morpholine was obtained as a yellow solid in the same manner as in Example 96 above, except that 4-iodo-2-methoxypyridine was used instead of 3-iodopyridine in (Step a).

¹H NMR (400 MHz, DMSO-*d*₆) *δ* 11.51 (s, 1H), 8.66 (s, 1H), 8.33 - 8.28 (m, 2H), 7.83 (dd, *J* = 5.7, 1.8 Hz, 1H), 7.65 (d, *J* = 1.6 Hz, 1H), 7.57 (d, *J* = 7.9 Hz, 1H), 7.51 (s, 1H), 7.34 (t, *J* = 7.6 Hz, 1H), 7.21 (d, *J* = 7.4 Hz, 1H), 3.92 (s, 3H), 3.76 (d, *J* = 4.6 Hz, 4H), 3.72 (d, *J* = 4.6 Hz, 4H), 2.36 (s, 3H); MS: m/z = 445.3 (M+1, ESI+)

### Example 102. Synthesis of (E)-4-(6-(2-(3-methylbenzylidene)hydrazinyl)-2-morpholino-9H-purin-9-yl)pyridin-2-ol

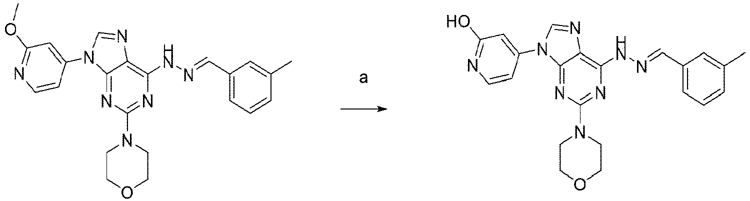

### (Step a) Synthesis of (E)-4-(6-(2-(3-methylbenzylidene)hydrazinyl)-2-morpholino-9H-purin-9-yl)pyridin-2-ol

(E)-4-(9-(2-Methoxypyridin-4-yl)-6-(2-(3-methylbenzylidene)hydrazinyl)-9H-purin-2-yl)morpholine (260 mg) was dissolved in acetonitrile (7 mL), then sodium iodide (351 mg) and trimethylsilyl chloride (253 mg) were added. The mixture was stirred at 80°C for 2 hours. After addition of ethyl acetate, the reaction was terminated with sodium thiosulfate. The resulting solid was filtered and water was added. The resulting product was recrystallized under dimethyl sulfoxide/methanol conditions to obtain the target compound (E)-4-(6-(2-(3-methylbenzylidene)hydrazinyl)-2-morpholino-9H-purin-9-yl)pyridin-2-ol (27 mg) as a white solid in a yield of 10.72%.

¹H NMR (400 MHz, DMSO-*d*₆) *δ* 11.72 (s, 1H), 11.49 (s, 1H), 8.57 (s, 1H), 8.29 (s, 1H), 7.58 (s, 2H), 7.51 (s, 1H), 7.34 (t, *J* = 6.8 Hz, 1H), 7.22 - 7.18 (m, 2H), 7.07 (d, *J =* 6.8 Hz, 1H), 3.74 - 3.72 (m, 8H), 2.36 (s, 3H); MS: m/z = 431.0 (M+1, ESI+).

### Example 103. Synthesis of (E)-4-(6-(2-(3-methylbenzylidene)hydrazinyl)-9-(pyridin-4-yl)-9H-purin-2-yl)morpholine

The target compound (E)-4-(6-(2-(3-methylbenzylidene)hydrazinyl)-9-(pyridin-4-yl)-9H-purin-2-yl)morpholine was obtained as a yellow solid in the same manner as in Example 96 above, except that 4-iodopyridine was used instead of 3-iodopyridine in (Step a), and copper iodide was used instead of copper bromide in (Step b).

¹H NMR (400 MHz, DMSO-*d*₆) *δ* 10.2 (s, 1H), 9.82 (s, 1H), 8.45 - 8.38 (m, 3H), 7.92 (d, *J* = 6.4 Hz, 2H), 7.75 - 7.65 (m, 2H), 7.45 (t, *J* = 7.6 Hz, 1H), 7. 43 - 7.38 (m, 1H), 3.74 (s, 8H), 2.40 (s, 3H); MS: m/z = 445 (M+1, ESI+).

### Example 104. Synthesis of (E)-4-(6-(2-(3-methylbenzylidene)hydrazinyl)-9-(4-(trifluoromethyl)phenyl)-9H-purin-2-yl)morpholine

The target compound (E)-4-(6-(2-(3-methylbenzylidene)hydrazinyl)-9-(4-(trifluoromethyl)phenyl)-9H-purin-2-yl)morpholine was obtained as a white solid in the same manner as in Example 119, except that (4-(trifluoromethyl)phenyl)boronic acid was used instead of phenylboronic acid in (Step a).

¹H NMR (400 MHz, DMSO-*d*₆) *δ* 11.50 (s, 1H), 8.55 (s, 1H), 8.34-8.22 (m, 3H), 7.96 (d, *J* = 8.6Hz, 2H), 7.58 (d, *J* = 7.6Hz, 1H), 7.52 (s, 1H), 7.34 (t, 1H), 7.20 (d, *J* = 7.5Hz, 1H), 3.72 (dd, *J* = 19.4 Hz, 4.7 Hz, 8H), 2.36 (s, 3H); MS: m/z = 482.48 (M+1, ESI+).

### Example 105. Synthesis of (E)-1-(6-(2-(3-methylbenzylidene)hydrazinyl)-9-(pyridin-3-yl)-9H-purin-2-yl)piperidin-4-ol

The target compound (E)-1-(6-(2-(3-methylbenzylidene)hydrazinyl)-9-(pyridin-3-yl)-9H-purin-2-yl)piperidin-4-ol was obtained as a white solid in the same manner as in Example 96 above, except that piperidin-4-ol was used instead of morpholine in (Step e).

¹H NMR (400 MHz, DMSO-*d*₆) *δ* 11.46 (s, 1H), 9.19 (d, *J* = 2.4 Hz, 1H), 8.61 (dd, *J* = 4.4, 1.2 Hz, 1H), 8.48 (s, 1H), 8.40 - 8.35 (m, 1H), 8.31 - 8.25 (m, 1H), 7.70 -7.62 (m, 1H), 7.59 (d, *J* = 8.0 Hz, 1H), 7.53 (s, 1H), 7.35 (t, J = 7.6 Hz, 1H), 7.21 (d, *J* = 7.6 Hz, 1H), 4.74 (d, *J* = 4.4 Hz, 1H), 4.40 - 4.30 (m, 1H), 3.75 - 3.65 (m, 1H), 3.30 - 3.20 (m, 2H), 2.37 (s, 3H), 1.85 - 1.75 (m, 2H), 1.45 - 1.30 (m, 2H); MS: m/z = 429 (M+1, ESI+).

### Example 106. Synthesis of (E)-1-(6-(2-(3-methylbenzylidene)hydrazinyl)-9-(pyridin-3-yl)-9H-purin-2-yl)azetidin-3-ol

The target compound (E)-1-(6-(2-(3-methylbenzylidene)hydrazinyl)-9-(pyridin-3-yl)-9H-purin-2-yl)azetidin-3-ol was obtained in the same manner as in Example 96 above, except that (Step e) was performed in the following manner:

### (Step e) Synthesis of (E)-1-(6-(2-(3-methylbenzylidene)hydrazinyl)-9-(pyridin-3-yl)-9H-purin-2-yl)azetidin-3-ol

(E)-2-Chloro-6-(2-(3-methylbenzylidene)hydrazinyl)-9-(pyridin-3-yl)-9H-purine (300 mg) was dissolved in isopropyl alcohol (8.25 mL), then N,N-diisopropylethylamine (0.72 mL) and 3-hydroxyazetidin-1-ium chloride (271 mg) were added, and the mixture was stirred at 150°C for 1 hour. The reaction solution was concentrated, followed by silica chromatography under the conditions of dichloromethane/ethyl acetate/methanol, to obtain the target compound (E)-1-(6-(2-(3-methylbenzylidene)hydrazinyl)-9-(pyridin-3-yl)-9H-purin-2-yl)azetidin-3-ol (20 mg) as a white solid in a yield of 6.06%.

¹H NMR (400 MHz, DMSO-*d*₆) *δ* 11.54 (s, 1H), 9.19 (d, *J* = 2.4 Hz, 1H), 8.62 (dd, J = 4.8, 1.6 Hz, 1H), 8.39 (s, 1H), 8.41 - 8.37 (m, 1H), 8.30 - 8.22 (m, 1H), 7.70 - 7.60 (m, 1H), 7.58 (d, *J* = 8.0 Hz, 1H), 7.54 (s, 1H), 7.35 (t, *J =* 7.2 Hz, 1H), 7.21 (d, *J* = 7.6 Hz, 1H), 5.70 - 5.60 (m, 1H), 4.60 - 4.50 (m, 1H), 4.29 - 4.20 (m, 2H), 3.85 - 3.75 (m, 2H), 2.37 (s, 3H); MS: m/z = 401 (M+1, ESI+).

### Example 107. Synthesis of 4-(9-(pyridin-4-yl)-6-(3-(m-tolyl)-1H-pyrazol-1-yl)-9H-purin-2-yl)morpholine

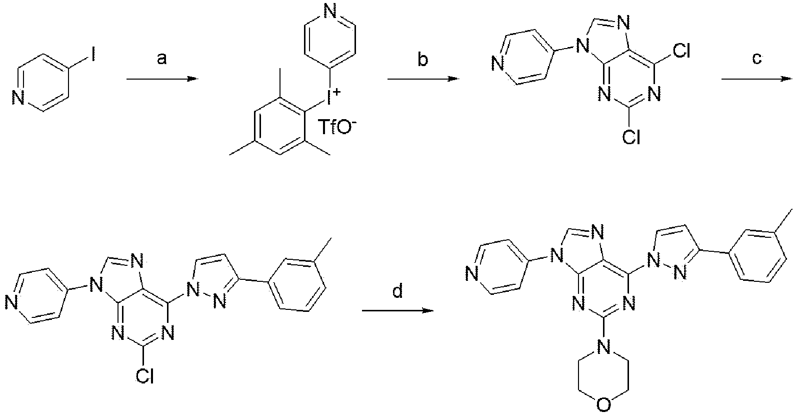

### (Step a) Synthesis of mesityl (pyridin-4-yl)iodonium trifluoromethane sulfonate

4-Iodopyridine (5.0 g) and m-chloroperoxybenzoic acid (7.43 g) were dissolved in dichloromethane (100.0 mL), then trifluoromethanesulfonic acid (14.63 g) was added at 0°C, and the reaction solution was stirred at 0°C for 2 hours. Then, mesitylene (3.22 g) was added and the mixture was stirred at 25°C for 3 hours. The reaction solution was extracted three times with water (30 mL) and ethyl acetate (40 mL), washed three times with a saturated NaCl solution (30 mL), and the organic layer was dried using anhydrous sodium sulfate and filtered under reduced pressure, and the filtrate was collected and concentrated under reduced pressure. The concentrate was subjected to silica chromatography to obtain the target compound mesityl (pyridin-4-yl) iodonium trifluoromethanesulfonate (4.3 g) as a yellow solid in a yield of 37.27%. MS: m/z = 324.1 (M-TfO⁻, ESI+).

### (Step b) Synthesis of 2,6-dichloro-9-(pyridin-4-yl)-9H-purine

Mesityl (pyridin-4-yl)iodonium trifluoromethanesulfonate (4.45 g) and 2,6-dichloro-9H-purine (1.37 g) were dissolved in dichloromethane (50 mL), then copper bromide (208 mg) and triethylamine (1.10 g) were added, and the mixture was stirred at 60°C for 8 hours. The reaction solution was extracted three times with water (25 mL) and dichloromethane (30 mL), washed three times with a saturated NaCl solution (25 mL), and the organic layer was dried using anhydrous sodium sulfate and filtered under reduced pressure, and the filtrate was collected and concentrated under reduced pressure. The concentrate was subjected to silica chromatography to obtain the target compound 2,6-dichloro-9-(pyridin-4-yl)-9H-purine (480 mg) as a yellow solid in a yield of 24.90%. MS: m/z = 266 (M+1, ESI+) .

### (Step c) Synthesis of 2-chloro-9-(pyridin-4-yl)-6-(3-(m-tolyl)-1H-pyrazol-1-yl)-9H-purine

3-(m-Tolyl)-1H-pyrazole (285 mg) was dissolved in dimethylformamide (15 mL), then sodium hydride (180 mg) was added at 0°C, and the mixture was stirred for 20 minutes. Then, 2,6-dichloro-9-(pyridin-4-yl)-9H-purine (400 mg) was added and stirred at 25°C for 3 hours. The reaction solution was extracted three times with water (15 mL) and ethyl acetate (15 mL), washed three times with a saturated NaCl solution (10 mL), and the organic layer was dried using anhydrous sodium sulfate and filtered under reduced pressure, and the filtrate was collected and concentrated under reduced pressure. The concentrate was subjected to silica chromatography to obtain the target compound 2-chloro-9-(pyridin-4-yl)-6-(3-(m-tolyl)-1H-pyrazol-1-yl)-9H-purine (100 mg) as a yellow solid in a yield of 17.23%. MS: m/z = 388.1(M+1, ESI+).

### (Step d) Synthesis of 4-(9-(pyridin-4-yl)-6-(3-(m-tolyl)-1H-pyrazol-1-yl)-9H-purin-2-yl)morpholine

2-Chloro-9-(pyridin-4-yl)-6-(3-(m-tolyl)-1H-pyrazol-1-yl)-9H-purine (100 mg) was dissolved in isopropyl alcohol (10 mL), then acetic acid (50 µL) and morpholine (225 mg) were added, and the mixture was stirred at 120°C overnight. The reaction solution was extracted three times with water (15 mL) and ethyl acetate (20 mL) and washed with a saturated NaCl solution, then the organic layer was dried using anhydrous sodium sulfate and filtered under reduced pressure, and the filtrate was collected and concentrated under reduced pressure. Then, the concentrate was subjected to silica chromatography under dichloromethane/methanol conditions to obtain the target compound 4-(9-(pyridin-4-yl)-6-(3-(m-tolyl)-1H-pyrazol-1-yl)-9H-purin-2-yl)morpholine (9 mg) as a yellow solid in a yield of 7.95%. MS: m/z = 439.2 (M+1, ESI+).

¹H NMR (400 MHz, DMSO-*d*₆) *δ* 9.12 (s, 1H), 8.89 (s, 1H), 8.77 (s, 2H), 8.16 (s, 2H), 8.0-7.75 (m, 2H), 7.40 - 7.30 (m, 1H), 7.28-7.15 (m, 2H), 3.84 (s, 4H), 3.74 (s, 4H), 2.40 (s, 3H).

### Example 108. Synthesis of (E)-4-(9-(5-methoxypyridin-2-yl)-6-(2-(3-methylbenzylidene)hydrazinyl)-9H-purin-2-yl)morpholine

The target compound (E)-4-(9-(5-methoxypyridin-2-yl)-6-(2-(3-methylbenzylidene)hydrazinyl)-9H-purin-2-yl)morpholine was obtained as a yellow solid in the same manner as in Example 96 above, except that 2-iodo-5-methoxypyridine was used instead of 3-iodopyridine in (Step a) .

¹H NMR (400 MHz, DMSO-*d*₆) *δ* 11.49 (s, 1H), 8.59 (s, 1H), 8.46 (d, *J* = 8.8 Hz, 1H), 8.32 (s, 1H), 8.26 (d, *J =* 2.8 Hz, 1H), 7.73 (dd, *J* = 8.8, 2.8 Hz, 1H), 7.58 (d, *J =* 7.6 Hz, 1H), 7.50 (s, 1H), 7.34 (t, *J* = 7.6 Hz, 1H), 7.21 (d, *J* = 7.6 Hz, 1H), 3.90 (s, 3H), 3.78 - 3.72 (m, 8H), 2.36 (s, 3H); MS: m/z = 445.3 (M+1, ESI+).

### Example 109. Synthesis of (E)-4-(6-(2-(3-methylbenzylidene)hydrazinyl)-9-(2-methylpyridin-3-yl)-9H-purin-2-yl)morpholine

The target compound (E)-4-(6-(2-(3-methylbenzylidene)hydrazinyl)-9-(2-methylpyridin-3-yl)-9H-purin-2-yl)morpholine was obtained as a yellow solid in the same manner as in Example 96 above, except that 3-iodo-2-methylpyridine was used instead of 3-iodopyridine in (Step a).

¹H NMR (400 MHz, DMSO-*d*₆) *δ* 11.46 (s, 1H), 8.60 (dd, J = 4.8, 1.6 Hz, 1H), 8.29 (s, 1H), 8.13 (s, 1H), 7.86 (dd, J = 8.0, 1.2 Hz, 1H), 7.59 (d, *J* = 7.6 Hz, 1H), 7.52 (s, 1H), 7.46 (dd, *J* = 8.0, 4.8 Hz, 1H), 7.34 (t, *J* = 7.2 Hz, 1H), 7.20 (d, *J* = 7.2 Hz, 1H), 3.62 (m, 8H), 2.37 (s, 3H), 2.36 (s, 3H); MS: m/z = 429.2 (M+1, ESI+).

### Example 110. Synthesis of (E)-4-(6-(2-(3-methylbenzylidene)hydrazinyl)-9-(6-methylpyridin-3-yl)-9H-purin-2-yl)morpholine

The target compound (E)-4-(6-(2-(3-methylbenzylidene)hydrazinyl)-9-(6-methylpyridin-3-yl)-9H-purin-2-yl)morpholine was obtained as a white solid in the same manner as in Example 96 above, except that 5-iodo-2-methylpyridine was used instead of 3-iodopyridine in (Step a).

¹H NMR (400 MHz, DMSO-*d*₆) *δ* 11.48 (s, 1H), 9.01 (d, *J* = 2.4 Hz, 1H), 8.43 (s, 1H), 8.30 (s, 1H), 8.21 (dd, *J* = 8.0, 2.4 Hz, 1H), 7.58 (d, *J* = 7.6 Hz, 1H), 7.52 (s, 1H), 7.48 (d, *J* = 8.4 Hz, 1H), 7.34 (t, *J* = 7.6 Hz, 1H), 7.20 (d, *J* = 7.2 Hz, 1H), 3.71 - 3.69 (m, 8H), 2.55 (s, 3H), 2.36 (s, 3H); MS: m/z = 429.3 (M+1, ESI+).

### Example 111. Synthesis of (E)-4-(9-(2,4-dimethylthiazol-5-yl)-6-(2-(3-methylbenzylidene)hydrazinyl)-9H-purin-2-yl)morpholine

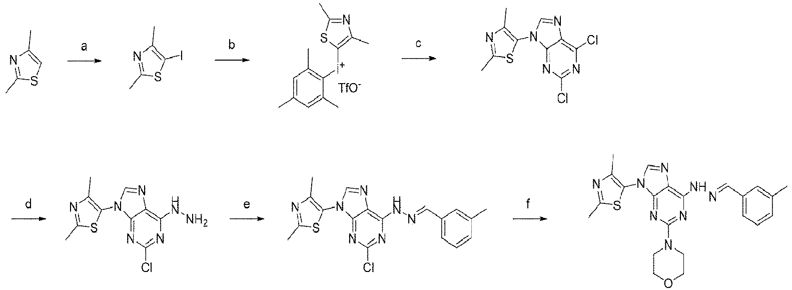

### (Step a) Synthesis of 5-iodo-2,4-dimethylthiazole

Chloroform (20 mL), N-iodosuccinimide (4.38 g), and trifluoroacetic acid (2.22 g) were added to 2,4-dimethylthiazole (1.1 g) at 0°C, and the mixture was stirred at 70°C for 16 hours. Distilled water was added to the reaction solution and extracted with ethyl acetate. After washing the organic solvent with a saturated NaCl solution, sodium sulfate was added, and the reaction mixture was filtered and concentrated. Then, the target compound 5-iodo-2,4-dimethylthiazole (2.12 g) was obtained as a white solid with a yield of 91.12% through silica chromatography. MS: m/z = 239.9 (M+1, ESI+).

The synthesis of (Step b) to (Step f) was performed in the same manner as in (Step a) to (Step e) of Example 96 to obtain the target compound (E)-4-(9-(2,4-dimethylthiazol-5-yl)-6-(2-(3-methylbenzylidene)hydrazinyl)-9H-purin-2-yl)morpholine as a white solid.

¹H NMR (400 MHz, DMSO-*d*₆) *δ* 11.47 (s, 1H), 8.27 (s, 1H), 8.16 (s, 1H), 7.58 (d, *J* = 7. 6 Hz, 1H), 7.51 (s, 1H), 7.33 (t, *J* = 7.6 Hz, 1H), 7.20 (d, *J* = 7.6 Hz, 1H), 3.66 (m, 8H), 2.67 (s, 3H), 2.36 (s, 3H), 2.24 (s, 3H); MS: m/z = 449.3 (M+1, ESI+).

### Example 112. Synthesis of (E)-4-(6-(2-((1H-indol-3-yl)methylene)hydrazinyl)-9-(pyridin-4-yl)-9H-purin-2-yl)morpholine

The target compound (E)-4-(6-(2-((1H-indol-3-yl)methylene)hydrazinyl)-9-(pyridin-4-yl)-9H-purin-2-yl)morpholine was obtained as a white solid in the same manner as in Example 96 above, except that 4-iodopyridine was used instead of 3-iodopyridine in (Step a), and 1H-indole-3-carbaldehyde was used instead of 3-methylbenzaldehyde in (Step d).

¹H NMR (400 MHz, DMSO-*d*₆) *δ* 11.42 - 11.27 (m, 2H), 8.75 - 8.43 (m, 5H), 8.24 (dd, *J* = 1.6, 4.8 Hz, 2H), 7.75 (d, *J* = 2.4 Hz, 1H), 7.42 (d, *J* = 8.0 Hz, 1H), 7.22 - 7.10 (m, 2H), 3.84 (s, 4H), 3.75 (s, 4H); MS: m/z = 440 (M+1, ESI+).

### Example 113. Synthesis of (E)-4-(9-(6-methoxypyridin-3-yl)-6-(2-(3-methylbenzylidene)hydrazinyl)-9H-purin-2-yl)morpholine

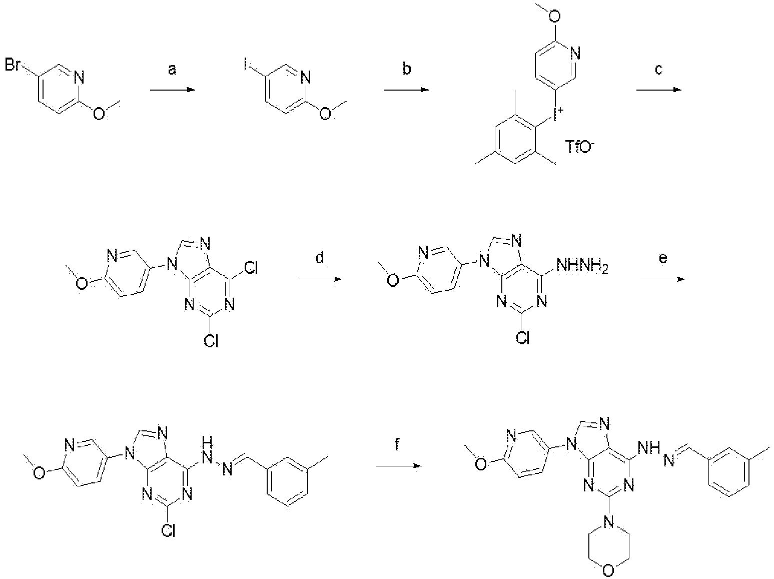

### (Step a) Synthesis of 5-iodo-2-methoxypyridine

5-Bromo-2-methoxypyridine (2.0 g) was dissolved in 1,4-dioxane (20 mL), and then sodium iodide (3.18 g), N¹,N²-dimethylethene-1,2-diamine (468 mg), and copper iodide (405 mg) were added. The mixture was stirred at 110°C for 16 hours. After filtering the reaction solution, ethyl acetate was added. The reaction solution was concentrated, followed by silica chromatography under benzene conditions, to obtain the target compound 5-iodo-2-methoxypyridine (1.5 g) as a brown oil with a yield of 59.68%. MS: m/z = 235.9 (M+1, ESI+).

The synthesis of (Step b) to (Step f) was performed in the same manner as in (Step a) to (Step e) of Example 96 to obtain the target compound (E)-4-(9-(6-methoxypyridin-3-yl)-6-(2-(3-methylbenzylidene)hydrazinyl)-9H-purin-2-yl)morpholine as a white solid.

¹H NMR (400 MHz, DMSO-*d*₆) *δ* 11.46 (s, 1H), 8.68 (s, 1H), 8.35 (s, 1H), 8.30 (s, 1H), 8.21 (d, *J* = 8.7 Hz, 1H), 7.58 (d, *J* = 7.3 Hz, 1H), 7.51 (s, 1H), 7.33 (t, *J* = 7.5 Hz, 1H), 7.20 (d, *J* = 7.2 Hz, 1H), 7.04 (d, *J* = 8.8 Hz, 1H), 3.92 (s, 3H), 3.69 (d, *J* = 7.2 Hz, 8H), 2.36 (s, 3H); MS: m/z = 445.1 (M+1, ESI+).

### Example 114. Synthesis of (E)-5-(6-(2-(3-methylbenzylidene)hydrazinyl)-2-morpholino-9H-purin-9-yl)pyridin-2-ol

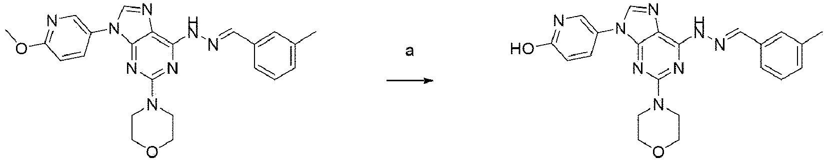

### (Step a) Synthesis of (E) -5-(6-(2-(3-methylbenzylidene)hydrazinyl)-2-morpholino-9H-purin-9-yl)pyridin-2-ol

The compound (E)-4-(9-(6-methoxypyridin-3-yl)-6-(2-(3-methylbenzylidene)hydrazinyl)-9H-purin-2-yl)morpholine (105 mg), which was prepared in Example 113 above, was dissolved in acetonitrile (5 mL), then sodium iodide (142 mg) and trimethylsilyl chloride (102 mg) were added. The mixture was stirred at 80°C for 2 hours. The pH of the reaction solution was adjusted to 8 using sodium carbonate. The solid formed by adding water was filtered to obtain the target compound (E)-5-(6-(2-(3-methylbenzylidene)hydrazinyl)-2-morpholino-9H-purin-9-yl)pyridin-2-ol (60 mg) as a white solid in a yield of 59%.

¹H NMR (400 MHz, DMSO-*d*₆) *δ* 11.41 (s, 1H), 8.28 (s, 1H), 8.17 (s, 1H), 7.97 (s, 1H), 7.80 (dd, *J* = 9.5, 2.7 Hz, 1H), 7.57 (d, *J* = 7.5 Hz, 1H), 7.50 (s, 1H), 7.33 (t, *J* = 7.6 Hz, 1H), 7.20 (d, *J* = 7.6 Hz, 1H), 6.45 (d, *J* = 9.5 Hz, 1H), 3.69 - 3.68 (m, 8H), 2.36 (s, 3H); MS: m/z = 431.0 (M+1, ESI+) .

### Example 115. Synthesis of (E)-4-(9-(2-chloropyridin-4-yl)-6-(2-(3-methylbenzylidene)hydrazinyl)-9H-purin-2-yl)morpholine

The target compound (E)-4-(9-(2-chloropyridin-4-yl)-6-(2-(3-methylbenzylidene)hydrazinyl)-9H-purin-2-yl)morpholine was obtained as a yellow solid in the same manner as in Example 96 above, except that 2-chloro-4-iodopyridine was used instead of 3-iodopyridine in (Step a).

¹H NMR (400 MHz, DMSO-*d*₆) *δ* 11.54 (s, 1H), 8.73 (s, 1H), 8.56 (d, *J* = 5.5 Hz, 1H), 8.32 (dd, *J* = 13.2, 7.6 Hz, 3H), 7.57 (d, *J* = 7.5 Hz, 1H), 7.51 (s, 1H), 7.33 (t, *J* = 7.6 Hz, 1H), 7.20 (d, *J* = 7.4 Hz, 1H), 3.76 (d, *J* = 3.8 Hz, 4H), 3.72 (d, *J* = 3.9 Hz, 4H), 2.35 (s, 3H); MS: m/z = 449.1 (M+1, ESI+).

### Example 116. Synthesis of (E)-4-(9-(5-chloropyridin-2-yl)-6-(2-(3-methylbenzylidene)hydrazinyl)-9H-purin-2-yl)morpholine

The target compound (E)-4-(9-(5-chloropyridin-2-yl)-6-(2-(3-methylbenzylidene)hydrazinyl)-9H-purin-2-yl)morpholine was obtained in the same manner as in Example 96 above, except that 5-chloro-2-iodopyridine was used instead of 3-iodopyridine in (Step a).

¹H NMR (400 MHz, DMSO-*d*₆) *δ* 11.51 (s, 1H), 8.67 (s, 1H), 8.64 - 8.62 (m, 2H), 8.30 (s, 1H), 8.22 (dd, *J* = 8.8, 2.8 Hz, 1H), 7.58 (d, *J* = 8.0 Hz, 1H), 7.51 (s, 1H), 7.34 (t, *J* = 7.6 Hz, 1H), 7.21 (d, *J* = 7.2 Hz, 1H), 3.80 - 3.72 (m, 8H), 2.36 (s, 3H); MS: m/z = 449.2 (M+1, ESI+).

### Example 117. Synthesis of (E)-4-(6-(2-(3-methylbenzylidene)hydrazinyl)-9-(thiazol-2-yl)-9H-purin-2-yl)morpholine

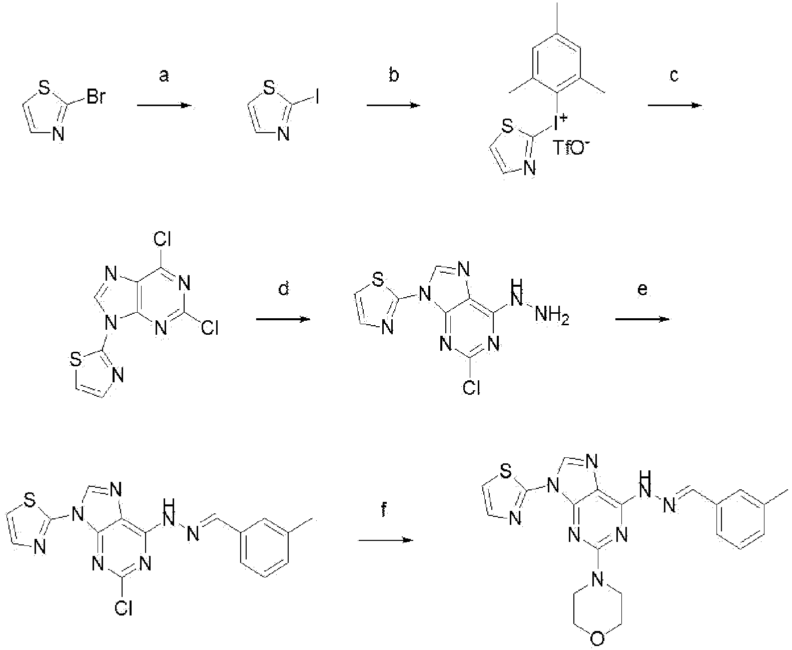

### (Step a) Synthesis of 2-iodothiazole

2-Bromothiazole (3.1 g) was dissolved in 1,4-dioxane (50 mL), and then sodium iodide (5.71 g), N¹,N²-dimethylethene-1,2-diamine (0.84 g), and copper iodide (0.72 g) were added. The mixture was stirred at 110°C overnight. Next, the reaction solution was filtered and concentrated, followed by silica chromatography, to obtain the target compound 2-iodothiazole (2.0 g) as a yellow solid in a yield of 49.84%. MS: m/z = 212.0 (M+1, ESI+).

The synthesis of (Step b) to (Step f) was performed in the same manner as (Step a) to (Step e) of Example 96 to obtain the target compound (E)-4-(6-(2-(3-methylbenzylidene)hydrazinyl)-9-(thiazol-2-yl)-9H-purin-2-yl)morpholine (15.0 mg) as a white solid in a yield of 18.3%.

¹H NMR (400 MHz, CDCl₃) *δ* 10.00 (s, 1H), 8.03 (s, 1H), 7.69 (s, 1H), 7.63 (d, *J* = 7.2 Hz, 1H), 7.53 (d, *J* = 3.6 Hz, 1H), 7.39 (t, *J* = 7.2 Hz, 1H), 7.33 (d, *J* = 8.0 Hz, 1H), 6.98 (d, *J* = 3.6 Hz, 1H), 3.96 - 3.88 (m, 8H), 2.45 (s, 3H); MS: m/z = 421.2 (M+1, ESI+).

### Example 118. Synthesis of 4-(9-(pyridin-3-yl)-6-(3-(m-tolyl)-1H-pyrazol-1-yl)-9H-purin-2-yl)morpholine

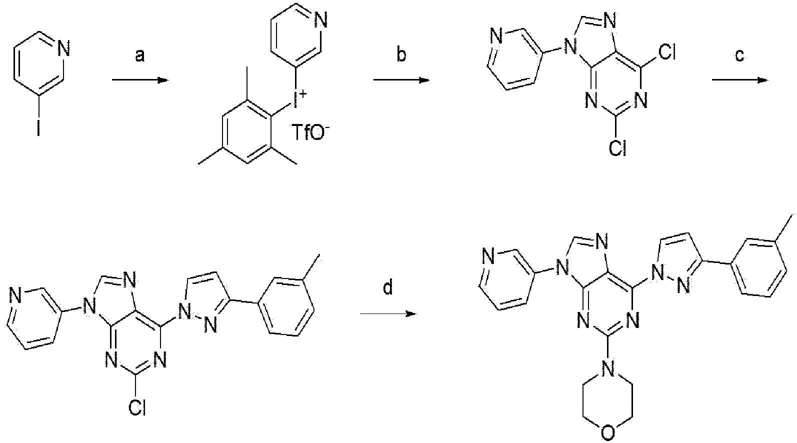

### (Step a) Synthesis of mesityl(pyridin-3-yl)iodonium trifluoromethane sulfonate

3-Iodopyridine (3.0 g) and 3-chlorobenzoperoxoic acid (4.46 g) were dissolved in dichloromethane (100 mL), then trifluoromethanesulfonic acid (8.78 g) was added at 0°C, and the reaction solution was stirred at 0°C for 2 hours. Then, mesitylene (1.93 g) was added at 0°C, and the reaction solution was stirred at 25°C for 3 hours. Distilled water was added to the reaction solution and extracted with ethyl acetate. After washing the organic solvent with a saturated NaCl solution, sodium sulfate was added, and the reaction mixture was filtered and concentrated. The concentrate was subjected to silica chromatography to obtain the target compound mesityl(pyridin-3-yl)iodonium trifluoromethanesulfonate (1.4 g) as a yellow solid in a yield of 29.51%. MS: m/z = 325.03 (M-TfO⁻, ESI+).

### (Step b) Synthesis of 2,6-dichloro-9-(pyridin-3-yl)-9H-purine

2,6-Dichloro-9H-purine (0.41 g) and mesityl(pyridin-3-yl)iodonium trifluoromethanesulfonate (1.4 g) were dissolved in dichloromethane (50 mL), then triethylamine (437 mg) and copper bromide (62 mg) were added, and the reaction solution was stirred at 60°C for 8 hours. Distilled water was added to the reaction solution and extracted with dichloromethane. After washing the organic solvent with a saturated NaCl solution, sodium sulfate was added, and the reaction mixture was filtered and concentrated. Then, the resulting product was subjected to silica chromatography to obtain the target compound 2,6-dichloro-9-(pyridin-3-yl)-9H-purine (500 mg) as a yellow solid in a yield of 87.03%. MS: m/z = 266.0 (M+1, ESI+).

### (Step c) Synthesis of 2-chloro-9-(pyridin-3-yl)-6-(3-(m-tolyl)-1H-pyrazol-1-yl)-9H-purine

Dimethylformamide (15 mL) was added to 3-(m-tolyl)-1H-pyrazole (200 mg), and sodium hydride (60%) (101 mg) was added at 0°C. After stirring at 0°C for 20 minutes, 2,6-dichloro-9-(pyridin-3-yl)-9H-purine (225 mg) was added. The reaction solution was stirred at 25°C for 3 hours. The reaction was terminated by adding distilled water, and then the reaction mixture was extracted with ethyl acetate. After washing the organic solvent with a saturated NaCl solution, sodium sulfate was added, and the reaction mixture was filtered and concentrated. The concentrate was subjected to silica chromatography to obtain the target compound 2-chloro-9-(pyridin-3-yl)-6-(3-(m-tolyl)-1H-pyrazol-1-yl)-9H-purine (200 mg) as a yellow solid in a yield of 60.99%. MS: m/z = 388.1 (M+1, ESI+).

### (Step d) Synthesis of 4-(9-(pyridin-3-yl)-6-(3-(m-tolyl)-1H-pyrazol-1-yl)-9H-purin-2-yl)morpholine

2-Chloro-9-(pyridin-3-yl)-6-(3-(m-tolyl)-1H-pyrazol-1-yl)-9H-purine (200 mg) and morpholine (449 mg) were dissolved in isopropyl alcohol (10 mL), then acetic acid (0.05 mL) was added, and the mixture was stirred at 120°C overnight. Distilled water was added to the reaction solution and extracted with ethyl acetate. After washing the organic solvent with a saturated NaCl solution, sodium sulfate was added, and the reaction mixture was filtered and concentrated. Then, the concentrate was subjected to silica chromatography under dichloromethane/methanol conditions to obtain the target compound 4-(9-(pyridin-3-yl)-6-(3-(m-tolyl)-1H-pyrazol-1-yl)-9H-purin-2-yl)morpholine (3.61 mg) as a yellow solid in a yield of 1.60%.

¹H NMR (400 MHz, DMSO-*d*₆) *δ* 9.19 (t, *J* = 2.9 Hz, 2H), 8.79 (s, 1H), 8.69 - 8.68 (m, 1H), 8.41 (d, *J* = 9.3 Hz, 1H), 7.84 (d, *J* = 10.3 Hz, 2H), 7.70 (dd, *J* = 8.2, 4.9 Hz, 1H), 7.41 (t, *J* = 7.6 Hz, 1H), 7.26 (d, *J* = 7.7 Hz, 1H), 7.22 (d, *J* = 2.7 Hz, 1H), 3.83 (d, *J* = 4.6 Hz, 4H), 3.75 (d, *J* = 4.8 Hz, 4H), 2.42 (s, 3H); MS: m/z = 439.3 (M+1, ESI+).

### Example 119. Synthesis of (E)-4-(6-(2-(3-methylbenzylidene)hydrazinyl)-9-phenyl-9H-purin-2-yl)morpholine

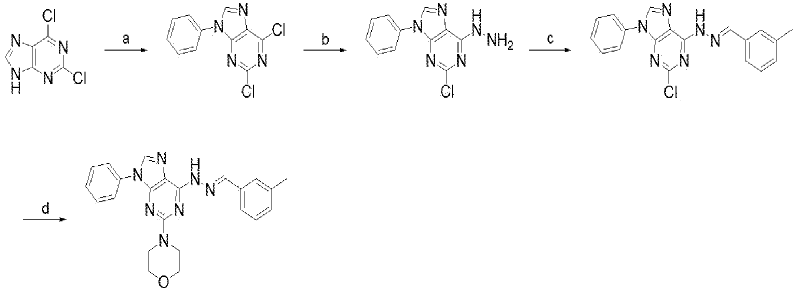

### (Step a) Synthesis of 2,6-dichloro-9-phenyl-9H-purine

2,6-Dichloro-9H-purine (5.0 g) and o-phenanthroline (9.53 g) were dissolved in dichloromethane (200 mL), then phenylboronic acid (9.68 g) and copper (II) acetate (4.81 g) were added, and the mixture was heated to 40°C under oxygen conditions and stirred for 24 hours. Distilled water was added to the reaction solution, and the resulting compound was extracted using dichloromethane, and then concentrated under reduced pressure. The concentrated product was subjected to silica chromatography under ethyl acetate/benzene conditions to obtain the target compound 2,6-dichloro-9-phenyl-9H-purine (2.2 g) as a white solid in a yield of 31%. MS: m/z = 264.9 (M+1, ESI+).

### (Step b) Synthesis of 2-chloro-6-hydrazinyl-9-phenyl-9H-purine

2,6-Dichloro-9-phenyl-9H-purine (1.9 g) was dissolved in tetrahydrofuran (30 mL), then hydrazine monohydrate (1.44 g) was added, and the mixture was stirred at room temperature for 20 minutes. After the reaction solution was concentrated under reduced pressure, distilled water was added to form a solid. The formed solid was filtered under reduced pressure and dried to obtain the target compound 2-chloro-6-hydrazinyl-9-phenyl-9H-purine (1.80 g) as a white solid with a yield of 96%. MS: m/z = 261.3 (M+1, ESI+)

### (Step c) Synthesis of (E)-2-chloro-6-(2-(3-methylbenzylidene)hydrazinyl)-9-phenyl-9H-purine

2-Chloro-6-hydrazinyl-9-phenyl-9H-purine (1.60 g), 3-methylbenzaldehyde (1.47 g), and acetic acid (37 mg) were dissolved in methanol (20 mL), and the mixture was stirred at 70°C for 30 minutes. After concentrating the reaction solution under reduced pressure, the target compound (E)-2-chloro-6-(2-(3-methylbenzylidene)hydrazinyl)-9-phenyl-9H-purine (1.1 g) was obtained as a white solid in a yield of 49% through silica chromatography under dichloromethane/methanol conditions. MS: m/z = 363.3(M+1, ESI+).

### (Step d) Synthesis of (E)-4-(6-(2-(3-methylbenzylidene)hydrazinyl)-9-phenyl-9H-purin-2-yl)morpholine

(E)-2-Chloro-6-(2-(3-methylbenzylidene)hydrazinyl)-9-phenyl-9H-purine (350 mg) and morpholine (420 mg) were dissolved in isopropyl alcohol (10 mL), then acetic acid (58 mg) was added, and the mixture was stirred at 120°C for 24 hours. Distilled water was added to the reaction solution, and the compound was extracted into the organic layer using ethyl acetate, followed by concentration. The concentrated product was subjected to silica chromatography under dichloromethane/methanol conditions to obtain the target compound (E)-4-(6-(2-(3-methylbenzylidene)hydrazinyl)-9-phenyl-9H-purin-2-yl)morpholine (156 mg) as a white solid in a yield of 13.8%.

¹H NMR (400 MHz, DMSO-*d*₆) *δ* 11.45 (s, 1H), 8.41 (s, 1H), 8.30 (s, 1H), 7.93 (d, *J* = 7.6 Hz, 2H), 7.62 - 7.55 (m, 3H), 7.52 (s, 1H), 7.41 (t, *J* = 7.2 Hz, 1H), 7.34 (t, *J* = 7.6 Hz, 1H), 7.20 (d, *J* = 7.6 Hz, 1H), 3.77 - 3.66 (m, 8H), 2.36 (s, 3H); MS: m/z = 414.1 (M+1, ESI+).

### Example 120. Synthesis of (E)-4-(9-(4-methoxyphenyl)-6-(2-(3-methylbenzylidene)hydrazinyl)-9H-purin-2-yl)morpholine

The target compound (E)-4-(9-(4-methoxyphenyl)-6-(2-(3-methylbenzylidene)hydrazinyl)-9H-purin-2-yl)morpholine was obtained as a white solid in the same manner as in Example 119, except that (4-methoxyphenyl)boronic acid was used instead of phenylboronic acid in (Step a).

¹H NMR (400 MHz, DMSO-*d*₆) *δ* 11.41 (s, 1H), 8.29 (s, 2H), 7.78 (d, *J* = 8.8 Hz, 2H), 7.58 (d, *J* = 7.6 Hz, 1H), 7.51 (s, 1H), 7.33 (t, *J* = 7.6 Hz, 1H), 7.19 (d, *J* = 7.5 Hz, 1H), 7.12 (d, *J* = 8.9 Hz, 2H), 3.82 (s, 3H), 3.69 (d, *J* = 7.5 Hz, 8H), 2.35 (s, 3H); MS: m/z = 444.5 (M+1, ESI+).

### Example 121. Synthesis of (E)-4-(9-(3-methoxyphenyl)-6-(2-(3-methylbenzylidene)hydrazinyl)-9H-purin-2-yl)morpholine

The target compound (E)-4-(9-(3-methoxyphenyl)-6-(2-(3-methylbenzylidene)hydrazinyl)-9H-purin-2-yl)morpholine was obtained as a white solid in the same manner as in Example 119, except that (3-methoxyphenyl)boronic acid was used instead of phenylboronic acid in (Step a).

¹H NMR (400 MHz, DMSO-*d*₆) *δ* 11.46 (s, 1H), 8.46 (s, 1H), 8.30 (s, 1H), 7.62-7.32 (m, 5H), 7.34 (t, *J* = 7.6 Hz, 1H), 7.20 (d, *J* = 7.5 Hz, 1H), 6.97 (d, *J* = 8.1 Hz, 1H), 3.84 (s, 3H), 3.71 (dd, *J* = 15.0, 4.2 Hz, 8H), 2.36 (s, 3H); MS: m/z = 444.5 (M+1, ESI+).

### Example 122. Synthesis of (E)-4-(9-(3-chlorophenyl)-6-(2-(3-methylbenzylidene)hydrazinyl)-9H-purin-2-yl)morpholine

The target compound (E)-4-(9-(3-chlorophenyl)-6-(2-(3-methylbenzylidene)hydrazinyl)-9H-purin-2-yl)morpholine was obtained as a white solid in the same manner as in Example 119, except that (3-chlorophenyl)boronic acid was used instead of phenylboronic acid in (Step a).

¹H NMR (400 MHz, DMSO-*d*₆) *δ* 11.47 (s, 1H), 8.49 (s, 1H), 8.31 (s, 1H), 8.13 (t, *J* = 1.9 Hz, 1H), 8.00 (d, *J =* 8.0 Hz, 1H), 7.63 - 7.56 (m, 2H), 7.46 (dd, *J* = 8.0, 1.8 Hz, 1H), 7.34 (t, *J* = 7.6 Hz, 1H), 7.20 (d, *J* = 7.5 Hz, 1H), 3.72 (dd, *J* = 15.6, 4.6 Hz, 8H), 2.36 (s, 3H); MS: m/z = 448.2 (M+1, ESI+).

### Example 123. Synthesis of (E)-N,N-dimethyl-4-(6-(2-(3-methylbenzylidene)hydrazinyl)-2-morpholino-9H-purin-9-yl)aniline

The target compound (E)-N,N-dimethyl-4-(6-(2-(3-methylbenzylidene)hydrazinyl)-2-morpholino-9H-purin-9-yl)aniline was obtained as a white solid in the same manner as in Example 119, except that (4-(dimethylamino)phenyl)boronic acid was used instead of phenylboronic acid in (Step a).

¹H NMR (400 MHz, DMSO-*d*₆) *δ* 11.39 (s, 1H), 8.30 (s, 1H), 8.21 (s, 1H), 7.64 - 7.55 (m, 3H), 7.51 (s, 1H), 7.40-7.25 (m, 1H), 7.19 (d, *J* = 6.6 Hz, 1H), 6.85 (d, *J* = 8.1 Hz, 2H), 3.69 (d, *J* = 9.2 Hz, 8H), 2.94 (s, 6H), 2.35 (s, 3H); MS: m/z = 456.2 (M+1, ESI+).

### Example 124. Synthesis of (E)-4-(9-(2-methoxyphenyl)-6-(2-(3-methylbenzylidene)hydrazinyl)-9H-purin-2-yl)morpholine

The target compound (E)-4-(9-(2-methoxyphenyl)-6-(2-(3-methylbenzylidene)hydrazinyl)-9H-purin-2-yl)morpholine was obtained as a white solid in the same manner as in Example 119, except that (2-methoxyphenyl)boronic acid was used instead of phenylboronic acid in (Step a).

¹H NMR (400 MHz, DMSO-*d*₆) *δ* 11.33 (s, 1H), 8.27 (s, 1H), 8.01 (s, 1H), 7.52 (t, *J* = 20.6 Hz, 4H), 7.30 (d, *J* = 15.9 Hz, 2H), 7.16 (d, *J* = 29.0 Hz, 2H), 3.79 (s, 4H), 3.62 (s, 7H), 2.34 (s, 3H); MS: m/z = 444.2 (M+1, ESI+).

### Example 125. Synthesis of (E)-4-(9-(3-cyclopropoxyphenyl)-6-(2-(3-methylbenzylidene)hydrazinyl)-9H-purin-2-yl)morpholine

The target compound (E)-4-(9-(3-cyclopropoxyphenyl)-6-(2-(3-methylbenzylidene)hydrazinyl)-9H-purin-2-yl)morpholine was obtained as a white solid in the same manner as in Example 119, except that (3-cyclopropoxyphenyl)boronic acid was used instead of phenylboronic acid in (Step a).

¹H NMR (400 MHz, DMSO-*d*₆) *δ* 11.43 (s, 1H), 8.41 (s, 1H), 8.31 (s, 1H), 7.74 (s, 1H), 7.59 (d, *J* = 7.6 Hz, 1H), 7.60 - 7.45 (m, 3H), 7.32 (t, *J* = 7.6 Hz, 1H), 7.18 (d, *J* = 7.4 Hz, 1H), 7.03 (d, *J* = 7.9 Hz, 1H), 4.00 - 3.85 (m, 1H), 3.75 (s, 4H), 3.68 (s, 4H), 2.34 (s, 3H), 0.85-0.60 (m, 4H); MS: m/z = 470.2 (M+1, ESI+).

### Example 126. Synthesis of (E)-3-(6-(2-(3-methylbenzylidene)hydrazinyl)-2-morpholino-9H-purin-9-yl)benzonitrile

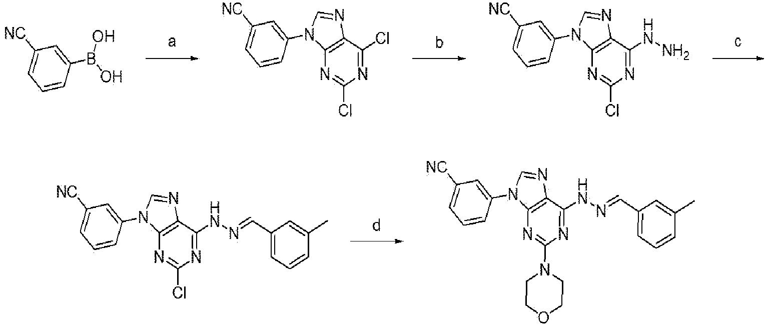

The target compound (E)-3-(6-(2-(3-methylbenzylidene)hydrazinyl)-2-morpholino-9H-purin-9-yl)benzonitrile was obtained as a white solid in the same manner as in Example 119, except that (3-cyanophenyl)boronic acid was used instead of phenylboronic acid in (Step a).

¹H NMR (400 MHz, DMSO-*d*₆) *δ* 11.48 (s, 1H), 8.52 (s, 1H), 8.47 - 8.38 (m, 2H), 8.30 (s, 1H), 7.87 (d, *J* = 7.6 Hz, 1H), 7.80 (t, *J* = 8.0 Hz, 1H), 7.58 (d, *J* = 7.6 Hz, 1H), 7.52 (s, 1H), 7.34 (t, *J* = 7.4 Hz, 1H), 7.21 (d, *J* = 7.2 Hz, 1H), 3.72 (dd, *J* = 15.2, 4.0 Hz, 8H), 2.36 (s, 3H); MS: m/z = 439.2 (M+1, ESI+).

### Example 127. Synthesis of (E)-4-(6-(2-(3-methylbenzylidene)hydrazinyl)-9-(m-tolyl)-9H-purin-2-yl)morpholine

The target compound (E)-4-(6-(2-(3-methylbenzylidene)hydrazinyl)-9-(m-tolyl)-9H-purin-2-yl)morpholine was obtained as a yellow solid in the same manner as in Example 119, except that m-tolylboronic acid was used instead of phenylboronic acid in (Step a).

¹H NMR (400 MHz, DMSO-*d*₆) *δ* 11.43 (s, 1H), 8.38 (s, 1H), 8.30 (s, 1H), 7.77 - 7.71 (m, 2H), 7.58 (d, *J* = 7.6 Hz, 1H), 7.51 (s, 1H), 7.46 (t, *J* = 8.0 Hz, 1H), 7.34 (t, *J* = 7.6 Hz, 1H), 7.21 (t, *J* = 8.4 Hz, 2H), 3.78 - 3.63 (m, 8H), 2.41 (s, 3H), 2.36 (s, 3H); MS: m/z = 428.2 (M+1, ESI+).

### Example 128. Synthesis of (E)-4-(6-(2-(3-methylbenzylidene)hydrazinyl)-9-(3-(trifluoromethoxy)phenyl)-9H-purin-2-yl)morpholine

The target compound (E)-4-(6-(2-(3-methylbenzylidene)hydrazinyl)-9-(3-(trifluoromethoxy)phenyl)-9H-purin-2-yl)morpholine was obtained as a yellow solid in the same manner as in Example 119, except that (3-(trifluoromethoxy)phenyl)boronic acid was used instead of phenylboronic acid in (Step a).

¹H NMR (400 MHz, DMSO-*d*₆) *δ* 11.49 (s, 1H), 8.53 (s, 1H), 8.30 (s, 1H), 8.18 (s, 1H), 8.01 (d, *J* = 8.0 Hz, 1H), 7.71 (t, *J* = 8.2 Hz, 1H), 7.58 (d, *J* = 7.5 Hz, 1H), 7.52 (s, 1H), 7.39 (d, *J* = 7.9 Hz, 1H), 7.33 (t, *J* = 7.5 Hz, 1H), 7.20 (d, *J* = 7.3 Hz, 1H), 3.71 (dd, *J* = 18.6, 3.7 Hz, 8H), 2.36 (s, 3H); MS: m/z = 498.3 (M+1, ESI+).

### Example 129. Synthesis of (E)-4-(9-(3-fluorophenyl)-6-(2-(3-methylbenzylidene)hydrazinyl)-9H-purin-2-yl)morpholine

The target compound (E)-4-(9-(3-fluorophenyl)-6-(2-(3-methylbenzylidene)hydrazinyl)-9H-purin-2-yl)morpholine was obtained as a white solid in the same manner as in Example 119, except that (3-fluorophenyl)boronic acid was used instead of phenylboronic acid in (Step a).

¹H NMR (400 MHz, DMSO-*d*₆) *δ* 11.49 (s, 1H), 8.50 (s, 1H), 8.30 (s, 1H), 7.97 - 7.91 (m, 1H), 7.91 - 7.86 (m, 1H), 7.67 - 7.56 (m, 2H), 7.52 (s, 1H), 7.34 (t, *J* = 7.6 Hz, 1H), 7.28 - 7.19 (m, 2H), 3.77 - 3.67 (m, 8H), 2.36 (s, 3H); MS: m/z = 432.2 (M+1, ESI+).

### Example 130. Synthesis of (E)-4-(6-(2-(3-methylbenzylidene)hydrazinyl)-9-(3-(trifluoromethyl)phenyl)-9H-purin-2-yl)morpholine

The target compound (E)-4- (6- (2- (3-methylbenzylidene)hydrazinyl)-9-(3-(trifluoromethyl)phenyl)-9H-purin-2-yl)morpholine was obtained as a pale yellow solid in the same manner as in Example 119, except that (3-(trifluoromethyl)phenyl)boronic acid was used instead of phenylboronic acid in (Step a).

¹H NMR (400 MHz, DMSO-*d*₆) *δ* 11.48 (s, 1H), 8.57 (s, 1H), 8.48 (s, 1H), 8.37 - 8.20 (m, 2H), 7.82 (t, *J* = 8.0 Hz, 1H), 7.75 (d, *J* = 7.6 Hz, 1H), 7.58 (d, *J* = 7.6 Hz, 1H), 7.52 (s, 1H), 7.33 (t, *J* = 7.2 Hz, 1H), 7.20 (d, *J* = 7.2 Hz, 1H), 3.79 - 3.64 (m, 8H), 2.36 (s, 3H); MS: m/z = 482.6 (M+1, ESI+) .

### Example 131. Synthesis of (E)-3-(6-(2-(3-methylbenzylidene)hydrazinyl)-2-morpholino-9H-purin-9-yl)phenol

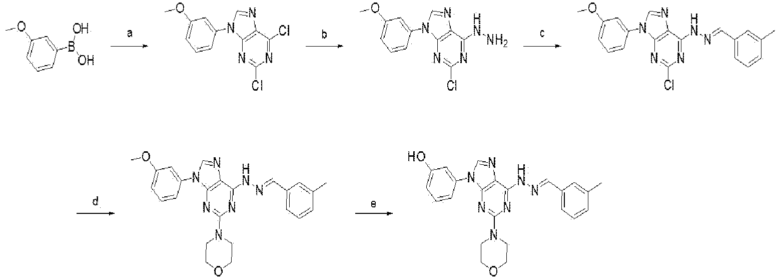

The target compound (E)-3-(6-(2-(3-methylbenzylidene)hydrazinyl)-2-morpholino-9H-purin-9-yl)phenol was obtained in the same manner as in Example 119, except that (3-methoxyphenyl)boronic acid was used instead of phenylboronic acid in (Step a), and (step e) was performed as follows.

### (Step e) Synthesis of (E)-3-(6-(2-(3-methylbenzylidene)hydrazinyl)-2-morpholino-9H-purin-9-yl)phenol

(E)-4-(9-(4-Methoxyphenyl)-6-(2-(3-methylbenzylidene)hydrazinyl)-9H-purin-2-yl)morpholine (100 mg) was dissolved in dichloromethane (10 mL), then boron tribromide (250 mg) was added at 0°C, and the mixture was stirred for 30 minutes. The reaction solution was extracted three times with water (20 mL) and ethyl acetate (30 mL) and washed with a saturated NaCl solution, then the organic layer was dried using anhydrous sodium sulfate and filtered under reduced pressure, and the filtrate was collected and concentrated under reduced pressure. The concentrated product was subjected to silica chromatography under dichloromethane/methanol conditions to obtain the target compound (E)-3-(6-(2-(3-methylbenzylidene)hydrazinyl)-2-morpholino-9H-purin-9-yl)phenol (48.5 mg) as a white solid in a yield of 50%.

¹H NMR (400 MHz, DMSO-*d*₆) *δ* 11.41 (s, 1H), 8.35 (s, 1H), 8.29 (s, 1H), 7.57 (d, *J* = 7.6 Hz, 1H), 7.51 (s, 1H), 7.40 (s, 1H), 7.35-7.25 (m, 3H), 7.20 (d, *J* = 7.4 Hz, 1H), 6.80 (d, *J* = 7.7 Hz, 1H), 3.71 (d, *J* = 11.1 Hz, 8H), 2.35 (s, 3H); MS: m/z = 430.4 (M+1, ESI+).

### Example 132. Synthesis of (E)-4-(6-(2-((1H-indol-3-yl)methylene)hydrazinyl)-9-phenyl-9H-purin-2-yl)morpholine

The target compound (E)-4-(6-(2-((1H-indol-3-yl)methylene)hydrazinyl)-9-phenyl-9H-purin-2-yl)morpholine was obtained as a white solid in the same manner as in Example 119 above, except that 1H-indole-3-carbaldehyde was used instead of 3-methylbenzaldehyde in (Step c).

¹H NMR (400 MHz, DMSO-*d*₆) *δ* 11.46 (s, 1H), 11.26 (s, 1H), 8.52 - 8.42 (m, 3H), 7.97 (d, *J* = 8.0 Hz, 2H), 7.73 (s, 1H), 7.59 (t, *J* = 8.0 Hz, 2H), 7.42 (t, *J* = 8.2 Hz, 2H), 7.22 - 7.20 (m, 1H), 7.18 - 7.15 (m, 1H), 3.80 (s, 4H), 3.73 (s, 4H).3.96 - 3.93 (m, 1H), 3.74 (d, *J* = 10.8 Hz, 1H), 3.63 - 3.59 (m, 1H), 3.4 (s, 1H), 3.2 - 3.1 (m, 1H), 2.37 (s, 3H), 1.2 (s, 3H); MS: m/z = 439 (M+1, ESI+).

### Example 133. Synthesis of (E)-4-(6-(2-((1-methyl-1H-indol-3-yl)methylene)hydrazinyl)-9-phenyl-9H-purin-2-yl)morpholine

The target compound (E)-4-(6-(2-((1-methyl-1H-indol-3-yl)methylene)hydrazinyl)-9-phenyl-9H-purin-2-yl)morpholine was obtained as a yellow solid in the same manner as in Example 119 above, except that 1-methyl-1H-indole-3-carbaldehyde was used instead of 3-methylbenzaldehyde in (Step c).

¹H NMR (400 MHz, DMSO-*d*₆) *δ* 11.20 (s, 1H), 8.81 - 8.68 (m, 1H), 8.49 (s, 1H), 8.41 (s, 1H), 7.94 (d, *J* = 12.4 Hz, 2H), 7.72 (s, 2H), 7.59 (t, *J* = 7.8 Hz, 2H), 7.48 (d, *J* = 8.0 Hz, 1H), 7.41 (t, *J* = 7.4 Hz, 1H), 7.27 (t, *J* = 7.6 Hz, 1H), 7.18 (t, *J* = 7.4 Hz, 1H), 3.83 (s, 3H), 3.79 (s, 4H), 3.73 (s, 4H); MS: m/z = 453 (M+1, ESI+).

### Example 134. Synthesis of 4-(9-(4-methoxyphenyl)-6-(3-(m-tolyl)-1H-pyrazol-1-yl)-9H-purin-2-yl)morpholine

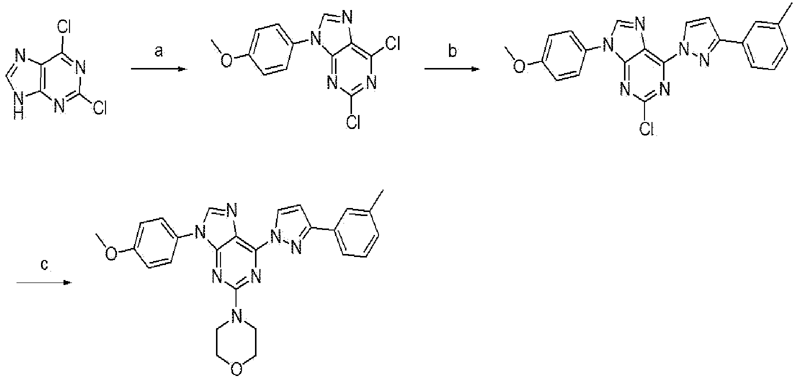

### (Step a) Synthesis of 2,6-dichloro-9-(4-methoxyphenyl)-9H-purine

2, 6-Dichloro-9H-purine (2.0 g) and (4-methoxyphenyl)boronic acid (4.82 g) were dissolved in dichloromethane (60 mL), then o-phenanthroline (3.81 g) and copper (II) acetate (1.92 g) were added, and the mixture was stirred overnight at 40°C under O₂ conditions. The reaction solution was filtered, and the filtrate was extracted three times with water (50 mL) and dichloromethane (30 mL), washed with a saturated NaCl solution, and the organic layer was dried using anhydrous sodium sulfate and filtered under reduced pressure, and the filtrate was collected and concentrated under reduced pressure. The concentrate was subjected to silica chromatography under ethyl acetate/petroleum ether conditions to obtain the target compound 2,6-dichloro-9-(4-methoxyphenyl)-9H-purine (1.05 g) as a white solid in a yield of 33.76%. MS: m/z = 295.0 (M+1, ESI+).

### (Step b) Synthesis of 2-chloro-9-(4-methoxyphenyl)-6-(3-(m-tolyl)-1H-pyrazol-1-yl)-9H-purine

3-(m-Tolyl)-1H-pyrazole (580 mg) was dissolved in tetrahydrofuran (8 mL), then sodium hydride (60%) (367 mg) was added, and the mixture was stirred at room temperature for 30 minutes. Then, 2,6-dichloro-9-(4-methoxyphenyl)-9H-purine (900 mg) was added, and the mixture was stirred at 0°C for 2 hours. After concentrating the reaction solution, the target compound 2-chloro-9-(4-methoxyphenyl)-6-(3-(m-tolyl)-1H-pyrazol-1-yl)-9H-purine (300 mg) was obtained as a yellow solid in a yield of 23.57% through silica chromatography under dichloromethane/methanol conditions. MS: m/z = 417.1 (M+1, ESI+).

### (Step c) Synthesis of 4-(9-(4-methoxyphenyl)-6-(3-(m-tolyl)-1H-pyrazol-1-yl)-9H-purin-2-yl)morpholine

2-Chloro-9-(4-methoxyphenyl)-6-(3-(m-tolyl)-1H-pyrazol-1-yl)-9H-purine (300 mg) was dissolved in isopropyl alcohol (5 mL), then acetic acid (43 mg) and morpholine (314 mg) were added, and the mixture was stirred at 120°C for 6 hours. The reaction solution was extracted three times with water (10 mL) and dichloromethane (5 mL), washed with a saturated NaCl solution, then the organic layer was dried using anhydrous sodium sulfate and filtered under reduced pressure, and the filtrate was collected and concentrated under reduced pressure. Then, the concentrate was subjected to silica chromatography under dichloromethane/methanol conditions to obtain the target compound 4-(9-(4-methoxyphenyl)-6-(3-(m-tolyl)-1H-pyrazol-1-yl)-9H-purin-2-yl)morpholine (37 mg) as a yellow solid in a yield of 10.99%.

¹H NMR (400 MHz, DMSO-*d*₆) δ 9.18 (d, *J* = 2.4 Hz, 1H), 8.59 (s, 1H), 7.85 - 7.78 (m, 4H), 7.39 (t, *J* = 7.6 Hz, 1H), 7.24 (d, *J* = 7.6 Hz, 1H), 7.21 - 7.14 (m, 3H), 3.84 (s, 3H), 3.83 - 3.76 (m, 4H), 3.76 - 3.69 (m, 4H), 2.41 (s, 3H); MS: m/z = 468.1(M+1, ESI+).

### Example 135. Synthesis of 4-(9-(3-chlorophenyl)-6-(3-(m-tolyl)-1H-pyrazol-1-yl)-9H-purin-2-yl)morpholine

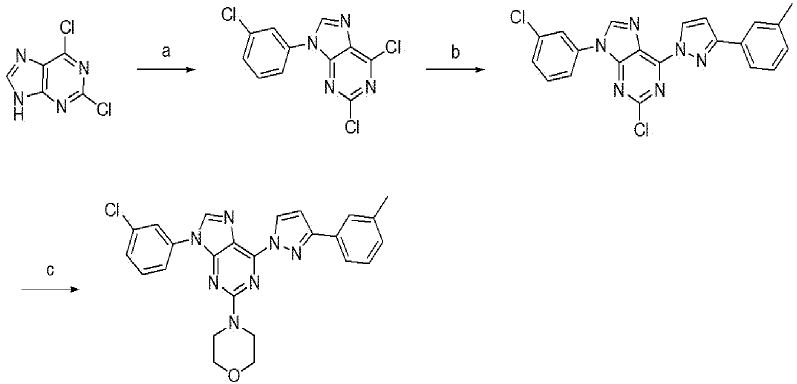

### (Step a) Synthesis of 2,6-dichloro-9-(3-chlorophenyl)-9H-purine

2,6-Dichloro-9H-purine (1.0 g) and (3-chlorophenyl)boronic acid (2.48 g) were dissolved in dichloromethane (100 mL), then copper(II) acetate (961 mg) and o-phenanthroline (1.91 g) were added, and the mixture was stirred at 40°C for 16 hours under O₂ conditions. The reaction solution was concentrated and the concentrate was subjected to silica chromatography under ethyl acetate/petroleum ether conditions to obtain the target compound 2,6-dichloro-9-(3-chlorophenyl)-9H-purine (750 mg) as a white solid in a yield of 47.57%. MS: m/z = 298.9(M+1, ESI+).

### (Step b) Synthesis of 2-chloro-9-(3-chlorophenyl)-6-(3-(m-tolyl)-1H-pyrazol-1-yl)-9H-purine

3-(m-Tolyl)-1H-pyrazole (742 mg) was dissolved in dimethylformamide (10 mL), then sodium hydride (60%) (282 mg) was added, and the mixture was stirred at room temperature for 30 minutes. Then, 2,6-dichloro-9-(3-chlorophenyl)-9H-purine (700 mg) was added and stirred at room temperature for 2 hours. After concentrating the reaction solution, the target compound 2-chloro-9-(3-chlorophenyl)-6-(3-(m-tolyl)-1H-pyrazol-1-yl)-9H-purine (430 mg) was obtained as a white solid in a yield of 43.58% through silica chromatography under ethyl acetate/petroleum ether conditions. MS: m/z = 421.1 (M+1, ESI+).

### (Step c) Synthesis of 4-(9-(3-chlorophenyl)-6-(3-(m-tolyl)-1H-pyrazol-1-yl)-9H-purin-2-yl)morpholine

2-Chloro-9-(3-chlorophenyl)-6-(3-(m-tolyl)-1H-pyrazol-1-yl)-9H-purine (430 mg) was dissolved in isopropyl alcohol (8 mL), then acetic acid (61 mg) and morpholine (445 mg) were added, and the mixture was stirred at 120°C overnight. The reaction solution was extracted three times with water (10 mL) and dichloromethane (5 mL), washed with a saturated NaCl solution, then the organic layer was dried using anhydrous sodium sulfate and filtered under reduced pressure, and the filtrate was collected and concentrated under reduced pressure. Then, the concentrate was subjected to silica chromatography under dichloromethane/methanol conditions to obtain the target compound 4-(9-(3-chlorophenyl)-6-(3-(m-tolyl)-1H-pyrazol-1-yl)-9H-purin-2-yl)morpholine (95 mg) as a white solid in a yield of 10.99%.

¹H NMR (400 MHz, DMSO-*d*₆) *δ* 9.16 (d, *J* = 2.6 Hz, 1H), 8.74 (s, 1H), 8.11 (s, 1H), 8.00 (d, *J* = 8.2 Hz, 1H), 7.85 - 7.80 (m, 2H) 7.65 (t, *J* = 8.1 Hz, 1H), 7.53 (d, *J* = 8.5 Hz, 1H), 7.39 (t, *J* = 7.5 Hz, 1H), 7.24 (d, *J* = 7.5 Hz, 1H), 7.19 (d, *J* = 2.7 Hz, 1H), 3.85 - 3.70 (m, 8H), 2.41 (s, 3H); MS: m/z = 472.4(M+1, ESI+).

### Example 136. Synthesis of 4-(9-phenyl-6-(3-(m-tolyl)-1H-pyrazol-1-yl)-9H-purin-2-yl)morpholine

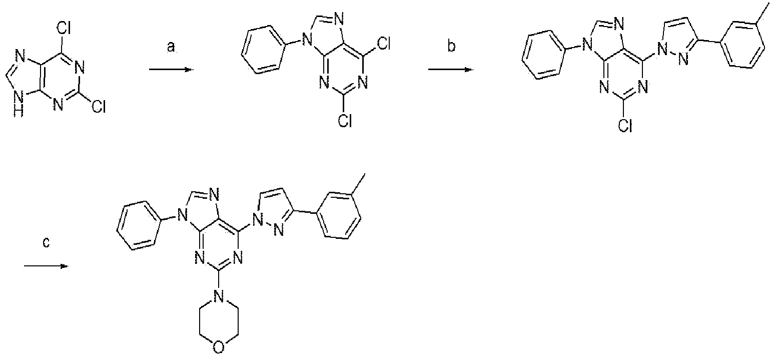

### (Step a) Synthesis of 2,6-dichloro-9-phenyl-9H-purine

2,6-Dichloro-9H-purine (1.0 g) and phenylboronic acid (1.94 g) were dissolved in dichloromethane (100 mL), then o-phenanthroline (1.92 g) and copper (II) acetate (0.96 g) were added, and the mixture was stirred at 40°C for 18 hours under O₂ conditions. Distilled water was added to the reaction solution and extracted with dichloromethane. After washing the organic solvent with a saturated NaCl solution, sodium sulfate was added, and the reaction mixture was filtered and concentrated. Then, the concentrated product was subjected to silica chromatography under ethyl acetate/benzene conditions to obtain the target compound 2,6-dichloro-9-phenyl-9H-purine (1.1 g) as a yellow solid in a yield of 78.42%. MS: m/z = 265.0 (M+1, ESI+).

### (Step b) Synthesis of 2-chloro-9-phenyl-6-(3-(m-tolyl)-1H-pyrazol-1-yl)-9H-purine

Dimethylformamide (15 mL) was added to 3-(m-tolyl)-1H-pyrazole (465 mg), and then sodium hydride (60%) (235 mg) was added at 0°C. After stirring at 0°C for 20 minutes, 2,6-dichloro-9-phenyl-9H-purine (518 mg) was added. The reaction solution was stirred at 25°C for 3 hours. The reaction was terminated by adding distilled water, and then the reaction mixture was extracted with ethyl acetate. After washing the organic solvent with a saturated NaCl solution, sodium sulfate was added, and the reaction mixture was filtered and concentrated. The concentrate was subjected to silica chromatography to obtain the target compound 2-chloro-9-phenyl-6-(3-(m-tolyl)-1H-pyrazol-1-yl)-9H-purine (200 mg) as a yellow solid in a yield of 26.41%. MS: m/z = 387.0 (M+1, ESI+).

### (Step c) Synthesis of 4-(9-phenyl-6-(3-(m-tolyl)-1H-pyrazol-1-yl)-9H-purin-2-yl)morpholine

2-Chloro-9-phenyl-6-(3-(m-tolyl)-1H-pyrazol-1-yl)-9H-purine (175 mg) and morpholine (225 mg) were dissolved in isopropyl alcohol (10 mL), acetic acid (0.05 mL) was added, and the mixture was stirred at 120°C overnight. Distilled water was added to the reaction solution and extracted with ethyl acetate. After washing the organic solvent with a saturated NaCl solution, sodium sulfate was added, and the reaction mixture was filtered and concentrated. Then, the concentrate was subjected to silica chromatography under dichloromethane/methanol conditions to obtain the target compound 4-(9-phenyl-6-(3-(m-tolyl)-1H-pyrazol-1-yl)-9H-purin-2-yl)morpholine (23 mg) as a yellow solid in a yield of 11.7%.

¹H NMR (400 MHz, DMSO-*d*₆) *δ* 9.19 (d, *J* = 2.6 Hz, 1H), 8.70 (s, 1H), 7.95 (d, *J* = 7.6 Hz, 2H), 7.85 - 7.83 (m, 2H), 7.63 (t, *J* = 7.8 Hz, 2H), 7.47 (t, *J* = 7.6 Hz, 1H), 7.39 (t, *J* = 7.6 Hz, 1H), 7.24 (d, *J* = 7.6 Hz, 1H), 7.20 (d, *J* = 2.8 Hz, 1H), 3.83 - 3.82 (m, 4H), 3.75 - 3.74 (m, 4H), 2.42 (s, 3H); MS: m/z = 438.2 (M+1, ESI+).

### Example 137. Synthesis of 4-(9-(3-methoxyphenyl)-6-(3-(m-tolyl)-1H-pyrazol-1-yl)-9H-purin-2-yl)morpholine

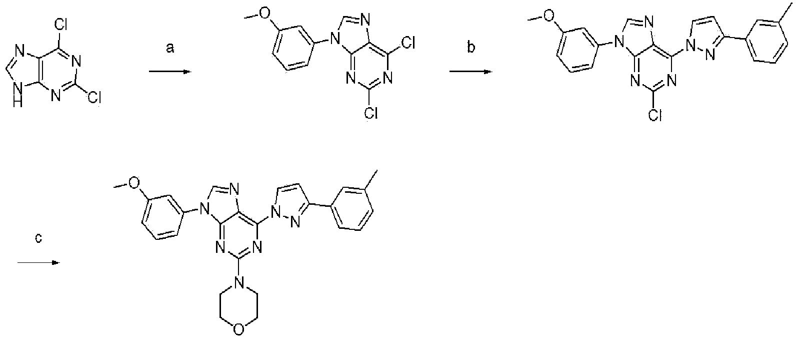

### (Step a) Synthesis of 2,6-dichloro-9-(3-methoxyphenyl)-9H-purine

2,6-Dichloro-9H-purine (1.5 g) and (3-methoxyphenyl)boronic acid (3.6 g) were dissolved in dichloromethane (80 mL), then o-phenanthroline (2.86 g) and copper(II) acetate (1.44 g) were added, and the mixture was stirred overnight at 50°C under O₂ conditions. Distilled water was added to the reaction solution and extracted with dichloromethane. After washing the organic solvent with a saturated NaCl solution, sodium sulfate was added, and the reaction mixture was filtered and concentrated. Then, the resulting product was subjected to silica chromatography to obtain the target compound 2,6-dichloro-9-(3-methoxyphenyl)-9H-purine (640 mg) as a yellow solid in a yield of 27.32%. MS: m/z = 296.1 (M+1, ESI+).

### (Step b) Synthesis of 2-chloro-9-(3-methoxyphenyl)-6-(3-(m-tolyl)-1H-pyrazol-1-yl)-9H-purine

Dimethylformamide (5 mL) was added to 3-(m-tolyl)-1H-pyrazole (347 mg), and then sodium hydride (60%) (132 mg) was added at 0°C. After stirring at room temperature for 60 minutes, 2,6-dichloro-9-(3-methoxyphenyl)-9H-purine (540 mg) was added. The reaction solution was stirred at room temperature overnight. The reaction was terminated by adding distilled water, and then the reaction mixture was extracted with ethyl acetate. After washing the organic solvent with a saturated NaCl solution, sodium sulfate was added, and the reaction mixture was filtered and concentrated. The concentrate was subjected to silica chromatography under benzene/ethyl acetate conditions to obtain the target compound 2-chloro-9-(3-methoxyphenyl)-6-(3-(m-tolyl)-1H-pyrazol-1-yl)-9H-purine (320 mg) as a purple solid in a yield of 42.03%. MS: m/z = 417.0 (M+1, ESI+).

### (Step c) Synthesis of 4-(9-(3-methoxyphenyl)-6-(3-(m-tolyl)-1H-pyrazol-1-yl)-9H-purin-2-yl)morpholine

2-Chloro-9-(3-methoxyphenyl)-6-(3-(m-tolyl)-1H-pyrazol-1-yl)-9H-purine (320 mg) and morpholine (669 mg) were dissolved in isopropyl alcohol (10 mL), then acetic acid (0.1 mL) was added, and the mixture was stirred at 120°C for 8 hours. Distilled water was added to the reaction solution and extracted with ethyl acetate. After washing the organic solvent with a saturated NaCl solution, sodium sulfate was added, and the reaction mixture was filtered and concentrated. Then, the concentrate was subjected to silica chromatography under benzene/ethyl acetate conditions to obtain the target compound 4-(9-(3-methoxyphenyl)-6-(3-(m-tolyl)-1H-pyrazol-1-yl)-9H-purin-2-yl)morpholine (18 mg) as a white solid in a yield of 5.01%.

¹H NMR (400 MHz, DMSO-*d*₆) δ 9.12 (s, 1H), 8.65 (s, 1H), 7.81 (s, 2H), 7.56 (s, 1H), 7.48 (s, 2H), 7.39 - 7.36 (m, 1H), 7.22 (d, *J* = 6.1 Hz, 1H), 7.14 (s, 1H), 7.00 (s, 1H), 3.83 (d, *J* = 6.1 Hz, 3H), 3.77 (s, 4H), 3.70 (s, 4H), 2.39 (s, 3H); MS: m/z = 468.2 (M+1, ESI+).

### Example 138. Synthesis of 4- (9- (3-cyclopropoxyphenyl)-6-(3-(m-tolyl)-1H-pyrazol-1-yl)-9H-purin-2-yl)morpholine

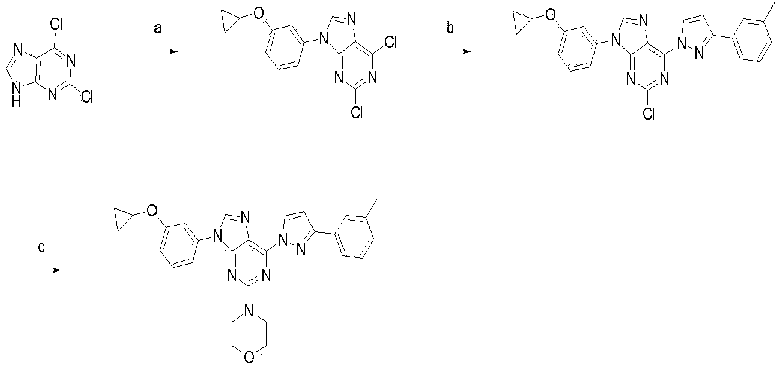

### (Step a) Synthesis of 2,6-dichloro-9-(3-cyclopropoxyphenyl)-9H-purine

2,6-Dichloro-9H-purine (1.5 g) and (3-cyclopropoxyphenyl)boronic acid (2.1 g) were dissolved in dichloromethane (60 mL), then o-phenanthroline (2.86 g) and copper(II) acetate (1.44 g) were added, and the mixture was stirred overnight at 40°C under O₂ conditions. Distilled water was added to the reaction solution and extracted with dichloromethane. After washing the organic solvent with a saturated NaCl solution, sodium sulfate was added, and the reaction mixture was filtered and concentrated. Then, the resulting product was subjected to silica chromatography to obtain the target compound 2,6-dichloro-9-(3-cyclopropoxyphenyl)-9H-purine (430 mg) as a yellow solid in a yield of 16.87%. MS: m/z = 322.1 (M+1, ESI+).

### (Step b) Synthesis of 2-chloro-9-(3-cyclopropoxyphenyl)-6-(3-(m-tolyl)-1H-pyrazol-1-yl)-9H-purine

Dimethylformamide (5 mL) was added to 3-(m-tolyl)-1H-pyrazole (195 mg), and sodium hydride (60%) (123 mg) was added at 0°C. After stirring at room temperature for 1 hour, 2,6-dichloro-9-(3-cyclopropoxyphenyl)-9H-purine (330 mg) was added. The reaction solution was stirred overnight at 25°C. The reaction was terminated by adding distilled water, and then the reaction mixture was extracted with ethyl acetate. After washing the organic solvent with a saturated NaCl solution, sodium sulfate was added, and the reaction mixture was filtered and concentrated. The concentrate was subjected to silica chromatography under benzene/ethyl acetate conditions to obtain the target compound 2-chloro-9-(3-cyclopropoxyphenyl)-6-(3-(m-tolyl)-1H-pyrazol-1-yl)-9H-purine (280 mg) as a purple solid in a yield of 61.62%. MS: m/z = 443.0 (M+1, ESI+).

### (Step c) Synthesis of 4-(9-(3-cyclopropoxyphenyl)-6-(3-(m-tolyl)-1H-pyrazol-1-yl)-9H-purin-2-yl)morpholine

2-Chloro-9-(3-cyclopropoxyphenyl)-6-(3-(m-tolyl)-1H-pyrazol-1-yl)-9H-purine (353 mg) and morpholine (695 mg) were dissolved in isopropyl alcohol (10 mL), then acetic acid (0.1 mL) was added, and the mixture was stirred at 120°C for 8 hours. Distilled water was added to the reaction solution and extracted with ethyl acetate. After washing the organic solvent with a saturated NaCl solution, sodium sulfate was added, and the reaction mixture was filtered and concentrated. Then, the concentrate was subjected to silica chromatography under dichloromethane/methanol conditions to obtain the target compound 4-(9-(3-cyclopropoxyphenyl)-6-(3-(m-tolyl)-1H-pyrazol-1-yl)-9H-purin-2-yl)morpholine (34.3 mg) as a white solid in a yield of 8.71%.

¹H NMR (400 MHz, DMSO-*d*₆) *δ* 9.11 (s, 1H), 8.61 (s, 1H), 7.80 (d, *J* = 10.4 Hz, 2H), 7.67 (s, 1H), 7.47 (d, *J* = 6.3 Hz, 2H), 7.36 (t, *J =* 7.5 Hz, 1H), 7.21 (d, *J* = 7.3 Hz, 1H), 7.13 (d, *J* = 2.7 Hz, 1H), 7.07 (dd, *J* = 5.8, 2.4 Hz, 1H), 3.91 (dd, *J* = 7.3, 4.3 Hz, 1H), 3.78 (s, 4H), 3.70 (s, 4H), 2.38 (s, 3H), 0.80 - 0.77 (m, 2H), 0.72 (d, *J* = 3.1 Hz, 2H); MS: m/z = 494.2 (M+1, ESI+).

### Example 139. Synthesis of (E)-4-(6-(2-(3-methylbenzylidene)hydrazinyl)-9-(2-(trifluoromethyl)pyridin-4-yl)-9H-purin-2-yl)morpholine

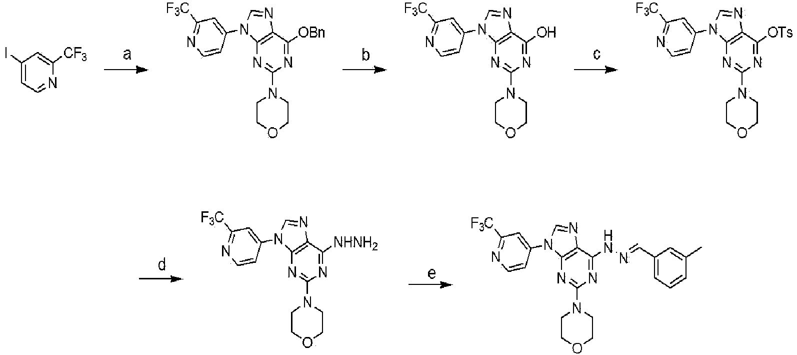

### (Step a) Synthesis of 4-(6-(benzyloxy)-9-(2-(trifluoromethyl)pyridin-4-yl)-9H-purin-2-yl)morpholine

4-(6-(Benzyloxy)-9H-purin-2-yl)morpholine (411 mg) and 4-iodo-2-(trifluoromethyl)pyridine (300 mg) were dissolved in dimethyl sulfoxide (30 mL), then L-proline (194 mg), tripotassium phosphate (820 mg), and copper iodide (255 mg) were added, and the mixture was stirred at 80°C for 5 hours. The reaction solution was extracted three times with water (100 mL) and dichloromethane (200 mL), washed with a saturated NaCl solution (100 mL), and the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 4-(6-(benzyloxy)-9-(2-(trifluoromethyl)pyridin-4-yl)-9H-purin-2-yl)morpholine (400 mg) as a yellow solid in a yield of 79.7%. MS: m/z = 457.2 (M+1, ESI+).

### (Step b) Synthesis of 2-morpholino-9-(2-(trifluoromethyl)pyridin-4-yl)-9H-purin-6-ol

4-(6-(Benzyloxy)-9-(2-(trifluoromethyl)pyridin-4-yl)-9H-purin-2-yl)morpholine (300 mg) was dissolved in dichloromethane (10 mL), then trifluoroacetic acid (2 mL) was added at 0°C, and the mixture was stirred at 25°C for 30 minutes. The reaction solution was concentrated under reduced pressure, then the pH was adjusted to 8 by alkalizing with a saturated sodium bicarbonate solution. The resulting product was extracted three times with dichloromethane (200 mL), and washed three times with a saturated NaCl solution (200 mL). The organic layer was dried using anhydrous sodium sulfate, filtered under reduced pressure, and the filtrate was concentrated under reduced pressure. After concentration, the target compound 2-morpholino-9-(2-(trifluoromethyl)pyridin-4-yl)-9H-purin-6-ol (200 mg) as a yellow solid was obtained in a yield of 88.2% and used in the next reaction in a crude state. MS: m/z = 313.1 (M+1, ESI+) .

### (Step c) Synthesis of 2-morpholino-9-(2-(trifluoromethyl)pyridin-4-yl)-9H-purin-6-yl 4-methylbenzenesulfonate

2-Morpholino-9-(2-(trifluoromethyl)pyridin-4-yl)-9H-purin-6-ol (200 mg) and triethylamine (165 mg) were dissolved in dichloromethane (20 mL), then tosyl chloride (156 mg) was added, and the mixture was stirred at 0°C for 2 hours. The reaction solution was extracted three times with water (30 mL) and dichloromethane (30 mL), washed with a saturated NaCl solution (30 mL), and the organic layer was dried using anhydrous sodium sulfate, filtered under reduced pressure, and the filtrate was collected and concentrated under reduced pressure. Then, the concentrated product was subjected to silica chromatography under dichloromethane/methanol conditions to obtain the target compound 2-morpholino-9-(2-(trifluoromethyl)pyridin-4-yl)-9H-purin-6-yl 4-methylbenzenesulfonate (240 mg) as a white solid in a yield of 80.1%. MS: m/z = 521.1 (M+1, ESI+).

### (Step d) Synthesis of 4-(6-hydrazinyl-9-(2-(trifluoromethyl)pyridin-4-yl)-9H-purin-2-yl)morpholine

2-Morpholino-9-(2-(trifluoromethyl)pyridin-4-yl)-9H-purin-6-yl 4-methylbenzenesulfonate (240 mg) was dissolved in tetrahydrofuran (10 mL), then hydrazine monohydrate (100 mg) was added, and the mixture was stirred at 25°C for 30 minutes. After the reaction was completed, the reaction solution was concentrated, ethyl acetate (10 mL) was added, and the resulting solid was filtered to obtain the target compound 4-(6-hydrazinyl-9-(2-(trifluoromethyl)pyridin-4-yl)-9H-purin-2-yl)morpholine (200 mg) as a white solid in a yield of 70.4%. MS: m/z = 381.2(M+1, ESI+).

### (Step e) Synthesis of (E)-4-(6-(2-(3-methylbenzylidene)hydrazinyl)-9-(2-(trifluoromethyl)pyridin-4-yl)-9H-purin-2-yl)morpholine

4-(6-Hydrazinyl-9-(2-(trifluoromethyl)pyridin-4-yl)-9H-purin-2-yl)morpholine (200 mg) was dissolved in methanol (15 mL), then 3-methylbenzaldehyde (78 mg) and acetic acid (0.1 mL) were added, and the mixture was stirred at 70°C for 30 minutes. After concentrating the reaction solution under reduced pressure, the concentrate was subjected to silica chromatography under dichloromethane/methanol conditions to obtain the target compound (E)-4-(6-(2-(3-methylbenzylidene)hydrazinyl)-9-(2-(trifluoromethyl)pyridin-4-yl)-9H-purin-2-yl)morpholine (82 mg) as a white solid in a yield of 32.4%.

¹H NMR (400 MHz, DMSO-*d*₆) *δ* 11.55 (s, 1H), 8.91 (d, *J* = 5.4 Hz, 1H), 8.82 (d, *J* = 3.5 Hz, 2H), 8.53 (d, *J* = 4.2 Hz, 1H), 8.30 (s, 1H), 7.62 - 7.48 (m, 2H), 7.34 (t, *J* = 7.6 Hz, 1H), 7.21 (d, *J* = 7.4 Hz, 1H), 3.84 - 3.66 (m, 8H), 2.36 (s, 3H); MS: m/z = 483.1 (M+1, ESI+).

### Example 140. Synthesis of (E)-4-(6-(2-(3-methylbenzylidene)hydrazinyl)-9-(2-methylpyridin-4-yl)-9H-purin-2-yl)morpholine

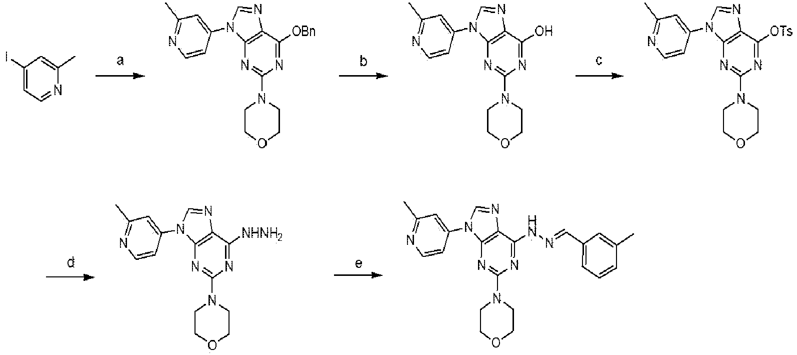

### (Step a) Synthesis of 4-(6-(benzyloxy)-9-(2-methylpyridin-4-yl)-9H-purin-2-yl)morpholine

4-(6-(Benzyloxy)-9H-purin-2-yl)morpholine (800 mg) and 4-iodo-2-methylpyridine (500 mg) were dissolved in dimethyl sulfoxide (30 mL), then L-proline (320 mg), tripotassium phosphate (1.17 g), and copper iodide (1.17 g) were added, and the mixture was stirred at 80°C for 5 hours. The reaction solution was extracted three times with water (100 mL) and dichloromethane (200 mL), washed with a saturated NaCl solution (100 mL), and the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 4-(6-(benzyloxy)-9-(2-methylpyridin-4-yl)-9H-purin-2-yl)morpholine (550 mg) as a yellow solid in a yield of 59.8%. MS: m/z = 403.2 (M+1, ESI+) .

### (Step b) Synthesis of 9-(2-methylpyridin-4-yl)-2-morpholino-9H-purin-6-ol

4-(6-(Benzyloxy)-9-(2-methylpyridin-4-yl)-9H-purin-2-yl)morpholine (550 mg) was dissolved in dichloromethane (10 mL), then trifluoroacetic acid (2 mL) was added at 0°C, and the mixture was stirred at 25°C for 30 minutes. The reaction solution was concentrated under reduced pressure, then the pH was adjusted to 8 by alkalizing with a saturated sodium bicarbonate solution. The resulting product was extracted three times with dichloromethane (200 mL), and washed three times with a saturated NaCl solution (200 mL). The organic layer was dried using anhydrous sodium sulfate, filtered under reduced pressure, and the filtrate was concentrated under reduced pressure. After concentration, the target compound 9-(2-methylpyridin-4-yl)-2-morpholino-9H-purin-6-ol (480 mg) as a yellow solid was obtained in a yield of 88.2% and used in the next reaction in a crude state. MS: m/z = 313.2 (M+1, ESI+).

### (Step c) Synthesis of 9-(2-methylpyridin-4-yl)-2-morpholino-9H-purin-6-yl 4-methylbenzenesulfonate

9-(2-Methylpyridin-4-yl)-2-morpholino-9H-purin-6-ol (480 mg) and triethylamine (906 mg) were dissolved in dichloromethane (20 mL), then tosyl chloride (686 mg) was added, and the mixture was stirred at 0°C for 2 hours. The reaction solution was extracted three times with water (30 mL) and dichloromethane (30 mL), washed with a saturated NaCl solution (30 mL), and the organic layer was dried using anhydrous sodium sulfate, filtered under reduced pressure, and the filtrate was collected and concentrated under reduced pressure. Then, the concentrated product was subjected to silica chromatography under dichloromethane/methanol conditions to obtain the target compound 9-(2-methylpyridin-4-yl)-2-morpholino-9H-purin-6-yl 4-methylbenzenesulfonate (300 mg) as a white solid in a yield of 41.8%. MS: m/z = 467.2 (M+1, ESI+).

### (Step d) Synthesis of 4-(6-hydrazinyl-9-(2-methylpyridin-4-yl)-9H-purin-2-yl)morpholine

9-(2-Methylpyridin-4-yl)-2-morpholino-9H-purin-6-yl 4-methylbenzenesulfonate (300 mg) was dissolved in tetrahydrofuran (10 mL), then hydrazine monohydrate (200 mg) was added, and the mixture was stirred at 25°C for 30 minutes. After the reaction was completed, the reaction solution was concentrated, ethyl acetate (10 mL) was added, and the resulting solid was filtered to obtain the target compound 4-(6-hydrazinyl-9-(2-methylpyridin-4-yl)-9H-purin-2-yl)morpholine (180 mg) as a white solid in a yield of 60%. MS: m/z = 327.2(M+1, ESI+).

### (Step e) Synthesis of (E)-4-(6-(2-(3-methylbenzylidene)hydrazinyl)-9-(2-methylpyridin-4-yl)-9H-purin-2-yl)morpholine

4-(6-Hydrazinyl-9-(2-methylpyridin-4-yl)-9H-purin-2-yl)morpholine (180 mg) was dissolved in methanol (15 mL), then 3-methylbenzaldehyde (100 mg) and acetic acid (0.1 mL) were added, and the mixture was stirred at 70°C for 30 minutes. After concentrating the reaction solution under reduced pressure, the concentrate was subjected to silica chromatography under dichloromethane/methanol conditions to obtain the target compound (E)-4-(6-(2-(3-methylbenzylidene)hydrazinyl)-9-(2-methylpyridin-4-yl)-9H-purin-2-yl)morpholine (32 mg) as a white solid in a yield of 13.5%.

¹H NMR (400 MHz, DMSO-*d*₆) *δ* 11.50 (s, 1H), 8.63 (s, 1H), 8.58 (d, *J* = 5.3 Hz, 1H), 8.29 (s, 1H), 8.05-7.95 (m, 2H), 7.57 (d, *J* = 7.4 Hz, 1H), 7.51 (s, 1H), 7.34 (t, *J =* 7.5 Hz, 1H), 7.21 (d, *J* = 6.8 Hz, 1H), 3.74 (dd, *J* = 18.8, 3.9 Hz, 8H), 2.56 (s, 3H), 2.36 (s, 3H); MS: m/z = 429.3 (M+1, ESI+).

### Example 141. Synthesis of (E)-4-(6-(2-(3-methylbenzylidene)hydrazinyl)-9-(6-methylpyridin-2-yl)-9H-purin-2-yl)morpholine

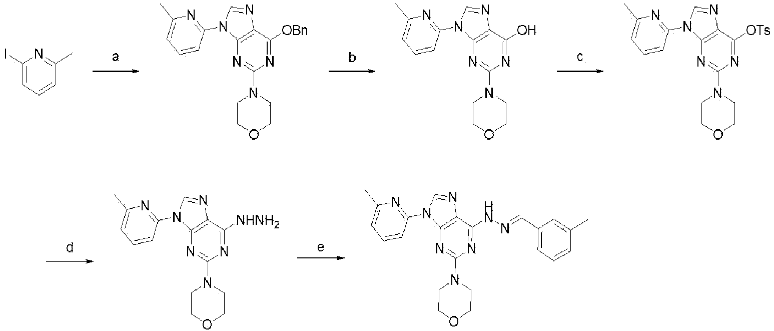

### (Step a) Synthesis of 4-(6-(benzyloxy)-9-(6-methylpyridin-2-yl)-9H-purin-2-yl)morpholine

4-(6-(Benzyloxy)-9H-purin-2-yl)morpholine (640 mg) and 2-iodo-6-methylpyridine (900 mg) were dissolved in dimethyl sulfoxide (20 mL), then L-proline (355 mg), tripotassium phosphate (1.31 g), and copper iodide (586 mg) were added, and the mixture was stirred at 80°C overnight. The reaction solution was filtered, extracted three times with water (30 mL) and ethyl acetate (50 mL), and washed with a saturated NaCl solution, then the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 4-(6-(benzyloxy)-9-(6-methylpyridin-2-yl)-9H-purin-2-yl)morpholine (600 mg) as a white solid in a yield of 72.45%. MS: m/z = 403.1 (M+1, ESI+)

### (Step b) Synthesis of 9-(6-methylpyridin-2-yl)-2-morpholino-9H-purin-6-ol

4-(6-(Benzyloxy)-9-(6-methylpyridin-2-yl)-9H-purin-2-yl)morpholine (600 mg) was dissolved in dichloromethane (10 mL), then trifluoroacetic acid (10 mL) was added, and the mixture was stirred at room temperature for 2 hours. The reaction solution was concentrated under reduced pressure to obtain the target compound 9-(6-methylpyridin-2-yl)-2-morpholino-9H-purin-6-ol (560 mg) as a white solid, which was used in the next reaction in a crude state. MS: m/z = 313.1 (M+1, ESI+).

### (Step c) Synthesis of 9-(6-methylpyridin-2-yl)-2-morpholino-9H-purin-6-yl 4-methylbenzenesulfonate

9-(6-Methylpyridin-2-yl)-2-morpholino-9H-purin-6-ol (560 mg) and triethylamine (906 mg) were dissolved in dichloromethane (15 mL), then tosyl chloride (686 mg) was added, and the mixture was stirred at room temperature for 2 hours. The reaction solution was concentrated, followed by silica chromatography under ethyl acetate/petroleum ether conditions, to obtain the target compound 9-(6-methylpyridin-2-yl)-2-morpholino-9H-purin-6-yl 4-methylbenzenesulfonate (200 mg) as a white solid in a yield of 23.98%. MS: m/z = 467.1 (M+1, ESI+).

### (Step d) Synthesis of 4-(6-hydrazinyl-9-(6-methylpyridin-2-yl)-9H-purin-2-yl)morpholine

9-(6-Methylpyridin-2-yl)-2-morpholino-9H-purin-6-yl 4-methylbenzenesulfonate (200 mg) was dissolved in tetrahydrofuran (5 mL), then hydrazine monohydrate (107.4 mg) was added, and the mixture was stirred at 25°C for 1 hour. After the reaction was completed, the reaction solution was concentrated, distilled water (5 mL) was added, and the resulting solid was filtered to obtain the target compound 4-(6-hydrazinyl-9-(6-methylpyridin-2-yl)-9H-purin-2-yl)morpholine (147 mg) as a yellow solid, which was used in the next reaction in a crude state. MS: m/z = 327.2 (M+1, ESI+) .

### (Step e) Synthesis of (E)-4-(6-(2-(3-methylbenzylidene)hydrazinyl)-9-(6-methylpyridin-2-yl)-9H-purin-2-yl)morpholine

4-(6-Hydrazinyl-9-(6-methylpyridin-2-yl)-9H-purin-2-yl)morpholine (179 mg) was dissolved in methanol (5 mL), then 3-methylbenzaldehyde (79.1 mg) and acetic acid (3.3 mg) were added, and the mixture was stirred at 70°C for 12 minutes. After concentrating the reaction solution under reduced pressure, the concentrate was subjected to silica chromatography under dichloromethane/methanol conditions to obtain the target compound (E)-4-(6-(2-(3-methylbenzylidene)hydrazinyl)-9-(6-methylpyridin-2-yl)-9H-purin-2-yl)morpholine (148 mg) as a white solid in a yield of 62.80%.

¹H NMR (400 MHz, DMSO-*d*₆) *δ* 11.50 (s, 1H), 8.69 (s, 1H), 8.37 (d, *J* = 8.1 Hz, 1H), 8.31 (s, 1H), 7.94 (t, *J =* 7.9 Hz, 1H), 7.57 (d, *J* = 7.7 Hz, 1H), 7.51 (s, 1H), 7.33 (t, *J* = 7.6 Hz, 1H), 7.25 (d, *J* = 7.5 Hz, 1H), 7.20 (d, *J =* 7.5 Hz, 1H), 3.78 (d, *J* = 4.5 Hz, 4H), 3.72 (d, *J* = 4.5 Hz, 4H), 2.53 (s, 3H), 2.35 (s, 3H); MS: m/z = 429.1 (M+1, ESI+).

### Example 142. Synthesis of (E)-4-(6-(2-(3-methylbenzylidene)hydrazinyl)-9-(3-methylpyridin-2-yl)-9H-purin-2-yl)morpholine

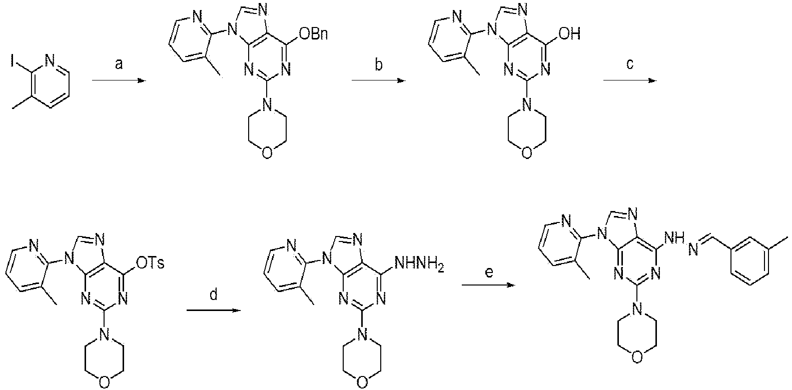

### (Step a) Synthesis of 4-(6-(benzyloxy)-9-(3-methylpyridin-2-yl)-9H-purin-2-yl)morpholine

4-(6-(Benzyloxy)-9H-purin-2-yl)morpholine (2.42 g) and 2-iodo-3-methylpyridine (1.55 g) were dissolved in dimethyl sulfoxide (40 mL), then L-proline (1.23 g), tripotassium phosphate (4.36 g), and copper iodide (2.02 g) were added, and the mixture was stirred at 95°C overnight. The reaction solution was extracted three times with water (40 mL) and ethyl acetate (10 mL) and washed with saturated NaCl solution, then the organic layer was dried using anhydrous sodium sulfate, filtered under reduced pressure, and the filtrate was collected and concentrated under reduced pressure. The concentrate was subjected to silica chromatography under dichloromethane/methanol conditions to obtain the target compound 4-(6-(benzyloxy)-9-(3-methylpyridin-2-yl)-9H-purin-2-yl)morpholine (145 mg) as a gray solid in a yield of 5.10%. MS: m/z = 403.1 (M+1, ESI+).

### (Step b) Synthesis of compound 9-(3-methylpyridin-2-yl)-2-morpholino-9H-purin-6-ol

4-(6-(Benzyloxy)-9-(3-methylpyridin-2-yl)-9H-purin-2-yl)morpholine (145 mg) was dissolved in dichloromethane (1 mL), then trifluoroacetic acid (10 mL) was added, and the mixture was stirred at room temperature for 4 hours. The reaction solution was concentrated under reduced pressure to obtain the target compound 9-(3-methylpyridin-2-yl)-2-morpholino-9H-purin-6-ol (105 mg) as a gray solid in a yield of 93.30%. MS: m/z = 313.1 (M+1, ESI+).

### (Step c) Synthesis of 9-(3-methylpyridin-2-yl)-2-morpholino-9H-purin-6-yl 4-methylbenzenesulfonate

9-(3-Methylpyridin-2-yl)-2-morpholino-9H-purin-6-ol (105 mg) and triethylamine (170 mg) were dissolved in dichloromethane (5 mL), then tosyl chloride (96 mg) was added, and the mixture was stirred at room temperature for 2 hours. The reaction solution was extracted three times with water (20 mL) and dichloromethane (10 mL), washed with a saturated NaCl solution, dried the organic layer using anhydrous sodium sulfate, filtered under reduced pressure, and the filtrate was collected and concentrated under reduced pressure to obtain the target compound 9-(3-methylpyridin-2-yl)-2-morpholino-9H-purin-6-yl 4-methylbenzenesulfonate (115 mg) as a brown solid, which was used in the next reaction in a crude state. MS: m/z = 467.1 (M+1, ESI+).

### (Step d) Synthesis of 4-(6-hydrazinyl-9-(3-methylpyridin-2-yl)-9H-purin-2-yl)morpholine

9-(3-Methylpyridin-2-yl)-2-morpholino-9H-purin-6-yl 4-methylbenzenesulfonate (115 mg) was dissolved in tetrahydrofuran (3 mL), then hydrazine monohydrate (62 mg) was added, and the mixture was stirred at room temperature for 20 minutes. After the reaction was completed, the reaction solution was concentrated to obtain the target compound 4-(6-hydrazinyl-9-(3-methylpyridin-2-yl)-9H-purin-2-yl)morpholine (90 mg) as a yellow solid in a yield of 89.50%. MS: m/z = 327.1 (M+1, ESI+).

### (Step e) Synthesis of (E)-4-(6-(2-(3-methylbenzylidene)hydrazinyl)-9-(3-methylpyridin-2-yl)-9H-purin-2-yl)morpholine

4-(6-Hydrazinyl-9-(3-methylpyridin-2-yl)-9H-purin-2-yl)morpholine (90 mg) was dissolved in methanol (3 mL), then acetic acid (7 mg) and 3-methylbenzaldehyde (53 mg) were added, and the mixture was stirred at 70°C for 30 minutes. After concentrating the reaction solution under reduced pressure, the concentrate was subjected to silica chromatography under dichloromethane/methanol conditions to obtain the target compound (E)-4-(6-(2-(3-methylbenzylidene)hydrazinyl)-9-(3-methylpyridin-2-yl)-9H-purin-2-yl)morpholine (18 mg) as a white solid in a yield of 19.04%.

¹H NMR (400 MHz, DMSO-*d*₆) *δ* 11.45 (s, 1H), 8.47 (d, J = 4.0 Hz, 1H), 8.30 (s, 1H), 8.15 (s, 1H), 7.96 (d, *J* = 7.6 Hz, 1H), 7.59 (d, *J* = 8.0 Hz, 1H), 7.54 - 7.49 (m, 2H), 7.34 (t, *J* = 8.0 Hz, 1H), 7.20 (d, *J* = 7.6 Hz, 1H), 3.68 - 3.59 (m, 8H), 2.36 (s, 3H), 2.26 (s, 3H) ; MS: m/z = 429.3 (M+1, ESI+) .

### Example 143. Synthesis of (E)-4-(6-(2-(3-methylbenzylidene)hydrazinyl)-9-(5-methylpyridin-2-yl)-9H-purin-2-yl)morpholine

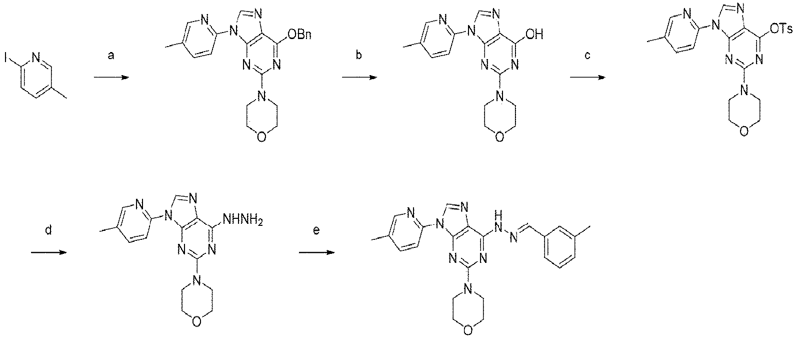

### (Step a) Synthesis of 4-(6-(benzyloxy)-9-(5-methylpyridin-2-yl)-9H-purin-2-yl)morpholine

4-(6-(Benzyloxy)-9H-purin-2-yl)morpholine (3.30 g) and potassium phosphate tribasic (4.36 g) were dissolved in dimethyl sulfoxide (40 mL), then 2-iodo-5-methylpyridine (1.55 g), copper iodide (2.02 g), and L-proline (1.23 g) were added. The reaction solution was stirred overnight at 95°C. Distilled water was added to the reaction solution and extracted with ethyl acetate. After washing the organic solvent with a saturated NaCl solution, sodium sulfate was added, then the resulting mixture was filtered and concentrated to obtain the target compound 4-(6-(benzyloxy)-9-(5-methylpyridin-2-yl)-9H-purin-2-yl)morpholine (1.05 g) as a gray solid in a yield of 36.9%. MS: m/z = 403.1 (M+1, ESI+) .

### (Step b) Synthesis of 9-(5-methylpyridin-2-yl)-2-morpholino-9H-purin-6-ol

4-(6-(Benzyloxy)-9-(5-methylpyridin-2-yl)-9H-purin-2-yl)morpholine (900 mg) was dissolved in dichloromethane (3 mL), then trifluoroacetic acid (3 mL) was added, and the mixture was stirred at room temperature for 4 hours. The reaction solution was concentrated to obtain the target compound 9-(5-methylpyridin-2-yl)-2-morpholino-9H-purin-6-ol (450 mg) as a gray solid in a yield of 64.42%. MS: m/z = 313.1 (M+1, ESI+)

### (Step c) Synthesis of 9-(5-methylpyridin-2-yl)-2-morpholino-9H-purin-6-yl 4-methylbenzenesulfonate

9-(5-Methylpyridin-2-yl)-2-morpholino-9H-purin-6-ol (450 mg) was dissolved in dichloromethane (10 mL), then triethylamine (728 mg) and 4-toluenesulfonyl chloride (412 mg) were added, and the mixture was stirred at room temperature for 2 hours. Distilled water was added to the reaction solution and extracted with dichloromethane. After washing the organic solvent with a saturated NaCl solution, sodium sulfate was added, then the resulting mixture was filtered and concentrated to obtain the target compound 9-(5-methylpyridin-2-yl)-2-morpholino-9H-purin-6-yl 4-methylbenzenesulfonate (300 mg) as a brown solid in a yield of 44.64%. MS: m/z = 467.1 (M+1, ESI+)

### (Step d) Synthesis of 4-(6-hydrazinyl-9-(5-methylpyridin-2-yl)-9H-purin-2-yl)morpholine

9-(5-Methylpyridin-2-yl)-2-morpholino-9H-purin-6-yl 4-methylbenzenesulfonate (350 mg) was dissolved in tetrahydrofuran (5 mL), then hydrazine monohydrate (188 mg) was added, and the mixture was stirred at room temperature for 20 minutes. The reaction solution was concentrated to obtain the target compound 4-(6-hydrazinyl-9-(5-methylpyridin-2-yl)-9H-purin-2-yl)morpholine (275 mg) as a yellow solid in a yield of 44.92%. MS: m/z = 327.1 (M+1, ESI+)

### (Step e) Synthesis of (E)-4-(6-(2-(3-methylbenzylidene)hydrazinyl)-9-(5-methylpyridin-2-yl)-9H-purin-2-yl)morpholine

4-(6-Hydrazinyl-9-(5-methylpyridin-2-yl)-9H-purin-2-yl)morpholine (275 mg) was dissolved in methanol (3 mL), then acetic acid (6 mg) and 3-methylbenzenaldehyde (82 mg) were added, and the mixture was stirred at 70°C for 30 minutes. After concentrating the reaction solution, the concentrate was subjected to silica chromatography under dichloromethane/methanol conditions to obtain the target compound (E)-4-(6-(2-(3-methylbenzylidene)hydrazinyl)-9-(5-methylpyridin-2-yl)-9H-purin-2-yl)morpholine (34 mg) as a yellow solid in a yield of 23.61%.

¹H NMR (400 MHz, CDCl₃) *δ* 9.22 (s, 1H), 8.69 (s, 1H), 8.48 (d, J = 8.4 Hz, 1H), 8.30 (s, 1H), 8.00 (s, 1H), 7.70 (d, *J* = 8.0 Hz, 1H), 7.60 - 7.56 (m, 2H), 7.30 (t, *J* = 7.6 Hz, 1H), 7.19 (d, *J* = 7.6 Hz, 1H), 3.93 - 3.92 (m, 4H), 3.86 - 3.84 (m, 4H), 2.40 (s, 6H); MS: m/z = 429.1 (M+1, ESI+).

### Example 144. Synthesis of (E)-4-(6-(2-(3-methylbenzylidene)hydrazinyl)-9-(4-methylpyridin-2-yl)-9H-purin-2-yl)morpholine

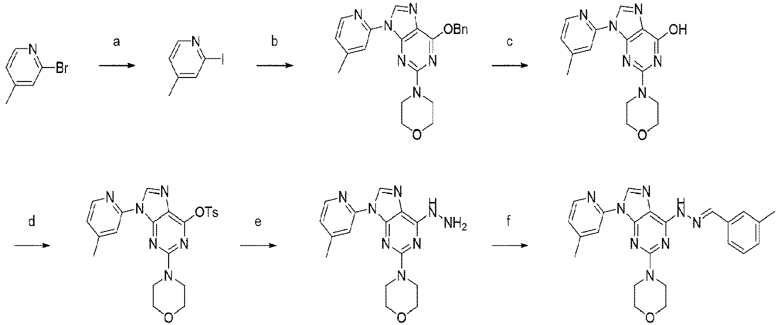

### (Step a) Synthesis of 2-iodo-4-methylpyridine

2-Bromo-4-methylpyridine (5.0 g) was dissolved in 1,4-dioxane (100 mL), then sodium iodide (8.77 g), N¹,N²-dimethylethene-1,2-diamine (1.29 g), and copper iodide (1.11 g) were added, and the mixture was stirred at 110°C for 16 hours. After filtering the reaction solution, dichloromethane was added. The reaction solution was concentrated, followed by silica chromatography, to obtain the target compound 2-iodo-4-methylpyridine (2.8 g) as yellow oil in a yield of 43.75%. MS: m/z = 219.9 (M+1, ESI+).

### (Step b) Synthesis of 4-(6-(benzyloxy)-9-(4-methylpyridin-2-yl)-9H-purin-2-yl)morpholine

4-(6-(Benzyloxy)-9H-purin-2-yl)morpholine (7.49 g) and potassium phosphate tribasic (15.31 g) were dissolved in dimethyl sulfoxide (100 mL), followed by the addition of 2-iodo-4-methylpyridine (5.8 g), copper iodide (6.89 g), and L-proline (4.19 g). The reaction solution was stirred at 90°C for 16 hours. After filtering the reaction solution, distilled water was added, and the mixture was subjected to silica chromatography to synthesize the target compound 4-(6-(benzyloxy)-9-(4-methylpyridin-2-yl)-9H-purin-2-yl)morpholine (4.0 g) as a yellow solid. MS: m/z = 403.1 (M+1, ESI+).

### (Step c) Synthesis of 9-(4-methylpyridin-2-yl)-2-morpholino-9H-purin-6-ol

4-(6-(Benzyloxy)-9-(4-methylpyridin-2-yl)-9H-purin-2-yl)morpholine (3.1 g) was dissolved in dichloromethane (15 mL), then trifluoroacetic acid (10 mL) was added, and the mixture was stirred at 25°C for 2 hours. After concentrating the reaction solution, the concentrate was subjected to silica chromatography to synthesize the target compound 9-(4-methylpyridin-2-yl)-2-morpholino-9H-purin-6-ol (1.5 g) as a yellow solid. MS: m/z = 313.1 (M+1, ESI+)

### (Step d) Synthesis of 9-(4-methylpyridin-2-yl)-2-morpholino-9H-purin-6-yl 4-methylbenzenesulfonate

9-(4-Methylpyridin-2-yl)-2-morpholino-9H-purin-6-ol (1.5 g) was dissolved in dichloromethane (20 mL), then triethylamine (1.17 g) was added, and the mixture was stirred at room temperature for 30 minutes. 4-Toluenesulfonyl chloride (1.10 g) was added, and the reaction mixture was stirred at room temperature for 2 hours. Distilled water was added to the reaction solution and extracted with ethyl acetate. After washing the organic solvent with a saturated NaCl solution, sodium sulfate was added, and the reaction mixture was filtered and concentrated. Then, the concentrated product was subjected to silica chromatography under dichloromethane/methanol conditions to obtain the target compound 9-(4-methylpyridin-2-yl)-2-morpholino-9H-purin-6-yl 4-methylbenzenesulfonate (270 mg) as a yellow solid in a yield of 81.12%. MS: m/z = 467.1 (M+1, ESI+)

### (Step e) Synthesis of 4-(6-hydrazinyl-9-(4-methylpyridin-2-yl)-9H-purin-2-yl)morpholine

9-(4-Methylpyridin-2-yl)-2-morpholino-9H-purin-6-yl 4-methylbenzenesulfonate (270 mg) was dissolved in tetrahydrofuran (5 mL), then hydrazine monohydrate (122 mg) was added, and the mixture was stirred at room temperature for 2 hours. After concentrating the reaction solution, the concentrate was subjected to silica chromatography under dichloromethane/methanol conditions to obtain the target compound 4-(6-hydrazinyl-9-(4-methylpyridin-2-yl)-9H-purin-2-yl)morpholine (100 mg) as a yellow solid in a yield of 45.82%. MS: m/z = 327.2 (M+1, ESI+)

### (Step f) Synthesis of (E)-4-(6-(2-(3-methylbenzylidene)hydrazinyl)-9-(4-methylpyridin-2-yl)-9H-purin-2-yl)morpholine

4-(6-Hydrazinyl-9-(4-methylpyridin-2-yl)-9H-purin-2-yl)morpholine (100 mg) was dissolved in methanol (5 mL), then acetic acid (0.1 mL) and 3-methylbenzenaldehyde (54 mg) were added, and the mixture was stirred at room temperature for 2 hours. After concentrating the reaction solution, the target compound (E)-4-(6-(2-(3-methylbenzylidene)hydrazinyl)-9-(4-methylpyridin-2-yl)-9H-purin-2-yl)morpholine (30 mg) was obtained as a white solid in a yield of 31.43% using prep-HPLC.

¹H NMR (400 MHz, DMSO-*d*₆) *δ* 12.52 (s, 1H), 8.89 (s, 1H), 8.59 (d, *J* = 4.6 Hz, 1H), 8.12 (s, 1H), 7.79 (s, 1H), 7.41 - 7.10 (m, 5H), 3.72 (dd, *J* = 14.7, 4.3 Hz, 8H), 2.35 (d, *J* = 21.6 Hz, 6H); MS: m/z = 429.1 (M+1, ESI+).

### Example 145. Synthesis of (E)-4-(6-(2-(3-methylbenzylidene)hydrazinyl)-9-(5-methylpyridin-3-yl)-9H-purin-2-yl)morpholine

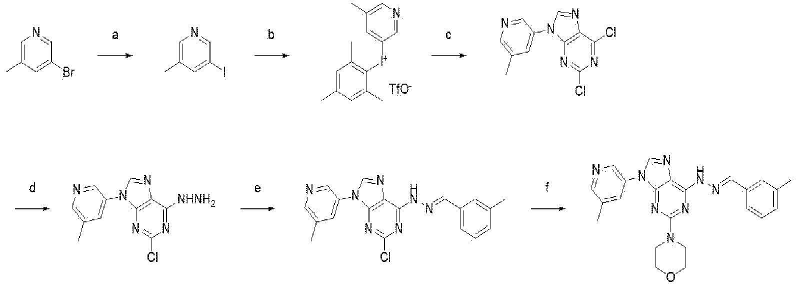

### (Step a) Synthesis of 3-iodo-5-methylpyridine

3-Bromo-5-methylpyridine (4.6 g) was dissolved in 1,4-dioxane (50 mL), then sodium iodide (8.02 g), N¹,N²-dimethylethene-1,2-diamine (1.18 g), and copper iodide (1.02 g) were added, and the mixture was stirred at 110°C overnight. Distilled water was added to the reaction solution and extracted with ethyl acetate. After washing the organic solvent with a saturated NaCl solution, sodium sulfate was added, filtered, and concentrated to obtain the target compound 3-iodo-5-methylpyridine (4.8 g) as a gray solid with a yield of 81.95%. MS: m/z = 219.9 (M+1, ESI+).

### (Step b) Synthesis of mesityl(5-methylpyridin-3-yl)iodonium trifluoromethane sulfonate

3-Iodo-5-methylpyridine (4.6 g) and m-chloroperoxybenzoic acid (6.4 g) were dissolved in dichloromethane (100.0 mL), then trifluoromethanesulfonic acid (12.60 g) was added at 0°C, and the mixture was stirred at room temperature for 2 hours. After adding mesitylene (2.77 g) at 0°C, the mixture was stirred overnight at room temperature. After adding distilled water, the reaction mixture was extracted with dichloromethane and concentrated under reduced pressure. After washing the organic solvent with a saturated NaCl solution, sodium sulfate was added, and the reaction mixture was filtered and concentrated. The target compound mesityl(5-methylpyridin-3-yl)iodonium trifluoromethanesulfonate (11 g) was synthesized as a gray solid through silica chromatography under dichloromethane/methanol conditions. MS: m/z = 337.8 (M-TfO⁻, ESI+).

### (Step c) Synthesis of 2,6-dichloro-9-(5-methylpyridin-3-yl)-9H-purine

Mesityl (5-methylpyridin-3-yl)iodonium trifluoromethanesulfonate (11 g), triethylamine (2.28 g), 2,6-dichloro-9H-purine (2.84 g), and copper bromide (432 mg) were dissolved in dichloromethane (60.0 mL), and the mixture was stirred at 60°C overnight. After adding distilled water, the reaction mixture was extracted with dichloromethane and concentrated under reduced pressure. After washing the organic solvent with a saturated NaCl solution, sodium sulfate was added, and the reaction mixture was filtered and concentrated. The resulting product was subjected to silica chromatography under dichloromethane/methanol conditions to obtain the target compound 2,6-dichloro-9-(5-methylpyridin-3-yl)-9H-purine (470.0 mg) as a yellow solid in a yield of 11.21%. MS: m/z = 279.9(M+1, ESI+).

### (Step d) Synthesis of 2-chloro-6-hydrazinyl-9-(5-methylpyridin-3-yl)-9H-purine

2,6-Dichloro-9-(5-methylpyridin-3-yl)-9H-purine (470.0 mg) was dissolved in tetrahydrofuran (6.0 mL), then hydrazine monohydrate (421.0 mg) was added, and the mixture was stirred at room temperature for 20 minutes. After concentrating the reaction solution under reduced pressure, distilled water was added, and the resulting yellow solid was filtered to obtain the target compound 2-chloro-6-hydrazinyl-9-(5-methylpyridin-3-yl)-9H-purine (320.0 mg) in a yield of 69.07%. MS: m/z = 276.1 (M+1, ESI+).

### (Step e) Synthesis of (E)-2-chloro-6-(2-(3-methylbenzylidene)hydrazinyl)-9-(5-methylpyridin-3-yl)-9H-purine

2-Chloro-6-hydrazinyl-9-(5-methylpyridin-3-yl)-9H-purine (320.0 mg), 3-methylbenzaldehyde (279.0 mg), and acetic acid (35.0 mg) were dissolved in methanol (5.0 mL), and the mixture was stirred at 70°C for 30 minutes. After concentrating the reaction solution under reduced pressure, the target compound (E)-2-chloro-6-(2-(3-methylbenzylidene)hydrazinyl)-9-(5-methylpyridin-3-yl)-9H-purine (270.0 mg) was obtained as a yellow solid in a yield of 61.55% through silica chromatography under dichloromethane/methanol conditions. MS: m/z = 378.0 (M+1, ESI+).

### (Step f) Synthesis of (E)-4-(6-(2-(3-methylbenzylidene)hydrazinyl)-9-(5-methylpyridin-3-yl)-9H-purin-2-yl)morpholine

(E)-2-Chloro-6-(2-(3-methylbenzylidene)hydrazinyl)-9-(5-methylpyridin-3-yl)-9H-purine (240.0 mg), morpholine (277.0 mg), and acetic acid (38 mg) were dissolved in isopropyl alcohol (6.0 mL), and the mixture was stirred at 120°C overnight. Distilled water was added to the reaction solution and extracted with ethyl acetate. After washing the organic solvent with a saturated NaCl solution, sodium sulfate was added, and the reaction mixture was filtered and concentrated. The target compound (E)-4-(6-(2-(3-methylbenzylidene)hydrazinyl)-9-(5-methylpyridin-3-yl)-9H-purin-2-yl)morpholine (60.0 mg) was obtained as a white solid in a yield of 19.04% through silica chromatography under dichloromethane/methanol conditions.

¹H NMR (400 MHz, DMSO-*d*₆) *δ* 11.52 (s, 1H), 9.01 (s, 1H), 8.48 - 8.47 (m, 2H), 8.30 (s, 1H), 8.19 (s, 1H), 7.58 (d, *J* = 7.6 Hz, 1H), 7.52 (s, 1H), 7.34 (t, *J =* 7.6 Hz, 1H), 7.21 (d, *J* = 7.2 Hz, 1H), 3.72 - 3.70 (m, 8H), 2.42 (s, 3H), 2.36 (s, 3H); MS: m/z = 429.3 (M+1, ESI+).

### Example 146. Synthesis of (E)-6-(6-(2-(3-methylbenzylidene)hydrazinyl)-2-morpholino-9H-purin-9-yl)nicotinonitrile

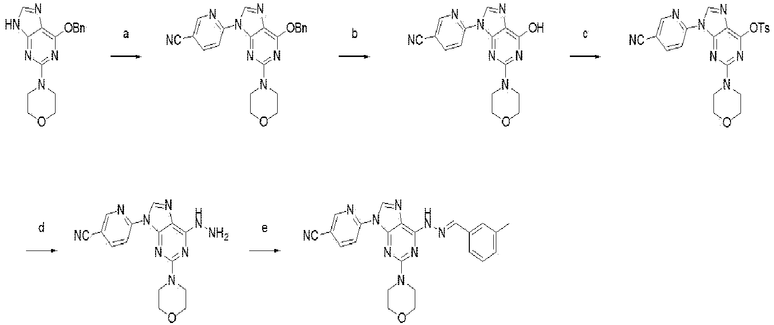

### (Step a) Synthesis of 6-(6-(benzyloxy)-2-morpholino-9H-purin-9-yl)nicotinonitrile

4-(6-(Benzyloxy)-9H-purin-2-yl)morpholine (2.10 g) and potassium phosphate tribasic (4.29 g) were dissolved in dimethyl sulfoxide (30 mL), then 6-iodonicotinonitrile (1.55 g), copper iodide (1.93 g), and L-proline (1.17 g) were added. The reaction solution was stirred overnight at 95°C. Distilled water was added to the reaction solution and extracted with ethyl acetate. After washing the organic solvent with a saturated NaCl solution, sodium sulfate was added, then the resulting mixture was filtered and concentrated to obtain the target compound 6-(6-(benzyloxy)-2-morpholino-9H-purin-9-yl)nicotinonitrile (410 mg) as a gray solid in a yield of 14.7%. MS: m/z = 414.1 (M+1, ESI+).

### (Step b) Synthesis of 6-(6-hydroxy-2-morpholino-9H-purin-9-yl)nicotinonitrile

6-(6-(Benzyloxy)-2-morpholino-9H-purin-9-yl)nicotinonitrile (410 mg) was dissolved in dichloromethane (2 mL), then trifluoroacetic acid (3 mL) was added, and the mixture was stirred at room temperature overnight. The reaction solution was concentrated to obtain the target compound 6-(6-hydroxy-2-morpholino-9H-purin-9-yl)nicotinonitrile (236 mg) as a gray solid in a yield of 30.91%. MS: m/z = 324.0 (M+1, ESI+).

### (Step c) Synthesis of 9-(5-cyanopyridin-2-yl)-2-morpholino-9H-purin-6-yl 4-methylbenzenesulfonate

6-(6-Hydroxy-2-morpholino-9H-purin-9-yl)nicotinonitrile (236 mg) was dissolved in dichloromethane (5 mL), then triethylamine (369 mg) and 4-toluenesulfonyl chloride (209 mg) were added, and the mixture was stirred at room temperature for 2 hours. Distilled water was added to the reaction solution and extracted with dichloromethane. After washing the organic solvent with a saturated NaCl solution, sodium sulfate was added, then the resulting mixture was filtered and concentrated to obtain the target compound 9-(5-cyanopyridin-2-yl)-2-morpholino-9H-purin-6-yl 4-methylbenzenesulfonate (170 mg) as a brown solid in a yield of 48.78%. MS: m/z = 478.0 (M+1, ESI+).

### (Step d) Synthesis of 6-(6-hydrazinyl-2-morpholino-9H-purin-9-yl)nicotinonitrile

9-(5-Cyanopyridin-2-yl)-2-morpholino-9H-purin-6-yl 4-methylbenzenesulfonate (170 mg) was dissolved in tetrahydrofuran (3 mL), then hydrazine monohydrate (89 mg) was added, and the mixture was stirred at room temperature for 20 minutes. The reaction solution was concentrated to obtain the target compound 6-(6-hydrazinyl-2-morpholino-9H-purin-9-yl)nicotinonitrile (130 mg) as a yellow solid in a yield of 5.41%. MS: m/z = 338.0 (M+1, ESI+).

### (Step e) Synthesis of (E)-6-(6-(2-(3-methylbenzylidene)hydrazinyl)-2-morpholino-9H-purin-9-yl)nicotinonitrile

6-(6-Hydrazinyl-2-morpholino-9H-purin-9-yl)nicotinonitrile (130 mg) was dissolved in methanol (5 mL), then acetic acid (0.05 mL) and 3-methylbenzaldehyde (5 mg) were added, and the mixture was stirred at 70°C for 30 minutes. After concentrating the reaction solution, the concentrate was subjected to silica chromatography under dichloromethane/methanol conditions to obtain the target compound (E)-6-(6-(2-(3-methylbenzylidene)hydrazinyl)-2-morpholino-9H-purin-9-yl)nicotinonitrile (2.3 mg) as a white solid in a yield of 27.16%.

¹H NMR (400 MHz, DMSO-*d*₆) *δ* 11.56 (s, 1H), 9.03 (s, 1H), 8.82 (d, *J* = 8.8 Hz, 1H), 8.76 (s, 1H), 8.56 (dd, *J* = 8.8, 1.6 Hz, 1H), 8.30 (s, 1H), 7.58 (d, *J* = 7.6 Hz, 1H), 7.51 (s, 1H), 7.34 (t, *J* = 7.6 Hz, 1H), 7.21 (d, *J* = 7.6 Hz, 1H), 3.81 - 3.73 (m, 8H), 2.36 (s, 3H); MS: m/z = 440.3 (M+1, ESI+).

### Example 147. Synthesis of (E)-4-(9-(1,5-dimethyl-1H-pyrazol-3-yl)-6-(2-(3-methylbenzylidene)hydrazinyl)-9H-purin-2-yl)morpholine

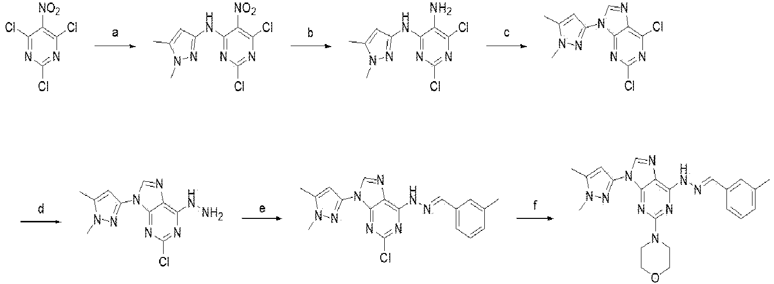

### (Step a) Synthesis of 2,6-dichloro-N-(1,5-dimethyl-1H-pyrazol-3-yl)-5-nitropyrimidin-4-amine

2,4,6-Trichloro-5-nitropyrimidine (500 mg) and N,N-diisopropylethylamine (426 mg) were dissolved in 1,4-dioxane (8 mL), then 1,5-dimethyl-1H-pyrazol-3-amine (196 mg) dissolved in 1,4-dioxane (7 mL) was added at 0°C, and the mixture was stirred at room temperature for 2 hours. The reaction solution was extracted three times with water (20 mL) and ethyl acetate (10 mL) and washed with a saturated NaCl solution, then the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 2,6-dichloro-N-(1,5-dimethyl-1H-pyrazol-3-yl)-5-nitropyrimidin-4-amine (160 mg) as a yellow solid in a yield of 24.04%. MS: m/z = 303.0 (M+1, ESI+).

### (Step b) Synthesis of 2,6-dichloro-N⁴-(1,5-dimethyl-1H-pyrazol-3-yl)pyrimidine-4,5-diamine

2,6-Dichloro-N-(1,5-dimethyl-1H-pyrazol-3-yl)-5-nitropyrimidin-4-amine (140 mg) was dissolved in acetic acid (6 mL), then iron (258 mg) was added, and the mixture was stirred at room temperature for 30 minutes. The reaction solution was extracted three times with water (15 mL) and ethyl acetate (10 mL), washed with a saturated NaCl solution, and the organic layer was dried using anhydrous sodium sulfate, and filtered under reduced pressure. The filtrate was concentrated under reduced pressure to obtain the target compound 2,6-dichloro-N⁴-(1,5-dimethyl-1H-pyrazol-3-yl)pyrimidine-4,5-diamine (120 mg) as a yellow solid in a yield of 95.23%. MS: m/z = 273.2(M+1, ESI+).

### (Step c) Synthesis of 2,6-dichloro-9-(1,5-dimethyl-1H-pyrazol-3-yl)-9H-purine

2,6-Dichloro-N⁴-(1,5-dimethyl-1H-pyrazol-3-yl)pyrimidine-4,5-diamine (120 mg) was dissolved in diethoxymethyl acetate (719 mg) and the mixture was stirred at 80°C overnight. The reaction solution was extracted three times with water (8 mL) and ethyl acetate (5 mL) and washed with a saturated NaCl solution, then the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 2,6-dichloro-9-(1,5-dimethyl-1H-pyrazol-3-yl)-9H-purine (120 mg) as a yellow solid in a yield of 96.77%. MS: m/z = 283.0 (M+1, ESI+).

### (Step d) Synthesis of 2-chloro-9-(1,5-dimethyl-1H-pyrazol-3-yl)-6-hydrazinyl-9H-purine

2,6-Dichloro-9-(1,5-dimethyl-1H-pyrazol-3-yl)-9H-purine (120 mg) was dissolved in tetrahydrofuran (3 mL), then hydrazine monohydrate (106 mg) was added, and the mixture was stirred at room temperature for 20 minutes. After the reaction was completed, the reaction solution was concentrated, distilled water (5 mL) was added, and the resulting solid was filtered to obtain the target compound 2-chloro-9-(1,5-dimethyl-1H-pyrazol-3-yl)-6-hydrazinyl-9H-purine (90 mg) as a yellow solid in a yield of 76.08%. MS: m/z = 279.0 (M+1, ESI+).

### (Step e) Synthesis of (E)-2-chloro-9-(1,5-dimethyl-1H-pyrazol-3-yl)-6-(2-(3-methylbenzylidene)hydrazinyl)-9H-purine

2-Chloro-9-(1,5-dimethyl-1H-pyrazol-3-yl)-6-hydrazinyl-9H-purine (90 mg) was dissolved in methanol (3 mL), then acetic acid (6 mg) and 3-methylbenzaldehyde (78 mg) were added, and the mixture was stirred at 70°C for 10 minutes. After concentrating the reaction solution under reduced pressure, the concentrate was subjected to silica chromatography under dichloromethane/methanol conditions to obtain the target compound (E)-2-chloro-9-(1,5-dimethyl-1H-pyrazol-3-yl)-6-(2-(3-methylbenzylidene)hydrazinyl)-9H-purine (110 mg) as a white solid in a yield of 89.43%. MS: m/z = 381.0 (M+1, ESI+).

### (Step f) Synthesis of (E)-4-(9-(1,5-dimethyl-1H-pyrazol-3-yl)-6-(2-(3-methylbenzylidene)hydrazinyl)-9H-purin-2-yl)morpholine

(E)-2-Chloro-9-(1,5-dimethyl-1H-pyrazol-3-yl)-6-(2-(3-methylbenzylidene)hydrazinyl)-9H-purine (110 mg) was dissolved in isopropyl alcohol (3 mL), then acetic acid (17 mg) and morpholine (126 mg) were added, and the mixture was stirred at 120°C overnight. The reaction solution was extracted three times with water (10 mL) and ethyl acetate (5 mL) and washed with a saturated NaCl solution, then the organic layer was dried using anhydrous sodium sulfate, filtered under reduced pressure, and the filtrate was collected and concentrated under reduced pressure. Then, the concentrate was subjected to silica chromatography under dichloromethane/methanol conditions to obtain the target compound (E)-4-(9-(1,5-dimethyl-1H-pyrazol-3-yl)-6-(2-(3-methylbenzylidene)hydrazinyl)-9H-purin-2-yl)morpholine (40 mg) as a white solid in a yield of 32.06%.

¹H NMR (400 MHz, DMSO-*d*₆) *δ* 11.39 (s, 1H), 8.28 (s, 1H), 8.25 (s, 1H), 7.57 (d, *J* = 8.0 Hz, 1H), 7.50 (s, 1H), 7.33 (t, *J =* 7.6 Hz, 1H), 7.20 (d, *J =* 7.2 Hz, 1H), 6.65 (s, 1H), 3.79 - 3.72 (m, 7H), 3.72 - 3.67 (m, 4H), 2.35 (d, *J* = 5.6 Hz, 6H); MS: m/z = 432.4 (M+1, ESI+).

### Example 148. Synthesis of (E)-4-(6-(2-(3-methylbenzylidene)hydrazinyl)-9-(3-methylisothiazol-5-yl)-9H-purin-2-yl)morpholine

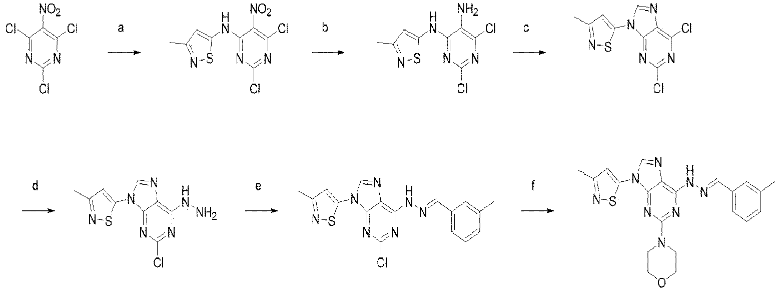

### (Step a) Synthesis of N-(2,6-dichloro-5-nitropyrimidin-4-yl)-3-methylisothiazol-5-amine

To 2,4,6-richloro-5-nitropyrimidine (600 mg) in tetrahydrofuran (20 mL), diethylamine (508 mg) and 3-methylisothiazol-5-amine hydrochloride (240 mg) in tetrahydrofuran (20 mL) were added at 0°C and stirred for 1 hour. Distilled water was added to the reaction solution and extracted with ethyl acetate. After washing the organic solvent with a saturated NaCl solution, sodium sulfate was added, and the reaction mixture was filtered and concentrated. The target compound N-(2,6-dichloro-5-nitropyrimidin-4-yl)-3-methylisothiazol-5-amine (200 mg) was obtained as a yellow solid in a yield of 24.91% through silica chromatography under benzene/ethyl acetate conditions. MS: m/z = 305.8 (M+1, ESI+).

### (Step b) Synthesis of 2,6-dichloro-N⁴-(3-methylisothiazol-5-yl)pyrimidine-4,5-diamine

Acetic acid (4 mL) and iron (366 mg) were added to N-(2,6-dichloro-5-nitropyrimidin-4-yl)-3-methylisothiazol-5-amine (200 mg), and the mixture was stirred at room temperature for 5 hours. Distilled water was added to the reaction solution and extracted with ethyl acetate. After washing the organic solvent with a saturated NaCl solution, sodium sulfate was added, filtered and concentrated to obtain the target compound 2,6-dichloro-N⁴-(3-methylisothiazol-5-yl)pyrimidine-4,5-diamine (135 mg) as a yellow solid in a yield of 75%. MS: m/z = 275.9 (M+1, ESI+).

### (Step c) Synthesis of 5-(2,6-dichloro-9H-purin-9-yl)-3-methylisothiazole

To 2,6-dichloro-N⁴-(3-methylisothiazol-5-yl)pyrimidine-4,5-diamine (135 mg), triethyl orthoformate (5 mL) and hydrochloric acid (0.3 mL) were added, and the mixture was stirred at 130°C for 2 hours. Distilled water was added to the reaction solution and extracted with dichloromethane. After washing the organic solvent with a saturated NaCl solution, sodium sulfate was added, then the resulting mixture was filtered and concentrated to obtain the target compound 5-(2,6-dichloro-9H-purin-9-yl)-3-methylisothiazole (86 mg) as a white solid in a yield of 62.43%. MS: m/z = 285.8 (M+1, ESI+).

### (Step d) Synthesis of 5-(2-chloro-6-hydrazinyl-9H-purin-9-yl)-3-methylisothiazole

5-(2,6-Dichloro-9H-purin-9-yl)-3-methylisothiazole (86.0 mg) was dissolved in tetrahydrofuran (3.0 mL), then hydrazine monohydrate (75.0 mg) was added, and the mixture was stirred at room temperature for 30 minutes. The reaction solution was concentrated under reduced pressure, distilled water was then added. The solid was filtered to obtain the target compound 5-(2-chloro-6-hydrazinyl-9H-purin-9-yl)-3-methylisothiazole (72.0 mg) as a yellow solid in a yield of 84.71%. MS: m/z = 281.9 (M+1, ESI+)

### (Step e) Synthesis of (E)-5-(2-chloro-6-(2-(3-methylbenzylidene)hydrazinyl)-9H-purin-9-yl)-3-methylisothiazole

5-(2-Chloro-6-hydrazinyl-9H-purin-9-yl)-3-methylisothiazole (79.0 mg), 3-methylbenzaldehyde (67.0 mg), and acetic acid (8 mg) were dissolved in methanol (3.0 mL), and the mixture was stirred at 70°C for 20 minutes. After concentrating the reaction solution, the concentrate was subjected to silica chromatography under dichloromethane/methanol conditions to obtain the target compound (E)-5-(2-chloro-6-(2-(3-methylbenzylidene)hydrazinyl)-9H-purin-9-yl)-3-methylisothiazole (45.0 mg) as a white solid in a yield of 35.52%. MS: m/z = 383.9 (M+1, ESI+)

### (Step f) Synthesis of (E)-4-(6-(2-(3-methylbenzylidene)hydrazinyl)-9-(3-methylisothiazol-5-yl)-9H-purin-2-yl)morpholine

(E)-5-(2-Chloro-6-(2-(3-methylbenzylidene)hydrazinyl)-9H-purin-9-yl)-3-methylisothiazole (45.0 mg), acetic acid (6 mg), and morpholine (43 mg) were dissolved in isopropyl alcohol (2.0 mL), and the mixture was stirred at 120°C overnight. Distilled water was added to the reaction solution and extracted with ethyl acetate. After washing the organic solvent with a saturated NaCl solution, sodium sulfate was added, and the reaction mixture was filtered and concentrated. The target compound (E)-4-(6-(2-(3-methylbenzylidene)hydrazinyl)-9-(3-methylisothiazol-5-yl)-9H-purin-2-yl)morpholine (1.96 mg) was obtained as a yellow solid in a yield of 4.52% through silica chromatography under dichloromethane/methanol conditions.

¹H NMR (400 MHz, DMSO-*d*₆) *δ* 10.72 (s, 1H), 9.80 (s, 1H), 8.41 (s, 1H), 7.73 (s, 1H), 7.70 (d, *J* = 7.2 Hz, 1H), 7.45 (t, *J =* 7.1 Hz, 1H), 7.39 (d, *J* = 7.2 Hz, 1H), 6.91 (s, 1H), 3.86 (s, 4H), 3.77 (s, 4H), 2.41 (s, 3H), 2.32 (s, 3H); MS: m/z = 435.2 (M+1, ESI+).

### Example 149. Synthesis of (E)-4-(9-(5-fluoropyridin-2-yl)-6-(2-(3-methylbenzylidene)hydrazinyl)-9H-purin-2-yl)morpholine

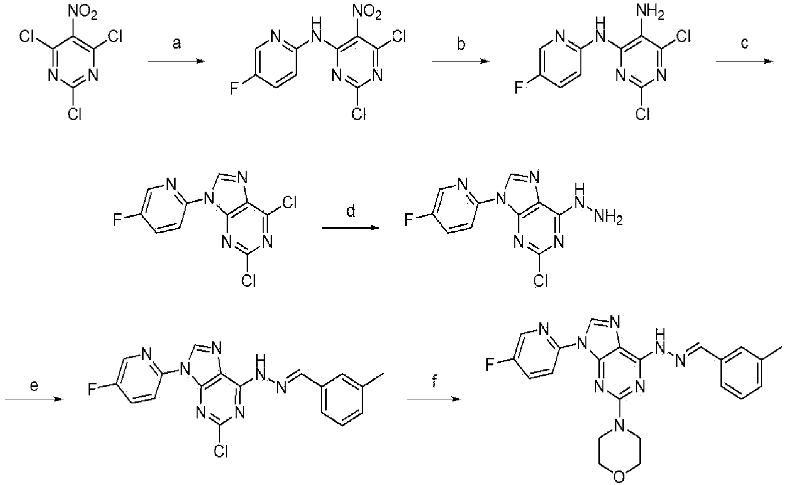

### (Step a) Synthesis of 2,6-dichloro-N-(5-fluoropyridin-2-yl)-5-nitropyrimidin-4-amine

2,4,6-Trichloro-5-nitropyrimidine (1.1 g) and N,N-diisopropylethylamine (932 mg) were dissolved in 1,4-dioxane (12 mL), then 5-fluoropyridin-2-amine (431 mg) dissolved in 1,4-dioxane (8 mL) was added at 0°C, and the mixture was stirred at room temperature for 3 hours. The reaction solution was extracted three times with water (20 mL) and ethyl acetate (10 mL) and washed with a saturated NaCl solution, then the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 2,6-dichloro-N-(5-fluoropyridin-2-yl)-5-nitropyrimidin-4-amine (440 mg) as a yellow solid in a yield of 30.15%. MS: m/z = 303.9 (M+1, ESI+).

### (Step b) Synthesis of 2, 6-dichloro-N⁴-(5-fluoropyridin-2-yl)pyrimidine-4,5-diamine

2,6-Dichloro-N-(5-fluoropyridin-2-yl)-5-nitropyrimidin-4-amine (440 mg) was dissolved in acetic acid (12 mL), then iron (811 mg) was added, and the mixture was stirred at room temperature for 1 hour. The reaction solution was extracted three times with water (20 mL) and ethyl acetate (10 mL), washed with a saturated NaCl solution, and the organic layer was dried using anhydrous sodium sulfate, and filtered under reduced pressure. The filtrate was collected and concentrated under reduced pressure to obtain the target compound 2,6-dichloro-N⁴-(5-fluoropyridin-2-yl)pyrimidine-4,5-diamine (350 mg) as a yellow solid in a yield of 88.29%. MS: m/z = 274.0 (M+1, ESI+).

### (Step c) Synthesis of 2, 6-dichloro-9- (5-fluoropyridin-2-yl)-9H-purine

2,6-Dichloro-N⁴-(5-fluoropyridin-2-yl)pyrimidine-4,5-diamine (300 mg) was dissolved in diethoxymethyl acetate (1.78 g), and the mixture was stirred at 80°C overnight. The reaction solution was extracted three times with water (8 mL) and ethyl acetate (5 mL) and washed with a saturated NaCl solution, then the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 2,6-dichloro-9-(5-fluoropyridin-2-yl)-9H-purine (205 mg) as a yellow solid in a yield of 65.91%.

¹H NMR (400 MHz, DMSO-*d*₆) *δ* 9.31 (s, 1H), 8.70 (d, *J* = 2.8 Hz, 1H), 8.33 (dd, *J* = 8.8, 3.6 Hz, 1H), 8.21 - 8.13 (m, 1H); MS: m/z = 283.9 (M+1, ESI+).

### (Step d) Synthesis of 2-chloro-9-(5-fluoropyridin-2-yl)-6-hydrazinyl-9H-purine

2,6-Dichloro-9-(5-fluoropyridin-2-yl)-9H-purine (205 mg) was dissolved in tetrahydrofuran (8 mL), then hydrazine monohydrate (181 mg) was added, and the mixture was stirred at room temperature for 20 minutes. After the reaction was completed, the reaction solution was concentrated, then distilled water (8 mL) was added, and the resulting solid was filtered to obtain the target compound 2-chloro-9-(5-fluoropyridin-2-yl)-6-hydrazinyl-9H-purine (180 mg) as a brown solid in a yield of 89.11%. MS: m/z = 280.0 (M+1, ESI+).

### (Step e) Synthesis of (E)-2-chloro-9-(5-fluoropyridin-2-yl)-6-(2-(3-methylbenzylidene)hydrazinyl)-9H-purine

2-Chloro-9-(5-fluoropyridin-2-yl)-6-hydrazinyl-9H-purine (180 mg) was dissolved in methanol (4 mL), then acetic acid (19 mg) and 3-methylbenzaldehyde (155 mg) were added, and the mixture was stirred at 70°C for 20 minutes. After concentrating the reaction solution under reduced pressure, the concentrate was subjected to silica chromatography under dichloromethane/methanol conditions to obtain the target compound (E)-2-chloro-9-(5-fluoropyridin-2-yl)-6-(2-(3-methylbenzylidene)hydrazinyl)-9H-purine (150 mg) as a yellow solid in a yield of 61.03%. MS: m/z = 382.0 (M+1, ESI+).

### (Step f) Synthesis of (E)-4-(9-(5-fluoropyridin-2-yl)-6-(2-(3-methylbenzylidene)hydrazinyl)-9H-purin-2-yl)morpholine

(E)-2-Chloro-9-(5-fluoropyridin-2-yl)-6-(2-(3-methylbenzylidene)hydrazinyl)-9H-purine (150 mg) was dissolved in isopropyl alcohol (6 mL), then acetic acid (24 mg) and morpholine (171 mg) were added, and the mixture was stirred at 120°C overnight. The reaction solution was extracted three times with water (20 mL) and dichloromethane (10 mL), washed with a saturated NaCl solution, then the organic layer was dried using anhydrous sodium sulfate and filtered under reduced pressure, and the filtrate was collected and concentrated under reduced pressure. Then, the concentrate was subjected to silica chromatography under dichloromethane/methanol conditions to obtain the target compound (E)-4-(9-(5-fluoropyridin-2-yl)-6-(2-(3-methylbenzylidene)hydrazinyl)-9H-purin-2-yl)morpholine (24 mg) as a yellow solid in a yield of 15.13%.

¹H NMR (400 MHz, DMSO-*d*₆) *δ* 11.50 (s, 1H), 8.67 - 8.60 (m, 2H), 8.58 (d, *J* = 2.4 Hz, 1H), 8.31 (s, 1H), 8.05 (t, *J* = 8.4 Hz, 1H), 7.58 (d, *J* = 7.2 Hz, 1H), 7.51 (s, 1H), 7.34 (t, *J* = 7.6 Hz, 1H), 7.21 (d, *J* = 7.2 Hz, 1H), 3.83 - 3.76 (m, 4H), 3.76 - 3.69 (m, 4H), 2.36 (s, 3H); MS: m/z = 433.3 (M+1, ESI+).

### Example 150. Synthesis of (E)-4-(9-(2-methoxypyridin-4-yl)-8-methyl-6-(2-(3-methylbenzylidene)hydrazinyl)-9H-purin-2-yl)morpholine

The target compound (E)-4-(9-(2-methoxypyridin-4-yl)-8-methyl-6-(2-(3-methylbenzylidene)hydrazinyl)-9H-purin-2-yl)morpholine was synthesized in the same manner as in Example 149 above, except that 2-methoxypyridin-4-amine was used in (Step a) and trimethyl orthoacetate was used in (Step c) .

¹H NMR (400 MHz, DMSO-*d*₆) *δ* 11.44 (s, 1H), 8.37 (d, *J* = 5.6 Hz, 1H), 8.29 (s, 1H), 7.55 - 7.51 (m, 2H), 7.33 (t, *J* = 7.6 Hz, 1H), 7.27 (dd, *J* = 5.6, 1.6 Hz, 1H), 7.19 (d, *J* = 7.6 Hz, 1H), 7.11 (d, *J* = 1.6 Hz, 1H), 3.94 (s, 3H), 3.64 (s, 8H), 2.48 (s, 3H), 2.36 (s, 3H); MS: m/z = 459 (M+1, ESI+).

### Example 151. Synthesis of (E)-4-(8-methyl-6-(2-(3-methylbenzylidene)hydrazinyl)-9-(2-methylpyridin-3-yl)-9H-purin-2-yl)morpholine

The target compound (E)-4-(8-methyl-6-(2-(3-methylbenzylidene)hydrazinyl)-9-(2-methylpyridin-3-yl)-9H-purin-2-yl)morpholine was synthesized in the same manner as in Example 149 above, except that 2-methylpyridin-3-amine was used in (Step a) and trimethyl orthoacetate was used in (Step c).

¹H NMR (400 MHz, DMSO-*d*₆) *δ* 11.43 (s, 1H), 8.63 (d, *J* = 5.2 Hz, 1H), 8.29 (s, 1H), 7.88 (d, *J* = 7.2 Hz, 1H), 7.56 - 7.52 (m, 2H), 7.47 (dd, *J* = 8.0, 4.8 Hz, 1H), 7.33 (t, *J* = 7.6 Hz, 1H), 7.19 (d, *J* = 7.2 Hz, 1H), 3.61-3.57 (m, 8H), 2.36 (s, 3H), 2.26 (s, 3H), 2.23 (s, 3H); MS: m/z = 443 (M+1, ESI+).

### Example 152. Synthesis of (E)-4-(9-(2,4-dimethylthiazol-5-yl)-8-methyl-6-(2-(3-methylbenzylidene)hydrazinyl)-9H-purin-2-yl)morpholine

The target compound (E)-4-(9-(2,4-dimethylthiazol-5-yl)-8-methyl-6-(2-(3-methylbenzylidene)hydrazinyl)-9H-purin-2-yl)morpholine was synthesized in the same manner as in Example 149 above, except that 2,4-dimethylthiazol-5-amine was used in (Step a) and trimethyl orthoacetate was used in (Step c).

¹H NMR (400 MHz, DMSO-*d*₆) δ 9.37 (s, 1H), 8.02 (s, 1H), 8.41 (s, 1H), 7.74 (s, 1H), 7.66 (d, *J* = 7.2 Hz, 1H), 7.45 (t, *J* = 7.1 Hz, 1H), 7.27 (d, *J* = 7.2 Hz, 1H), 3.73 - 3.64 (m, 8H), 3.86 (s, 4H), 2.77 (s, 3H), 2.62 (s, 3H), 2.42 (s, 3H), 2.22 (s, 3H); MS: m/z = 463 (M+1, ESI+).

### Example 153. Synthesis of (E)-4-(9-(1,5-dimethyl-1H-pyrazol-3-yl)-8-methyl-6-(2-(3-methylbenzylidene)hydrazinyl)-9H-purin-2-yl)morpholine

The target compound (E)-4-(9-(1,5-dimethyl-1H-pyrazol-3-yl)-8-methyl-6-(2-(3-methylbenzylidene)hydrazinyl)-9H-purin-2-yl)morpholine was synthesized in the same manner as in Example 149 above, except that 1,5-dimethyl-1H-pyrazol-3-amine was used in (Step a) and trimethyl orthoacetate was used in (Step c).

¹H NMR (400 MHz, DMSO-*d*₆) *δ* 11.38 (s, 1H), 8.28 (s, 1H), 7.54 (d, *J* = 7.6 Hz, 1H), 7.51 (s, 1H), 7.33 (t, *J* = 7.6 Hz, 1H), 7.19 (d, *J* = 7.6 Hz, 1H), 6.41 (s, 1H), 3.79 (s, 3H), 3.66 (s, 8H), 2.49 (s, 3H), 2.35 (s, 6H); MS: m/z = 446.3 (M+1, ESI+).

### Example 154. Synthesis of 4-(8-methyl-9-phenyl-6-(3-(m-tolyl)-1H-pyrazol-1-yl)-9H-purin-2-yl)morpholine

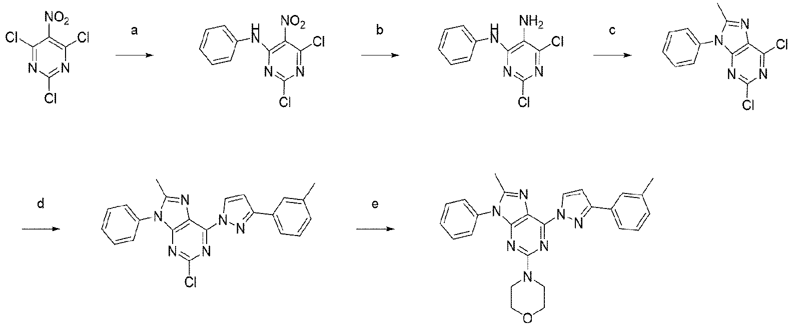

The target compound 4-(8-methyl-9-phenyl-6-(3-(m-tolyl)-1H-pyrazol-1-yl)-9H-purin-2-yl)morpholine was synthesized in the same manner as in Example 149 above, except that aniline was used in (Step a), trimethyl orthoacetate was used in (Step c), and (Step d) was performed in the same manner as in (Step a) of Example 64.

¹H NMR (400 MHz, DMSO-*d*₆) *δ* 9.21 (d, *J* = 2.4 Hz, 1H), 7.82 (s, 1H), 7.80 (s, 1H), 7.64 - 7.58 (m, 5H), 7.39 (t, *J* = 7.6 Hz, 1H), 7.24 (d, *J* = 7.6 Hz, 1H), 7.19 (d, *J* = 2.4 Hz, 1H), 3.67 (m, 8H), 2.45 (s, 3H), 2.41 (s, 3H); MS: m/z = 452 (M+1, ESI+).

### Example 155. Synthesis of (E)-4-(6-(2-(3-methylbenzylidene)hydrazinyl)-9-(pyrimidin-2-yl)-9H-purin-2-yl)-morpholine

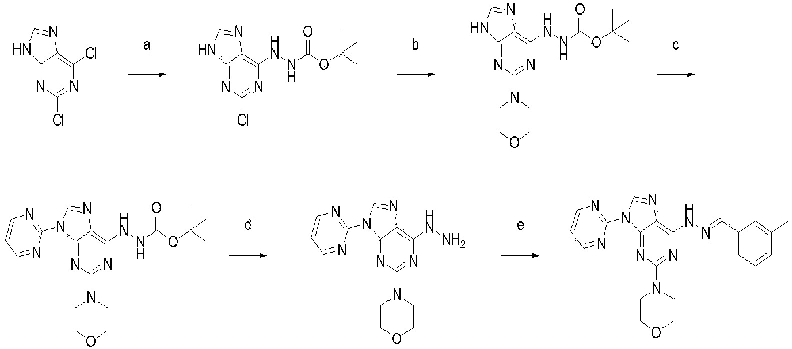

### (Step a) Synthesis of tert-butyl 2-(2-chloro-9H-purin-6-yl)hydrazinyl-1-carboxylate

To 2,6-dichloro-9H-purine (1.0 g), tert-butyl hydrazinecarboxylate (1.4 g) and tetrahydrofuran (26.5 mL) were added, and the mixture was stirred at 50°C overnight. After drying the reaction solution, the target compound tert-butyl 2-(2-chloro-9H-purin-6-yl)hydrazinyl-1-carboxylate (2.9 g) was synthesized through silica chromatography under dichloromethane/methanol conditions.

### (Step b) Synthesis of tert-butyl 2-(2-morpholino-9H-purin-6-yl)hydrazinyl-1-carboxylate

Morpholine (17.4 g) was added to tert-butyl 2-(2-chloro-9H-purin-6-yl)hydrazinyl-1-carboxylate (1.0 g), and the mixture was stirred at 90°C for 4 hours. After adding ethyl acetate and distilled water to the reaction solution, the organic solvent was concentrated to obtain the target compound tert-butyl 2-(2-morpholino-9H-purin-6-yl)hydrazinyl-1-carboxylate (840 mg) as a solid in a yield of 71.3%.

### (Step c) Synthesis of tert-butyl 2-(2-morpholino-9-(pyrimidin-2-yl)-9H-purin-6-yl)hydrazinyl-1-carboxylate

2-Iodopyrimidine (314.5 mg), cesium carbonate (1.87 g), and dimethylformamide (9.5 mL) were added to tert-butyl 2-(2-morpholino-9H-purin-6-yl)hydrazinyl-1-carboxylate (640 mg), and the mixture was stirred at 80°C for 3 hours. After adding ethyl acetate and distilled water to the reaction solution, the organic solvent was concentrated, followed by silica chromatography under dichloromethane/isopropyl alcohol conditions, to obtain the target compound tert-butyl 2-(2-morpholino-9-(pyrimidin-2-yl)-9H-purin-6-yl)hydrazinyl-1-carboxylate (50 mg) in a yield of 6.3%.

### (Step d) Synthesis of 4-(6-hydrazinyl-9-(pyrimidin-2-yl)-9H-purin-2-yl)morpholine

Tert-Butyl 2-(2-morpholino-9-(pyrimidin-2-yl)-9H-purin-6-yl)hydrazinyl-1-carboxylate (30 mg) was dissolved in 1,4-dioxane (181.4 uL), then 4 M hydrochloric acid (26.5 mg) in 1,4-dioxane as a solvent was added, and the mixture was stirred at 30°C for 8.5 hours. The reaction solution was concentrated to synthesize the target compound 4-(6-hydrazinyl-9-(pyrimidin-2-yl)-9H-purin-2-yl)morpholine (30 mg) .

### (Step e) Synthesis of (E)-4-(6-(2-(3-methylbenzylidene)hydrazinyl)-9-(pyrimidin-2-yl)-9H-purin-2-yl)-morpholine

To 4-(6-hydrazinyl-9-(pyrimidin-2-yl)-9H-purin-2-yl)morpholine (30 mg), ethanol (957.5 uL) and 3-methylbenzaldehyde (11.5 mg) were added, and the mixture was stirred at 30°C for 18 hours. After concentrating the reaction solution, the concentrate was subjected to silica chromatography under isopropyl alcohol/dichloromethane conditions to obtain the target compound (E)-4-(6-(2-(3-methylbenzylidene)hydrazinyl)-9-(pyrimidin-2-yl)-9H-purin-2-yl)-morpholine (5 mg) as a gray color in a yield of 12.6%.

¹H NMR (400 MHz, DMSO-*d*₆) *δ* 12.76 (s, 1H), 9.10 (s, 1H), 9.06 (d, *J* = 4.8 Hz, 2H), 8.27 (s, 1H), 7.56 - 7.50 (m, 3H), 7.32 (t, *J* = 7.6 Hz, 1H), 7.21 - 7.19 (m, 1H), 3.77 - 3.69 (m, 8H), 2.32 (s, 3H); MS: m/z = 416 (M+1, ESI+).

### Example 156. Synthesis of (E)-2-(8-methyl-6-(2-(3-methylbenzylidene)hydrazinyl)-2-morpholino-9H-purin-9-yl)-1-phenylethan-1-one

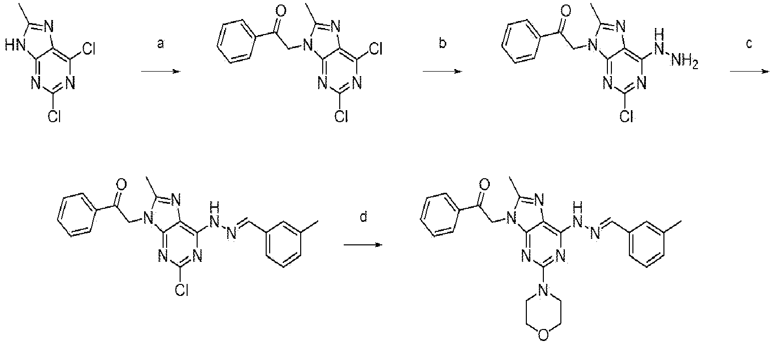

### (Step a) Synthesis of 2-(2,6-dichloro-8-methyl-9H-purin-9-yl)-1-phenylethan-1-one

Dichloromethane (22.2 mL), triethylamine (1.35 g), and 2-bromoacetophenone (1.15 g) were added to 2,6-dichloro-8-methyl-9H-purine (900 mg) at 60°C, followed by stirring for 6 hours. The target compound 2-(2,6-dichloro-8-methyl-9H-purin-9-yl)-1-phenylethan-1-one (1.0 g) was synthesized in a yield of 70.2% through silica chromatography under dichloromethane/methanol conditions.

### (Step b) Synthesis of 2-(2-chloro-6-hydrazinyl-8-methyl-9H-purin-9-yl)-1-phenylethan-1-one

To 2-(2,6-dichloro-8-methyl-9H-purin-9-yl)-1-phenylethan-1-one (950 mg), tetrahydrofuran (5.9 mL) and hydrazine monohydrate (296.2 mg) were added, and the mixture was stirred at 70°C for 1.5 hours. The resulting solid was filtered and distilled water was added. The solid was concentrated under reduced pressure to synthesize the target compound 2-(2-chloro-6-hydrazinyl-8-methyl-9H-purin-9-yl)-1-phenylethan-1-one (0.89 g) in a yield of 95%.

### (Step c) Synthesis of (E)-2-(2-chloro-8-methyl-6-(2-(3-methylbenzylidene)hydrazinyl)-9H-purin-9-yl)-1-phenylethan-1-one

To 2-(2-chloro-6-hydrazinyl-8-methyl-9H-purin-9-yl)-1-phenylethan-1-one (0.89 g), ethanol (28.1 mL), acetic acid (10 drops), and 3-methylbenzaldehyde (337.6 mg) were added, and the mixture was stirred at 70°C for 1 hour. The resulting solid was filtered and concentrated under reduced pressure to synthesize the target compound (E)-2-(2-chloro-8-methyl-6-(2-(3-methylbenzylidene)hydrazinyl)-9H-purin-9-yl)-1-phenylethan-1-one (1.1 g) in a yield of 93.5%.

### (Step d) Synthesis of (E)-2-(8-methyl-6-(2-(3-methylbenzylidene)hydrazinyl)-2-morpholino-9H-purin-9-yl)-1-phenylethan-1-one

To (E)-2-(2-chloro-8-methyl-6-(2-(3-methylbenzylidene)hydrazinyl)-9H-purin-9-yl)-1-phenylethan-1-one (0.89 g), morpholine (10.6 g) was added, and the mixture was stirred at 90°C for 4 hours. After adding distilled water and ethyl acetate, the organic solvent was extracted. After concentration of the solvent under reduced pressure, the target compound (E)-2-(8-methyl-6-(2-(3-methylbenzylidene)hydrazinyl)-2-morpholino-9H-purin-9-yl)-1-phenylethan-1-one (600 mg) was synthesized as a yellow solid in a yield of 60.1% through silica chromatography under ethyl acetate/hexane conditions.

¹H NMR (400 MHz, DMSO-*d*₆) *δ* 11.33 (s, 1H), 8.26 (s, 1H), 8.16 - 8.14 (m, 2H), 7.76 (t, *J =* 7.4 Hz, 1H), 7.63 (t, *J* = 7.8 Hz, 2H), 7.53 (d, *J* = 8.0 Hz, 1H), 7.50 (s, 1H), 7.32 (t, *J =* 7.6 Hz, 1H), 7.18 (d, *J* = 7.6 Hz, 1H), 5.76 (d, *J* = 4.0 Hz, 2H), 3.63 (s, 8H), 2.36 (s, 3H), 2.35 (s, 3H); MS: m/z = 470 (M+1, ESI+).

### Example 157. Synthesis of (E)-2-(8-methyl-6-(2-(3-methylbenzylidene)hydrazinyl)-2-morpholino-9H-purin-9-yl)-1-(pyridin-2-yl)ethan-1-one

The target compound (E)-2-(8-methyl-6-(2-(3-methylbenzylidene)hydrazinyl)-2-morpholino-9H-purin-9-yl)-1-(pyridin-2-yl)ethan-1-one was synthesized as a yellow solid in the same manner as in Example 156 above, except that 2-(bromoacetyl)pyridine hydrobromide was used in (Step a).

¹H NMR (400 MHz, DMSO-*d*₆) *δ* 11.33 (s, 1H), 8.86 (dd, J = 2.6, 3.4 Hz, 1H), 8.26 (s, 1H), 8.10 (td, *J* = 1.6, 7.6 Hz, 1H), 8.04 (d, *J =* 7.6 Hz, 1H), 7.82 - 7.78 (m, 1H), 7.53 (d, *J* = 7.6 Hz, 1H), 7.50 (s, 1H), 7.32 (t, *J* = 7.6 Hz, 1H), 7.19 (d, *J* = 8.0 Hz, 1H), 5.83 (s, 2H), 3.61 (s, 8H), 2.38 (s, 3H), 2.35 (s, 3H); MS: m/z = 471 (M+1, ESI+).

### Example 158. Synthesis of (E)-2-(8-methyl-6-(2-(3-methylbenzylidene)hydrazinyl)-2-morpholino-9H-purin-9-yl)-1-(pyridin-3-yl)ethan-1-one

The target compound (E)-2-(8-methyl-6-(2-(3-methylbenzylidene)hydrazinyl)-2-morpholino-9H-purin-9-yl)-1-(pyridin-3-yl)ethan-1-one was synthesized as a white solid in the same manner as in Example 156 above, except that 3-(bromoacetyl)pyridine hydrobromide was used in (Step a).

¹H NMR (400 MHz, DMSO-*d*₆) *δ* 11.30 (s, 1H), 9.32 (d, J = 2.0 Hz, 1H), 8.90 (dd, *J* = 1.6, 4.8 Hz, 1H), 8.46 (dt, J = 2.0, 8.1 Hz, 1H), 8.26 (s, 1H), 7.66 (dd, *J* = 4.8, 8.0 Hz, 1H), 7.53 (d, *J* = 7.6 Hz, 1H), 7.50 (s, 1H), 7.32 (t, *J* = 7.6 Hz, 1H), 7.19 (d, *J* = 8.0 Hz, 1H), 5.80 (s, 2H), 3.63 (s, 8H), 2.38 (s, 3H), 2.35 (s, 3H); MS: m/z = 471 (M+1, ESI+).

### Example 159. Synthesis of (E)-2-(8-methyl-6-(2-(3-methylbenzylidene)hydrazinyl)-2-morpholino-9H-purin-9-yl)-1-(pyridin-4-yl)ethan-1-one

The target compound (E)-2-(8-methyl-6-(2-(3-methylbenzylidene)hydrazinyl)-2-morpholino-9H-purin-9-yl)-1-(pyridin-4-yl)ethan-1-one was synthesized as a yellow solid in the same manner as in Example 156 above, except that 4-(bromoacetyl)pyridine hydrobromide was used in (Step a).

¹H NMR (400 MHz, DMSO-*d*₆) *δ* 11.31 (s, 1H), 8.91 (dd, J = 1.8, 4.6 Hz, 2H), 8.26 (s, 1H), 8.00 (dd, *J* = 1.6, 4.4 Hz, 2H), 7.53 (d, *J* = 7.6 Hz, 1H), 7.50 (s, 1H), 7.32 (t, *J* = 7.4 Hz, 1H), 7.18 (d, *J* = 7.6 Hz, 1H), 5.78 (s, 2H), 3.63 (s, 8H), 2.38 (s, 3H), 2.35 (s, 3H); MS: m/z = 471 (M+1, ESI+).

### Example 160. Synthesis of (E)-4-(9-(3-methoxyphenyl)-8-methyl-6-(2-(3-methylbenzylidene)hydrazinyl)-9H-purin-2-yl)morpholine

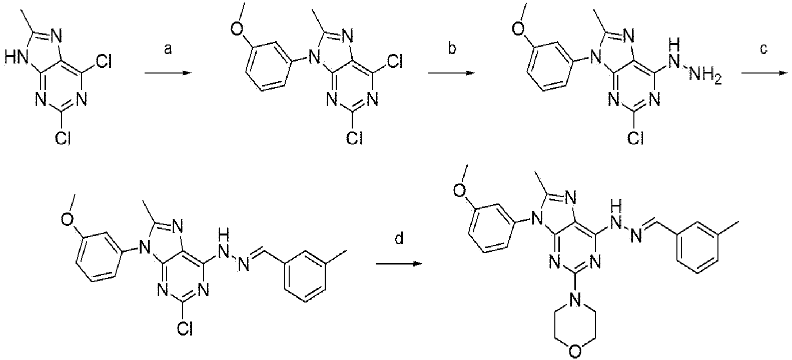

### (Step a) Synthesis of 2,6-dichloro-9-(3-methoxyphenyl)-8-methyl-9H-purine

Copper(II) acetate (0.45 g), o-phenanthroline (0.89 g), and acetonitrile (16.4 mL) were added to 2,6-dichloro-8-methyl-9H-purine (0.5 g), and the mixture was stirred at 40°C for 21 hours. The target compound 2,6-dichloro-9-(3-methoxyphenyl)-8-methyl-9H-purine (439 mg) was synthesized in a yield of 57.6% through silica chromatography under ethyl acetate/hexane conditions.

### (Step b) Synthesis of 2-chloro-6-hydrazinyl-9-(3-methoxyphenyl)-8-methyl-9H-purine

To 2,6-dichloro-9-(3-methoxyphenyl)-8-methyl-9H-purine (440 mg), tetrahydrofuran (2.85 mL) and hydrazine monohydrate (142.5 mg) were added, and the mixture was stirred at 60°C for 2 hours. The resulting solid was filtered and distilled water was added. The solid was concentrated under reduced pressure to synthesize the target compound 2-chloro-6-hydrazinyl-9-(3-methoxyphenyl)-8-methyl-9H-purine (340 mg) in a yield of 78.4%.

### (Step c) Synthesis of (E)-2-chloro-9-(3-methoxyphenyl)-8-methyl-6-(2-(3-methylbenzylidene)hydrazinyl)-9H-purine

To 2-chloro-6-hydrazinyl-9-(3-methoxyphenyl)-8-methyl-9H-purine (340 mg), ethanol (11.16 mL), 3-methylbenzaldehyde (268.1 mg), and acetic acid (3.35 mg) were added, and the mixture was stirred at 60°C for 1 hour. The resulting solid was filtered and concentrated under reduced pressure to synthesize the target compound (E)-2-chloro-9-(3-methoxyphenyl)-8-methyl-6-(2-(3-methylbenzylidene)hydrazinyl)-9H-purine (0.39 g) in a yield of 85.9%.

### (Step d) Synthesis of (E)-4-(9-(3-methoxyphenyl)-8-methyl-6-(2-(3-methylbenzylidene)hydrazinyl)-9H-purin-2-yl)morpholine

To (E)-2-chloro-9-(3-methoxyphenyl)-8-methyl-6-(2-(3-methylbenzylidene)hydrazinyl)-9H-purine (0.39 g), morpholine (4.79 g) was added, and the mixture was stirred at 90°C for 1 hour. The resulting mixture was subjected to silica chromatography under ethyl acetate/dichloromethane/hexane conditions to obtain the target compound (E)-4-(9-(3-methoxyphenyl)-8-methyl-6-(2-(3-methylbenzylidene)hydrazinyl)-9H-purin-2-yl)morpholine (111 mg) as a gray solid in a yield of 25.3%.

¹H NMR (400 MHz, DMSO-*d*₆) *δ* 11.37 (s, 1H), 8.29 (s, 1H), 7.55 - 7.47 (m, 3H), 7.33 (t, *J =* 7.6 Hz, 1H), 7.19 (d, *J* = 7.6 Hz, 2H), 7.12 (t, *J* = 2.0 Hz, 1H), 7.09 - 7.06 (m, 1H), 3.83 (s, 3H), 3.62 (s, 8H), 2.37 (s, 3H), 2.36 (s, 3H). MS: m/z = 458 (M+1, ESI+); MS: m/z = 458 (M+1, ESI+).

### Example 161. Synthesis of (E)-4-(9-(4-methoxyphenyl)-8-methyl-6-(2-(3-methylbenzylidene)hydrazinyl)-9H-purin-2-yl)morpholine

The target compound (E)-4-(9-(4-methoxyphenyl)-8-methyl-6-(2-(3-methylbenzylidene)hydrazinyl)-9H-purin-2-yl)morpholine was obtained as a white solid in the same manner as in Example 160 above, except using (4-methoxyphenyl)boronic acid in (Step a).

¹H NMR (400 MHz, DMSO-*d*₆) *δ* 11.36 (s, 1H), 7.53 (d, *J* = 7.6 Hz, 1H), 7.50 (s, 1H), 7.44 - 7.40 (m, 2H), 7.32 (t, *J* = 7.6 Hz, 1H), 7.19 (d, *J* = 7.6 Hz, 1H), 7.14 - 7.11 (m, 2H), 3.84 (s, 3H), 3.61 (s, 8H), 2.35 (s, 3H), 2.34 (s, 3H); MS: m/z = 458 (M+1, ESI+).

### Example 162. Synthesis of (E)-4-(9-(3-fluorophenyl)-8-methyl-6-(2-(3-methylbenzylidene)hydrazinyl)-9H-purin-2-yl)morpholine

The target compound (E)-4-(9-(3-fluorophenyl)-8-methyl-6-(2-(3-methylbenzylidene)hydrazinyl)-9H-purin-2-yl)morpholine was synthesized as a gray solid in the same manner as in Example 160 above, except using (3-fluorophenyl)boronic acid in (Step a).

¹H NMR (400 MHz, DMSO-*d*₆) *δ* 11.41 (s, 1H), 8.29 (s, 1H), 7.67 - 7.61 (m, 1H), 7.55 - 7.50 (m, 3H), 7.42 - 7.30 (m, 3H), 7.20 - 7.15 (m, 1H), 3.62 (s, 8H), 2.41 (s, 3H), 2.35 (s, 3H). MS: m/z = 458 (M+1, ESI+); MS: m/z = 446 (M+1, ESI+).

### Example 163. Synthesis of (E)-4-(9-(3-chlorophenyl)-8-methyl-6-(2-(3-methylbenzylidene)hydrazinyl)-9H-purin-2-yl)morpholine

The target compound (E)-4-(9-(3-chlorophenyl)-8-methyl-6-(2-(3-methylbenzylidene)hydrazinyl)-9H-purin-2-yl)morpholine was synthesized as a yellow solid in the same manner as in Example 160 above, except using (3-chlorophenyl)boronic acid in (Step a).

¹H NMR (400 MHz, DMSO-*d*₆) *δ* 11.40 (s, 1H), 8.29 (s, 1H), 7.71 (t, *J* = 1.8 Hz, 1H), 7.63 (d, *J* = 8.0 Hz, 1H), 7.61 - 7.59 (m, 1H), 7.57 (t, *J* = 1.8 Hz, 1H), 7.55 - 7.51 (m, 3H), 7.33 (t, *J =* 7.6 Hz, 1H), 7.19 (t, *J =* 7.6 Hz, 1H), 3.62 (s, 8H), 2.37 (s, 3H), 2.32 (s, 3H); MS: m/z = 462 (M+1,

ESI+).

### Example 164. Synthesis of (E)-4-(8-methyl-6-(2-(3-methylbenzylidene)hydrazinyl)-9-(pyridin-2-yl)-9H-purin-2-yl)morpholine

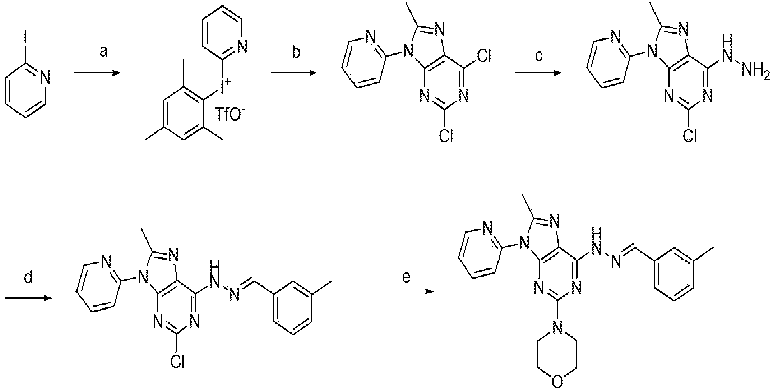

### (Step a) Synthesis of mesityl (pyridin-2-yl) iodonium trifluoromethane sulfonate

2-Iodopyridine (3.0 g) and m-chloroperoxybenzoic acid (3.79 g) were dissolved in dichloromethane (200 mL), then trifluoromethanesulfonic acid (8.82 g) was added at 0°C, and the mixture was stirred at room temperature for 1 hour. Then, mesitylene (1.93 g) was added at 0°C, and the mixture was stirred overnight at room temperature. The reaction solution was extracted three times with water (50 mL) and dichloromethane (30 mL), washed with a saturated NaCl solution, then the organic layer was dried using anhydrous sodium sulfate and filtered under reduced pressure, and the filtrate was collected and concentrated under reduced pressure. The concentrated product was subjected to silica chromatography under dichloromethane/methanol conditions to synthesize the target compound mesityl (pyridin-2-yl) iodonium trifluoromethanesulfonate (2.2 g) as a brown solid in a yield of 31.78%. MS: m/z = 324.0 (M-TfO-, ESI+).

### (Step b) Synthesis of 2,6-dichloro-8-methyl-9-(pyridin-2-yl)-9H-purine

Mesityl (pyridin-2-yl)iodonium trifluoromethanesulfonate (2.0 g) and 2,6-dichloro-8-methyl-9H-purine (657 mg) were dissolved in dichloromethane (30 mL), then copper bromide (93 mg) and triethylamine (493 mg) were added, and the mixture was stirred at 60°C overnight. The reaction solution was extracted three times with water (20 mL) and ethyl acetate (10 mL) and washed with a saturated NaCl solution, then the organic layer was dried using anhydrous sodium sulfate, filtered under reduced pressure, and the filtrate was collected and concentrated under reduced pressure. The concentrate was subjected to silica chromatography under dichloromethane/methanol conditions to obtain the target compound 2,6-dichloro-8-methyl-9-(pyridin-2-yl)-9H-purine (240 mg) as a brown solid in a yield of 26.45%. MS: m/z = 280.0 (M+1, ESI+).

### (Step c) Synthesis of 2-chloro-6-hydrazinyl-8-methyl-9-(pyridin-2-yl)-9H-purine

2,6-Dichloro-8-methyl-9-(pyridin-2-yl)-9H-purine (240 mg) was dissolved in tetrahydrofuran (5 mL), then hydrazine monohydrate (215 mg) was added, and the mixture was stirred at room temperature for 20 minutes. After the reaction was completed, the reaction solution was concentrated, then distilled water (8 mL) was added, and the resulting solid was filtered to obtain the target compound 2-chloro-6-hydrazinyl-8-methyl-9-(pyridin-2-yl)-9H-purine (170 mg) as a brown solid in a yield of 71.86%. MS: m/z = 276.1 (M+1, ESI+).

### (Step d) Synthesis of (E)-2-chloro-8-methyl-6-(2-(3-methylbenzylidene)hydrazinyl)-9-(pyridin-2-yl)-9H-purine

2-Chloro-6-hydrazinyl-8-methyl-9-(pyridin-2-yl)-9H-purine (170 mg) was dissolved in methanol (6 mL), and acetic acid (18 mg) and 3-methylbenzaldehyde (148 mg) were stirred at 70°C for 20 minutes. After concentrating the reaction solution under reduced pressure, the concentrate was subjected to silica chromatography under dichloromethane/methanol conditions to obtain the target compound (E)-2-chloro-8-methyl-6-(2-(3-methylbenzylidene)hydrazinyl)-9-(pyridin-2-yl)-9H-purine (90 mg) as a yellow solid in a yield of 23.79%. MS: m/z = 378.1 (M+1, ESI+).

### (Step e) Synthesis of (E)-4-(8-methyl-6-(2-(3-methylbenzylidene)hydrazinyl)-9-(pyridin-2-yl)-9H-purin-2-yl)morpholine

(E)-2-Chloro-8-methyl-6-(2-(3-methylbenzylidene)hydrazinyl)-9-(pyridin-2-yl)-9H-purine (70 mg) was dissolved in isopropyl alcohol (3 mL), then acetic acid (11 mg) and morpholine (81 mg) were added, and the mixture was stirred at 120°C overnight. The reaction solution was extracted three times with water (20 mL) and ethyl acetate (10 mL) and washed with a saturated NaCl solution, then the organic layer was dried using anhydrous sodium sulfate, filtered under reduced pressure, and the filtrate was collected and concentrated under reduced pressure. Then, the target compound (E)-4-(8-methyl-6-(2-(3-methylbenzylidene)hydrazinyl)-9-(pyridin-2-yl)-9H-purin-2-yl)morpholine (9.58 mg) was obtained as a yellow solid in a yield of 12.05% through silica chromatography under dichloromethane/methanol conditions.

¹H NMR (400 MHz, DMSO-*d*₆) *δ* 11.42 (s, 1H), 8.65 (dd, J = 4.8, 1.2 Hz, 1H), 8.31 (s, 1H), 8.10 (td, *J* = 8.0, 2.0 Hz, 1H), 7.88 (d, *J* = 8.0 Hz, 1H), 7.58 - 7.48 (m, 3H), 7.33 (t, *J* = 7.6 Hz, 1H), 7.20 (d, *J* = 7.2 Hz, 1H), 3.71 - 3.60 (m, 8H), 2.57 (s, 3H), 2.36 (s, 3H); MS: m/z = 429.4 (M+1, ESI+).

### Example 165. Synthesis of (E)-4-(8-methyl-6-(2-(3-methylbenzylidene)hydrazinyl)-9-phenyl-9H-purin-2-yl)morpholine

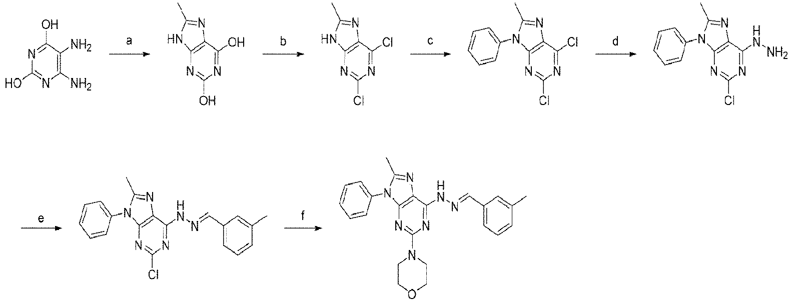

### (Step a) Synthesis of 8-methyl-9H-purin-2,6-diol

5,6-Diaminopyrimidine-2,4-diol (4.00 g) was dissolved in acetic anhydride (50 mL), and the mixture was stirred at 145°C for 15 hours. After the reaction solution was distilled under reduced pressure, 1.5 N sodium hydroxide solution (200 mL) was added and boiled for 30 minutes, followed by treatment with charcoal, and the resulting solution was acidified with acetic acid while the product was hot. The target compound 8-methyl-9H-purin-2,6-diol (4.00 g) was obtained as a colored solid during cooling in a yield of 85.1%. MS: m/z = 167.1 (M+1, ESI+).

### (Step b) Synthesis of 2,6-dichloro-8-methyl-9H-purine

8-Methyl-9H-purin-2,6-diol (4.00 g) was dissolved in phosphoryl chloride (50 mL), N,N-diisopropylethylamine (9.27 g) was added, and the mixture was stirred at 100°C overnight. The reaction solution was extracted three times with water (200 mL) and dichloromethane (200 mL), washed with a saturated NaCl solution, then the organic layer was dried using anhydrous sodium sulfate and filtered under reduced pressure, and the filtrate was collected and concentrated under reduced pressure. The concentrate was subjected to silica chromatography under dichloromethane/methanol conditions to obtain the target compound 2,6-dichloro-8-methyl-9H-purine (3.50 g) as a brown solid in a yield of 72.9%. MS: m/z = 202.1 (M+1, ESI+).

### (Step c) Synthesis of 2,6-dichloro-8-methyl-9-phenyl-9H-purine

2, 6-Dichloro-8-methyl-9H-purine (500 mg) and phenylboronic acid (1.50 g) were dissolved in dichloromethane (30 mL), then o-phenanthroline (887 mg) and copper (II) acetate (806 mg) were added, and the mixture was stirred overnight at 40°C under O₂ conditions. The reaction solution was filtered, and the filtrate was extracted three times with water (100 mL) and dichloromethane (100 mL), washed with a saturated NaCl solution, and the organic layer was dried using anhydrous sodium sulfate, filtered under reduced pressure, and the filtrate was collected and concentrated under reduced pressure. The concentrate was subjected to silica chromatography under ethyl acetate/petroleum ether conditions to obtain the target compound 2,6-dichloro-8-methyl-9-phenyl-9H-purine (180 mg) as a white solid in a yield of 26.0%. MS: m/z = 279.1 (M+1, ESI+).

### (Step d) Synthesis of 2-chloro-6-hydrazinyl-8-methyl-9-phenyl-9H-purine

2,6-Dichloro-8-methyl-9-phenyl-9H-purine (160 mg) was dissolved in tetrahydrofuran (10 mL), hydrazine monohydrate (113 mg) was added, and the mixture was stirred at room temperature for 30 minutes. After the reaction was completed, the reaction solution was concentrated, then distilled water (10 mL) was added, and the resulting solid was filtered to obtain the target compound 2-chloro-6-hydrazinyl-8-methyl-9-phenyl-9H-purine (140 mg) as a white solid in a yield of 87.5%. MS: m/z = 275.1 (M+1, ESI+).

### (Step e) Synthesis of (E)-2-chloro-8-methyl-6-(2-(3-methylbenzylidene)hydrazinyl)-9-phenyl-9H-purine

2-Chloro-6-hydrazinyl-8-methyl-9-phenyl-9H-purine (120 mg) was dissolved in methanol (10 mL), then acetic acid (0.1 mL) and 3-methylbenzaldehyde (90 mg) were added, and the mixture was stirred at 70°C for 30 minutes. After concentrating the reaction solution under reduced pressure, the concentrate was subjected to silica chromatography under ethyl acetate/petroleum ether conditions to obtain the target compound (E)-2-chloro-8-methyl-6-(2-(3-methylbenzylidene)hydrazinyl)-9-phenyl-9H-purine (120 mg) as a white solid in a yield of 72.7%. MS: m/z = 377.2 (M+1, ESI+).

### (Step f) Synthesis of (E)-4-(8-methyl-6-(2-(3-methylbenzylidene)hydrazinyl)-9-phenyl-9H-purin-2-yl)morpholine

(E)-2-Chloro-8-methyl-6-(2-(3-methylbenzylidene)hydrazinyl)-9-phenyl-9H-purine (120 mg) was dissolved in isopropyl alcohol (10 mL), then acetic acid (0.1 mL) and morpholine (0.5 mL) were added, and the mixture was stirred at 120°C overnight. The reaction solution was extracted three times with water (30 mL) and ethyl acetate (50 mL) and washed with a saturated NaCl solution, then the organic layer was dried using anhydrous sodium sulfate, filtered under reduced pressure, and the filtrate was collected and concentrated under reduced pressure. Then, the target compound (E)-4-(8-methyl-6-(2-(3-methylbenzylidene)hydrazinyl)-9-phenyl-9H-purin-2-yl)morpholine (45 mg) was obtained as a white solid in a yield of 33.0% through silica chromatography under dichloromethane/methanol conditions.

¹H NMR (400 MHz, DMSO-*d*₆) *δ* 11.37 (s, 1H), 8.29 (s, 1H), 7.65 - 7.49 (m, 7H), 7.32 (t, *J =* 7.6 Hz, 1H), 7.19 (d, *J* = 7.5 Hz, 1H), 3.61 (s, 8H), 2.38 (s, 3H), 2.36 (s, 3H); MS: m/z = 428.3 (M+1, ESI+).

### Example 166. Synthesis of (E)-9-((3-methylbenzylidene)amino)-2-morpholino-N-(pyridin-4-yl)-9H-purin-6-amine

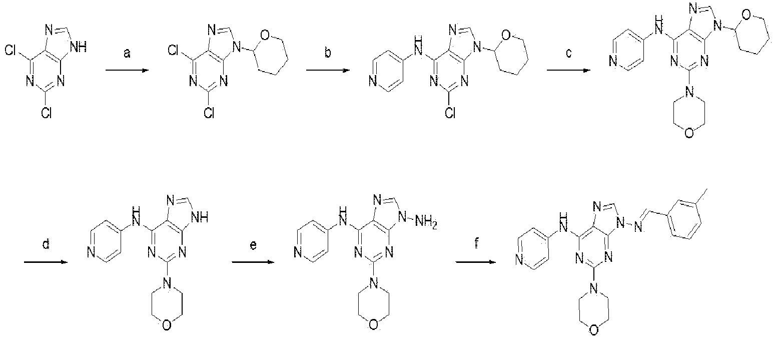

### (Step a) Synthesis of 2,6-dichloro-9-(tetrahydro-2H-pyran-2-yl)-9H-purine

2,6-Dichloro-9H-purine (10 g) was dissolved in ethyl acetate (105.8 mL), then 3,4-dihydro-2H-pyran (7.57 g) and p-toluenesulfonic acid monohydrate (80.52 mg) were added, and the mixture was stirred at 50°C for 2 hours and then stirred at room temperature for 66 hours. The reaction solution was concentrated, followed by silica chromatography under ethyl acetate/hexane conditions, to obtain the target compound 2,6-dichloro-9-(tetrahydro-2H-pyran-2-yl)-9H-purine (13.6 g) as a pale yellow solid in a yield of 94.1%.

¹H NMR (400 MHz, DMSO-*d*₆) *δ* 8.96 (s, 1H), 5.74 (dd, J = 10.8, 2.4 Hz, 1H), 4.05 - 4.00 (m, 1H), 3.78 - 3.70 (m, 1H), 2.31 - 2.20 (m, 1H), 2.05 - 1.95 (m, 2H), 1.80 - 1.73 (m, 1H), 1.63 - 1.57 (m, 2H).

### (Step b) Synthesis of 2-chloro-N-(pyridin-4-yl)-9-(tetrahydro-2H-pyran-2-yl)-9H-purin-6-amine

2,6-Dichloro-9-(tetrahydro-2H-pyran-2-yl)-9H-purine (13.6 g), 4-aminopyridine (4.22 g), BINAP (3.1 g), Pd(OAc)₂ (558.9 mg), and t-BuOK (16.76 g) were dissolved in toluene (276.6 mL), and the mixture was stirred at 80°C for 17 hours. After concentrating the reaction solution, the target compound 2-chloro-N-(pyridin-4-yl)-9-(tetrahydro-2H-pyran-2-yl)-9H-purin-6-amine (2.6 g) was obtained as a yellow solid in a yield of 15.7% through silica chromatography under dichloromethane/methanol/triethylamine conditions.

¹H NMR (400 MHz, DMSO-*d*₆) *δ* 8.66 (s, 1H), 8.47 (dd, *J* = 4.8, 1.6 Hz, 2H), 7.95 (dd, *J* = 4.8, 1.2 Hz, 2H), 4.05 - 4.01 (m, 1H), 3.80 - 3.70 (m, 1H), 2.38 - 2.20 (m, 1H), 2.05 - 1.50 (m, 5 H).

### (Step c) Synthesis of 2-morpholino-N-(pyridin-4-yl)-9-(tetrahydro-2H-pyran-2-yl)-9H-purin-6-amine

2-Chloro-N-(pyridin-4-yl)-9-(tetrahydro-2H-pyran-2-yl)-9H-purin-6-amine (1.66 g) was dissolved in isopropyl alcohol (20.1 mL), then acetic acid (0.290 mL) and morpholine (2.19 mL) were added, and the mixture was stirred at 120°C for 10 hours. After the reaction solution was concentrated under reduced pressure, distilled water was added, and the reaction mixture was extracted with dichloromethane/methanol and concentrated. Then, the target compound 2-morpholino-N-(pyridin-4-yl)-9-(tetrahydro-2H-pyran-2-yl)-9H-purin-6-amine (1.67 g) was obtained as a yellow solid in a yield of 87.2% through silica chromatography under dichloromethane/methanol conditions.

¹H NMR (400 MHz, DMSO-*d*₆) *δ* 10.09 (s, 1H), 8.39 (d, *J* = 6.4 Hz, 2H), 8.22 (s, 1H), 7.90 (dd, *J* = 4.8, 1.2 Hz, 2H), 5.56 (dd, *J* = 4.8, 1.2 Hz, 1H), 4.01 (d, *J* = 11.2 Hz, 1H), 2.35 - 2.20 (m, 1H), 2.02 - 1.89 (m, 2H), 1.70 - 1.51 (m, 4H) .

### (Step d) Synthesis of 2-morpholino-N-(pyridin-4-yl)-9H-purin-6-amine

2-Morpholino-N-(pyridin-4-yl)-9-(tetrahydro-2H-pyran-2-yl)-9H-purin-6-amine (1.05 g) was dissolved in methanol (36.5 mL), then 1.25 M hydrogen chloride (28.9 mL) in methanol as a solvent was added, and the mixture was stirred at 70°C for 30 minutes. The reaction solution was concentrated under reduced pressure, and the concentrate was adjusted to neutral pH using ethyl acetate/methanol and 1 N sodium hydroxide solution, and then extracted four times. Then, the reaction product was dried using anhydrous magnesium sulfate, filtered under reduced pressure, and the filtrate was collected and concentrated under reduced pressure to obtain the target compound 2-morpholino-N-(pyridin-4-yl)-9H-purin-6-amine (1.22 g) as a yellow solid with a yield of 93.7%.

¹H NMR (400 MHz, DMSO-*d*₆) *δ* 12.63 (br s, 1H), 10.01 (s, 1H), 8.39 (t, *J* = 1.2 Hz, 2H), 7.99 (s, 1H), 7.91 (d, *J* = 5.6 Hz, 2H), 3.75 - 3.65 (m, 8H).

### (Step e) Synthesis of 2-morpholino-N⁶-(pyridin-4-yl)-9H-purin-6,9-diamine

2-Morpholino-N-(pyridin-4-yl)-9H-purin-6-amine (1.2 g) was dissolved in tetrahydrofuran (20 mL), then sodium hydride (164 mg) was added, and O-(diphenylphosphinyl)hydroxylamine (1.48 g) dissolved in tetrahydrofuran (10 mL) was added, and the mixture was stirred at room temperature for 3 hours. Distilled water and 1 N HCl were sequentially added to the reaction solution to adjust the pH to neutral, and the resulting product was extracted under ethyl acetate/methanol conditions, dried using anhydrous magnesium sulfate, and filtered under reduced pressure. The filtrate was collected and concentrated under reduced pressure. Then, the target compound 2-morpholino-N⁶-(pyridin-4-yl)-9H-purin-6,9-diamine (233 mg) was obtained as a pale yellow solid in a yield of 18% through silica chromatography under chloroform/methanol/triethylamine conditions.

¹H NMR (400 MHz, DMSO-*d*₆) *δ* 10.03 (s, 1H), 8.41 - 8.37 (m, 2H), 7.94 - 7. 88 (m, 3H), 5.92 (s, 2H), 3.75 - 3.70 (m, 8H) .

### (Step f) Synthesis of (E)-9-((3-methylbenzylidene)amino)-2-morpholino-N-(pyridin-4-yl)-9H-purin-6-amine

3-Methylbenzaldehyde (23.1 mg) was dissolved in ethanol (0.6 mL), then acetic acid (0.006 mL) was added, and the mixture was stirred for 10 minutes. 2-morpholino-N⁶-(pyridin-4-yl)-9H-purin-6,9-diamine (30 mg) was added, and the mixture was stirred at 100°C for 88 hours. After concentrating the reaction solution under reduced pressure, distilled water was added, the resulting mixture was extracted under dichloromethane/methanol conditions, dried using anhydrous magnesium sulfate, and filtered under reduced pressure, and the filtrate was collected and concentrated under reduced pressure. Then, the concentrated product was subjected to silica chromatography under dichloromethane/methanol conditions to obtain the target compound (E)-9-((3-methylbenzylidene)amino)-2-morpholino-N-(pyridin-4-yl)-9H-purin-6-amine (41 mg) as a white solid in a yield of 47.5%.

¹H NMR (400 MHz, DMSO-*d*₆) *δ* 10.30 (s, 1H), 9.82 (s, 1H), 8.45 - 8.40 (m, 3H), 7.92 (dd, *J* = 4.8, 1.2 Hz, 2H), 7.74 (s, 1H), 7.70 (d, *J* = 7.6 Hz, 1H), 7.45 (t, *J* = 7.6 Hz, 1H), 7.39 (d, *J* = 7.6 Hz, 1H), 3.75 (s, 8H), 2.41 (s, 3H); MS: m/z = 415 (M+1, ESI+).

### Example 167. Synthesis of (E)-9-((2-methylbenzylidene)amino)-2-morpholino-N-(pyridin-4-yl)-9H-purin-6-amine

The target compound (E)-9-((2-methylbenzylidene)amino)-2-morpholino-N-(pyridin-4-yl)-9H-purin-6-amine (41 mg) was obtained as a white solid in a yield of 47.5% in the same manner as in Example 166 above, except using 2-methylbenzaldehyde (50 mg) in (Step f).

¹H NMR (400 MHz, DMSO-*d*₆) *δ* 10.25 - 10.20 (m, 2H), 8.45 - 8.40 (m, 3H), 8.01 - 8.00 (m, 1H), 7.93 (dd, *J* = 4.8, 1.2 Hz, 2H), 7.48 - 7.43 (m, 1H), 7.40 - 7.35 (m, 2H), 3.75 (s, 8H), 3.56 (s, 3H); MS: m/z = 415 (M+1, ESI+).

### Example 168. Synthesis of (E)-9-((4-methylbenzylidene)amino)-2-morpholino-N-(pyridin-4-yl)-9H-purin-6-amine

The target compound (E)-9-((4-methylbenzylidene)amino)-2-morpholino-N-(pyridin-4-yl)-9H-purin-6-amine (65 mg) was obtained as a white solid in a yield of 75.4% in the same manner as in Example 166 above, except using 4-methylbenzaldehyde (50 mg) in (Step f).

¹H NMR (400 MHz, DMSO-*d*₆) *δ* 10.20 (s, 1H), 9.82 (s, 1H), 8.45 - 8.40 (m, 3H), 7.93 (dd, *J* = 4.8, 1.2 Hz, 2H), 7.82 (d, *J* = 8.4 Hz, 2H), 7.38 (d, *J* = 8.4 Hz, 2H), 3.75 (s, 8H), 2.41 (s, 3H); MS: m/z = 415 (M+1, ESI+).

### Example 169. Synthesis of (E)-9-(benzylideneamino)-2-morpholino-N-(pyridin-4-yl)-9H-purin-6-amine

The target compound (E)-9-(benzylideneamino)-2-morpholino-N-(pyridin-4-yl)-9H-purin-6-amine (42 mg) was obtained as a white solid in a yield of 50.4% in the same manner as in Example 166 above, except using benzaldehyde (44.2 mg) in (Step f).

¹H NMR (400 MHz, DMSO-*d*₆) *δ* 10.22 (s, 1H), 9.88 (s, 1H), 8.45 (s, 1H), 8.45 - 8.40 (m, 2H), 7.95 - 7.90 (m, 4H), 7.60 - 7.52 (m, 3H), 3.76 (s, 8H); MS: m/z = 401 (M+1, ESI+).

### Example 170. Synthesis of (E)-2-morpholino-N-(pyridin-4-yl)-9-((3-(trifluoromethyl)benzylidene)amino)-9H-purin-6-amine

The target compound (E)-2-morpholino-N-(pyridin-4-yl)-9-((3-(trifluoromethyl)benzylidene)amino)-9H-purin-6-amine (52 mg) was obtained as a pale yellow solid in a yield of 29% in the same manner as in Example 166 above, except using 3-(trifluoromethyl)benzaldehyde (201 mg) in (Step f).

¹H NMR (400 MHz, DMSO-*d*₆) *δ* 10.23 (s, 1H), 9.95 (s, 1H), 8.48 (s, 1H), 8.45 - 8.40 (m, 2H), 8.25 (s, 1H), 8.22 (d, *J* = 8 Hz, 1H), 7.96 - 7.90 (m, 3H), 7.81 (t, *J* = 8 Hz, 1H), 3.80 - 3.70 (m, 8H); MS: m/z = 469 (M+1, ESI+).

### Example 171. Synthesis of (E)-9-((3-chlorobenzylidene)amino)-2-morpholino-N-(pyridin-4-yl)-9H-purin-6-amine

The target compound (E)-9-((3-chlorobenzylidene)amino)-2-morpholino-N-(pyridin-4-yl)-9H-purin-6-amine (30 mg) was obtained as a pale yellow solid in a yield of 17.96% in the same manner as in Example 166 above, except using 3-chlorobenzaldehyde (162 mg) in (Step f).

¹H NMR (400 MHz, DMSO-*d*₆) *δ* 10.22 (s, 1H), 9.87 (s, 1H), 8.44 - 8.40 (m, 3H), 7.98 (t, *J* = 1.6 Hz, 1H), 7.93 - 7.87 (m, 3H), 7.70 - 7.50 (m, 2H), 3.75 (s, 8H); MS: m/z = 435 (M+1, ESI+).

### Example 172. Synthesis of (E)-9-((3-methoxybenzylidene)amino)-2-morpholino-N-(pyridin-4-yl)-9H-purin-6-amine

The target compound (E)-9-((3-methoxybenzylidene)amino)-2-morpholino-N-(pyridin-4-yl)-9H-purin-6-amine (30 mg) was obtained as a pale yellow solid in a yield of 17.96% in the same manner as in Example 166 above, except using 3-methoxybenzaldehyde (157 mg) in (Step f).

¹H NMR (400 MHz, DMSO-*d*₆) *δ* 10.21 (s, 1H), 9.83 (s, 1H), 8.44 - 8.40 (m, 3H), 7.93 - 7.90 (m, 2H), 7.50 - 7.45 (m, 3H), 7.20 - 7.14 (m, 1H), 3.85 (s, 3H), 3.75 (s, 8H); MS: m/z = 431 (M+1, ESI+).

### Example 173. Synthesis of (E)-9-((4-methoxybenzylidene)amino)-2-morpholino-N-(pyridin-4-yl)-9H-purin-6-amine

The target compound (E)-9-((4-methoxybenzylidene)amino)-2-morpholino-N-(pyridin-4-yl)-9H-purin-6-amine (30 mg) was obtained as a pale yellow solid in a yield of 17.96% in the same manner as in Example 166 above, except using 4-methoxybenzaldehyde (123 mg) in (Step f).

¹H NMR (400 MHz, DMSO-*d*₆) *δ* 10.19 (s, 1H), 9.75 (s, 1H), 8.43 - 8.40 (m, 3H), 7.95 - 7.91 (m, 2H), 7.90 - 7.85 (m, 2H), 7.15 - 7.10 (m, 2H), 3.86 (s, 3H), 3.74 (s, 8H); MS: m/z = 431 (M+1, ESI+).

### Example 174. Synthesis of (E)-2-morpholino-N-(pyridin-4-yl)-9-((pyridin-4-ylmethylene)amino)-9H-purin-6-amine

The target compound (E)-2-morpholino-N-(pyridin-4-yl)-9-((pyridin-4-ylmethylene)amino)-9H-purin-6-amine (30 mg) was obtained as an off-white solid in a yield of 17.96% in the same manner as in Example 166 above, except using isonicotinaldehyde (123 mg) in (Step f).

¹H NMR (400 MHz, DMSO-*d*₆) *δ* 10.24 (s, 1H), 9.91 (s, 1H), 8.79 - 8.77 (m, 2H), 8.48 (s, 1H), 8.43 - 8.41 (m, 2H), 7.92 - 7.91 (m, 2H), 7.86 - 7.84 (m, 2H), 3.76 (s, 8H); MS: m/z = 402 (M+1, ESI+).

### Example 175. Synthesis of (E)-9-((4-chlorobenzylidene)amino)-2-morpholino-N-(pyridin-4-yl)-9H-purin-6-amine

The target compound (E)-9-((4-chlorobenzylidene)amino)-2-morpholino-N-(pyridin-4-yl)-9H-purin-6-amine (27 mg) was obtained as an off-white solid in a yield of 16.15% in the same manner as in Example 166 above, except using 4-chlorobenzaldehyde (162 mg) in (Step f).

¹H NMR (400 MHz, DMSO-*d*₆) *δ* 10.21 (s, 1H), 9.87 (s, 1H), 8.44 - 8.40 (m, 3H), 7.96 - 7.91 (m, 4H), 7.64 (d, *J* = 8.4 Hz, 2H), 3.75 (s, 8H); MS: m/z = 435 (M+1, ESI+).

### Example 176. Synthesis of (E) -N- (6-(1H-imidazol-1-yl)-2-morpholino-9H-purin-9-yl)-1-(m-tolyl)methanimine

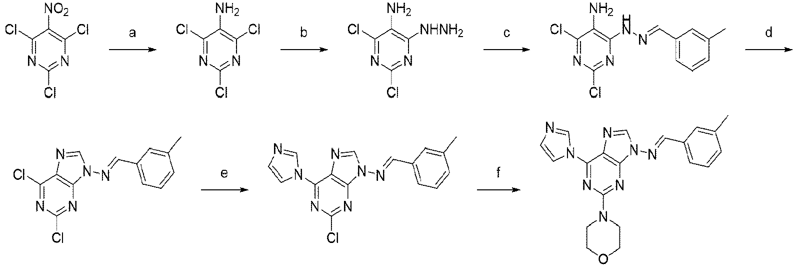

### (Step a) Synthesis of 2,4,6-trichloropyrimidine-5-amine

2,4,6-Trichloro-5-nitro-pyrimidine (8.65 g) was dissolved in acetic acid (35 mL), then iron (8.45 g) was added, and the mixture was stirred at room temperature for 1 hour and 30 minutes. The reaction solution was filtered with methanol, and the filtrate was concentrated under reduced pressure, and extracted with ethyl acetate/water. The organic layer was dried using anhydrous sodium sulfate, filtered under reduced pressure, and the filtrate was concentrated under reduced pressure. Then, the concentrated product was subjected to silica chromatography under ethyl acetate/hexane conditions to obtain the target compound 2,4,6-trichloropyrimidine-5-amine (5.33 g) as a white solid in a yield of 70.9%. MS: m/z = 198 (M+1, ESI+).

### (Step b) Synthesis of 2,4-dichloro-6-hydrazinylpyrimidine-5-amine

2,4,6-Trichloropyrimidine-5-amine (2.38 g) was dissolved in tetrahydrofuran (46 mL), then hydrazine hydrate (2.29 mL) was added, and the mixture was stirred at 30°C for 1 hour. Water was added to the reaction solution, the resulting solid was filtered and dried to obtain the target compound 2,4-dichloro-6-hydrazinylpyrimidine-5-amine (1.7 g) as a yellow solid in a yield of 73%.

¹H NMR (400 MHz, DMSO-*d*₆) *δ* 8.47 (br s, 1H), 5.16 (s, 2H), 4.52 (br s, 2 H).

### (Step c) Synthesis of (E)-2,4-dichloro-6-(2-(3-methylbenzylidene)hydrazinyl)pyrimidine-5-amine

2,4-Dichloro-6-hydrazinylpyrimidine-5-amine (500 mg) was dissolved in isopropyl alcohol (25.8 mL), then 3-methylbenzaldehyde (340.6 mg) was added, and the mixture was stirred at 60°C for 30 minutes. The reaction solution was concentrated under reduced pressure, and the concentrate was subjected to silica chromatography under ethyl acetate/hexane conditions to obtain the target compound (E)-2,4-dichloro-6-(2-(3-methylbenzylidene)hydrazinyl)pyrimidine-5-amine (775 mg) as a yellow solid in a yield of 99%.

¹H NMR (400 MHz, DMSO-*d*₆) *δ* 11.47 (br s, 1H), 8.11 (s, 1H), 7.50 - 7.45 (m, 2H), 7.38 - 7.32 (m, 1H), 7.25 (d, *J* = 7.6 Hz, 1H), 5.77 (br s, 2H), 2.36 (s, 3H).

### (Step d) Synthesis of (E)-N-(2,6-dichloro-9H-purin-9-yl)-1-(m-tolyl)methanimine

(E)-2,4-dichloro-6-(2-(3-methylbenzylidene)hydrazinyl)pyrimidine-5-amine (776 mg) was dissolved in triethyl orthoformate (7.63 mL), then formic acid (0.15 mL) was added, and the mixture was stirred at 90°C for 30 minutes. After concentrating the reaction solution, the formed solid was filtered under hexane/dichloromethane conditions and dried to obtain the target compound (E)-N-(2,6-dichloro-9H-purin-9-yl)-1-(m-tolyl)methanimine (700 mg) as a white solid with a yield of 87.3%.

¹H NMR (400 MHz, DMSO-*d*₆) *δ* 9.44 (s, 1H), 9.26 (s, 1H), 7.79 (s, 1H), 7.78 - 7.72 (m, 1H), 7.52 - 7.46 (m, 2H), 2.42 (s, 3H).

### (Step e) Synthesis of (E)-N-(2-chloro-6-(1H-imidazol-1-yl)-9H-purin-9-yl)-1-(m-tolyl)methanimine

(E)-N-(2,6-Dichloro-9H-purin-9-yl)-1-(m-tolyl)methanimine (150 mg) was dissolved in tetrahydrofuran (2.45 mL), then triethylamine (0.09 mL) and imidazole (73.4 mg) were added, and the mixture was stirred at 60°C for 17.5 hours. The reaction solution was extracted with ethyl acetate, methanol/water, then the organic layer was dried using anhydrous sodium sulfate, filtered under reduced pressure, and the filtrate was collected and concentrated under reduced pressure. The concentrate was subjected to silica chromatography under dichloromethane/methanol conditions to obtain the target compound (E)-N-(2-chloro-6-(1H-imidazol-1-yl)-9H-purin-9-yl)-1-(m-tolyl)methanimine (140 mg) as a white solid in a yield of 84.6%.

¹H NMR (400 MHz, DMSO-*d*₆) *δ* 9.49 (s, 1H), 9.27 (s, 1H), 9.04 (s, 1H), 8.37 (t, *J* = 1.2 Hz, 1H), 7.81 (s, 1H), 7.77 (d, *J* = 6.8 Hz, 1H), 7.55 - 7.45 (m, 2H), 7.30 (s, 1H), 2.43 (s, 3H).

### (Step f) Synthesis of (E)-N-(6-(1H-imidazol-1-yl)-2-morpholino-9H-purin-9-yl)-1-(m-tolyl)methanimine

(E)-N-(2-Chloro-6-(1H-imidazol-1-yl)-9H-purin-9-yl)-1-(m-tolyl)methanimine (132 mg) was dissolved in isopropyl alcohol (1.96 mL), then morpholine (0.17 mL) and acetic acid (22.6 µL) were added, and the mixture was stirred at 90°C for 1 hour and 30 minutes. The reaction solution was extracted with dichloromethane, methanol/water, and the organic layer was dried using anhydrous sodium sulfate, filtered under reduced pressure, and the filtrate was collected and concentrated under reduced pressure. The concentrate was subjected to silica chromatography under dichloromethane/methanol conditions to obtain the target compound (E)-N-(6-(1H-imidazol-1-yl)-2-morpholino-9H-purin-9-yl)-1-(m-tolyl)methanimine (107 mg) as a pale yellow solid in a yield of 70%.

¹H NMR (400 MHz, DMSO-*d*₆) *δ* 9.76 (s, 1H), 9.06 (t, *J* = 0.8 Hz, 1H), 8.69 (s, 1H), 8.40 (t, *J* = 1.6 Hz, 1H), 7.76 (s, 1H), 7.73 (d, *J* = 7.6 Hz, 1H), 7.50 - 7.40 (m, 2H), 7.73 - 7.72 (m, 1H), 3.85 - 3.83 (m, 4H), 3.76 - 3.72 (m, 4H); MS: m/z = 389 (M+1, ESI+).

### Example 177. Synthesis of (E)-N-(2-morpholino-6-(1H-pyrazol-1-yl)-9H-purin-9-yl)-1-(m-tolyl)methanimine

The target compound (E)-N-(2-morpholino-6-(1H-pyrazol-1-yl)-9H-purin-9-yl)-1-(m-tolyl)methanimine was obtained as a white solid in the same manner as in Example 176 above, except using pyrazole in (Step e).

¹H NMR (400 MHz, DMSO-*d*₆) 9.77 (s, 1H), 9.12 - 9.11 (m, 1H), 8.65 (s, 1H), 7.96 (t, *J* = 0.8 Hz, 1H), 7.77 (s, 1H), 7.73 (d, *J* = 8.0 Hz, 1H), 7.49 - 7.40 (m, 2H), 6.71 - 6.69 (m, 1H), 3.85 - 3.83 (m, 4H), 3.76 - 3.72 (m, 4H), 2.42 (s, 3H); MS: m/z = 389 (M+1, ESI+).

### Example 178. Synthesis of (E)-N-(4-methoxyphenyl)-9-((3-methylbenzylidene)amino)-2-morpholino-9H-purin-6-amine

The target compound (E)-N-(4-methoxyphenyl)-9-((3-methylbenzylidene)amino)-2-morpholino-9H-purin-6-amine was obtained as a white solid in the same manner as in Example 176 above, except using 4-methoxyaniline in (Step e).

¹H NMR (400 MHz, DMSO-*d*₆) *δ* 9.86 (s, 1H), 9.57 (s, 1H), 8.28 (s, 1H), 7.77 - 7.71 (m, 3H), 7.68 (d, *J =* 7.6 Hz, 1H), 7.44 (t, *J* = 7.6 Hz, 1H), 7.38 (d, *J* = 7.6 Hz, 1H), 6.96 - 6.87 (m, 2H), 3.75 (s, 3H), 3.73 - 3.66 (m, 8H), 2.40 (s, 3H); MS: m/z = 444.2 (M+1, ESI+).

### Example 179. Synthesis of (E)-N-methyl-9-((3-methylbenzylidene)amino)-2-morpholino-9H-purin-6-amine

The target compound (E)-N-methyl-9-((3-methylbenzylidene)amino)-2-morpholino-9H-purin-6-amine was obtained as a white solid in the same manner as in Example 176 above, except using methanamine in (Step e).

¹H NMR (400 MHz, DMSO-*d*₆) *δ* 9.88 (s, 1H), 8.13 (s, 1H), 7.71 (s, 1H), 7.66 (d, *J* = 7.6 Hz, 1H), 7.57 (s, 1H), 7.43 (t, *J* = 7.5 Hz, 1H), 7.37 (d, *J* = 7.7 Hz, 1H), 3.70 (s, 8H), 2.93 (s, 3H), 2.40 (s, 3H); MS: m/z = 352.0 (M+1, ESI+).

### Example 180. Synthesis of (E)-9-((3-methylbenzylidene)amino)-2-morpholino-N-phenyl-9H-purin-6-amine

The target compound (E)-9-((3-methylbenzylidene)amino)-2-morpholino-N-phenyl-9H-purin-6-amine was obtained as a white solid in the same manner as in Example 176 above, except using aniline in (Step e).

¹H NMR (400 MHz, DMSO-*d*₆) *δ* 9.86 (s, 1H), 9.72 (s, 1H), 8.33 (s, 1H), 7.87 (d, *J* = 8.0 Hz, 2H), 7.73 (s, 1H), 7.69 (d, *J* = 8.0 Hz, 1H), 7.45 (t, *J* = 7.6 Hz, 1H), 7.39 (d, *J* = 7.6 Hz, 1H), 7.33 (t, *J* = 7.6 Hz, 2H), 7.03 (t, *J* = 7.6 Hz, 1H), 3.76 - 3.67 (m, 8H), 2.41 (s, 3H); MS: m/z = 414.2(M+1, ESI+).

### Example 181. Synthesis of (E)-9-((3-methylbenzylidene)amino)-2-morpholino-N-(p-tolyl)-9H-purin-6-amine

The target compound (E)-9-((3-methylbenzylidene)amino)-2-morpholino-N-(p-tolyl)-9H-purin-6-amine was obtained as a white solid in the same manner as in Example 176 above, except using 4-methylaniline in (Step e).

¹H NMR (400 MHz, DMSO-*d*₆) *δ* 9.86 (s, 1H), 9.61 (s, 1H), 8.30 (s, 1H), 7.77 - 7.71 (m, 3H), 7.68 (d, *J* = 7.2 Hz, 1H), 7.44 (t, *J* = 7.6 Hz, 1H), 7.38 (d, *J* = 7.6 Hz, 1H), 7.13 (d, *J* = 8.4 Hz, 2H), 3.75 - 3.67 (m, 8H), 2.40 (s, 3H), 2.27 (s, 3H); MS: m/z = 428.3 (M+1, ESI+).

### Example 182. Synthesis of (E)-9-((3-methylbenzylidene)amino)-N-(1-methylpiperidin-4-yl)-2-morpholino-9H-purin-6-amine

The target compound (E)-9-((3-methylbenzylidene)amino)-N-(1-methylpiperidin-4-yl)-2-morpholino-9H-purin-6-amine was obtained as a white solid in the same manner as in Example 176 above, except using 1-methylpiperidin-4-amine in (Step e).

¹H NMR (400 MHz, DMSO-*d*₆) *δ* 9.87 (s, 1H), 8.15 (s, 1H), 7.70 (s, 1H), 7.66 (d, *J* = 7.2 Hz, 1H), 7.50 - 7.39 (m, 2H), 7.37 (d, *J* = 7.2 Hz, 1H), 4.08 - 3.83 (m, 1H), 3.72 - 3.64 (m, 8H), 2.78 (d, *J* = 10.8 Hz, 2H), 2.40 (s, 3H), 2.17 (s, 3H), 1.97 (t, *J* = 11.6 Hz, 2H), 1.87 - 1.80 (m, 2H), 1.71 - 1.60 (m, 2H); MS: m/z = 435.0 (M+1, ESI+).

### Example 183. Synthesis of (E)-N-(2-morpholino-6-(2-(pyridin-2-yl)ethoxy)-9H-purin-9-yl)-1-(m-tolyl)methanimine

The target compound (E)-N-(2-morpholino-6-(2-(pyridin-2-yl)ethoxy)-9H-purin-9-yl)-1-(m-tolyl)methanimine was obtained as a white solid in the same manner as in Example 176 above, except using 2-(pyridin-2-yl)ethan-1-ol in (Step e).

¹H NMR (400 MHz, DMSO-*d*₆) *δ* 9.97 (s, 1H), 9.83 (s, 1H), 8.36 (s, 1H), 8.12 (s, 1H), 7.82 (d, *J* = 8.5 Hz, 1H), 7.72 (s, 1H), 7.69 (d, *J =* 7.6 Hz, 1H), 7.44 (t, *J* = 7.5 Hz, 1H), 7.38 (d, *J =* 9.5 Hz, 1H), 7.34 (d, *J =* 8.1 Hz, 1H), 7.06 (d, *J* = 8.0 Hz, 1H), 3.77 - 3.68 (m, 8H), 2.40 (s, 3H); MS: m/z = 444.3(M+1, ESI+).

### Example 184. Synthesis of (E)-N-(3-methoxyphenyl)-9-((3-methylbenzylidene)amino)-2-morpholino-9H-purin-6-amine

The target compound (E)-N-(3-methoxyphenyl)-9-((3-methylbenzylidene)amino)-2-morpholino-9H-purin-6-amine was obtained as a white solid in the same manner as in Example 176 above, except using 3-methoxyaniline in (Step e).

¹H NMR (400 MHz, DMSO-*d*₆) *δ* 9.84 (s, 1H), 9.66 (s, 1H), 8.32 (s, 1H), 7.72 (s, 1H), 7.68 (d, *J* = 7.5 Hz, 1H), 7.63 (s, 1H), 7.44 (t, *J* = 7.9 Hz, 2H), 7.38 (d, *J* = 7.4 Hz, 1H), 7.21 (t, *J* = 8.1 Hz, 1H), 6.60 (dd, *J* = 8.0, 1.4 Hz, 1H), 3.78 - 3.68 (m, 11H), 2.40 (s, 3H); MS: m/z = 444.0(M+1, ESI+).

### Example 185. Synthesis of (E)-N-(3-chlorophenyl)-9-((3-methylbenzylidene)amino)-2-morpholino-9H-purin-6-amine

The target compound (E)-N-(3-chlorophenyl)-9-((3-methylbenzylidene)amino)-2-morpholino-9H-purin-6-amine was obtained as a white solid in the same manner as in Example 176 above, except using 3-chloroaniline in (Step e).

¹H NMR (400 MHz, DMSO-*d*₆) *δ* 9.97 (s, 1H), 9.83 (s, 1H), 8.36 (s, 1H), 8.12 (s, 1H), 7.82 (d, *J* = 8.5 Hz, 1H), 7.72 (s, 1H), 7.69 (d, *J =* 7.6 Hz, 1H), 7.44 (t, *J =* 7.5 Hz, 1H), 7.38 (d, *J =* 9.5 Hz, 1H), 7.34 (d, *J =* 8.1 Hz, 1H), 7.06 (d, *J* = 8.0 Hz, 1H), 3.77 - 3.68 (m, 8H), 2.40 (s, 3H); MS: m/z = 448.2( M+1, ESI+).

### Example 186. Synthesis of (E)-N-(4-chlorophenyl)-9-((3-methylbenzylidene)amino)-2-morpholino-9H-purin-6-amine

The target compound (E)-N-(4-chlorophenyl)-9-((3-methylbenzylidene)amino)-2-morpholino-9H-purin-6-amine was obtained as a white solid in the same manner as in Example 176 above, except using 4-chloroaniline in (Step e).

¹H NMR (400 MHz, DMSO-*d*₆) *δ* 9.87 (s, 1H), 9.83 (s, 1H), 8.33 (s, 1H), 7.90 (d, *J* = 8.8 Hz, 2H), 7.72 (s, 1H), 7.68 (d, *J* = 7.5 Hz, 1H), 7.43 (d, *J* = 7.5 Hz, 1H), 7.37 (d, *J =* 8.7 Hz, 3H), 3.70 (s, 8H), 2.40 (s, 3H); MS: m/z = 448.1 (M+1, ESI+).

### Example 187. Synthesis of (E)-N-(2-methoxyphenyl)-9-((3-methylbenzylidene)amino)-2-morpholino-9H-purin-6-amine

The target compound (E)-N-(2-methoxyphenyl)-9-((3-methylbenzylidene)amino)-2-morpholino-9H-purin-6-amine was obtained as a white solid in the same manner as in Example 176 above, except using 2-methoxyaniline in (Step e).

¹H NMR (400 MHz, DMSO-*d*₆) *δ* 9.84 (s, 1H), 8.34 (d, *J* = 11.7 Hz, 2H), 8.28 (s, 1H), 7.74 (s, 1H), 7.70 (d, *J* = 7.6 Hz, 1H), 7.45 (t, *J* = 7.6 Hz, 1H), 7.39 (d, *J* = 7.9 Hz, 1H), 7.13 - 7.05 (m, 2H), 7.02 (t, *J* = 7.6 Hz, 1H), 3.93 (s, 3H), 3.73 (s, 8H), 2.41 (s, 3H); MS: m/z = 444.3 (M+1, ESI+).

### Example 188. Synthesis of (E)-N-(1,5-dimethyl-1H-pyrazol-3-yl)-9-((3-methylbenzylidene)amino)-2-morpholino-9H-purin-6-amine

The target compound (E)-N-(1,5-dimethyl-1H-pyrazol-3-yl)-9-((3-methylbenzylidene)amino)-2-morpholino-9H-purin-6-amine was obtained as a white solid in the same manner as in Example 176 above, except using 1,5-dimethyl-1H-pyrazol-3-amine in (Step e).

¹H NMR (400 MHz, DMSO-*d*₆) *δ* 9.85 (s, 1H), 9.38 (s, 1H), 8.25 (s, 1H), 7.72 (s, 1H), 7.68 (d, *J* = 7.4 Hz, 1H), 7.44 (t, *J* = 7.6 Hz, 1H), 7.38 (d, *J* = 7.1 Hz, 1H), 6.41 (s, 1H), 3.74 - 3.69 (m, 8H), 3.65 (s, 3H), 2.40 (s, 3H), 2.26 (s, 3H); MS: m/z = 432.3(M+1, ESI+).

### Example 189. Synthesis of (E)-N-(2-morpholino-6-(pyridin-2-yloxy)-9H-purin-9-yl)-1-(m-tolyl)methanimine

The target compound (E)-N-(2-morpholino-6-(pyridin-2-yloxy)-9H-purin-9-yl)-1-(m-tolyl)methanimine was obtained as a white solid in the same manner as in Example 176 above, except using pyridin-2-ol in (Step e).

¹H NMR (400 MHz, DMSO-*d*₆) *δ* 9. 72 (s, 1H), 8.63 (s, 1H), 7.79 - 7.71 (m, 3H), 7.62 - 7.56 (m, 1H), 7.46 (t, *J* = 7.6 Hz, 1H), 7.41 (d, *J* = 7.6 Hz, 1H), 6.53 (d, *J* = 9.2 Hz, 1H), 6.39 (td, *J* = 6.8, 1.2 Hz, 1H), 3.80 - 3.75 (m, 4H), 3.75 - 3.69 (m, 4H), 2.41 (s, 3H); MS: m/z = 416.2 (M+1, ESI+).

### Example 190. Synthesis of (E)-2-morpholino-9-((pyridin-3-ylmethylene)amino)-N-(m-tolyl)-9H-purin-6-amine

The target compound (E)-2-morpholino-9-((pyridin-3-ylmethylene)amino)-N-(m-tolyl)-9H-purin-6-amine was obtained as a white solid in the same manner as in Example 176 above, except using nicotinaldehyde in (Step c) and m-toluidine in (Step e).

¹H NMR (400 MHz, DMSO-*d*₆) *δ* 9.99 (s, 1H), 9.66 (s, 1H), 9.05 (d, *J* = 1.6 Hz, 1H), 8.73 (dd, *J* = 4.8, 1.6 Hz, 1H), 8.34 (s, 1H), 8.30 (dt, *J* = 8.0, 1.6 Hz, 1H), 7.81 - 7.74 (m, 1H), 7.63 (d, *J* = 8.4 Hz, 1H), 7.58 (dd, *J* = 7.6, 4.8 Hz, 1H), 7.21 (t, *J* = 7.6 Hz, 1H), 6.85 (d, *J* = 7.6 Hz, 1H), 3.72 (s, 8H), 2.30 (s, 3H); MS: m/z = 415.2(M+1, ESI+).

### Example 191. Synthesis of (E)-9-((3-methylbenzylidene)amino)-2-morpholino-N-(pyridin-3-yl)-9H-purin-6-amine

The target compound (E)-9-((3-methylbenzylidene)amino)-2-morpholino-N-(pyridin-3-yl)-9H-purin-6-amine (130 mg) was obtained as a yellow solid in a yield of 72.2% in the same manner as in Example 96 above, but with purification and separation in the final stage.

¹H NMR (400 MHz, DMSO-*d*₆) *δ* 9.98 (s, 1H), 9.87 (s, 1H), 9.06 (d, *J* = 2.4 Hz, 1H), 8.37 (s, 1H), 8.26 - 8.22 (m, 2H), 7.73 (s, 1H), 7.69 (d, *J* = 7.6 Hz, 1H), 7.47 - 7.43 (m, 1H), 7.40 - 7.35 (m, 2H), 3.71 (s, 8H), 2.41 (3H); MS: m/z = 415 (M+1, ESI+).

### Example 192. Synthesis of (E)-9-((3-methylbenzylidene)amino)-N-(5-methylpyridin-3-yl)-2-morpholino-9H-purin-6-amine

The target compound (E)-9-((3-methylbenzylidene)amino)-N-(5-methylpyridin-3-yl)-2-morpholino-9H-purin-6-amine was synthesized as a white solid (7 mg) in a yield of 2.22% in the same manner as in Example 145, but with purification and separation in the final stage.

¹H NMR (400 MHz, DMSO-*d*₆) *δ* 9.96 (s, 1H), 9.84 (s, 1H), 8.86 (s, 1H), 8.38 (s, 1H), 8.12 (s, 1H), 8.09 (s, 1H), 7.73 (s, 1H), 7.70 (d, *J* = 7.6 Hz, 1H), 7.45 (t, *J* = 7.6 Hz, 1H), 7.39 (d, *J* = 7.2 Hz, 1H), 3.75 - 3.67 (m, 8H), 2.41 (s, 3H), 2.30 (s, 3H); MS: m/z = 429.3 (M+1, ESI+).

### Example 193. Synthesis of (E)-N-(2-chloropyridin-4-yl)-9-((3-methylbenzylidene)amino)-2-morpholino-9H-purin-6-amine

The target compound (E)-N-(2-chloropyridin-4-yl)-9-((3-methylbenzylidene)amino)-2-morpholino-9H-purin-6-amine (25 mg) was synthesized as a white solid in a yield of 11.1% in the same manner as in Example 115, but with purification and separation in the final stage.

¹H NMR (400 MHz, DMSO-*d*₆) *δ* 10.43 (s, 1H), 9.76 (s, 1H), 8.41 (s, 1H), 8.22 (d, *J* = 5.6 Hz, 1H), 8.09 (d, *J =* 1.2 Hz, 1H), 7.88 (dd, *J* = 5.6, 1.2 Hz, 1H), 7.71 - 7.63 (m, 2H), 7.43 (t, *J* = 7.6 Hz, 1H), 7.37 (d, *J* = 7.6 Hz, 1H), 3.80 - 3.68 (m, 8H), 2.39 (s, 3H); MS: m/z = 449.1 (M+1, ESI+) .

### Example 194. Synthesis of (E)-9-((3-methylbenzylidene)amino)-N-(2-methylpyridin-3-yl)-2-morpholino-9H-purin-6-amine

The target compound (E)-9-((3-methylbenzylidene)amino)-N-(2-methylpyridin-3-yl)-2-morpholino-9H-purin-6-amine (25 mg) was synthesized as a white solid in a yield of 11.1% in the same manner as in Example 109, but with purification and separation in the final stage.

¹H NMR (400 MHz, DMSO-*d*₆) *δ* 9.86 (s, 1H), 9.32 (s, 1H), 8.29 (s, 1H), 7.85 (d, *J* = 7.2 Hz, 1H), 7.73 (s, 1H), 7.68 (d, *J* = 7.6 Hz, 1H), 7.44 (t, *J* = 7.2 Hz, 1H), 7.38 (d, *J* = 7.2 Hz, 1H), 7.25 (dd, *J* = 8.0, 4.8 Hz, 1H), 3.63 - 3.56 (m, 8H), 2.45 (s, 3H), 2.40 (s, 3H); MS: m/z = 429.3 (M+1, ESI+).

### Example 195. Synthesis of (E)-9-((3-methylbenzylidene)amino)-2-morpholino-N-(pyridin-4-ylmethyl)-9H-purin-6-amine

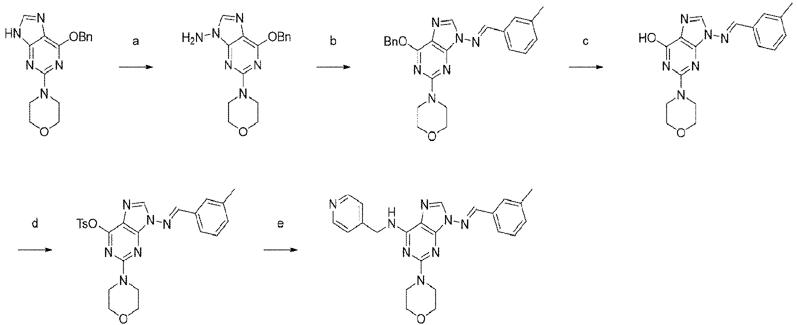

### (Step a) Synthesis of 6-(benzyloxy)-2-morpholino-9H-purin-9amine

4-(6-(Benzyloxy)-9H-purin-2-yl)morpholine (5.0 g) was added to tetrahydrofuran (100 mL), followed by addition of sodium hydride (10.93 g) at 0°C, and the mixture was stirred for 30 minutes. After adding (aminooxy)diphenylphosphine oxide (5.62 g), the mixture was stirred at 25°C for 3 hours. Distilled water was added to the reaction solution and extracted with ethyl acetate. After washing the organic solvent with a saturated NaCl solution, sodium sulfate was added, and the reaction mixture was filtered and concentrated. The concentrate was subjected to silica chromatography to synthesize the target compound 6-(benzyloxy)-2-morpholino-9H-purin-9amine (3.0 g) as a yellow solid in a yield of 57.24%. MS: m/z = 327.0 (M+1, ESI+).

### (Step b) Synthesis of (E)-N-(6-(benzyloxy) -2-morpholino-9H-purin-9-yl)-1-(m-tolyl)methanimine

To 6-(benzyloxy)-2-morpholino-9H-purin-9amine (3.0 g), 3-methylbenzaldehyde (1.33 g), acetic acid (55 mg), and methanol (50 mL) were added, and the mixture was stirred at 70°C for 3 hours. Distilled water was added to the reaction solution and extracted with ethyl acetate. After washing the organic solvent with a saturated NaCl solution, sodium sulfate was added, and the reaction mixture was filtered and concentrated. The concentrate was subjected to silica chromatography to synthesize the target compound (E)-N-(6-(benzyloxy)-2-morpholino-9H-purin-9-yl)-1-(m-tolyl)methanimine (2 g) as a yellow solid in a yield of 50.78%. MS: m/z = 429.10 (M+1, ESI+).

### (Step c) Synthesis of (E)-9-((3-methylbenzylidene)amino)-2-morpholino-9H-purin-6-ol

To (E)-N-(6-(benzyloxy)-2-morpholino-9H-purin-9-yl)-1-(m-tolyl)methanimine (2.2 g), dichloromethane (10 mL) and trifluoroacetic acid (10 mL) were added, and the mixture was stirred at 25°C for 18 hours. Distilled water was added to the reaction solution and extracted with ethyl acetate. After washing the organic solvent with a saturated NaCl solution, sodium sulfate was added, and the reaction mixture was filtered and concentrated. The concentrate was subjected to silica chromatography to synthesize the target compound (E)-9-((3-methylbenzylidene)amino)-2-morpholino-9H-purin-6-ol (1.5 g) as a yellow solid in a yield of 86.34%. MS: m/z = 339.1(M+1, ESI+).

### (Step d) Synthesis of (E)-9-((3-methylbenzylidene)amino)-2-morpholino-9H-purin-6-yl 4-methylbenzenesulfonate

To (E)-9-((3-methylbenzylidene)amino)-2-morpholino-9H-purin-6-ol (1.5 g), 4-toluenesulfonyl chloride (1.69 g), triethylamine (0.45 g), and dichloromethane (50 mL) were added, and the mixture was stirred at 25°C for 3 hours. Distilled water was added to the reaction solution and extracted with ethyl acetate. After washing the organic solvent with a saturated NaCl solution, sodium sulfate was added, and the reaction mixture was filtered and concentrated. The concentrate was subjected to silica chromatography to synthesize the target compound (E)-9-((3-methylbenzylidene)amino)-2-morpholino-9H-purin-6-yl 4-methylbenzenesulfonate (1.2 g) as a yellow solid in a yield of 54.96%. MS: m/z = 493.1 (M+1, ESI+).

### (Step e) Synthesis of (E)-9-((3-methylbenzylidene)amino)-2-morpholino-N-(pyridin-4-ylmethyl)-9H-purin-6-amine

To (E)-9-((3-methylbenzylidene)amino)-2-morpholino-9H-purin-6-yl 4-methylbenzenesulfonate (150 mg), tetrahydrofuran (10 mL) and pyridin-4-ylmethanamine (0.05 g) were added, and the mixture was stirred at 25°C for 3 hours. The resulting product was subjected to silica chromatography under dichloromethane/methanol conditions to obtain the target compound (E)-9-((3-methylbenzylidene)amino)-2-morpholino-N-(pyridin-4-ylmethyl)-9H-purin-6-amine (40 mg) as a white solid in a yield of 30.65%.

¹H NMR (400 MHz, DMSO-*d*₆) *δ* 9.85 (s, 1H), 8.48 (dd, *J* = 4.5, 1.5 Hz, 2H), 8.36 (s, 1H), 8.19 (s, 1H), 7.70 (s, 1H), 7.66 (d, *J* = 7.6 Hz, 1H), 7.43 (t, *J* = 7.5 Hz, 1H), 7.35 (dd, *J* = 13.9, 6.7 Hz, 3H), 4.63 (s, 2H), 3.60 (s, 8H), 2.40 (s, 3H); MS: m/z = 429.2 (M+1, ESI+).

### Example 196. Synthesis of (E)-N-isopropyl-9-((3-methylbenzylidene)amino)-2-morpholino-9H-purin-6-amine

The target compound (E)-N-isopropyl-9-((3-methylbenzylidene)amino)-2-morpholino-9H-purin-6-amine (33 mg) was synthesized as a yellow solid in a yield of 28.56% in the same manner as in Example 195 above, except using propane-2-amine in (Step e).

¹H NMR (400 MHz, DMSO-*d*₆) *δ* 9.87 (s, 1H), 8.13 (s, 1H), 7.70 (s, 1H), 7.65 (d, *J* = 7.7 Hz, 1H), 7.42 (t, *J* = 7.6 Hz, 1H), 7.36 (d, *J* = 7.5 Hz, 2H), 4.37 (s, 1H), 3.68 (s, 8H), 2.39 (s, 3H), 1.23 (s, 3H), 1.21 (s, 3H); MS: m/z = 380.2 (M+1, ESI+).

### Example 197. Synthesis of (E)-N-(2-morpholino-6-(pyridin-4-yloxy)-9H-purin-9-yl)-1-(m-tolyl)methanimine

Synthesis was performed in the same manner as in Example 195 above, but (Step e) was performed as follows.

### (Step e) Synthesis of (E)-N-(2-morpholino-6-(pyridin-4-yloxy)-9H-purin-9-yl)-1-(m-tolyl)methanimine

Sodium hydride (20 mg) and tetrahydrofuran (5 mL) were added to pyridin-4-ol (46 mg), and the mixture was stirred at 25°C for 1 hour. (E)-9-((3-Methylbenzylidene)amino) -2-morpholino-9H-purin-6-yl 4-methylbenzenesulfonate (200 mg) was added, and the mixture was stirred at 25°C for 12 hours. The target compound (E)-N-(2-morpholino-6-(pyridin-4-yloxy)-9H-purin-9-yl)-1-(m-tolyl)methanimine (12 mg) was synthesized as a white solid in a yield of 7.1% through Prep-HPLC.

¹H NMR (400 MHz, DMSO-*d*₆) *δ* 9.77 (s, 1H), 9.29 (d, *J* = 8.1 Hz, 2H), 8.77 (s, 1H), 7.84 - 7.73 (m, 2H), 7.57 - 7.43 (m, 2H), 6.44 (d, *J* = 8.1 Hz, 2H), 3.84 (dd, *J* = 26.9, 4.1 Hz, 8H), 2.47 (s, 3H); MS: m/z = 416.3(M+1, ESI+).

### Example 198. Synthesis of (E)-3-methyl-N-(9-((3-methylbenzylidene)amino)-2-morpholino-9H-purin-6-yl)isoxazol-5-amine

The target compound (E)-3-methyl-N-(9-((3-methylbenzylidene)amino)-2-morpholino-9H-purin-6-yl)isoxazol-5-amine as a white solid was synthesized in the same manner as in Example 195 above, except that 3-methylisoxazol-5-amine was used in (Step e), and the same method as in (Step e) of Example 197 was employed.

¹H NMR (400 MHz, DMSO-*d*₆) *δ* 11.42 (s, 1H), 9.80 (s, 1H), 8.42 (s, 1H), 7.74 (s, 1H), 7.70 (d, *J* = 7.6 Hz, 1H), 7.45 (t, *J* = 7.6 Hz, 1H), 7.39 (d, *J* = 7.8 Hz, 1H), 6.25 (s, 1H), 3.74 (s, 8H), 2.41 (s, 3H), 2.23 (s, 3H). MS: m/z = 419.2(M+1, ESI+).

### Example 199. Synthesis of (E)-9-((3-methylbenzylidene)amino)-2-morpholino-N-(pyridin-2-yl)-9H-purin-amine

The target compound (E)-9-((3-methylbenzylidene)amino)-2-morpholino-N-(pyridin-2-yl)-9H-purin-amine was synthesized as a white solid in the same manner as in (Step a) to (Step d) of Example 195, and then in the same manner as in (Step e) of Example 197, except that pyridin-2-amine was used instead of pyridin-4-ol.

¹H NMR (400 MHz, DMSO-*d*₆) *δ* 9.82 (s, 1H), 9.01 (s, 1H), 8.38 (s, 1H), 8.34 (d, *J =* 4.5 Hz, 1H), 8.26 (d, *J* = 8.4 Hz, 1H), 7.85 (t, *J* = 8.0 Hz, 1H), 7.74 (s, 1H), 7.70 (d, *J =* 7.7 Hz, 1H), 7.45 (t, *J* = 7.6 Hz, 1H), 7.39 (d, *J* = 7.0 Hz, 1H), 7.12 - 7.07 (m, 1H), 3.74 (s, 8H), 2.40 (s, 3H); MS: m/z = 415.2(M+1, ESI+).

### Example 200. Synthesis of (E)-2-morpholino-9-((pyridin-3-ylmethylene)amino)-N-(pyridin-4-yl)-9H-purin-amine

The target compound (E)-2-morpholino-9-((pyridin-3-ylmethylene)amino)-N-(pyridin-4-yl)-9H-purin-amine was synthesized in the same manner as in Example 195, except that nicotinaldehyde was used instead of 3-methylbenzaldehyde in (Step b), and then in the same manner as in (Step e) of Example 197, except that pyridin-4-amine was used instead of pyridin-4-ol.

¹H NMR (400 MHz, DMSO-*d*₆) *δ* 9.14 (s, 1H), 8.73 (s, 1H), 8.68 (s, 1H), 8.46 (d, *J =* 7.2 Hz, 2H), 8.38 (d, *J* = 7.2 Hz, 1H), 7.95 (s, 1H), 7.58 (t, *J* = 7.1 Hz, 1H), 7.57 (s, 1H), 6.99 (d, *J* = 7.2 Hz, 2H), 3.73 - 3.64 (m, 8H); MS: m/z = 402 (M+1, ESI+).

### Example 201. Synthesis of (E)-N-(9-((3-methylbenzylidene)amino)-2-morpholino-9H-purin-6-yl)oxazol-5-amine

The target compound (E)-N-(9-((3-methylbenzylidene)amino)-2-morpholino-9H-purin-6-yl)oxazol-5-amine was synthesized in the same manner as in (Step a) to (Step d) of Example 195, and then in the same manner as in (Step e) of Example 197, except that oxazol-5-amine was used instead of pyridin-4-ol.

¹H NMR (400 MHz, DMSO-*d*₆) δ 9.97 (s, 1H), 8.68 (s, 1H), 8.47 (s, 1H), 7.90 (s, 1H), 7.74 (s, 1H), 7.45 (t, *J* = 7.1 Hz, 1H), 7.66 (d, *J* = 7.2 Hz, 1H), 7.45 (t, *J* = 7.2 Hz, 1H), 7.27 (d, *J* = 7.2 Hz, 1H), 7.09 (s, 1H), 3.73 - 3.64 (m, 8H), 2.42 (s, 3H); MS: m/z = 405 (M+1, ESI+).

### Example 202. Synthesis of (E)-N,N-dimethyl-3-(6-(2-(3-methylbenzylidene)hydrazinyl)-2-morpholino-9H-purin-9-yl)aniline

The target compound (E)-N,N-dimethyl-3-(6-(2-(3-methylbenzylidene)hydrazinyl)-2-morpholino-9H-purin-9-yl)aniline was obtained as a white solid in the same manner as in Example 119, except that (3-(dimethylamino)phenyl)boronic acid was used instead of phenylboronic acid in (Step a).

¹H NMR (400 MHz, DMSO-*d*₆) δ 11.41 (s, 1H), 8.40 (s, 1H), 8.30 (s, 1H), 7.57 (d, *J* = 7.1 Hz, 1H), 7.51 (s, 1H), 7.35 - 7.30 (m, 3H), 7.20 (d, *J* = 7.4 Hz, 1H), 7.12 (d, *J* = 7.6 Hz, 1H), 6.74 (d, *J* = 8.3 Hz, 1H), 3.71 (d, *J* = 14.4 Hz, 8H), 2.98 (s, 6H), 2.36 (s, 3H); MS: m/z = 457.2 (M+1, ESI+).

### Example 203. Synthesis of N⁹-(3-methylbenzyl)-2-morpholino-N⁶-(pyridin-4-yl)-9H-purin-6,9-diamine

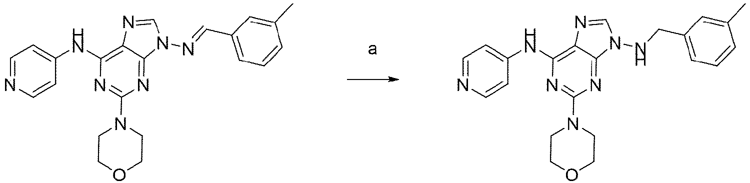

### (Step a) Synthesis of N⁹-(3-methylbenzyl)-2-morpholino-N⁶-(pyridin-4-yl)-9H-purin-6,9-diamine

To the compound (E)-9-((3-methylbenzylidene)amino)-2-morpholino-N-(pyridin-4-yl)-9H-purin-6-amine (200 mg) prepared by the method of Example 166 above, methanol (5 mL) and sodium borohydride (146 mg) were added, and the mixture was stirred at room temperature for 3 hours. Distilled water was added to the reaction solution and extracted with ethyl acetate. After washing the organic solvent with a saturated NaCl solution, sodium sulfate was added, and the reaction mixture was filtered and concentrated. The resulting product was subjected to silica chromatography under dichloromethane/methanol conditions to synthesize the target compound N⁹-(3-methylbenzyl)-2-morpholino-N⁶-(pyridin-4-yl)-9H-purin-6,9-diamine (104 mg) as a white solid in a yield of 51.75%.

¹H NMR (400 MHz, DMSO-*d*₆) *δ* 10.01 (s, 1H), 8.39 (d, J = 5.5 Hz, 2H), 7.90 (d, *J* = 6.3 Hz, 2H), 7.71 (s, 1H), 7.20 - 7.14 (m, 2H), 7.11 - 7.02 (m, 3H), 4.33 (d, *J* = 4.1 Hz, 2H), 3.78 - 3.71 (m, 8H), 2.26 (s, 3H); MS: m/z = 417.0(M+1, ESI+) .

### Example 204. Synthesis of 4-(9-phenyl-6-(pyridin-4-yloxy)-9H-purin-2-yl)morpholine

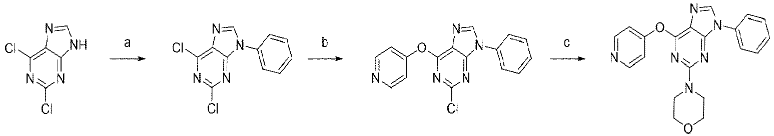

### (Step a) Synthesis of 2,6-dichloro-9-phenyl-9H-purine

Phenylboronic acid (12.06 g), dichloromethane (50 mL), copper(II) acetate (2.88 g), and o-phenanthroline (5.72 g) were added to 2,6-dichloro-9H-purine (3.0 g), and then the mixture was stirred at 40°C overnight. The reaction solution was filtered and concentrated under reduced pressure. The target compound 2,6-dichloro-9-phenyl-9H-purine (900 mg) was synthesized as a white solid in a yield of 21.4% through silica chromatography. MS: m/z = 265.1(M+1, ESI+).

### (Step b) Synthesis of 2-chloro-9-phenyl-6-(pyridin-4-yloxy)-9H-purine

Sodium hydride (117 mg) and tetrahydrofuran (10 mL) were added to pyridin-4-ol (277 mg), and the mixture was stirred at room temperature for 1 hour. 2,6-Dichloro-9-phenyl-9H-purine (700 mg) was added, and the mixture was stirred at room temperature for 2 hours. The reaction solution was concentrated under reduced pressure, followed by silica chromatography, to obtain the target compound 2-chloro-9-phenyl-6-(pyridin-4-yloxy)-9H-purine (264 mg) as a white solid. MS: m/z = 324.0(M+1, ESI+).

### (Step c) Synthesis of 4-(9-phenyl-6-(pyridin-4-yloxy)-9H-purin-2-yl)morpholine

To 2-chloro-9-phenyl-6-(pyridin-4-yloxy)-9H-purine (264 mg), isopropyl alcohol (10 mL), morpholine (357 mg), and acetic acid (0.1 mL) were added, and the mixture was stirred at 120°C overnight. Distilled water was added to the reaction solution and extracted with ethyl acetate. After washing the organic solvent with a saturated NaCl solution, sodium sulfate was added, and the reaction mixture was filtered and concentrated. The target compound 4-(9-phenyl-6-(pyridin-4-yloxy)-9H-purin-2-yl)morpholine (88 mg) was synthesized as a white solid in a yield of 28.79% through Prep-HPLC.

¹H NMR (400 MHz, CDCl₃) *δ* 9.28 (d, *J* = 8.4 Hz, 2H), 8.07 (s, 1H), 7.71 (d, *J* = 8.0 Hz, 2H), 7.59 (t, *J* = 7.6 Hz, 2H), 7.48 (t, *J* = 7.6 Hz, 1H), 6.55 (d, *J* = 8.0 Hz, 2H), 3.88 - 3.83 (m, 4H), 3.83 - 3.78 (m, 4H); MS: m/z = 375.2 (M+1, ESI+) .

### Example 205. Synthesis of (E)-N-(4-methoxyphenyl)-8-methyl-9-((3-methylbenzylidene)amino)-2-morpholino-9H-purin-6-amine

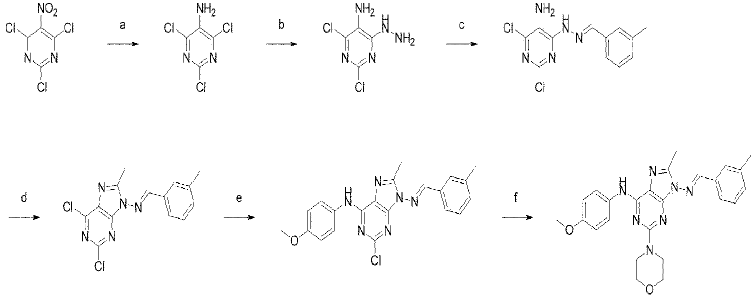

### (Step a) Synthesis of 2,4,6-trichloropyrimidine-5-amine

To 2,4, 6-trichloro-5-nitropyrimidine (8.65 g), isopropyl alcohol (86.5 mL), acetic acid (35 mL), and iron (8.45 g) were added, and the mixture was stirred at 25°C for 1.5 hours. The resulting product was filtered to remove byproducts, and the organic solvent was concentrated under reduced pressure. After adding ethyl acetate and distilled water, the organic solvent was extracted. After adding magnesium sulfate to the organic solvent, the resulting product was filtered and concentrated under reduced pressure. The target compound 2,4,6-trichloropyrimidine-5-amine (5.33 g) was synthesized in a yield of 70.9% through silica chromatography under ethyl acetate/hexane conditions.

### (Step b) Synthesis of 2,4-dichloro-6-hydrazinylpyrimidine-5-amine

Tetrahydrofuran (17.8 mL) and hydrazine monohydrate (928.4 mg) were added to 2,4,6-trichloropyrimidin-5-amine (920 mg), and the mixture was stirred at 30°C for 1 hour. The target compound 2,4-dichloro-6-hydrazinylpyrimidine-5-amine (680 mg) was synthesized in a yield of 75.6% through silica chromatography under dichloromethane/methanol conditions.

### (Step c) Synthesis of (E)-2,4-dichloro-6-(2-(3-methylbenzylidene)hydrazinyl)pyrimidine-5-amine

Ethanol (35 mL), acetic acid (10.5 mg), and 3-methylbenzaldehyde (547.4 mg) were added to 2,4-dichloro-6-hydrazinylpyrimidine-5-amine (680 mg), and the mixture was stirred at 60°C for 1 hour. The target compound (E)-2,4-dichloro-6-(2-(3-methylbenzylidene)hydrazinyl)pyrimidine-5-amine (790 mg) was synthesized in a yield of 76.1% through silica chromatography under ethyl acetate/hexane conditions.

### (Step d) Synthesis of (E)-N-(2,6-dichloro-8-methyl-9H-purin-9yl)-1-(m-tolyl)methanimine

Triethyl orthoacetate (10.2 mL) was added to (E)-2,4-dichloro-6-(2-(3-methylbenzylidene)hydrazinyl)pyrimidin-5-amine (790 mg), and the mixture was stirred at 100°C for 1 hour. The resulting solid was filtered, and ether and distilled water were added. The solid was softened with ether and concentrated under reduced pressure to obtain the target compound (E)-N-(2,6-dichloro-8-methyl-9H-purin-9-yl)-1-(m-tolyl)methanimine (700 mg) in a yield of 82%.

### (Step e) Synthesis of (E)-2-chloro-N-(4-methoxyphenyl)-8-methyl-9-((3-methylbenzylidene)amino)-9H-purin-6-amine

Isopropyl alcohol (4.7 mL), 4-methoxyaniline (57.7 mg), and triethylamine (71.1 mg) were added to (E)-N-(2,6-dichloro-8-methyl-9H-purin-9yl)-1-(m-tolyl)methanimine (150 mg), and the mixture was stirred at 80°C for 5 hours. The reaction mixture was concentrated under reduced pressure, then softened with methanol and concentrated again under reduced pressure, thereby synthesizing the target compound (E)-2-chloro-N-(4-methoxyphenyl)-8-methyl-9-((3-methylbenzylidene)amino)-9H-purin-6-amine (146 mg) in a yield of 76.6%.

### (Step f) Synthesis of (E)-N-(4-methoxyphenyl)-8-methyl-9-((3-methylbenzylidene)amino)-2-morpholino-9H-purin-6-amine

Isopropyl alcohol (2.15 mL), acetic acid (20.6 mg), and morpholine (149.9 mg) were added to (E)-2-chloro-N-(4-methoxyphenyl)-8-methyl-9-((3-methylbenzylidene)amino)-9H-purin-6-amine (140 mg), and the mixture was stirred at 120°C for 115 hours. The resulting product was subjected to silica chromatography under dichloromethane/methanol conditions to obtain the target compound (E)-N-(4-methoxyphenyl)-8-methyl-9-((3-methylbenzylidene)amino)-2-morpholino-9H-purin-6-amine (50 mg) in a yield of 31.8%.

¹H NMR (400 MHz, DMSO-*d*₆) *δ* 10.04 (s, 1H), 9.49 (s, 1H), 7.77 - 7.69 (m, 4H), 7.43 (t, *J =* 7.6 Hz, 1H), 7.37 (d, *J* = 7.6 Hz, 1H), 6.89 (d, *J* = 8.0 Hz, 2H), 3.74 (s, 3H), 3.71 - 3.66 (m, 8H), 2.66 (s, 3H), 2.41 (s, 3H); MS: m/z = 458 (M+1, ESI+).

### Example 206. Synthesis of (E)-8-methyl-9-((3-methylbenzylidene)amino)-2-morpholino-N-phenyl-9H-purin-6-amine

The target compound (E)-8-methyl-9-((3-methylbenzylidene)amino)-2-morpholino-N-phenyl-9H-purin-6-amine was synthesized as a pink solid in the same manner as in Example 205 above, except using aniline in (Step e).

¹H NMR (400 MHz, DMSO-*d*₆) *δ* 10.03 (s, 1H), 9.63 (s, 1H), 7.89 (d, *J* = 1.2 Hz, 2H), 7.74 (s, 1H), 7.70 (d, *J =* 7.6 Hz, 1H), 7.43 (t, *J* = 7.4 Hz, 1H), 7.37 (d, *J* = 7.6 Hz, 1H), 7.32 (t, *J* = 3.2 Hz, 1H), 7.00 (t, *J* = 3.2 Hz, 1H), 3.69 - 3.68 (m, 8H), 2.59 (s, 3H), 2.33 (s, 3H); MS: m/z = 428 (M+1, ESI+).

### Example 207. Synthesis of (E)-8-methyl-9-((3-methylbenzylidene)amino)-2-morpholino-N-(pyridin-4-yl)-9H-purin-6-amine

The target compound (E)-8-methyl-9-((3-methylbenzylidene)amino)-2-morpholino-N-(pyridin-4-yl)-9H-purin-6-amine was synthesized as a yellow solid in the same manner as in Example 166 above, except that 2,6-dichloro-8-methyl-9H-purine was used in (Step a).

¹H NMR (400 MHz, DMSO-*d*₆) *δ* 10.12 (s, 1H), 10.01 (s, 1H), 8.39 (d, *J* = 3.6 Hz, 2H), 7.91 (d, *J* = 3.2 Hz, 2H), 7.75 (s, 1H), 7.72 (d, *J* = 7.6 Hz, 1H), 7.44 (t, *J* = 7.6 Hz, 1H), 7.39 (d, *J* = 7.6 Hz, 1H), 3.74 - 3.72 (m, 8H), 2.62 (s, 3H), 2.41 (s, 3H); MS: m/z = 429 (M+1, ESI+).

### Example 208. Synthesis of (E)-N-8-methyl-2-morpholino-(6-(2-pyridin-2-yl)ethoxy)-9H-purin-9-yl-1-(m-tolyl)methanimine

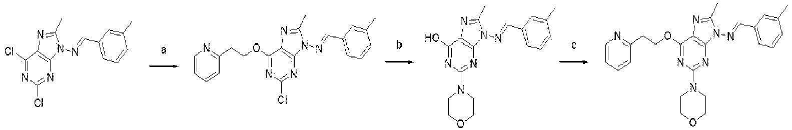

### (Step a) Synthesis of (E)-N-(6-(2-(pyridin-2-yl) ethoxy) -2-chloro-8-methyl-9H-purin-9-yl) (m-tolyl)methanimine

Tetrahydrofuran (1.5 mL) was added to 2-(2-pyridyl)ethanol (30.8 mg), and NaHMDS (137.5 mg) was added, and the mixture was stirred at room temperature for 10 minutes. (E)-N-(2,6-dichloro-8-methyl-9H-purin-9-yl)-1-(m-tolyl)methanimine (80 mg) was dissolved in tetrahydrofuran (1.0 mL), then added thereto, and the mixture was stirred at 30°C for 1 hour. The target compound (E)-N-(6-(2-(pyridin-2-yl)ethoxy)-2-chloro-8-methyl-9H-purin-9-yl)(m-tolyl)methanimine (53 mg) was synthesized in a yield of 52.1% through silica chromatography under ethyl acetate/hexane conditions.

### (Step b) Synthesis of (E)-N-(8-methyl-2-morpholino-9H-purin-9-yl-6-ol) (m-tolyl)methanimine

To (E)-N-(6-(2-(pyridin-2-yl)ethoxy)-2-chloro-8-methyl-9H-purin-9-yl) (m-tolyl)methanimine (53 mg), isopropyl alcohol (0.81 mL), morpholine (56.7 mg), and acetic acid (7.8 mg) were added, and the mixture was stirred at 120°C for 16 hours. The reaction solution was concentrated under reduced pressure and softened with methanol to synthesize the target compound (E)-N-(8-methyl-2-morpholino-9H-purin-9-yl-6-ol) (m-tolyl)methanimine (25 mg) in a yield of 54.6%.

### (Step c) Synthesis of (E)-N-8-methyl-2-morpholino-(6-(2-pyridin-2-yl)ethoxy)-9H-purin-9-yl-1-(m-tolyl)methanimine

To (E)-N-(8-methyl-2-morpholino-9H-purin-9-yl-6-ol) (m-tolyl)methanimine (25 mg), 1,4-dioxane (0.71 mL), 2-(2-pyridyl)ethanol (13.1 mg), PPh₃ (27.9 mg), and DEAD (27.1 mg) were added, and the mixture was stirred at 30°C for 1 hour. The target compound (E)-N-8-methyl-2-morpholino-(6-(2-pyridin-2-yl)ethoxy)-9H-purin-9-yl-1-(m-tolyl)methanimine (15 mg) was synthesized as a gray solid in a yield of 46.2% through silica chromatography under dichloromethane/methanol conditions.

¹H NMR (400 MHz, DMSO-*d*₆) *δ* 9.96 (s, 1H), 8.53 - 8.51 (m, 1H), 7.76 - 7.69 (m, 3H), 7.37 (t, *J* = 7.6 Hz, 1H), 7.39 (d, *J* = 8.0 Hz, 2H), 7.28 - 7.26 (m, 1H), 4.84 (t, *J* = 6.6 Hz, 2H), 3.71 (s, 8H), 3.26 (t, *J* = 6.6 Hz, 2H), 2.54 (s, 3H), 2.40 (s, 3H); MS: m/z = 458 (M+1, ESI+).

### Example 209. Synthesis of (E)-4-(8-(1-methyl-1H-pyrazol-4-yl)-4-(2-(3-methylbenzylidene)hydrazinyl)quinazolin-2-yl)morpholine

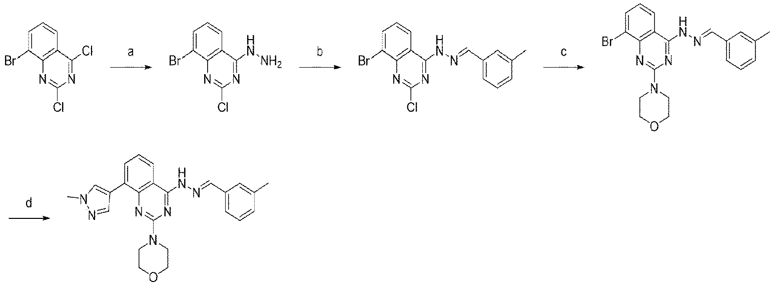

### (Step a) Synthesis of 8-bromo-2-chloro-4-hydrazinylquinazoline

8-Bromo-2,4-dichloroquinazoline (950.0 mg) was dissolved in tetrahydrofuran (8.3 mL), then hydrazine monohydrate (406.5 µL) was added, and the mixture was stirred at 40°C for 0.5 hours. The reaction solution was filtered with distilled water to obtain the target compound 8-bromo-2-chloro-4-hydrazinylquinazoline (702 mg) as a yellow solid in a yield of 75%. MS: m/z = 275 (M+1, ESI+)

### (Step b) Synthesis of (E)-8-bromo-2-chloro-4-(2-(3-methylbenzylidene)hydrazinyl)quinazoline

8-Bromo-2-chloro-4-hydrazinylquinazoline (0.82 g) and 3-methylbenzaldehyde (615 µL) were dissolved in absolute ethanol (26 mL), and a catalytic equivalent of acetic acid was added, followed by stirring at 60°C for 1 hour. The reaction solution was filtered with ethanol to obtain the target compound (E)-8-bromo-2-chloro-4-(2-(3-methylbenzylidene)hydrazinyl)quinazoline (816 mg) as a yellow solid in a yield of 85%. MS: m/z = 377 (M+1, ESI+)

### (Step c) Synthesis of (E)-4-(8-bromo-4-(2- (3-methylbenzylidene)hydrazinyl)quinazolin-2-yl)morpholine

(E)-8-Bromo-2-chloro-4-(2-(3-methylbenzylidene)hydrazinyl)quinazoline (816 mg) was dissolved in morpholine (10.8 mL), and the mixture was stirred at 90°C for 16 hours. The reaction solution was distilled under reduced pressure, followed by silica chromatography under hexane/ethyl acetate conditions, to obtain the target compound (E)-4-(8-bromo-4-(2-(3-methylbenzylidene)hydrazinyl)quinazolin-2-yl)morpholine (530 mg) as a yellow solid in a yield of 57%. MS: m/z = 427 (M+1, ESI+)

### (Step d) Synthesis of (E)-4-(8-(1-methyl-1H-pyrazol-4-yl)-4-(2-(3-methylbenzylidene)hydrazinyl)quinazolin-2-yl)morpholine

(E)-4-(8-Bromo-4-(2-(3-methylbenzylidene)hydrazinyl)quinazolin-2-yl)morpholine (150 mg) and (1-methyl-1H-pyrazol-4-yl)boronic acid (88.6 mg) were dissolved in 1,4-dioxane (1.8 mL) and distilled water (0.4 mL), then Pd(dppf)Cl₂ (25.7 mg) and cesium carbonate (344 mg) were added, and the mixture was stirred at 95°C for 16 hours. After the reaction solution was concentrated under reduced pressure, distilled water was added, and the reaction mixture was extracted with ethyl acetate, and concentrated. Then, the target compound (E)-4-(8-(1-methyl-1H-pyrazol-4-yl)-4-(2-(3-methylbenzylidene)hydrazinyl)quinazolin-2-yl)morpholine (62 mg) was obtained as a white solid in a yield of 41% through silica chromatography under hexane/ethyl acetate conditions.

¹H NMR (400 MHz, DMSO-*d*₆) *δ* 11.41 (s, 1H), 8.44 (s, 1H), 8.42 (s, 1H), 8.23 - 8.21 (m, 1H), 8.14 (s, 1H), 7.90 (dd, *J* = 7.6, 1.2 Hz, 1H), 7.60 (d, *J* = 8.0 Hz, 1H), 7.55 (s, 1H), 7.38 (t, *J* = 7.6 Hz, 1H), 7.26 - 7.19 (m, 2H), 3.92 (s, 3H), 3.86 - 3.84 (m, 4H), 3.77 - 3.74 (m, 4H), 2.39 (s, 3H); MS: m/z = 428 (M+1, ESI+).

### Example 210. Synthesis of (E)-4-(8-(3,5-dimethylisoxazol-4-yl)-4-(2-(3-methylbenzylidene)hydrazinyl)quinazolin-2-yl)morpholine

The target compound (E)-4-(8-(3,5-dimethylisoxazol-4-yl)-4-(2-(3-methylbenzylidene)hydrazinyl)quinazolin-2-yl)morpholine (31 mg) was obtained as a yellow solid in a yield of 30% in the same manner as in Example 209 above, except that 3,5-dimethyl-4-(4,4,5,5-tetramethyl-1,3,2-dioxaborolan-2-yl)isoxazole (104.7 mg) was used in (Step d).

¹H NMR (400 MHz, DMSO-*d*₆) *δ* 11.50 (s, 1H), 8.44 (s, 2H), 7.61 - 7.55 (m, 3H), 7.38 (t, *J* = 7.6 Hz, 1H), 7.28 - 7.24 (m, 2H), 3.78 -3.70 (m, 4H), 3.68 - 3.64 (m, 4H), 2.39 (s, 3H), 2.30 (s, 3H), 2.13 (s, 3H); MS: m/z = 443 (M+1, ESI+) .

### Example 211. Synthesis of (E)-4-(8-(3-methoxyphenyl)-4-(2-(3-methylbenzylidene)hydrazinyl)quinazolin-2-yl)morpholine

The target compound (E)-4-(8-(3-methoxyphenyl)-4-(2-(3-methylbenzylidene)hydrazinyl)quinazolin-2-yl)morpholine (69 mg) was obtained as a yellow solid in a yield of 66% in the same manner as in Example 209 above, except that (3-methoxyphenyl)boronic acid (53 mg) and tetrakis (5.4 mg) were used in (Step d).

¹H NMR (400 MHz, DMSO-*d*₆) *δ* 11.47 (s, 1H), 8.45 (s, 1H), 8.38(br s,1H), 7.68 (d, *J* = 7.2 Hz, 1H), 7.61 (d, *J* = 7.6 Hz, 1H), 7.56 (s, 1H), 7.40 - 7.25 (m, 6H), 6.91 (dd, *J* = 8.0, 2.4 Hz, 1H), 3.80 - 3.75 (m, 7H), 3.68 -3.66 (m, 4H), 2.39 (s, 3H); MS: m/z = 454 (M+1, ESI+).

### Example 212. Synthesis of (E)-4-(4-(2-(3-methylbenzylidene)hydrazinyl)-8-(pyridin-4-yl)quinazolin-2-yl)morpholine

The target compound (E)-4-(4-(2-(3-methylbenzylidene)hydrazinyl)-8-(pyridin-4-yl)quinazolin-2-yl)morpholine (56 mg) was obtained as a yellow solid in a yield of 64% in the same manner as in Example 209 above, except that pyridin-4-ylboronic acid (50.7 mg) was used in (Step d).

¹H NMR (400 MHz, DMSO-*d*₆) *δ* 11.52 (S, 1H), 8.63 - 8.61 (m, 2H), 8.45 (br s, 2H), 7.77 - 7.75 (m, 3H), 7.61 (d, J = 7.6 Hz, 1H), 7.56 (s, 1H), 7.38 (t, J = 7.6 Hz, 1H), 7.33 - 7.29 (m, 2H), 3.80 - 3.65 (m, 8H), 2.39 (s, 3H); MS: m/z = 425 (M+1, ESI+).

### Example 213. Synthesis of (E)-4-(8-(4-methoxyphenyl)-4-(2-(3-methylbenzylidene)hydrazinyl)quinazolin-2-yl)morpholine

The target compound (E)-4-(8-(4-methoxyphenyl)-4-(2-(3-methylbenzylidene)hydrazinyl)quinazolin-2-yl)morpholine (67.6 mg) was obtained as a yellow solid in a yield of 42.4% in the same manner as in Example 209 above, except that (4-methoxyphenyl)boronic acid (53 mg) and tetrakis (8.13 mg) were used in (Step d).

¹H NMR (400 MHz, DMSO-*d*₆) *δ* 11.43 (S, 1H), 8.44 (s, 1H), 8.35 - 8. 25 (br s, 1H), 7.69 (d, *J* = 8.8 Hz, 2H), 7.65 - 7.55 (m, 2H), 7.55 (s, 1H), 7.38 (t, *J* = 7.6 Hz, 1H), 7.27 - 7. 20 (m, 2H), 7.00 (d, *J* = 8.8 Hz, 2H), 3.81 (s, 3H), 3.77 - 3.70 (m, 4H), 3.70 - 3.60 (m, 3H), 2.39 (s, 3H); MS: m/z = 454 (M+1, ESI+).

### Example 214. Synthesis of (E)-4-(4-(2-(3-methylbenzylidene)hydrazinyl)-8-phenylquinazolin-2-yl)morpholine

The target compound (E)-4-(4-(2-(3-methylbenzylidene)hydrazinyl)-8-phenylquinazolin-2-yl)morpholine (67.6 mg) was obtained as a yellow solid in a yield of 42.4% in the same manner as in Example 209 above, except that phenylboronic acid (64.4 mg) and tetrakis (8.13 mg) were used in (Step d).

¹H NMR (400 MHz, DMSO-*d*₆) *δ* 11.47 (s, 1H), 8.44 (s, 1H), 8.41 - 8.25 (m, 1H), 7.74 - 7.51 (m, 2H), 7.68 - 7.63 (m, 1H), 7.61 (d, *J* = 8.0 Hz, 1H), 7.56 (s, 1H), 7.44 (t, *J* = 7.2 Hz, 2H), 7.40 - 7.30 (m, 2H), 7.29 - 7.22 (m, 2H), 3.80 - 3.70 (m, 4H), 3.66 (t, *J* = 4.8 Hz, 4H), 2.39 (s, 3H); MS: m/z = 424 (M+1, ESI+).

### Example 215. Synthesis of (E)-4-(4-(2-(3-methylbenzylidene)hydrazinyl)-8-(pyridin-3-yl)quinazolin-2-yl)morpholine

The target compound (E)-4-(4-(2-(3-methylbenzylidene)hydrazinyl)-8-(pyridin-3-yl)quinazolin-2-yl)morpholine (145 mg) was obtained as a yellow solid in a yield of 58.2% in the same manner as in Example 209 above, except that 3-pyridylboronic acid (144.2 mg) was used in (Step d).

¹H NMR (400 MHz, DMSO-*d*₆) *δ* 11.52 (s, 1H), 8.90 (d, *J* = 1.6 Hz, 1H), 8.53 (dd, *J* = 4.8, 1.6 Hz, 1H), 8.48 - 8.38 (m, 2H), 8.16 - 8.11 (m, 1H), 7.75 - 7.72 (m, 1H), 7.61 (d, *J* = 8.0 Hz, 1H), 7.56 (s, 1H), 7.50 - 7.45 (m, 1H), 7.38 (t, *J* = 7.6 Hz, 1H), 7.35 - 7.24 (m, 2H), 3.79 - 3.70 (m, 4H), 3.66 (t, *J* = 4.8 Hz, 4H), 2.39 (s, 3H); MS: m/z = 425 (M+1, ESI+).

### Example 216. Synthesis of (E)-4-(4-(2-(3-methylbenzylidene)hydrazinyl)-7-(pyridin-3-yl)pyrido[2,3-d]pyrimidin-2-yl)morpholine

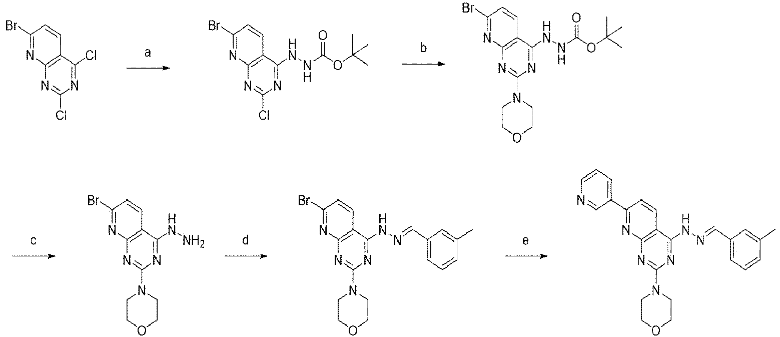

### (Step a) Synthesis of tert-butyl 2-(7-bromo-2-chloropyrido[2,3-d]pyrimidin-4-yl)hydrazine-1-carboxylate

7-Bromo-2,4-dichloropyrido[2,3-d]pyrimidine (790.0 mg) was dissolved in dimethylformamide (10.0 mL), then tert-butyl carbazate (374.3 mg) and diisopropylethylamine (592.1 µL) were added, and the mixture was stirred at 25°C for 20.5 hours. Then, the reaction solution was concentrated under reduced pressure, and the target compound tert-butyl 2-(7-bromo-2-chloropyrido[2,3-d]pyrimidin-4-yl)hydrazine-1-carboxylate (1.0 g) was synthesized as a white solid in a yield of 94.0% through silica chromatography under ethyl acetate/hexane conditions. MS: m/z = 374 (M+1, ESI+)

### (Step b) Synthesis of tert-butyl 2-(7-bromo-2-morpholinopyrido[2,3-d]pyrimidin-4-yl)hydrazine-1-carboxylate

Tert-Butyl 2-(7-bromo-2-chloropyrido[2,3-d]pyrimidin-4-yl)hydrazine-1-carboxylate (1.0 g) was dissolved in dimethylformamide, then morpholine (232.6 µL) and diisopropylethylamine (557.9 µL) were added, and the mixture was stirred at 70°C for 2 hours. Then, the reaction solution was concentrated under reduced pressure, and the target compound tert-butyl 2-(7-bromo-2-morpholinopyrido[2,3-d]pyrimidin-4-yl)hydrazine-1-carboxylate (1.0 g) was synthesized as a white solid in a yield of 90.3% through silica chromatography under ethyl acetate/hexane conditions. MS: m/z = 425 (M+1, ESI+)

### (Step c) Synthesis of 4-(7-bromo-4-hydrazinylpyrido[2,3-d]pyrimidin-2-yl)morpholine

Tert-Butyl 2-(7-bromo-2-morpholinopyrido[2,3-d]pyrimidin-4-yl)hydrazine-1-carboxylate (1.0 g, 2.4 mmol) was dissolved in dichloromethane (20.0 mL), then trifluoroacetic acid (1.0 mL) was added, and the mixture was stirred at room temperature for 95.0 hours. After adjusting the pH to 7-8 with 1N sodium hydroxide aqueous solution, the resulting product was extracted with dichloromethane and concentration to synthesize the target compound 4-(7-bromo-4-hydrazinylpyrido[2,3-d]pyrimidin-2-yl)morpholine (740.0 mg) in a yield of 96.8%. MS: m/z = 325 (M+1, ESI+)

### (Step d) Synthesis of (E)-4-(7-bromo-4-(2-(3-methylbenzylidene)hydrazinyl)pyrido[2,3-d]pyrimidin-2-yl)morpholine

4-(7-Bromo-4-hydrazinylpyrido[2,3-d]pyrimidin-2-yl)morpholine (750.0 mg) was dissolved in ethanol (20.0 mL), then acetic acid (2.5 µL) and 3-methylbenzaldehyde (277.1 mg) were added, and the mixture was stirred at 70°C for 73.5 hours. After stirring, the resulting solid was filtered to synthesize the target compound (E)-4-(7-bromo-4-(2-(3-methylbenzylidene)hydrazinyl)pyrido[2,3-d]pyrimidin-2-yl)morpholine (640.0 mg) in a yield of 64.9%. MS: m/z = 427 (M+1, ESI+)

### (Step e) Synthesis of (E)-4-(4-(2-(3-methylbenzylidene)hydrazinyl)-7-(pyridin-3-yl)pyrido[2,3-d]pyrimidin-2-yl)morpholine

(E)-4-(7-Bromo-4-(2-(3-methylbenzylidene)hydrazinyl)pyrido[2,3-d]pyrimidin-2-yl)morpholine (100.0 mg) was dissolved in 1,4-dioxane (8.0 mL), then Pd(dppf)Cl₂ (17.1 mg), potassium carbonate (97.0 mg), 3-pyridylboronic acid (31.6 mg), and water (2.0 mL) were added, and the mixture was stirred in a microwave at 120°C for 17 hours. The reaction solution was concentrated under reduced pressure, followed by silica chromatography under dichloromethane/methanol conditions, to synthesize the target compound (E)-4-(4-(2-(3-methylbenzylidene)hydrazinyl)-7-(pyridin-3-yl)pyrido[2,3-d]pyrimidin-2-yl)morpholine (10.0 mg) in a yield of 10.0%.

### ¹H NMR (DMSO-d₆) : δ 11.81 (s, 1H), 9.09 (d, J = 2.0 Hz, 1H), 8.81 (d, J = 2.0 Hz, 1H), 8.68 (dd, J = 4.6, 1.4 Hz, 1H), 8.65 (s, 1H), 8.31 (dt, J = 8.0 Hz, 2.0 Hz, 1H), 8.05 (d, J = 2.0 Hz, 1H), 7.59 - 7.56 (m, 2H), 7.51 (s, 1H), 7.37 (t, J = 7.6 Hz, 1H), 7.25 (d, J = 7.6 Hz, 1H), 3.90 - 3.89 (m, 4H), 3.71 (t, J = 4.6 Hz, 4H), 2.37 (s, 3H); MS: m/z = 426 (M+1, ESI+)

### Example 217. Synthesis of (E)-4-(4-(2-(3-methylbenzylidene)hydrazinyl)-7-phenylpyrido[2,3-d]pyrimidin-2-yl)morpholine

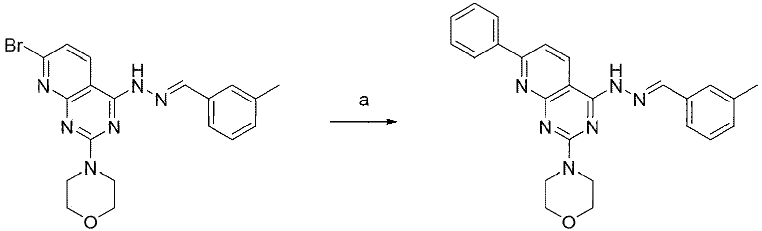

(E)-4-(7-bromo-4-(2-(3-methylbenzylidene)hydrazinyl)pyrido[2,3-d]pyrimidin-2-yl)morpholine (100.0 mg) was dissolved in 1,4-dioxane (8.0 mL), then Pd(dppf)Cl₂ (17.1 mg), potassium carbonate (97.0 mg), phenylboronic acid (31.3 mg), and water (2.0 mL) were added. The mixture was stirred in a microwave at 120°C for 17 hours. The reaction solution was concentrated under reduced pressure, followed by silica chromatography under dichloromethane/methanol conditions, to synthesize the target compound (E)-4-(4-(2-(3-methylbenzylidene)hydrazinyl)-7-phenylpyrido[2,3-d]pyrimidin-2-yl)morpholine (20.0 mg) in a yield of 20.0%.

¹H NMR (CDCl₃): *δ* 10.10 (s, 1H), 8.63 (s, 1H), 8.21 (s, 1H), 7.94 (s, 1H), 7.71-7.70 (m, 2H), 7.69 (d, 1H), 7.56-7.54 (m, 2H), 7.49-7.33 (m, 2H), 4.08 - 4.02 (m, 4H), 3.88 - 3.83 (t, *J* = 4.6 Hz, 4H), 2.49 (s, 3H); MS: m/z = 425 (M+1, ESI+)

### <Experimental Examples>

### Experimental Example 1. Evaluation of enzyme inhibition activity against human PIKFYVE

The enzyme inhibition activity values for the compounds according to Examples against human PIKFYVE were calculated using the ADP-Glo^{™} kinase assay service of Carna biosciences, Inc. The enzyme activity evaluation method provided by Carna biosciences Inc., is shown below.

Enzyme inhibition activity was evaluated by reacting the compounds according to Examples with human GST-PIKFYVE (11-118, Carna biosciences), which was purified using glutathione Sepharose chromatography. The reaction buffer used was composed of 50 mmol/L MOPS, 1 mmol/L DTT, and pH 7.2, and all reactions of the test distilled water were performed in the buffer.

Compounds were prepared as solutions in 100% DMSO as 100X stocks and adjusted to have a final sample concentration of 10 nM.

The substrate-enzyme reaction for PIKFYVE was performed by mixing 5 µL human GST-PIKFYVE, 5 µL compounds according to Examples, 5 µL enzyme substrate, and 5 µL purified ATP and reacting for 1 hour at room temperature. After the substrate-enzyme reaction, 20 µL ADP-GloTM Reagent was added and reacted at room temperature for 40 minutes, then 40 µL Kinase Detection Reagent was added and reacted at room temperature for 40 minutes.

After completion of the reaction, the degree of enzyme activity inhibition was measured by luminescence. The fluorescence of the enzyme activity in the solvent control group without PIKfyve treatment was set to 100%, and the fluorescence of the enzyme activity in the solvent control group without compound treatment was set to 0%. The inhibitory activity of each compound according to Examples was calculated based on these values. Results thereof are shown in Table 2 below.

**[Table 2]**

| **Exam ple No.** | **ADP-Glo kinase assay (%, 10nM)** | **Exam ple No.** | **ADP-Glo kinase assay (%, 10nM)** | **Examp le No.** | **ADP-Glo kinase assay (%, 10nM)** |
|---|---|---|---|---|---|
| 9 | 51.47 | 90 | 66.88 | 145 | 94.29 |
| 13 | 51.68 | 91 | 73.33 | 146 | 79.77 |
| 16 | 63.02 | 92 | 61.82 | 147 | 88.80 |
| 17 | 69.26 | 93 | 66.78 | 148 | 73.88 |
| 18 | 89.87 | 94 | 58.50 | 149 | 83.75 |
| 19 | 58.67 | 96 | 85.95 | 153 | 63.58 |
| 20 | 78.53 | 97 | 78.47 | 156 | 65.90 |
| 21 | 60.95 | 98 | 91.62 | 157 | 71.16 |
| 22 | 66.19 | 99 | 87.24 | 158 | 63.93 |
| 23 | 90.82 | 100 | 83.22 | 159 | 65.33 |
| 24 | 72.15 | 101 | 91.20 | 160 | 77.75 |
| 25 | 72.46 | 102 | 91.19 | 161 | 79.72 |
| 26 | 80.87 | 103 | 91.20 | 162 | 78.79 |
| 28 | 65.48 | 104 | 68.18 | 163 | 54.29 |
| 29 | 55.15 | 107 | 82.62 | 164 | 63.89 |
| 30 | 76.90 | 108 | 80.35 | 165 | 73.72 |
| 32 | 59.60 | 109 | 79.21 | 166 | 90.77 |
| 34 | 74.72 | 110 | 88.60 | 167 | 68.47 |
| 35 | 70.11 | 111 | 89.77 | 168 | 87.26 |
| 36 | 76.04 | 112 | 89.10 | 169 | 89.56 |
| 37 | 89.07 | 113 | 87.10 | 170 | 79.60 |
| 38 | 82.96 | 114 | 80.06 | 171 | 86.73 |
| 39 | 74.99 | 115 | 92.24 | 172 | 90.96 |
| 40 | 86.29 | 116 | 87.75 | 173 | 73.94 |
| 41 | 76.69 | 117 | 67.79 | 176 | 56.91 |
| 42 | 81.60 | 118 | 81.33 | 178 | 55.44 |
| 43 | 80.86 | 119 | 80.61 | 180 | 63.03 |
| 44 | 62.80 | 120 | 88.76 | 181 | 53.92 |
| 45 | 67.70 | 121 | 92.33 | 184 | 55.65 |
| 46 | 73.96 | 122 | 87.32 | 186 | 52.43 |
| 47 | 60.82 | 123 | 84.48 | 187 | 52.07 |
| 48 | 90.25 | 124 | 87.81 | 188 | 65.93 |
| 49 | 78.87 | 125 | 65.27 | 190 | 78.59 |
| 50 | 54.37 | 126 | 87.21 | 191 | 76.59 |
| 51 | 66.19 | 127 | 84.12 | 192 | 61.25 |
| 52 | 67.10 | 128 | 65.10 | 193 | 64.35 |
| 53 | 70.90 | 129 | 89.94 | 194 | 61.38 |
| 54 | 58.10 | 130 | 69.14 | 198 | 75.59 |
| 64 | 82.48 | 131 | 91.40 | 202 | 80.47 |
| 68 | 56.03 | 132 | 86.76 | 203 | 53.14 |
| 73 | 80.98 | 134 | 59.84 | 205 | 53.74 |
| 74 | 69.71 | 135 | 51.18 | 206 | 53.87 |
| 80 | 83.61 | 136 | 78.65 | 207 | 75.57 |
| 81 | 60.34 | 137 | 81.44 | 208 | 71.38 |
| 82 | 60.95 | 138 | 64.23 | 209 | 94.99 |
| 83 | 63.61 | 139 | 84.50 | 210 | 65.10 |
| 85 | 70.06 | 140 | 92.50 | 211 | 64.14 |
| 87 | 63.65 | 141 | 88.29 | 212 | 88.34 |
| 88 | 64.66 | 142 | 68.43 | 213 | 68.30 |
| 89 | 76.44 | 143 | 90.79 | 215 | 81.70 |
| | | | | 216 | 63.00 |

### Experimental Example 2. Evaluation of interactions with PIKFYVE and compounds

To predict the efficacy of the compounds according to Example on PIKFYVE in humans, the interaction between PIKFYVE and the compounds was tested using the HEK293 cell line derived from human embryonic kidney (ATCC, CRL-1573) via NanoBRET^{™} TE Intracellular Kinase Assay Service of Carna biosciences Inc., (Japan). The evaluation method provided by Carna biosciences Inc., was shown below and the NanoBRET^{™} TE Intracellular Kinase Assay, K-8 Kit (Promega, CS1810C88) was used.

The HEK-293 cells cultured in Opti-MEM^{™} I Reduced Serum Medium (Thermo Fisher, 11058-021) were transfected with Transfection Carrier DNA (Promega, E4881), PIKFYVE-NanoLuc Fusion Vector (Promega, CS1810C225), and FuGENE HD Transfection Reagent (Promega, E2311) to produce cells expressing genes combining PIKFYVE and NanoLuc, followed by culturing 100 µl (2×10⁵ cells/ml) each in 96-well plates (Corning, 3917).

10X compounds according to Examples or Comparative Examples prepared by concentration in 1X NanoBRET^{™} tracer reagent and DMSO were reacted in 96-well plates and incubated for 2 hours in a 37°C, 5% CO₂ incubator.

After the reaction was completed, 3X Complete NanoBRET^{™} Nano-Glo Substrate solution was added to each well, and the reaction was allowed to run for 2-3 minutes at room temperature before BRET was measured using a GloMax Discover Multimode Microplate Reader (Promega). Based on the BRET measured in each well, IC₅₀ values were calculated and results thereof are shown in Table 3 below.

**[Table 3]**

| **Example No.** | **NanoBRET assay** | **Example No.** | **NanoBRET assay** | **Example No.** | **NanoBRET assay** |
|---|---|---|---|---|---|
| 17 | ** | 87 | ** | 124 | *** |
| 18 | *** | 88 | * | 125 | *** |
| 20 | ** | 89 | ** | 126 | *** |
| 21 | * | 90 | * | 127 | *** |
| 22 | ** | 91 | ** | 128 | *** |
| 23 | *** | 92 | * | 129 | *** |
| 24 | ** | 93 | * | 131 | *** |
| 25 | ** | 96 | *** | 132 | *** |
| 26 | ** | 97 | ** | 136 | *** |
| 28 | * | 99 | *** | 137 | *** |
| 30 | ** | 100 | *** | 139 | *** |
| 32 | * | 101 | *** | 140 | *** |
| 35 | ** | 102 | *** | 141 | *** |
| 36 | ** | 103 | *** | 143 | *** |
| 37 | *** | 104 | ** | 145 | *** |
| 39 | ** | 107 | *** | 146 | *** |
| 40 | *** | 108 | *** | 147 | *** |
| 41 | *** | 109 | ** | 148 | *** |
| 42 | *** | 110 | *** | 149 | *** |
| 43 | *** | 111 | *** | 157 | ** |
| 44 | * | 112 | *** | 165 | ** |
| 45 | * | 113 | *** | 166 | *** |
| 46 | ** | 114 | * | 191 | ** |
| 48 | *** | 115 | *** | 202 | *** |
| 49 | *** | 116 | *** | 209 | *** |
| 52 | * | 118 | *** | 210 | * |
| 53 | * | 119 | *** | 211 | * |
| 64 | *** | 120 | *** | 212 | *** |
| 73 | ** | 121 | *** | 213 | ** |
| 74 | * | 122 | *** | 215 | *** |
| 80 | *** | 123 | *** | | |
| Activity range | *** ≤ 10nM | | | | |
| | 10nM< ** ≤ 20nM | | | | |
| | 20nM< * ≤ 30nM | | | | |

As shown in Table 3 above, the compounds of the present disclosure were found to have excellent interaction with PIKFYVE.

### Experimental Example 3. Evaluation of compounds of microsomal metabolic stability

The microsomal metabolic stability assay was performed using pooled human or CD1 male mouse interspecies microsomes. Supplemental cofactors (NADPH, UDPGA) and microsomal membrane pore-forming factor (Alamethicin) were added to promote cytochrome P450 (CYP), UGT reactivity to the target compound. Test compounds (1 µM incubation concentration) and microsomes (0.5 mg/ml incubation concentration) were diluted in 0.1 M phosphate buffer, pH 7.4, and incubated (37°C). The reaction was initiated by the addition of NADPH (1 mM), UDPGA (1 mM) and Alamethicin (25 µg/mg). The incubation times were 0, 5, 15, 30, and 45 minutes, and at each time point, the reaction was terminated by adding acetonitrile containing internal standard (IS) (1 : 3 ratio) . The plate was then sealed, centrifuged (3000 rpm, 20 min, 4°C), and the supernatant was analyzed by LC-MS/MS. Hepatic extraction ratio values were calculated from the measured in vitro clearance and Intrinsic clearance (Clᵢₙₜ) values were calculated from the assay results, and the respective Clᵢₙₜ values were categorized into Low, Medium, and High categories based on the reference document (ref. Prostaglandins & other lipid mediators 136 (2018): 90-95) . Results thereof are shown in Table 4 below.

**[Table 4]**

| **Example No.** | **Microsomal metabolic stability** | **Exampl e No.** | **Microsomal metabolic stability** |
|---|---|---|---|
| **23** | Medium | **127** | Low |
| **24** | Medium | **128** | Low |
| **34** | Medium | **129** | Low |
| **39** | Medium | **132** | Medium |
| **42** | Low | **136** | Medium |
| **43** | Medium | **139** | low |
| **48** | Low | **140** | Medium |
| **64** | Medium | **141** | medium |
| **85** | Medium | **147** | Medium |
| **96** | Medium | **149** | Medium |
| **101** | Low | **157** | Medium |
| **109** | Medium | **165** | medium |
| **111** | Medium | **166** | medium |
| **120** | medium | **202** | low |
| **121** | Medium | **209** | medium |
| **122** | Medium | **212** | Low |
| **126** | Low | **215** | Medium |
| Clᵢₙₜ (µL/min/mg protein) | Low < 8.8, | | |
| | Medium 8.8~48, | | |
| | High > 48 | | |

As can be seen in Table 4, the microsomal metabolic stability evaluation showed that the compounds according to the present disclosure had Low or Medium results, indicating significant metabolic stability and suitable bioavailability as drugs.

### Experimental Example 4. Pharmacodynamic evaluation of compounds in tumor tissue

To evaluate the pharmacodynamics of the PIKFYVE inhibition of the compounds according to Examples in vivo, tumor tissues were obtained from xenograft mice at NDIC (Republic of Korea), and protein analysis was performed by Western blot. The xenotransplantation test method provided by NDIC and the Western blot analysis after obtaining tumor tissue samples are described below.

KMS-26 (JCRB, JCRB1187), a multiple myeloma cell line, was transplanted subcutaneously into the flanks of NOD.SCID mice (Coatech) with Matrigel (Corning, 356237) at a density of 6x10⁶ cells. After the tumor size reached approximately 130 mm³, the test compound was prepared in 5% DMSO + 5% Solutol + 90% 1X DPBS pH7.4 excipient and administered orally at 30 mg/kg.

Tumors were removed and homogenized in PBS solvent, and protease & phosphatase inhibitors (Roche, 05892970001, 04906837001) were added, followed by centrifugation (13200 rpm, 15 minutes, 4°C). The supernatant was recovered, sample reducing agent (Invitrogen B0009) and LDS sample buffer (Invitrogen B0007) were added, and the mixture was heated at 70°C for 10 minutes, followed by Western blotting. In order to observe lysosomal immaturation and apoptosis induced by PIKfyve inhibition, proteins were detected using antibodies (Cell Signaling Technology 9542, Abcam 75852, Sigma A5441) that detect the lysosomal enzyme Cathepsin D (CTSD), the apoptosis-activation marker Poly (ADP-ribose) polymerase (PARP), and the control protein Beta-actin (ACTB), respectively. Results thereof are shown in FIG. 1.

As shown in FIG. 1, pharmacodynamic evaluation in tumor tissues showed that compounds according to the present disclosure inhibited PIKfyve in tumor tissues of xenograft mice, thereby interfering with lysosomal maturation and inducing apoptotic cell death.

As described above, the present disclosure has been described in an exemplary manner, as an example, and various modifications may be made by those skilled in the art to which the present disclosure pertains without departing from the essential characteristics of the present disclosure. Therefore, exemplary embodiments disclosed in the present specification are intended to explain the present disclosure rather than limiting it, and the spirit and scope of the present disclosure are not limited by these exemplary embodiments. The protection scope of the present disclosure should be construed by the following claims, and all techniques within the equivalent range should be construed as being included in the scope of the present disclosure.

## Claims

1. A compound represented by the following Chemical Formula 1, an optical isomer thereof, or a pharmaceutically acceptable salt thereof: 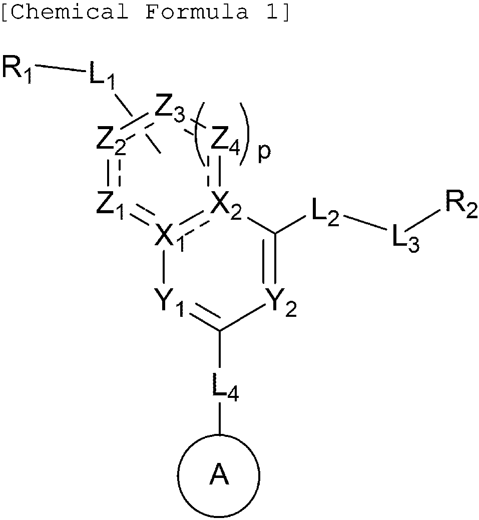 in Chemical Formula 1 above,
each of X₁ and X₂ is independently C or N;
each of Y₁ and Y₂ is independently C or N;
each of Z₁, Z₂, Z₃ and Z₄ is independently C, C-R', CH, CH₂, N, N-R' or S;
p is 0 or 1;
R' is a hydrogen atom or C₁-C₆ alkyl;
each of -̅ -̅ -̅ is optionally a single bond or a double bond, provided that two double bonds are not adjacent to each other;
L₁, L₂ and L₃ are each independently a single bond, C₁-C₆ alkyl, C₁-C₆ haloalkyl, C₁-C₆ alkyl-C=O, -C=O-, -C=O-C₁-C₆ alkyl, -C=O-NH, -SO₂, C₁-C₆ alkyl-Rₐ, C₁-C₆ alkyl-SO₂, -NH, - NH-C₁-C₆ alkyl, C₁-C₆ alkyl-NH-, C₁-C₆ alkyl-O-, -NH-N=CH-, - N=CH-, -NH-NH-C=O-, -C=O-NH-N=CH-, -O-, -O-C₁-C₆ alkyl, substituted or unsubstituted C₂-C₈ heterocycloalkyl containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted heteroaryl is substituted with at least one halogen atom, C₁-C₆ alkyl, C₁-C₆ haloalkyl, -O-C₁-C₆ alkyl, CN or NH₂;
Rₐ is substituted or unsubstituted C₃-C₈ cycloalkyl or substituted or unsubstituted C₂-C₈ heterocycloalkyl containing a heteroatom selected from oxygen, nitrogen and sulfur;
R₁ is a hydrogen atom, substituted or unsubstituted C₁-C₆ alkyl, C₁-C₆ haloalkyl, C₁-C₆ alkyl-OH, -O-C₁-C₆ alkyl, -O-C₁-C₆ haloalkyl, -O-C₃-C₈ cycloalkyl, -SO₂-C₁-C₆ alkyl, -N-(C₁-C₆ alkyl)₂, -C=O-R_{b}, substituted or unsubstituted C₃-C₈ cycloalkyl, substituted or unsubstituted C₂-C₈ heterocycloalkyl containing a heteroatom selected from oxygen, nitrogen and sulfur, substituted or unsubstituted C₂-C₈ heterocycloalkene containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted alkyl, cycloalkyl, heterocycloalkyl, heterocycloalkene, aryl, or heteroaryl is substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, or C₂-C₈ heterocycloalkyl containing a heteroatom selected from oxygen, nitrogen and sulfur;
R_{b} is C₁-C₆ alkyl, -N-(C₁-C₆ alkyl)₂, -O-C₁-C₆ alkyl, substituted or unsubstituted C₂-C₈ heterocycloalkyl containing a heteroatom selected from oxygen, nitrogen and sulfur, substituted or unsubstituted C₆-C₁₂ aryl, substituted or unsubstituted C₃-C₁₂ heteroaryl containing a heteroatom selected from oxygen, nitrogen and sulfur, wherein the substituted heterocycloalkyl, aryl and heteroaryl are substituted with at least one halogen atom, C₁-C₆ alkyl, C₁-C₆ haloalkyl, -O-C₁-C₆ alkyl, oxo(=O), -OH, -CN or -NH₂;
R₂ is a hydrogen atom, C₁-C₆ haloalkyl, substituted or unsubstituted C₂-C₈ heterocycloalkyl containing a heteroatom selected from oxygen, nitrogen and sulfur, substituted or unsubstituted C₆-C₁₂ aryl, or substituted or unsubstituted C₃-C₁₂ heteroaryl containing a heteroatom selected from oxygen, nitrogen and sulfur, wherein the substituted heterocycloalkyl, aryl and heteroaryl are substituted with at least one halogen atom, C₁-C₆ alkyl, C₁-C₆ haloalkyl, -O-C₁-C₆ alkyl, CN or NH₂;
L₄ is a single bond, C₁-C₆ alkyl, -NH, -NH-C₁-C₆ alkyl, C₁-C₆ haloalkyl, or -O-C₁-C₆ alkyl; and 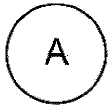
is substituted or unsubstituted C₂-C₈ heterocycloalkyl containing a heteroatom selected from oxygen, nitrogen and sulfur or substituted or unsubstituted C₂-C₈ heterocycloalkene containing a heteroatom selected from oxygen, nitrogen and sulfur, wherein the substituted heterocycloalkyl and heterocycloalkene are substituted with at least one halogen, C₁-C₆ alkyl, C₁-C₆ haloalkyl, C₁-C₆ alkyl-OH, -O-C₁-C₆ alkyl, oxo(=O), CN or NH₂.

2. The compound, the optical isomer, or the pharmaceutically acceptable salt of claim 1, wherein
each X₁ and X₂ are independently C or N, wherein at least one of X₁ and X₂ is C; and
each Y₁ and Y₂ are independently C or N, wherein at least one of Y₁ and Y₂ is N.

3. The compound, the optical isomer, or the pharmaceutically acceptable salt of claim 1, wherein
Z₁ is C, CH or N;
Z₂ is C, C-R', CH, CH₂ or N;
Z₃ is CH, CH₂, N, N-R' or S; or
Z₄ is CH or N,
wherein R' is a hydrogen atom or C₁-C₆ alkyl.

4. The compound, the optical isomer, or the pharmaceutically acceptable salt of claim 1, wherein
when p is 0,
Z₁ is C or N,
Z₂ is C-R', CH, CH₂ or N,
Z₃ is CH, CH₂, N, N-R' or S,
wherein R' is a hydrogen atom or C₁-C₆ alkyl.

5. The compound, the optical isomer, or the pharmaceutically acceptable salt of claim 1, wherein
when p is 1,
Z₁ is C, CH or N,
Z₂ is C or CH,
Z₃ is CH,
Z₄ is CH or N,
wherein R' is a hydrogen atom or C₁-C₆ alkyl.

6. The compound, the optical isomer, or the pharmaceutically acceptable salt of claim 1, wherein
L₁ is a single bond, C₁-C₆ alkyl, C₁-C₆ haloalkyl, C₁-C₆ alkyl-C=O, -O-, -NH, -N=CH-, -NH-C₁-C₆ alkyl, substituted or unsubstituted C₆-C₁₂ aryl, or 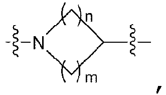 and
n and m are each independently either an integer of 1 and 2.

7. The compound, the optical isomer, or the pharmaceutically acceptable salt of claim 1, wherein
R₁ is a hydrogen atom, substituted or unsubstituted C₁-C₆ alkyl, C₁-C₆ alkyl-OH, -SO₂-C₁-C₆ alkyl, -N-(C₁-C₆ alkyl)₂, -C=O-R_{b}, -O-C₁-C₆ alkyl, -O-O₁-C₆ haloalkyl, substituted or unsubstituted C₃-C₆ cycloalkyl, -O-C₃-C₈ cycloalkyl, substituted or unsubstituted 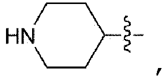 substituted or unsubstituted 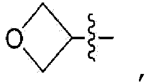 substituted or unsubstituted 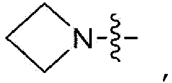 substituted or unsubstituted 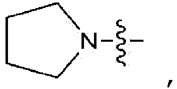 substituted or unsubstituted 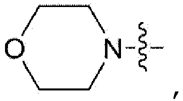 substituted or unsubstituted 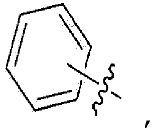 substituted or unsubstituted 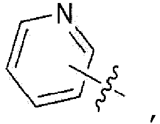 substituted or unsubstituted 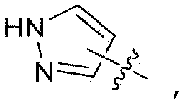 substituted or unsubstituted 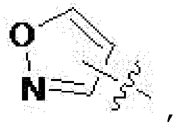 substituted or unsubstituted 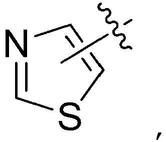 substituted or unsubstituted 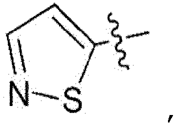 substituted or unsubstituted 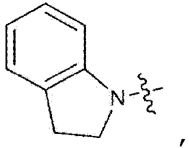 substituted or unsubstituted 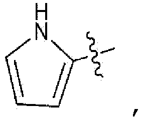 substituted or unsubstituted 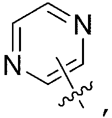 substituted or unsubstituted 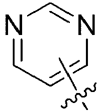 (wherein the substituted C₁-C₆ alkyl, 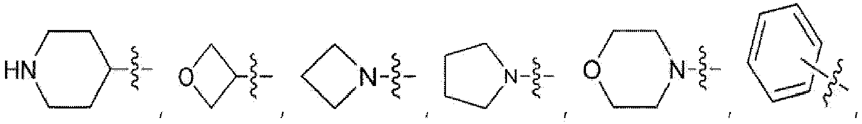 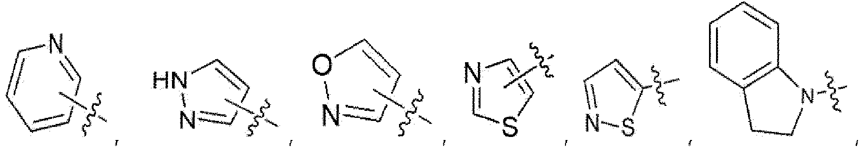 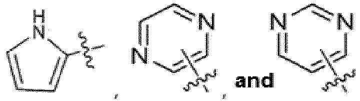 are each independently substituted with at least one halogen atom, C₁-C₆ alkyl, C₁-C₆ haloalkyl, -O-C₁-C₆ alkyl, -O-C₃-C₈ cycloalkyl, -O-C₁-C₆ haloalkyl, oxo(=O), -OH, -CN, -N-(C₁-C₆ alkyl)₂, -NH₂, C₂-C₈ heterocycloalkyl containing a heteroatom selected from oxygen, nitrogen and sulfur, C₆-C₁₂ aryl or C₃-C₁₂ heteroaryl containing a heteroatom selected from oxygen, nitrogen and sulfur), and
R_{b} is N- (C1-C6 alkyl) 2, O-C₁-C₆ alkyl,

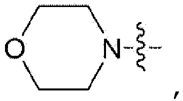

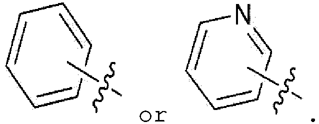

8. The compound, the optical isomer, or the pharmaceutically acceptable salt of claim 1, wherein
R₁ is a hydrogen atom, substituted or unsubstituted C₁-C₆ alkyl, C₁-C₆ alkyl-OH, -SO₂-C₁-C₆ alkyl, -N-(C₁-C₆ alkyl)₂, -C=O-N-(C₁-C₆ alkyl)₂, -C=O-O-C₁-C₆ alkyl, -O-C₁-C₆ alkyl,

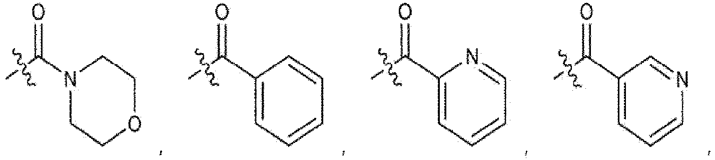

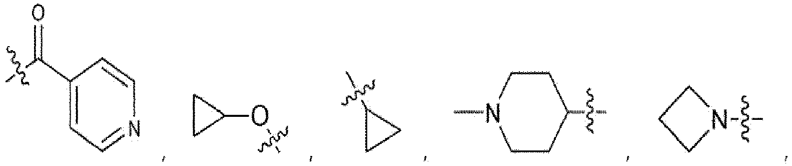

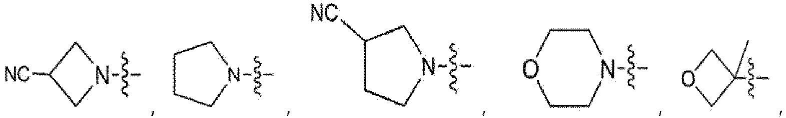

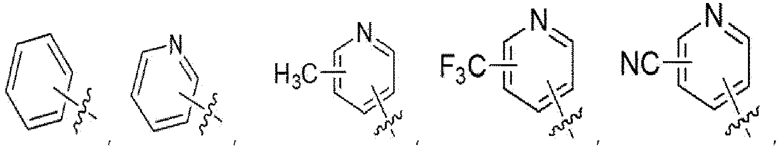

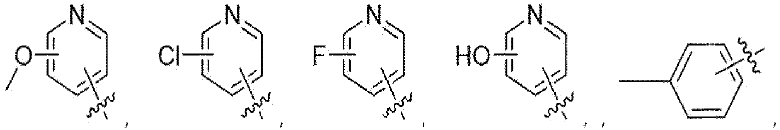

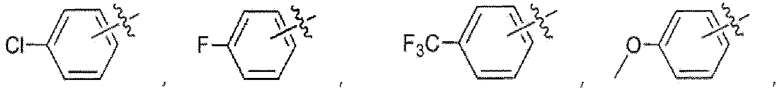

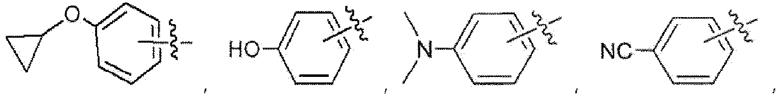

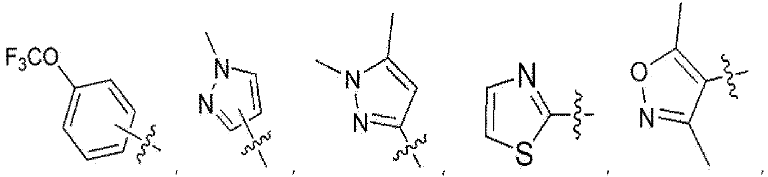

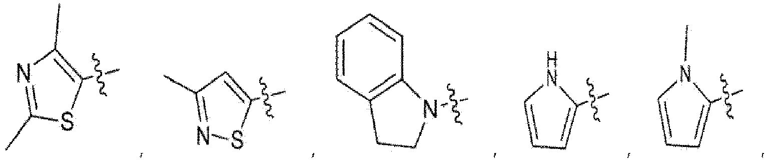

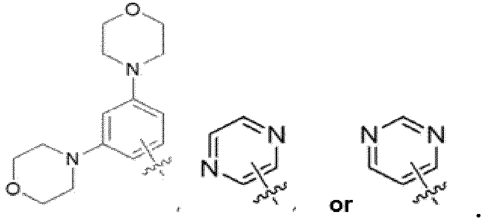

9. The compound, the optical isomer, or the pharmaceutically acceptable salt of claim 1, wherein
L₂ is a single bond, -NH-N=CH-, -NH-, -NH-NH-C=O-, -NH-C₁-C₆ alkyl, -O-, -O-C₁-C₆ alkyl, or

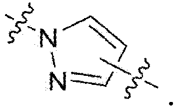

10. The compound, the optical isomer, or the pharmaceutically acceptable salt of claim 1, wherein
L₃ is a single bond, C₁-C₆ alkyl, -NH-N=CH-, -NH-, -NH-NH-C=O-, -NH-C₁-C₆ alkyl, -O-, -O-C₁-C₆ alkyl-, -C=O-NH-, or

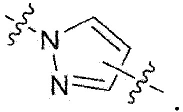

11. The compound, the optical isomer, or the pharmaceutically acceptable salt of claim 1, wherein
R₂ is a hydrogen atom, C₁-C₆ alkyl, C₁-C₆ haloalkyl,
substituted or unsubstituted 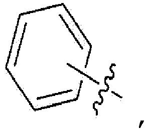 substituted or unsubstituted 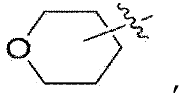 substituted or unsubstituted 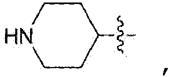 substituted or unsubstituted 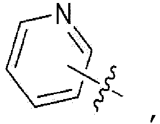 substituted or unsubstituted 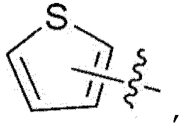 substituted or unsubstituted 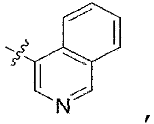 substituted or unsubstituted 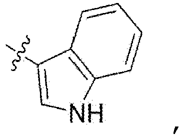 substituted or unsubstituted 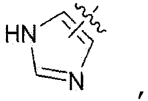 substituted or unsubstituted 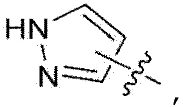 substituted or unsubstituted 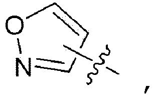 substituted or unsubstituted 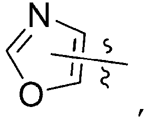 substituted or unsubstituted 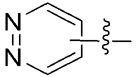 (wherein the substituted 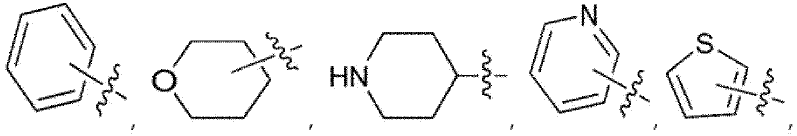 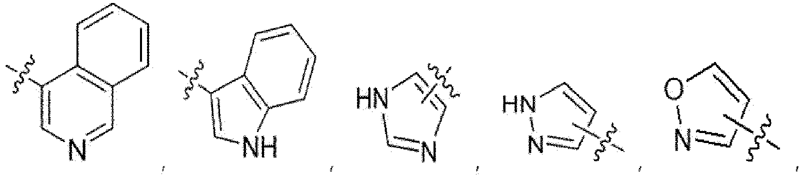 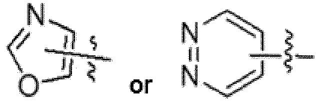 is substituted with at least one halogen atom, C₁-C₆ alkyl, C₁-C₆ haloalkyl, -O-C₁-C₆ alkyl, or CN) .

12. The compound, the optical isomer, or the pharmaceutically acceptable salt of claim 1, wherein
R₂ is a hydrogen atom, C₁-C₆ alkyl,

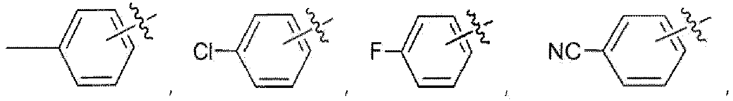

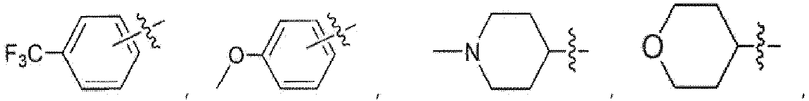

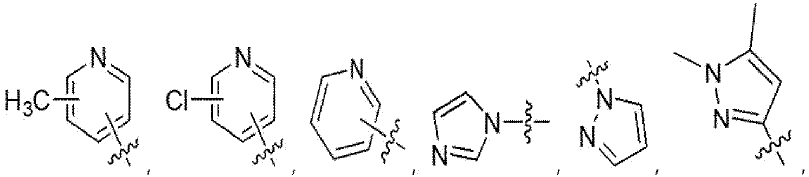

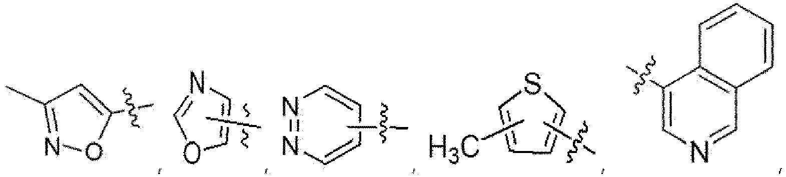

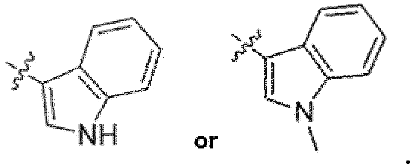

13. The compound, the optical isomer, or the pharmaceutically acceptable salt of claim 1, wherein 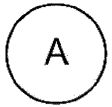 is 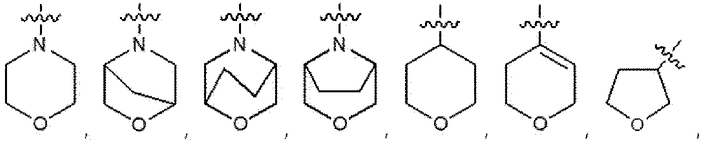 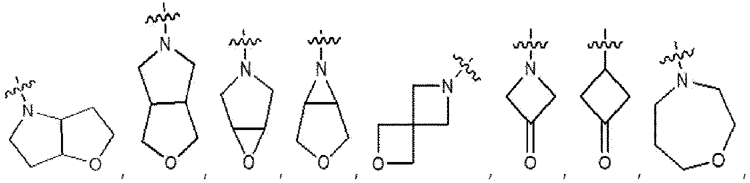 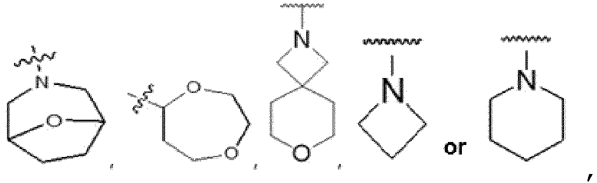 and the 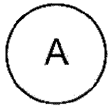 is unsubstituted or substituted with at least one halogen, C₁-C₆ alkyl, C₁-C₆ haloalkyl, C₁-C₆ alkyl-OH, -O-C₁-C₆ alkyl, -OH, CN or NH₂.

14. The compound, the optical isomer, or the pharmaceutically acceptable salt of claim 1, wherein 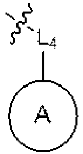 is 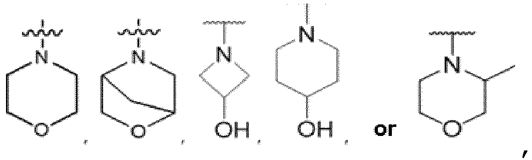 and L₄ is a single bond.

15. The compound, the optical isomer, or the pharmaceutically acceptable salt of claim 1, wherein the compound represented by Chemical Formula 1 above is any one selected from the group consisting of the following compounds:
(E)-4-(4-(2-(3-methylbenzylidene)hydrazinyl)-7-phenethyl-6,7-dihydro-5H-pyrrolo[2,3-d]pyrimidin-2-yl)morpholine;
(E)-4-(7-isobutyl-4-(2-(3-methylbenzylidene)hydrazinyl)-6,7-dihydro-5H-pyrrolo[2,3-d]pyrimidin-2-yl)morpholine;
(E)-4-(7-isobutyl-4-(2-(3-methylbenzylidene)hydrazinyl)-7H-pyrrolo[2,3-d]pyrimidin-2-yl)morpholine;
(E)-4-(4-(2-(3-methylbenzylidene)hydrazinyl)-7-phenethyl-pyrrolo[2,3-d]pyrimidin-2-yl)morpholine;
(E)-4-(4-(2-(3-methylbenzylidene)hydrazinyl)-7-(2-(pyridin-2-yl)ethyl)-7H-pyrrolo[2,3-d]pyrimidin-2-yl)morpholine;
(E)-4-(7-(2,2-difluoro-2-(pyridin-2-yl)ethyl)-4-(2-(3-methylbenzylidene)hydrazinyl)-7H-pyrrolo[2,3-d]pyrimidin-2-yl)morpholine;
(E)-4-(8-(2-(3-methylbenzylidene)hydrazinyl)-3-phenylimidazo[1,2-b]pyridazin-6-yl)morpholine;
(E)-4-(4-(2-(3-methylbenzylidene)hydrazinyl)-7-phenyl-5H-pyrrolo[3,2-d]pyrimidin-2-yl)morpholine;
(E)-4-(5-methyl-4-(2-(3-methylbenzylidene)hydrazinyl)-7-phenyl-5H-pyrrolo[3,2-d]pyrimidin-2-yl)morpholine;
(E)-4-(5-isopropyl-4-(2-(3-methylbenzylidene)hydrazinyl)-7-phenyl-5H-pyrrolo[3,2-d]pyrimidin-2-yl)morpholine;
(E)-4-(4-(2-(3-methylbenzylidene)hydrazinyl)-7-phenylthieno[3,2-d]pyrimidin-2-yl)morpholine;
(E)-4-(4-(2-benzylidenehydrazinyl)-7-(1-methyl-1H-pyrazol-4-yl)thieno[3,2-d]pyrimidin-2-yl)morpholine;
(E)-4-(4-(2-(3-methylbenzylidene)hydrazinyl)-1-(pyridin-3-yl)-1H-pyrazolo[3,4-d]pyrimidin-6-yl)morpholine;
(E)-4-(4-(2-(3-methylbenzylidene)hydrazinyl)-1-phenyl-1H-pyrazolo[3,4-d]pyrimidin-6-yl)morpholine;
(E)-4-(4-(2-(3-methylbenzylidene)hydrazinyl)-1-(pyridin-2-yl)-1H-pyrazolo[3,4-d]pyrimidin-6-yl)morpholine;
(E)-4-(4-(2-(3-methylbenzylidene)hydrazinyl)-1-(pyridin-4-yl)-1H-pyrazolo[3,4-d]pyrimidin-6-yl)morpholine;
(E)-4-(9-isobutyl-6-(2-(3-methylbenzylidene)hydrazinyl)-9H-purin-2-yl)morpholine;
(E)-4-(6-(2-(3-methylbenzylidene)hydrazinyl)-9-phenethyl-9H-purin-2-yl)morpholine;
(E)-1-cyclopropyl-2-(6-(2-(3-methylbenzylidene)hydrazinyl)-2-morpholino-9H-purin-9-yl)ethan-1-one;
(E)-4-(6-(2-(3-methylbenzylidene)hydrazinyl)-9-(pyridin-2-ylmethyl)-9H-purin-2-yl)morpholine;
(E)-4-(9-(cyclopropylmethyl)-6-(2-(3-methylbenzylidene)hydrazinyl)-9H-purin-2-yl)morpholine;
(E)-2-(6-(2-(3-methylbenzylidene)hydrazinyl)-2-morpholino-9H-purin-9-yl)-1-phenylethan-1-one;
(E)-2-(6-(2-(3-methylbenzylidene)hydrazinyl)-2-morpholino-9H-purin-9-yl)-1-(pyridin-2-yl)ethan-1-one;
(E)-2-(6-(2-((1H-indol-3-yl)methylene)hydrazinyl)-2-morpholino-9H-purin-9-yl)-1-(pyridin-2-yl)ethan-1-one;
(E)-1-(4-chlorophenyl)-2-(6-(2-(3-methylbenzylidene)hydrazinyl)-2-morpholino-9H-purin-9-yl)ethan-1-one;
(E)-4-(9-(2,2-difluoro-2-(pyridin-2-yl)ethyl)-6-(2-(3-methylbenzylidene)hydrazinyl)-9H-purin-2-yl)morpholine;
(E)-2-(6-(2-(3-methylbenzylidene)hydrazinyl)-2-morpholino-9H-purin-9-yl)-1-(pyrrolidin-1-yl)ethan-1-one;
(E)-2-(6-(2-(3-methylbenzylidene)hydrazinyl)-2-morpholino-9H-purin-9-yl)-1-(pyridin-3-yl)ethan-1-one;
(E)-1-(indolin-1-yl)-2-(6-(2-(3-methylbenzylidene)hydrazinyl)-2-morpholino-9H-purin-9-yl)ethan-1-one;
(E)-1-(1-methyl-1H-pyrrol-2-yl)-2-(6-(2-(3-methylbenzylidene)hydrazinyl)-2-morpholino-9H-purin-9-yl)ethan-1-one;
(E)-1-(2-(6-(2-(3-methylbenzylidene)hydrazinyl)-2-morpholino-9H-purin-9-yl)acetyl)azetidine-3-carbonitrile;
(E)-2-(6-(2-(3-methylbenzylidene)hydrazinyl)-2-morpholino-9H-purin-9-yl)-1-(pyridin-4-yl)ethan-1-one;
(E)-1-(2-(6-(2-(3-methylbenzylidene)hydrazinyl)-2-morpholino-9H-purin-9-yl)acetyl)pyrrolidine-3-carbonitrile;
(E)-2-(6-(2-(3-methylbenzylidene)hydrazinyl)-2-morpholino-9H-purin-9-yl)-1-(1H-pyrrol-2-yl)ethan-1-one;
(E)-4-(2-(6-(2-(3-methylbenzylidene)hydrazinyl)-2-morpholino-9H-purin-9-yl)acetyl)benzonitrile;
(E)-3-(2-(6-(2-(3-methylbenzylidene)hydrazinyl)-2-morpholino-9H-purin-9-yl)acetyl)benzonitrile;
(E)-1-(2-methoxyphenyl)-2-(6-(2-(3-methylbenzylidene)hydrazinyl)-2-morpholino-9H-purin-9-yl)ethan-1-one;
(E)-1-(3-methoxyphenyl)-2-(6-(2-(3-methylbenzylidene)hydrazinyl)-2-morpholino-9H-purin-9-yl)ethan-1-one;
(E)-1-(4-methoxyphenyl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(o-tolyl)ethan-1-one;
(E)-2-(6-(2-(3-methylbenzylidene)hydrazinyl)-2-morpholino-9H-purin-9-yl)-1-(m-tolyl)ethan-1-one;
(E)-2-(6-(2-(3-methylbenzylidene)hydrazinyl)-2-morpholino-9H-purin-9-yl)-1-(p-tolyl)ethan-1-one;
(E)-2-(6-(2-(3-methylbenzylidene)hydrazinyl)-2-morpholino-9H-purin-9-yl)-1-(2-(trifluoromethyl)phenyl)ethan-1-one;
(E)-2-(6-(2-(3-methylbenzylidene)hydrazinyl)-2-morpholino-9H-purin-9-yl)-1-(3-(trifluoromethyl)phenyl)ethan-1-one;
(E)-2-(6-(2-(3-methylbenzylidene)hydrazinyl)-2-morpholino-9H-purin-9-yl)-1-(4-(trifluoromethyl)phenyl)ethan-1-one;
(E)-1-(3-fluorophenyl)-2-(6-(2-(3-methylbenzylidene)hydrazinyl)-2-morpholino-9H-purin-9-yl)ethan-1-one;
(E)-1-(4-fluorophenyl)-2-(6-(2-(3-methylbenzylidene)hydrazinyl)-2-morpholino-9H-purin-9-yl)ethan-1-one;
(E)-1-(2-chlorophenyl)-2-(6-(2-(3-methylbenzylidene)hydrazinyl)-2-morpholino-9H-purin-9-yl)ethan-1-one;
(E)-1-(3-chlorophenyl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(3-(trifluoromethoxy)phenyl)ethan-1-one;
(E)-1-(2,4-dimorpholinophenyl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(4-(trifluoromethyl)pyridin-2-yl)ethan-1-one;
(E)-1-(5-fluoropyridin-2-yl)-2-(6-(2-(3-methylbenzylidene)hydrazinyl)-2-morpholino-9H-purin-9-yl)ethan-1-one;
(E)-2-(6-(2-(3-methylbenzylidene)hydrazinyl)-2-morpholino-9H-purin-9-yl)-1-(pyrazin-2-yl)ethan-1-one;
(E)-2-(6-(2-((5-methylthiophen-2-yl)methylene)hydrazinyl)-2-morpholino-9H-purin-9-yl)-1-(pyridin-2-yl)ethan-1-one;
(E)-3-((2-(2-morpholino-9-(2-oxo-2-(pyridin-2-yl)ethyl)-9H-purin-6-yl)hydrazinylidene)methyl)benzonitrile;
(E)-2-(2-morpholino-6-(2-(3-(trifluoromethyl)benzylidene)hydrazinyl)-9H-purin-9-yl)-1-(pyridin-2-yl)ethan-1-one;
(E)-2-(6-(2-(4-methylbenzylidene)hydrazinyl)-2-morpholino-9H-purin-9-yl)-1-(pyridin-2-yl)ethan-1-one;
(E)-2-(6-(2-(2-methylbenzylidene)hydrazinyl)-2-morpholino-9H-purin-9-yl)-1-(pyridin-2-yl)ethan-1-one;
(E)-2-(2-morpholino-6-(2-(pyridin-3-ylmethylene)hydrazinyl)-9H-purin-9-yl)-1-(pyridin-2-yl)ethan-1-one;
(E)-2-(6-(2-(3-methoxybenzylidene)hydrazinyl)-2-morpholino-9H-purin-9-yl)-1-(pyridin-2-yl)ethan-1-one;
3-methyl-N'-(2-morpholino-9-(2-oxo-2-(pyridin-2-yl)ethyl)-9H-purin-6-yl)benzohydrazide;
2-(2-morpholino-6-(3-(pyridin-3-yl)-1H-pyrazol-1-yl)-9H-purin-9-yl)-1-(pyridin-2-yl)-ethan-1-one;
2-(2-morpholino-6-(3-(m-tolyl)-1H-pyrazol-1-yl)-9H-purin-9-yl)-1-(pyridin-2-yl)ethan-1-one;
2-(2-morpholino-6-(3-(3-(trifluoromethyl)phenyl)-1H-pyrazol-1-yl)-9H-purin-9-yl)-1-(pyridin-2-yl)ethan-1-one;
2-(6-(3-(3-fluorophenyl)-1H-pyrazol-1-yl)-2-morpholino-9H-purin-9-yl)-1-(pyridin-2-yl)ethan-1-one;
3-(1-(2-morpholino-9-(2-oxo-2-(pyridin-2-yl)ethyl)-9H-purin-6-yl)-1H-pyrazol-3-yl)benzonitrile;
2-(6-(3-(3-methoxyphenyl)-1H-pyrazol-1-yl)-2-morpholino-9H-purin-9-yl)-1-(pyridin-2-yl)ethan-1-one;
2-(2-morpholino-6-(3-(tetrahydro-2H-pyran-4-yl)-1H-pyrazol-1-yl)-9H-purin-9-yl)-1-(pyridin-2-yl)ethan-1-one;
2-(6-(3-(6-methylpyridin-2-yl)-1H-pyrazol-1-yl)-2-morpholino-9H-purin-9-yl)-1-(pyridin-2-yl)ethan-1-one;
1-(2-morpholino-9-(2-oxo-2-(pyridin-2-yl)ethyl)-9H-purin-6-yl)-N-(m-tolyl)-1H-pyrazole-3-carboxamide;
(E)-1-(azetidin-1-yl)-2-(6-(2-(3-methylbenzylidene)hydrazinyl)-2-morpholino-9H-purin-9-yl)ethan-1-one;
(E)-4-(6-(2-(3-methylbenzylidene)hydrazinyl)-9-(2-(pyridin-2-yl)ethyl)-9H-purin-2-yl)morpholine;
(E)-4-(6-(2-(3-methylbenzylidene)hydrazinyl)-9-((3-methyloxetan-3-yl)methyl)-9H-purin-2-yl)morpholine;
(E)-4-(2-(6-(2-(3-methylbenzylidene)hydrazinyl)-2-morpholino-9H-purin-9-yl)ethylmorpholine;
(E)-4-(6-(2-(3-methylbenzylidene)hydrazinyl)-9-(1-methylpiperidin-4-yl)-9H-purin-2-yl)morpholine;
(E)-4-(6-(2-(3-methylbenzylidene)hydrazinyl)-9-(pyridin-4-ylmethyl)-9H-purin-2-yl)morpholine;
(E)-4-(6-(2-(3-methylbenzylidene)hydrazinyl)-9-(pyridin-3-ylmethyl)-9H-purin-2-yl)morpholine;
(E)-4-(6-(2-(isoquinolin-4-ylmethylene)hydrazinyl)-9-(2-pyridin-2-yl)ethyl)-9H-purin-2-yl)morpholine;
(E)-4-(6-(2-(3-methylbenzylidene)hydrazinyl)-9-(2-(pyridin-3-yl)ethyl)-9H-purin-2-yl)morpholine;
(E)-(3-(6-(2-(3-methylbenzylidene)hydrazinyl)-2-morpholino-9H-purin-9-yl)azetidin-1-yl) (phenyl)methanone;
(E)-3-(6-(2-(3-methylbenzylidene)hydrazinyl)-2-morpholino-9H-purin-9-yl)azetidin-1-yl(pyridin-3-yl)methanone;
methyl (E)-3-(6-(2-(3-methylbenzylidene)hydrazinyl)-2-morpholino-9H-purin-9-yl)azetidine-1-carboxylate;
(E)-N,N-dimethyl-3-(6-(2-(3-methylbenzylidene)hydrazinyl)-2-morpholino-9H-purin-9-yl)azetidine-1-carboxamide;
(E)-4-(6-(2-(3-methylbenzylidene)hydrazinyl)-9-(1-(methylsulfonyl)azetidin-3-yl)-9H-purin-2-yl)morpholine;
(E)-3-(6-(2-(3-methylbenzylidene)hydrazinyl)-2-morpholino-9H-purin-9-yl)azetidin-1-yl(morpholino)methanone;
(E)-(4-(6-(2-(3-methylbenzylidene)hydrazinyl)-2-morpholino-9H-purin-9-yl)piperidin-1-yl) (pyridin-3-yl)methanone;
(E)-(4-(6-(2-(3-methylbenzylidene)hydrazinyl)-2-morpholino-9H-purin-9-yl)piperidin-1-yl) (morpholino)methanone;
(E)-(4-(6-(2-(3-methylbenzylidene)hydrazinyl)-2-morpholino-9H-purin-9-yl)piperidin-1-yl) (phenyl)methanone;
(E)-N,N-dimethyl-4-(6-(2-(3-methylbenzylidene)hydrazinyl)-2-morpholino-9H-purin-9-yl)piperidine-1-carboxamide;
methyl (E)-4-(6-(2-(3-methylbenzylidene)hydrazinyl)-2-morpholino-9H-purin-9-yl)piperidine-1-carboxylate;
(E)-4-(6-(2-(3-methylbenzylidene)hydrazinyl)-9-(1-(methylsulfonyl)piperidin-4-yl)-9H-purin-2-yl)morpholino;
(E)-(4-(6-(2-(3-methylbenzylidene)hydrazinyl)-2-morpholino-9H-purin-9-yl)piperidin-1-yl) (pyridin-4-yl)methanone;
(E)-(4-(6-(2-(3-methylbenzylidene)hydrazinyl)-2-morpholino-9H-purin-9-yl)piperidin-1-yl) (pyridin-2-yl)methanone;
(E)-4-(9-(1-methyl-1H-pyrazol-4-yl)-6-(2-(3-methylbenzylidene)hydrazinyl)-9H-purin-2-yl)-morpholine;
(E)-4-(6-(2-(3-methylbenzylidene)hydrazinyl)-9-(pyridin-3-yl)-9H-purin-2-yl)morpholine;
(S,E)-3-methyl-4-(6-(2-(3-methylbenzylidene)hydrazinyl)-9-(pyridin-3-yl)-9H-purin-2-yl)morpholine;
(1R,4R)-5-(6-(2-(3-methylbenzylidene)hydrazinyl)-9-(pyridin-3-yl)-9H-purin-2-yl)-oxa-5-azabicyclo[2.2.1]heptane;
(E)-4-(6-(2-(3-methylbenzylidene)hydrazinyl)-9-(pyridin-2-yl)-9H-purin-2-yl)morpholine;
(E)-4-(9-(4-chlorophenyl)-6-(2-(3-methylbenzylidene)hydrazinyl)-9H-purin-2-yl)morpholine;
(E)-4-(9-(2-methoxypyridin-4-yl)-6-(2-(3-methylbenzylidene)hydrazinyl)-9H-purin-2-yl)morpholine;
(E)-4-(6-(2-(3-methylbenzylidene)hydrazinyl)-2-morpholino-9H-purin-9-yl)pyridin-2-ol;
(E)-4-(6-(2-(3-methylbenzylidene)hydrazinyl)-9-(pyridin-4-yl)-9H-purin-2-yl)morpholine;
(E)-4-(6-(2-(3-methylbenzylidene)hydrazinyl)-9-(4-(trifluoromethyl)phenyl)-9H-purin-2-yl)morpholine;
(E)-1-(6-(2-(3-methylbenzylidene)hydrazinyl)-9-(pyridin-3-yl)-9H-purin-2-yl)piperidin-4-ol;
(E)-1-(6-(2-(3-methylbenzylidene)hydrazinyl)-9-(pyridin-3-yl)-9H-purin-2-yl)azetidin-3-ol;
4-(9-(pyridin-4-yl)-6-(3-(m-tolyl)-1H-pyrazol-1-yl)-9H-purin-2-yl)morpholine;
(E)-4-(9-(5-methoxypyridin-2-yl)-6-(2-(3-methylbenzylidene)hydrazinyl)-9H-purin-2-yl)morpholine;
(E)-4-(6-(2-(3-methylbenzylidene)hydrazinyl)-9-(2-methylpyridin-3-yl)-9H-purin-2-yl)morpholine;
(E)-4-(6-(2-(3-methylbenzylidene)hydrazinyl)-9-(6-methylpyridin-3-yl)-9H-purin-2-yl)morpholine;
(E)-4-(9-(2,4-dimethylthiazol-5-yl)-6-(2-(3-methylbenzylidene)hydrazinyl)-9H-purin-2-yl)morpholine;
(E)-4-(6-(2-((1H-indol-3-yl)methylene)hydrazinyl)-9-(pyridin-4-yl)-9H-purin-2-yl)morpholine;
(E)-4-(9-(6-methoxypyridin-3-yl)-6-(2-(3-methylbenzylidene)hydrazinyl)-9H-purin-2-yl)morpholine;
(E)-5-(6-(2-(3-methylbenzylidene)hydrazinyl)-2-morpholino-9H-purin-9-yl)pyridin-2-ol;
(E)-4-(9-(2-chloropyridin-4-yl)-6-(2-(3-methylbenzylidene)hydrazinyl)-9H-purin-2-yl)morpholine;
(E)-4-(9-(5-chloropyridin-2-yl)-6-(2-(3-methylbenzylidene)hydrazinyl)-9H-purin-2-yl)morpholine;
(E)-4-(6-(2-(3-methylbenzylidene)hydrazinyl)-9-(thiazol-2-yl)-9H-purin-2-yl)morpholine;
4-(9-(pyridin-3-yl)-6-(3-(m-tolyl)-1H-pyrazol-1-yl)-9H-purin-2-yl)morpholine;
(E)-4-(6-(2-(3-methylbenzylidene)hydrazinyl)-9-phenyl-9H-purin-2-yl)morpholine;
(E)-4-(9-(4-methoxyphenyl)-6-(2-(3-methylbenzylidene)hydrazinyl)-9H-purin-2-yl)morpholine;
(E)-4-(9-(3-methoxyphenyl)-6-(2-(3-methylbenzylidene)hydrazinyl)-9H-purin-2-yl)morpholine;
(E)-4-(9-(3-chlorophenyl)-6-(2-(3-methylbenzylidene)hydrazinyl)-9H-purin-2-yl)morpholine;
(E)-N,N-dimethyl-4-(6-(2-(3-methylbenzylidene)hydrazinyl)-2-morpholino-9H-purin-9-yl)aniline;
(E)-4-(9-(2-methoxyphenyl)-6-(2-(3-methylbenzylidene)hydrazinyl)-9H-purin-2-yl)morpholine;
(E)-4-(9-(3-cyclopropoxyphenyl)-6-(2-(3-methylbenzylidene)hydrazinyl)-9H-purin-2-yl)morpholine;
(E)-3-(6-(2-(3-methylbenzylidene)hydrazinyl)-2-morpholino-9H-purin-9-yl)benzonitrile;
(E)-4-(6-(2-(3-methylbenzylidene)hydrazinyl)-9-(m-tolyl)-9H-purin-2-yl)morpholine;
(E)-4-(6-(2-(3-methylbenzylidene)hydrazinyl)-9-(3-(trifluoromethoxy)phenyl)-9H-purin-2-yl)morpholine;
(E)-4-(9-(3-fluorophenyl)-6-(2-(3-methylbenzylidene)hydrazinyl)-9H-purin-2-yl)morpholine;
(E)-4-(6-(2-(3-methylbenzylidene)hydrazinyl)-9-(3-(trifluoromethyl)phenyl)-9H-purin-2-yl)morpholine;
(E)-3-(6-(2-(3-methylbenzylidene)hydrazinyl)-2-morpholino-9H-purin-9-yl)phenol;
(E)-4-(6-(2-((1H-indol-3-yl)methylene)hydrazinyl)-9-phenyl-9H-purin-2-yl)morpholine;
(E)-4-(6-(2-((1-methyl-1H-indol-3-yl)methylene)hydrazinyl)-9-phenyl-9H-purin-2-yl)morpholine;
4-(9-(4-methoxyphenyl)-6-(3-(m-tolyl)-1H-pyrazol-1-yl)-9H-purin-2-yl)morpholine;
4-(9-(3-chlorophenyl)-6-(3-(m-tolyl)-1H-pyrazol-1-yl)-9H-purin-2-yl)morpholine;
4-(9-phenyl-6-(3-(m-tolyl)-1H-pyrazol-1-yl)-9H-purin-2-yl)morpholine;
4-(9-(3-methoxyphenyl)-6-(3-(m-tolyl)-1H-pyrazol-1-yl)-9H-purin-2-yl)morpholine;
4-(9-(3-cyclopropoxyphenyl)-6-(3-(m-tolyl)-1H-pyrazol-1-yl)-9H-purin-2-yl)morpholine;
(E)-4-(6-(2-(3-methylbenzylidene)hydrazinyl)-9-(2-(trifluoromethyl)pyridin-4-yl)-9H-purin-2-yl)morpholine;
(E)-4-(6-(2-(3-methylbenzylidene)hydrazinyl)-9-(2-methylpyridin-4-yl)-9H-purin-2-yl)morpholine;
(E)-4-(6-(2-(3-methylbenzylidene)hydrazinyl)-9-(6-methylpyridin-2-yl)-9H-purin-2-yl)morpholine;
(E)-4-(6-(2-(3-methylbenzylidene)hydrazinyl)-9-(3-methylpyridin-2-yl)-9H-purin-2-yl)morpholine;
(E)-4-(6-(2-(3-methylbenzylidene)hydrazinyl)-9-(5-methylpyridin-2-yl)-9H-purin-2-yl)morpholine;
(E)-4-(6-(2-(3-methylbenzylidene)hydrazinyl)-9-(4-methylpyridin-2-yl)-9H-purin-2-yl)morpholine;
(E)-4-(6-(2-(3-methylbenzylidene)hydrazinyl)-9-(5-methylpyridin-3-yl)-9H-purin-2-yl)morpholine;
(E)-6-(6-(2-(3-methylbenzylidene)hydrazinyl)-2-morpholino-9H-purin-9-yl)nicotinonitrile;
(E)-4-(9-(1,5-dimethyl-1H-pyrazol-3-yl)-6-(2-(3-methylbenzylidene)hydrazinyl)-9H-purin-2-yl)morpholine;
(E)-4-(6-(2-(3-methylbenzylidene)hydrazinyl)-9-(3-methylisothiazol-5-yl)-9H-purin-2-yl)morpholine;
(E)-4-(9-(5-fluoropyridin-2-yl)-6-(2-(3-methylbenzylidene)hydrazinyl)-9H-purin-2-yl)morpholine;
(E)-4-(9-(2-methoxypyridin-4-yl)-8-methyl-6-(2-(3-methylbenzylidene)hydrazinyl)-9H-purin-2-yl)morpholine;
(E)-4-(8-methyl-6-(2-(3-methylbenzylidene)hydrazinyl)-9-(2-methylpyridin-3-yl)-9H-purin-2-yl)morpholine;
(E)-4-(9-(2,4-dimethylthiazol-5-yl)-8-methyl-6-(2-(3-methylbenzylidene)hydrazinyl)-9H-purin-2-yl)morpholine;
(E)-4-(9-(1,5-dimethyl-1H-pyrazol-3-yl)-8-methyl-6-(2-(3-methylbenzylidene)hydrazinyl)-9H-purin-2-yl)morpholine;
4-(8-methyl-9-phenyl-6-(3-(m-tolyl)-1H-pyrazol-1-yl)-9H-purin-2-yl)morpholine;
(E)-4-(6-(2-(3-methylbenzylidene)hydrazinyl)-9-(pyrimidin-2-yl)-9H-purin-2-yl)-morpholine;
(E)-2-(8-methyl-6-(2-(3-methylbenzylidene)hydrazinyl)-2-morpholino-9H-purin-9-yl)-1-phenylethan-1-one
(E)-2-(8-methyl-6-(2-(3-methylbenzylidene)hydrazinyl)-2-morpholino-9H-purin-9-yl)-1-(pyridin-2-yl)ethan-1-one;
(E)-2-(8-methyl-6-(2-(3-methylbenzylidene)hydrazinyl)-2-morpholino-9H-purin-9-yl)-1-(pyridin-3-yl)ethan-1-one;
(E)-2-(8-methyl-6-(2-(3-methylbenzylidene)hydrazinyl)-2-morpholino-9H-purin-9-yl)-1-(pyridin-4-yl)ethan-1-one;
(E)-4-(9-(3-methoxyphenyl)-8-methyl-6-(2-(3-methylbenzylidene)hydrazinyl)-9H-purin-2-yl)morpholine;
(E)-4-(9-(4-methoxyphenyl)-8-methyl-6-(2-(3-methylbenzylidene)hydrazinyl)-9H-purin-2-yl)morpholine;
(E)-4-(9-(3-fluorophenyl)-8-methyl-6-(2-(3-methylbenzylidene)hydrazinyl)-9H-purin-2-yl)morpholine;
(E)-4-(9-(3-chlorophenyl)-8-methyl-6-(2-(3-methylbenzylidene)hydrazinyl)-9H-purin-2-yl)morpholine;
(E)-4-(8-methyl-6-(2-(3-methylbenzylidene)hydrazinyl)-9-(pyridin-2-yl)-9H-purin-2-yl)morpholine;
(E)-4-(8-methyl-6-(2-(3-methylbenzylidene)hydrazinyl)-9-phenyl-9H-purin-2-yl)morpholine;
(E)-9-((3-methylbenzylidene)amino)-2-morpholino-N-(pyridin-4-yl)-9H-purin-6-amine;
(E)-9-((2-methylbenzylidene)amino)-2-morpholino-N-(pyridin-4-yl)-9H-purin-6-amine;
(E)-9-((4-methylbenzylidene)amino)-2-morpholino-N-(pyridin-4-yl)-9H-purin-6-amine;
(E)-9-(benzylideneamino)-2-morpholino-N-(pyridin-4-yl)-9H-purin-6-amine;
(E)-2-morpholino-N-(pyridin-4-yl)-9-((3-(trifluoromethyl)benzylidene)amino)-9H-purin-6-amine;
(E)-9-((3-chlorobenzylidene)amino)-2-morpholino-N-(pyridin-4-yl)-9H-purin-6-amine;
(E)-9-((3-methoxybenzylidene)amino)-2-morpholino-N-(pyridin-4-yl)-9H-purin-6-amine;
(E)-9-((4-methoxybenzylidene)amino)-2-morpholino-N-(pyridin-4-yl)-9H-purin-6-amine;
(E)-2-morpholino-N-(pyridin-4-yl)-9-((pyridin-4-ylmethylene)amino)-9H-purin-6-amine;
(E)-9-((4-chlorobenzylidene)amino)-2-morpholino-N-(pyridin-4-yl)-9H-purin-6-amine;
(E)-N-(6-(1H-imidazol-1-yl)-2-morpholino-9H-purin-9-yl)-1-(m-tolyl)methanimine;
(E)-N-(2-morpholino-6-(1H-pyrazol-1-yl)-9H-purin-9-yl)-1-(m-tolyl)methanimine;
(E)-N-(4-methoxyphenyl)-9-((3-methylbenzylidene)amino)-2-morpholino-9H-purin-6-amine;
(E)-N-methyl-9-((3-methylbenzylidene)amino)-2-morpholino-9H-purin-6-amine;
(E)-9-((3-methylbenzylidene)amino)-2-morpholino-N-phenyl-9H-purin-6-amine;
(E)-9-((3-methylbenzylidene)amino)-2-morpholino-N-(p-tolyl)-9H-purin-6-amine;
(E)-9-((3-methylbenzylidene)amino)-N-(1-methylpiperidin-4-yl)-2-morpholino-9H-purin-6-amine;
(E)-N-(2-morpholino-6-(2-(pyridin-2-yl)ethoxy)-9H-purin-9-yl)-1-(m-tolyl)methanimine;
(E)-N-(3-methoxyphenyl)-9-((3-methylbenzylidene)amino)-2-morpholino-9H-purin-6-amine;
(E)-N-(3-chlorophenyl)-9-((3-methylbenzylidene)amino)-2-morpholino-9H-purin-6-amine;
(E)-N-(4-chlorophenyl)-9-((3-methylbenzylidene)amino)-2-morpholino-9H-purin-6-amine;
(E)-N-(2-methoxyphenyl)-9-((3-methylbenzylidene)amino)-2-morpholino-9H-purin-6-amine;
(E)-N-(1,5-dimethyl-1H-pyrazol-3-yl)-9-((3-methylbenzylidene)amino)-2-morpholino-9H-purin-6-amine;
(E)-N-(2-morpholino-6-(pyridin-2-yloxy)-9H-purin-9-yl)-1-(m-tolyl)methanimine;
(E)-2-morpholino-9-((pyridin-3-ylmethylene)amino)-N-(m-tolyl)-9H-purin-6-amine;
(E)-9-((3-methylbenzylidene)amino)-2-morpholino-N-(pyridin-3-yl)-9H-purin-6-amine;
(E)-9-((3-methylbenzylidene)amino)-N-(5-methylpyridin-3-yl)-2-morpholino-9H-purin-6-amine;
(E)-N-(2-chloropyridin-4-yl)-9-((3-methylbenzylidene)amino)-2-morpholino-9H-purin-6-amine;
(E)-9-((3-methylbenzylidene)amino)-N-(2-methylpyridin-3-yl)-2-morpholino-9H-purin-6-amine;
(E)-9-((3-methylbenzylidene)amino)-2-morpholino-N-(pyridin-4-ylmethyl)-9H-purin-6-amine;
(E)-N-isopropyl-9-((3-methylbenzylidene)amino)-2-morpholino-9H-purin-6-amine;
(E)-N-(2-morpholino-6-(pyridin-4-yloxy)-9H-purin-9-yl)-1-(m-tolyl)methanimine;
(E)-3-methyl-N-(9-((3-methylbenzylidene)amino)-2-morpholino-9H-purin-6-yl)isoxazol-5-amine;
(E)-9-((3-methylbenzylidene)amino)-2-morpholino-N-(pyridin-2-yl)-9H-purin-amine;
(E)-2-morpholino-9-((pyridin-3-ylmethylene)amino)-N-(pyridin-4-yl)-9H-purin-amine;
(E)-N-(9-((3-methylbenzylidene)amino)-2-morpholino-9H-purin-6-yl)oxazol-5-amine;
(E)-N,N-dimethyl-3-(6-(2-(3-methylbenzylidene)hydrazinyl)-2-morpholino-9H-purin-9-yl)aniline;
N⁹-(3-methylbenzyl)-2-morpholino-N⁶-(pyridin-4-yl)-9H-purin-6,9-diamine;
4-(9-phenyl-6-(pyridin-4-yloxy)-9H-purin-2-yl)morpholine;
(E)-N-(4-methoxyphenyl)-8-methyl-9-((3-methylbenzylidene)amino)-2-morpholino-9H-purin-6-amine;
(E)-8-methyl-9-((3-methylbenzylidene)amino)-2-morpholino-N-phenyl-9H-purin-6-amine;
(E)-8-methyl-9-((3-methylbenzylidene)amino)-2-morpholino-N-(pyridin-4-yl)-9H-purin-6-amine;
(E)-N-8-methyl-2-morpholino-(6-(2-pyridin-2-yl)ethoxy)-9H-purin-9-yl-1-(m-tolyl)methanimine;
(E)-4-(8-(1-methyl-1H-pyrazol-4-yl)-4-(2-(3-methylbenzylidene)hydrazinyl)quinazolin-2-yl)morpholine;
(E)-4-(8-(3,5-dimethylisoxazol-4-yl)-4-(2-(3-methylbenzylidene)hydrazinyl)quinazolin-2-yl)morpholine;
(E)-4-(8-(3-methoxyphenyl)-4-(2-(3-methylbenzylidene)hydrazinyl)quinazolin-2-yl)morpholine;
(E)-4-(4-(2-(3-methylbenzylidene)hydrazinyl)-8-(pyridin-4-yl)quinazolin-2-yl)morpholine;
(E)-4-(8-(4-methoxyphenyl)-4-(2-(3-methylbenzylidene)hydrazinyl)quinazolin-2-yl)morpholine;
(E)-4-(4-(2-(3-methylbenzylidene)hydrazinyl)-8-phenylquinazolin-2-yl)morpholine;
(E)-4-(4-(2-(3-methylbenzylidene)hydrazinyl)-8-(pyridin-3-yl)quinazolin-2-yl)morpholine;
(E)-4-(4-(2-(3-methylbenzylidene)hydrazinyl)-7-(pyridin-3-yl)pyrido[2,3-d]pyrimidin-2-yl)morpholine; and
(E)-4-(4-(2-(3-methylbenzylidene)hydrazinyl)-7-phenylpyrido[2,3-d]pyrimidin-2-yl)morpholine.

16. A pharmaceutical composition for preventing or treating cancer, comprising: the compound according to any one of claims 1 to 15, an optical isomer thereof, or a pharmaceutically acceptable salt thereof.

17. A pharmaceutical composition for preventing or treating inflammatory diseases, comprising: the compound according to any one of claims 1 to 15, an optical isomer thereof, or a pharmaceutically acceptable salt thereof.

18. A pharmaceutical composition for preventing or treating lysosomal storage diseases (LSDs), comprising: the compound according to any one of claims 1 to 15, an optical isomer thereof, or a pharmaceutically acceptable salt thereof.

19. A pharmaceutical composition for preventing or treating neurological diseases, comprising: the compound according to any one of claims 1 to 15, an optical isomer thereof, or a pharmaceutically acceptable salt thereof.

20. A PIKfyve inhibitor comprising the compound according to any one of claims 1 to 15, an optical isomer thereof, or a pharmaceutically acceptable salt thereof.
